(19) Europäisches Patentamt
European Patent Office
Office européen des brevets

(11) EP 0 635 004 B1

(12) EUROPEAN PATENT SPECIFICATION

(45) Date of publication and mention of the grant of the patent:
**17.07.2002 Bulletin 2002/29**

(21) Application number: **93907333.4**

(22) Date of filing: **10.03.1993**

(51) Int Cl.[7]: **C07D 211/14**, C07D 211/22, C07D 211/70

(86) International application number:
**PCT/US93/02103**

(87) International publication number:
**WO 93/21156 (28.10.1993 Gazette 1993/26)**

# (54) 4-DIPHENYLMETHYL PIPERIDINE DERIVATIVES AND PROCESS FOR THEIR PREPARATION

4-DIPHENYLMETHYLPIPERIDINE UND VERFAHREN ZU IHRER HERSTELLUNG

DERIVES DE PIPERIDINE 4-DIPHENYMETHYLIQUE ET LEUR PROCEDE DE PREPARATION

(84) Designated Contracting States:
**AT BE CH DE DK ES FR GB GR IE IT LI LU MC NL PT SE**

(30) Priority: **10.04.1992 US 867261**
**25.02.1993 US 17251**

(43) Date of publication of application:
**25.01.1995 Bulletin 1995/04**

(73) Proprietor: **MERRELL PHARMACEUTICALS INC.**
**Cincinnati, Ohio 45215-6300 (US)**

(72) Inventors:
- **KING, Chi-Hsin, R.**
  **Cincinnati, OH 45241 (US)**
- **KAMINSKI, Michele A.**
  **West Chester, OH 45069 (US)**

(74) Representative: **VOSSIUS & PARTNER**
**Siebertstrasse 4**
**81675 München (DE)**

(56) References cited:
**DE-A- 3 005 948** **DE-A- 3 007 498**

- **J. PHARM. BIOMED. ANAL. (JPBADA,07317085); 91; VOL.9 (10-12); PP.929-33 CHEN T. M. ET. AL. 'Determination of the metabolites of terfenadine in human urine by thermospray liquid chromatography-mass spectrometry'**
- **TETRAHEDRON, (INCL. TETRAHEDRON REPORTS) vol. 34, 1978, OXFORD GB pages 1651 - 1660 K. OMURA, D. SWERN 'Oxidation of Alcohols by "Activated" Dimethyl Sulfoxide.'**
- **JOURNAL OF ORGANIC CHEMISTRY. vol. 50, no. 25, 13 December 1985, EASTON US pages 5446 - 5448 H. C. BROWN ET. AL. 'Diisopinocampheylchloroborane, a Remarkably Efficient Chiral Reducing Agent for Aromatic Prochiral Ketones.'**
- **JOURNAL OF THE CHEMICAL SOCIETY, DALTON TRANSACTIONS 1982, LETCHWORTH GB pages 1091 - 1097 L. D. BURKE, J. F. HEALY 'The Involvement of Perruthenate in the RuCl3-catalysed Oxidation of Alcohols in the Presence of Peroxodisulphate.'**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 635 004 B1

Printed by Jouve, 75001 PARIS (FR)

## Description

The present invention is related to a novel process for preparing certain piperidine derivatives which are useful as antihistamines, antiallergy agents and bronchodilators [United States Patent No. 4,254,129, March 3, 1981, United States Patent No. 4,254,130, March 3, 1981 and United States Patent No. 4,285,958, April 25, 1981].

These piperidine derivatives can be described by the following formulas:

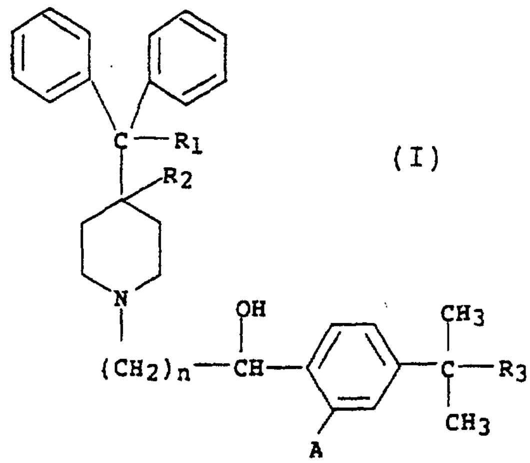

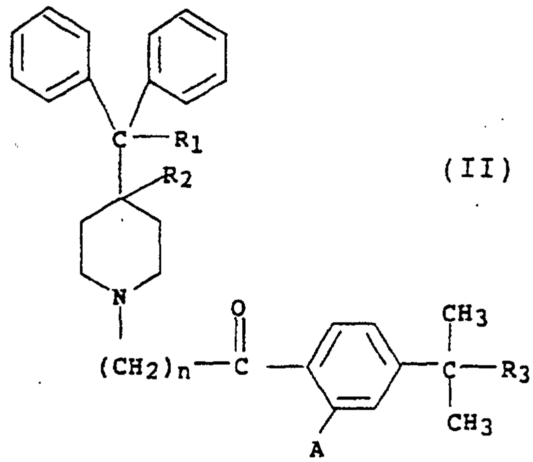

wherein

$R_1$ represents hydrogen or hydroxy;

$R_2$ represents hydrogen; or

$R_1$ and $R_2$ taken together form a second bond between the carbon atoms bearing $R_1$ and $R_2$;

n is an integer of from 1 to 5;

$R_3$ is $-CH_2OH$, -COOH or -COOalkyl wherein the alkyl moiety has from 1 to 6 carbon atoms and is straight or branched;

each of A is hydrogen; and

pharmaceutically acceptable salts and individual optical isomers thereof.

The novel process for preparing the piperidine derivatives of formula (I) and formula (II) of the present invention offers high yields and ease of purification.

SUMMARY OF THE INVENTION

The present invention provides a novel process for preparing the piperidine derivatives of formula (I) and formula (II)

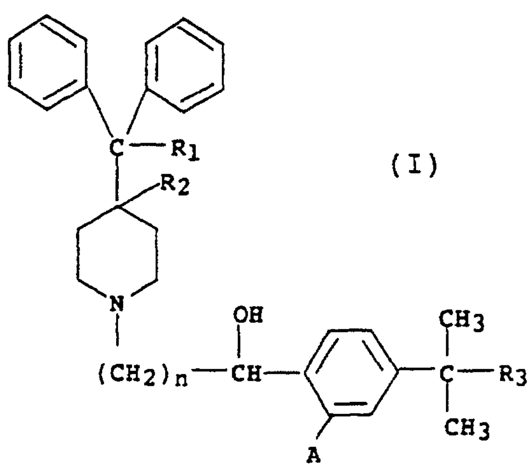

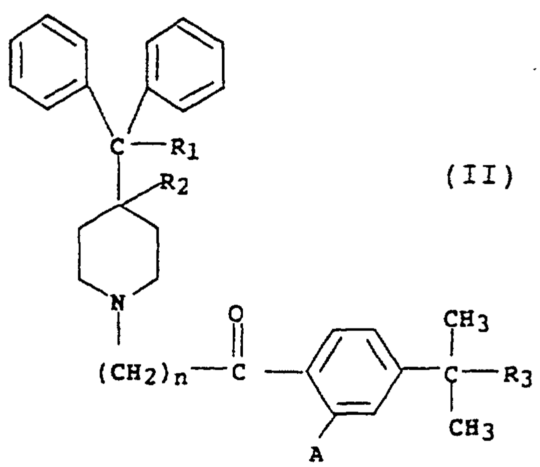

wherein

$R_1$ represents hydrogen or hydroxy;

$R_2$ represents hydrogen; or

$R_1$ and $R_2$ taken together form a second bond between the carbon atoms bearing $R_1$ and $R_2$;

n is an integer of from 1 to 5;

$R_3$ is $-CH_2OH$, -COOH or -COOalkyl wherein the alkyl moiety has from 1 to 6 carbon atoms and is straight or branched;

each of A is hydrogen; and

pharmaceutically acceptable salts and individual optical isomers thereof comprising the steps of:

(a) reacting a benzeneacetic acid compound of the formula

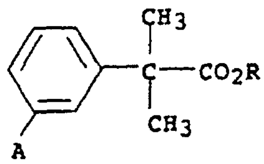

wherein A is as defined above and R is hydrogen or $C_1$-$C_6$ alkyl with a suitable reducing agent to give a phenethyl alcohol;

(b) reacting the phenethyl alcohol with a ω-halo compound of the formula

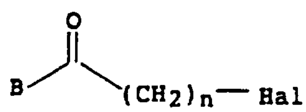

wherein B is halo or hydroxy, Hal represents Cl, Br or I and n is as defined above, in the presence of a suitable Lewis acid to produce a ω-halo hydroxyethylphenylketone; and

(c) reacting the ω-halo hydroxyethylphenylketone with a piperidine compound of the formula

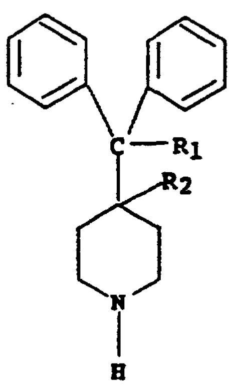

wherein $R_1$ and $R_2$ are as defined above in the presence of a suitable non-nucleophilic base to produce a piperidine hydroxyethylphenylketone derivative of formula (II) wherein $R_3$ is $-CH_2OH$;

(d) optionally reacting the piperidine hydroxyethylphenylketone derivative of formula (II) wherein $R_3$ is $-CH_2OH$ with a suitable oxidizing agent to produce a piperidine carboxyphenylketone derivative of formula (II) wherein $R_3$ is -COOH;

(e) optionally reacting the piperidine carboxyphenylketone derivative of formula (II) wherein $R_3$ is -COOH to produce a piperidine carboxyphenylketone ester derivative of formula (II) wherein $R_3$ is -COOalkyl.

(f) optionally reacting the piperidine carboxyphenylketone derivative of formula (II) wherein $R_3$ is -COOH or the piperidine carboxyphenylketone ester derivative of formula (II) wherein $R_3$ is -COOalkyl with a suitable reducing agent to produce a piperidine carboxyphenylalcohol derivative of formula (I) wherein $R_3$ is -COOH or the piperidine carboxyphenylalcohol ester of formula (I) wherein $R_3$ is -COOalkyl;

(g) optionally reacting the piperidine carboxyphenylalcohol derivative of formula (I) wherein $R_3$ is -COOH to produce a piperidine carboxyphenylalcohol ester derivative of formula (I) wherein $R_3$ is -COOalkyl; and

(h) optionally reacting the piperidine hydroxyethylphenylketone derivative of formula (II) wherein $R_3$ is $-CH_2OH$, the piperidine carboxyphenylketone derivative of formula (II) wherein $R_3$ is -COOH, the piperidine carboxyphenylketone ester derivative of formula (II) wherein $R_3$ is -COOalkyl, the piperidine carboxyphenylalcohol derivative of formula (I) wherein $R_3$ is -COOH or the piperidine carboxyphenylalcohol ester of formula (I) wherein $R_3$ is -COOalkyl with an appropriate deprotecting reagent,

with the proviso that each of the hydroxy groups present in the compounds described in steps a-g are optionally protected or unprotected.

Alternatively, the present invention provides a novel process for preparing the piperidine derivatives of formula (I) comprising the steps of:

(a) reacting the ω-halo hydroxyethylphenylketone with a suitable reducing agent to produce a ω-halo hydroxyethylphenylalcohol;

(b) reacting the ω-halo hydroxyethylphenylalcohol with a piperidine compound of the formula

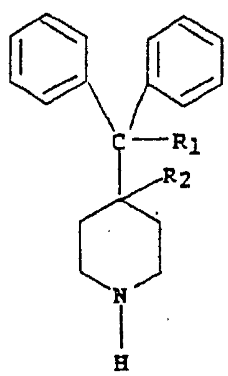

wherein $R_1$ and $R_2$ are as defined above, in the presence of a suitable non-nucleophilic base to produce a piperidine hydroxyethylphenylalcohol derivative of formula (I) wherein $R_3$ is $-CH_2OH$;

(c) optionally reacting the piperidine hydroxyethylphenylalcohol derivative of formula (I) wherein $R_3$ is $-CH_2OH$ with a suitable oxidizing agent to produce a piperidine carboxyphenylalcohol derivative of formula (I) wherein $R_3$ is -COOH; and

(d) optionally reacting the piperidine carboxyphenylalcohol derivative of formula (I) wherein $R_3$ is -COOH to produce the piperidine carboxyphenylalcohol ester derivative of formula (I) wherein $R_3$ is -COOalkyl,

(e) optionally reacting the piperidine hydroxyethylphenylalcohol derivative of formula (I) wherein $R_3$ is $-CH_2OH$, the piperidine carboxyphenylalcohol derivative of formula (I) wherein $R_3$ is -COOH or the piperidine carboxyphenylalcohol ester derivative of formula (I) wherein $R_3$ is -COOalkyl with an appropriate deprotecting reagent,

with the proviso that each of the hydroxy groups present in the compounds described in steps a-d are optionally protected or unprotected.

In addition, the present invention provides a novel process for preparing the piperidine derivatives of formula (I) comprising the steps of:

(a) reacting the piperidine hydroxyethylphenylketone derivative of formula (II) wherein $R_3$ is $-CH_2OH$ with a suitable reducing agent to produce a piperidine hydroxyethylphenylalcohol derivative of formula (I) wherein $R_3$ is $-CH_2OH$;

(b) optionally reacting the piperidine hydroxyethylphenylalcohol derivative of formula (I) wherein $R_3$ is $-CH_2OH$ with a suitable oxidizing agent to produce a piperidine carboxyphenylalcohol derivative of formula (I) wherein $R_3$ is -COOH; and

(c) optionally reacting the piperidine carboxyphenylalcohol derivative of formula (I) wherein $R_3$ is -COOH to produce the piperidine carboxyphenylalcohol ester derivative of formula (I) wherein $R_3$ is -COOalkyl.

(d) optionally reacting the piperidine hydroxyethylphenylketone derivative of formula (II) wherein $R_3$ is $-CH_2OH$, the piperidine hydroxyethylphenylalcohol derivative of formula (I) wherein $R_3$ is $-CH_2OH$, the piperidine carboxyphenylalcohol derivative of formula (I)

wherein $R_3$ is -COOH or the piperidine carboxyphenylalcohol ester derivative of formula (I) wherein $R_3$ is -COOalkyl with an appropriate deprotecting reagent,with the proviso that each of the hydroxy groups present in the compounds described in steps a-c are optionally protected or unprotected.

As used herein, straight or branched alkyl groups having from 1 to 6 carbon atoms as referred to herein are methyl, ethyl, n-propyl, isopropyl, n-butyl, sec-butyl, tert-butyl, n-pentyl, neopentyl and n-hexyl.

The piperidine derivatives of the formula (I) and formula (II) can form pharmaceutically acceptable salts. Pharmaceutically acceptable acid addition salts of the compounds of this invention are those of any suitable inorganic or organic acid. Suitable inorganic acids are, for example, hydrochloric, hydrobromic, sulfuric, and phosphoric acids. Suitable organic acids include carboxylic acids, such as, acetic, propionic, glycolic, lactic, pyruvic, malonic, succinic, fumaric, malic, tartaric, citric, cyclamic, ascorbic, maleic, hydroxymaleic, and dihydroxymaleic, benzoic, phenylacetic, 4-aminobenzoic, 4-hydroxybenzoic, anthranillic, cinnamic, salicyclic, 4-aminosalicyclic, 2-phenoxybenzoic, 2-acetoxybenzoic, and mandelic acid, sulfonic acids, such as, methanesulfonic, ethanesulfonic and β-hydroxyethanesulfonic acid. Non-toxic salts of the compounds of the above-identified formula formed with inorganic or organic bases are also included within the scope of this invention and include, for example, those of alkali metals, such as, sodium, potassium and lithium, alkaline earth metals, for example, calcium and magnesium, light metals of group IIIA, for example, aluminum, organic amines, such as, primary, secondary or tertiary amines, for example, cyclohexylamine, ethylamine, pyridine, methylaminoethanol and piperazine. The salts are prepared by conventional means as, for example, by treating a piperidine derivative of formula (I) or formula (II) with an appropriate acid or base.

The novel process for preparing the piperidine derivatives of formula (I) and formula (II) is outlined in Scheme A. In Scheme A, all substituents are as previously defined unless otherwise indicated.

Scheme A

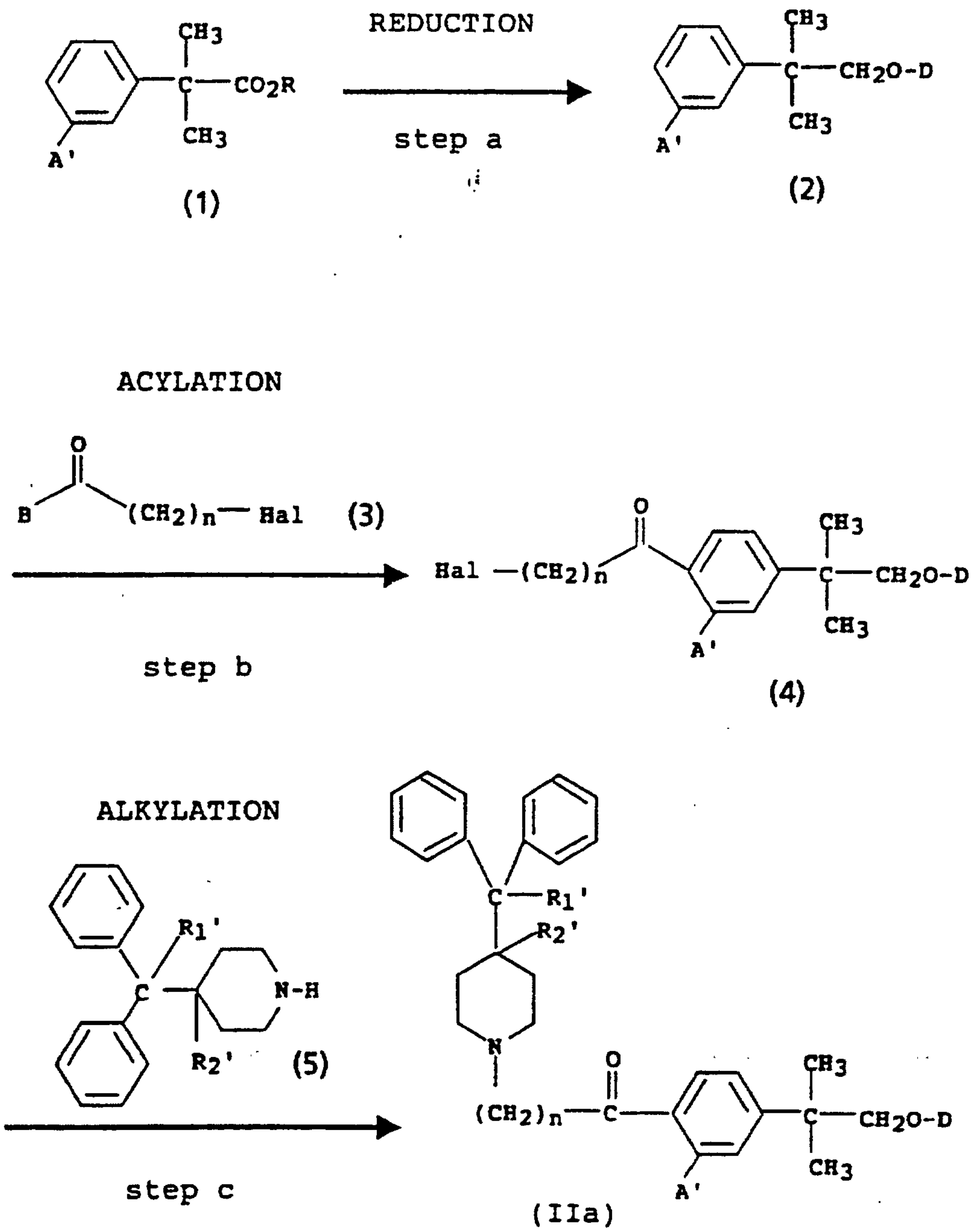


(1) (2) (3) (4) (5) (IIa)

Scheme A Cont.

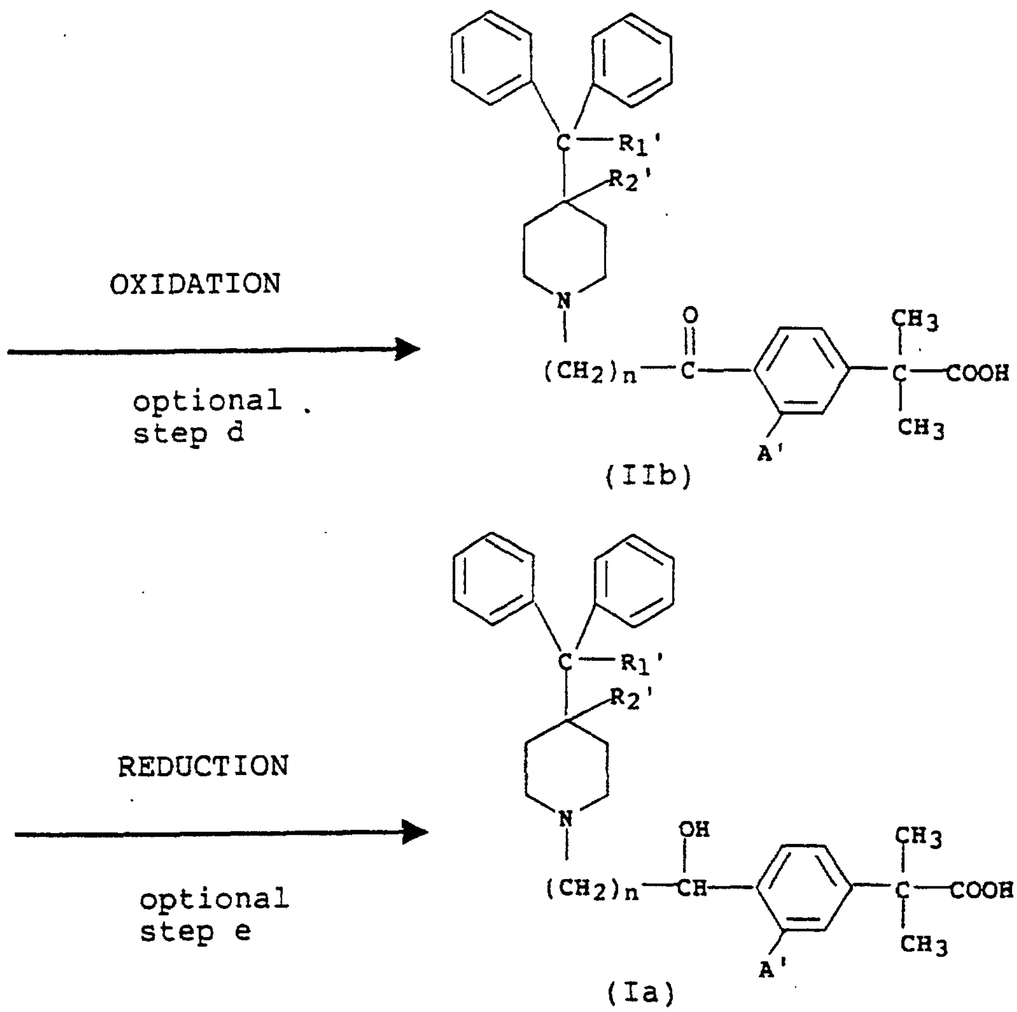


(IIb)

(Ia)

| | |
|---|---|
| A' = | hydrogen |
| B = | Halo or OH |
| D = | hydrogen or a suitable protecting group |
| $R_1$' and $R_2$' = | hydrogen, protected hydroxy, hydroxy or taken togther to form a second bond between the carbon atoms bearing $R_1$' and $R_2$' |
| R = | $C_1$-$C_6$ alkyl or hydrogen |

Scheme A provides a general synthetic procedure for preparing the piperidine derivatives of formula (I) and formula (II).

In step a, the carboxy functionality of an appropriate benzeneacetic acid compound of structure (1), wherein R is hydrogen or $C_1$-$C_6$ alkyl, is reduced to give the corresponding phenethyl alcohol of structure (2), wherein D is hydrogen.

For example, reduction of the appropriate benzeneacetic acid of structure (1), wherein R is hydrogen, using, for example, sodium bis(2-methoxyethoxy)aluminum hydride, lithium aluminum hydride, diborane or aluminum hydride with diborane being preferred. Reduction of the appropriate benzeneactic acid of structure (1), wherein R is $C_1$-$C_6$ alkyl, using, for example, lithium aluminum hydride, lithium borohydride, sodium bis(2-methoxyethoxy)aluminum hydride, aluminum hydride, lithium triethylborohydride and lithium tri-sec-butylborohydride with lithium aluminum hydride being preferred. Suitable solvents are ethers, for example, diethyl ether, tetrahydrofuran or dioxane. These reduction reactions are carried out at temperatures ranging from about 0°C to the reflux temperature of the solvent, and the reaction time varies from about 1/2 hour to 8 hours.

The starting benzeneacetic acid compounds of structure (1) are known in the art of are prepared by procedures well known in the art.

Alternatively, the phenethyl alcohol compounds of structure (2) may be prepared by Friedel-Crafts acylation of an appropriate benzene compound with a suitable protected 2-methyl-2-propenyl alcohol compound. For example, the phenethyl alcohol compound of structure (2) wherein A' is hydrogen, may be prepared by reacting an appropriate 2-methyl-2-propenyl alcohol compound with an appropriate benzene compound in the presence of $AlCl_3$.

In step b, the appropriate phenethyl alcohol of structure (2), wherein A' is hydrogen, is acylated with the ω-halo compound of structure (3) wherein B is halo under Friedel-Crafts conditions to give the corresponding ω-halo hydroxyethylphenylketone of structure (4) wherein A' is described as above.

For example, the ω-halo hydroxyethylphenylketone of structure (4), wherein A' is hydrogen, may be prepared by reacting an appropriate phenyethyl alcohol of structure (2), wherein A' is hydrogen, with a appropriate ω-halo compound of structure (3) wherein B is halo, which are known in the art or are prepared by procedures well known in the art, under the general conditions of a Friedel-Crafts acylation using a suitable Lewis acid. The reaction is carried out in a solvent, such as carbon disulfide, methylene chloride, tetrachloroethane or nitrobenzene with methylene chloride being the preferred solvent. The reaction time varies from about 1/2 hour to 8 hours, preferably 1 to 5 hours and the reaction temperature varies from about 0°C to 25°C. The ω-halo hydroxyethylphenylketone of structure (4) wherein A' is hydrogen, is recovered from the reaction zone by an aqueous quench followed by extraction as is known in the art. The ω-halo hydroxyethylphenylketone of structure (4) wherein A' is hydrogen, may be purified by procedures well known in the art, such as crystallization.

While not necessary for utilization in the acylation reaction of step b, the hydroxyethyl functionality of those phenethyl alcohols of structure (2) may be protected with a suitable protecting group. The selection and utilization of suitable protecting groups for the phenethyl alcohols of structure (2) is well known by one of ordinary skill in the art and is described in "Protective Groups in Organic Syntheses", Theodora W. Greene, Wiley (1981). For example, suitable protecting groups for the hydroxyethyl functionality include ethers such as tetrahydrothiopyranyl, tetrahydrothiofuranyl, 2-(phenylselenyl)ethyl ether, o-nitrobenzyl ether, trimethylsilyl ether, isopropyldimethylsilyl ether, t-butyldimethylsilyl ether, t-butyldiphenylsilyl ether, tribenzylsilyl ether, triisopropylsilyl ether; and esters, such as acetate ester, isobutyrate ester, pivaloate ester, adamantoate ester, benzoate ester, 2,4,6-trimethylbenzoate (mesitoate) ester, methyl carbonate, p-nitrophenyl carbonate, p-nitrobenzyl carbonate, S-benzyl thiocarbonate and N-phenylcarbamate, with acetoxy being preferred.

For those ω-halo hydroxyethylphenylketone of structure (4), wherein A' is hydrogen, and D is hydrogen, the Friedel-Crafts acylation may result in acylation of the hydroxyethyl functionality and will require deprotection prior to the oxidation reaction described in optional step d. Suitable deprotecting agents and methods are described in optional step d, infra.

In step c, the ω-halo functionality of the appropriate ω-halo hydroxyethylphenylketone of structure (4) is alkylated with the appropriate piperidine compound of structure (5) to give the corresponding piperidine hydroxyethylphenylketone derivative of formula (II) wherein $R_3$ is $-CH_2OH$.

For example, the alkylation reaction is carried out in a suitable solvent preferably in the present of a base and optionally in the presence of a catalytic amount of an iodide source, such as potassium or sodium iodide, for about 4 to 120 hours and at temperatures of about 70°C to the reflux temperature of the solvent. Suitable solvents for the alkylation reaction include alcohol solvents such as, methanol, ethanol, isopropyl alcohol, or n-butanol; ketone solvents, such as, methyl isobutyl ketone; hydrocarbon solvents, such as, benzene, toluene or xylene; halogenated hydrocarbons, such as, chlorobenzene or methylene chloride or dimethylformamide. Suitable bases for the alkylation reaction include inorganic bases, for example, sodium bicarbonate, potassium carbonate, or potassium bicarbonate or organic bases, such as, a trialkylamine, for example, triethylamine or pyridine, or an excess of an appropriate piperidine compound of structure (5) may be used.

For those piperidine compounds of structure (5), wherein $R_1$ is hydroxy, it is preferred that $R_1$ be unprotected for utilization in the alkyation reaction of step c, but those hydroxy functionalities present in the piperidine compounds of structure (5), wherein $R_1$ is hydroxy may be protected with a suitable protecting group. The selection and utilization of suitable protecting groups for the piperidine compounds of structure (5), wherein $R_1$ is hydroxy is well known by one of ordinary skill in the art and is described in "Protective Groups in Organic Syntheses", Theodora W. Greene, Wiley (1981). For example, suitable protecting groups for those hydroxy functionalities present include ethers such as tetrahydrothiopyranyl, tetrahydrothiofuranyl, 2-(phenylselenyl)ethyl ether, o-nitrobenzyl ether, trimethylsilyl ether, isopropyldimethylsilyl ether, t-butyldimethylsilyl ether, t-butyldiphenylsilyl ether, tribenzylsilyl ether, triisopropylsilyl ether; and esters, such as acetate ester, isobutyrate ester, pivaloate ester, adamantoate ester, benzoate ester, 2,4,6-trimethylbenzoate (mesitoate) ester, methyl carbonate, p-nitrophenyl carbonate, p-nitrobenzyl carbonate, S-benzyl thiocarbonate and N-phenylcarbamate.

The piperidine compounds of structure (5) wherein $R_1$ and $R_2$ are hydrogen and where $R_1$ is hydroxy and $R_2$ is hydrogen are readily available to one or ordinary skill in the art. The piperidine compounds of structure (5) wherein

$R_1$ and $R_2$ form a second bond between the carbon atoms bearing $R_1$ and $R_2$ may be prepared by dehydration of the corresponding compound wherein $R_1$ is hydroxy by procedures generally known in the art.

In optional step d, the hydroxyethyl functionality of the appropriate piperidine hydroxyethylphenylketone derivative of formula (II) wherein $R_3$ is $-CH_2OH$ is oxidized to give the corresponding piperidine carboxyphenylketone derivative of formula (II) wherein $R_3$ is-COOH.

For example, oxidation of the appropriate piperidine hydroxyethylphenylketone derivative of formula (II) wherein $R_3$ is $-CH_2OH$ may be achieved using a variety of oxidizing agents and methods.

One such method involves a two-step procedure in which the hydroxyethyl functionality of the appropriate piperidine hydroxyethylphenylketone derivative of formula (II) wherein $R_3$ is $-CH_2OH$ is first oxidized to the corresponding aldehyde functionality using, for example, Swern Oxidation conditions (dimethyl sulfoxide, oxyalyl chloride and triethylamine), as is known in the art. The Swern Oxidation is carried out in suitable aprotic organic solvents such as methylene chloride at temperatures ranging from about -78°C to room temperature, and the reaction time varies from about 1/2 hour to 8 hours. Other suitable reagents for the oxidation of the hydroxyethyl functionality of the appropriate piperidine hydroxyethylphenylketone derivative of formula (II) wherein $R_3$ is $-CH_2OH$ to the corresponding aldehyde functionality are Dess-Martin reagent, chromium (IV) oxide, nickel peroxide, sodium dichromate, potassium dichromate, t-butyl chromate, silver oxide, argentic picolinate manganese dioxide lead tetraacetate, dicyclohexylcarbodiimide, 2,3-dichloro-5,6-dicyanoquinone, tetrachloro-1,2-benzoquinone, 2,2,6,6-tetramethylpiperidinyl-1-oxy (TEMPO) or quinolinium chlorochromate.

The intermediate aldehyde compound is then oxidized further to give the corresponding piperidine carboxyphenylketone derivative of formula (II) wherein $R_3$ is -COOH using, for example, potassium permanganate. The potassium permanganate oxidation is carried out in a suitable acidic medium such as hydrochloric acid/acetone at a temperature ranging from about 0°C to room temperature and the reaction time varies from about 1/2 hour to 8 hours. Other suitable reagents for the oxidation of the intermediate aldehyde to the corresponding piperidine carboxyphenylketone derivative of formula (II) wherein $R_3$ is -COOH are chromium (IV) oxide, silver (I) oxide, silver oxide, argentic picolinate, peroxide, nitric acid, m-chloroperbenzoic acid and peracetic acid.

Another method involves a one-step procedure in which the hydroxyethyl functionality of the appropriate piperidine hydroxyethylphenylketone derivative of formula (II) wherein $R_3$ is $-CH_2OH$ is oxidized directly to the carboxy functionality to give the corresponding piperidine carboxyphenylketone derivative of formula (II) wherein $R_3$ is -COOH. Oxidizing reagents suitable for direct, one-step oxidation of the hydroxyethyl functionality to the carboxy functionality include, for example, chromium (IV) oxide, potassium permanganate, nitric acid, nitrogen dioxide, ruthenium (VIII) oxide, nickel peroxide, silver oxide, t-butyl chromate and xenic acid.

Oxidation using Swern Oxidation conditions, nickel peroxide, chromium (IV) oxide, silver oxide, sodium dichromate, potassium dichromate, manganese dioxide, 2,3-dichloro-5,6-dicyanoquinone and tetrachloro-1,2-benzoquinone is preferred for those piperidine hydroxyethylphenylketone derivatives of formula (II) wherein $R_3$ is $-CH_2OH$ wherein $R_1$ and $R_2$ taken together form a second bond between the carbon atoms bearing $R_1$ and $R_2$.

As one skilled in the art would appreciate, those piperidine hydroxyethylphenylketone derivatives of formula (II) wherein $R_3$ is $-CH_2OH$ wherein the hydroxyethyl functionality is protected must be reacted with an appropriate deprotecting reagent prior to the oxidation reaction described in step d. The selection and utilization of appropriate deprotecting reagents is well known by one of ordinary skill in the art and is described in "Protective Groups in Organic Syntheses", Theodora W. Greene, Wiley (1981). Examples of appropriate deprotecting reagents are mineral acids, strong organic acids, Lewis acids, aqueous mineral bases, catalytic hydrogenation and the like. For example, cleavage of an acetate ester protecting group on the hydroxyethyl functionality of the piperidine hydroxyethylphenylketone derivatives of formula (II) wherein $R_3$ is $-CH_2OH$ can be achieved by using a base, such as sodium methoxide in methanol as is known in the art. Other methods known in the art for acetate ester cleavage include potassium carbonate in methanol, methanolic ammonia, sodium hydroxide/pyridine in methanol and potassium cyanide in ethanol.

In optional step e, the ketone functionality of the appropriate piperidine carboxyphenylketone derivatives of formula (II) wherein $R_3$ is $-CH_2OD$ or -COOH is reduced to give the corresponding piperidine carboxyphenylalcohol derivatives of formula (I) wherein $R_3$ is $-CH_2OD$ or -COOH.

For example, reduction of the appropriate piperidine carboxyphenylketone derivatives of formula (II) wherein $R_3$ is $-CH_2OD$ or -COOH, using, for example, sodium borohydride, potassium borohydride, sodium cyanoborohydride, or tetramethylammonium borohydride is carried out in lower alcohol solvents, such as, methanol, ethanol, isopropyl alcohol or n-butanol at temperatures ranging from about 0°C to the reflux temperature of the solvent, and the reaction time varies from about 1/2 hour to 8 hours. Other suitable reducing agents are, for example, lithium tri-tert-butylaluminohydride and diisobutylaluminum hydride. These reduction reactions are carried out in suitable solvents, such as diethyl ether, tetrahydrofuran or dioxane at temperatures ranging from about 0°C to the reflux temperature of the solvent, and the reaction time varies from about 1/2 hour to 8 hours.

Catalytic reduction may also be employed in the preparation of appropriate piperidine carboxyphenylalcohol derivatives of formula (I) wherein $R_3$ is $-CH_2OD$ or -COOH from an appropriate piperidine carboxyphenylketone derivatives of formula (II) wherein $R_3$ is $-CH_2OD$ or -COOH, using, for example, Raney nickel, palladium, platinum or rhodium catalysts in lower alcohol solvents, such as, methanol, ethanol, isopropyl alcohol or n-butanol or acetic acid or their aqueous mixtures, or by the use of aluminum isopropoxide in isopropyl alcohol.

Reduction using sodium borohydride or potassium borohydride is preferred over catalytic reduction for those piperidine carboxyphenylketone derivatives of formula (II) wherein $R_3$ is $-CH_2OD$ or -COOH wherein $R_1$ and $R_2$ taken together form a second bond between the carbon atoms bearing $R_1$ and $R_2$.

In addition, a chiral reduction of the appropriate piperidine carboxyphenylketone derivatives of formula (II) wherein $R_3$ is $-CH_2OD$ or -COOH, using, for example, (+)-B-chlorodiisopinocamphenylborane gives the corresponding (R)-piperidine carboxyphenylalcohol derivatives of formula (I) wherein $R_3$ is $-CH_2OD$ or -COOH and (-)-B-chlorodiisopinocamphenylborane gives the corresponding (S)-piperidine carboxyphenylalcohol derivatives of formula (I) wherein $R_3$ is $-CH_2OD$ or -COOH. Other suitable chiral reducing agents are, (R) and (S)-oxazaborolidine/$BH_3$, potassium 9-O-(1,2,5,6-di-O-isopropylidine-α-D-glucofuransoyl)-9-boratabicyclo[3.3.1]nonane, (R) and (S)-B-3-pinanyl-9-borabicyclo[3.3.1]nonane, NB-Enantride, Lithium (R)-(+) and (S)-(-)-2,2'-dihydroxy-1,1'-binaphthyl alkoxyl aluminum hydride, (R)-(+) and (S)-(-)-2,2'-dihydroxy-6,6'-dimethylbiphenyl borane-amine complex, tris[[(1S,2S,5R)-2-isopropyl-5-methylcyclohex-1-yl]methyl]aluminum, [[(1R,3R)-2,2-dimethylbicyclo[2.2.1]hept-3-yl]methyl]beryllium chloride, (R)-BINAP-ruthenium complex/$H_2$ and 6,6'-bis(diphenylphosphino)-3,3'-dimethoxy-2,2',4,4'-tetramethyl-1,1'-biphenyl.

As one skilled in the art would appreciate, the carboxy functionalities of the piperidine carboxyphenylketone derivatives of formula (II) wherein $R_3$ is -COOH and piperidine carboxyphenylalcohol derivatives of formula (I) wherein $R_3$ is -COOH may be esterified by techniques and procedures well known and appreciated by one of ordinary skill in the art to give the corresponding piperidine carboxyphenylketone ester derivatives of formula (II) wherein $R_3$ is -COOalkyl and piperidine carboxyphenylalcohol ester derivatives of formula (I) wherein $R_3$ is -COOalkyl.

For example, one such method involves reacting an appropriate piperidine carboxyphenylketone derivative of formula (II) wherein $R_3$ is -COOH with an excess of an appropriate HOalkyl wherein the alkyl moiety has from 1 to 6 carbon atoms and is straight or branched in the presence of a small amount of mineral acid, e.g. sulfuric acid at reflux. Another suitable method involves reacting an appropriate piperidine carboxyphenylketone derivative of formula (II) wherein $R_3$ is -COOH or piperidine carboxyphenylalcohol derivative of formula (I) wherein $R_3$ is -COOH with an excess of diazomethane in a suitable solvent such as ether at room temperature to give the methyl ester. In addition, the piperidine carboxyphenylketone ester derivatives of formula (II) wherein $R_3$ is -COOalkyl or piperidine carboxyphenylalcohol ester derivatives of formula (I) wherein $R_3$ is -COOalkyl may also be prepared by reacting an appropriate piperidine carboxyphenylketone derivative of formula (II) wherein $R_3$ is -COOH or piperidine carboxyphenylalcohol derivative of formula (I) wherein $R_3$ is -COOH with an excess of 2,2-dimethoxypropane in a suitable solvent such as methanol at 0°C to room temperature to give the methyl ester. Another suitable method involves first reacting an appropriate piperidine carboxyphenylketone derivative of formula (II) wherein $R_3$ is -COOH with thionyl chloride in a suitable solvent such as methylene chloride to give an intermediate acid chloride, followed by addition of a suitable alcohol of the formula HOalkyl wherein the alkyl moiety has from 1 to 6 carbon atoms and is straight or branched.

As one skilled in the art would appreciate, the reduction of the ketone functionality of the appropriate piperidine carboxyphenylketone derivatives of formula (II) wherein $R_3$ is -COOH described in optional step e can be conducted on the ω-halo hydroxyethylphenylketone of structure (4) or piperidine hydroxyethylphenylketone derivatives of formula (II) wherein $R_3$ is $-CH_2OH$.

For example, reduction of an appropriate ω-halo hydroxyethylphenylketone of structure (4) using the techniques and methods described previously in step e gives the corresponding ω-halo hydroxyethylphenylalcohol. The resulting benzylic alcohol functionality may be optionally protected using the protecting groups described previously for hydroxyethyl in step b. The ω-halo hydroxyethylphenylalcohol so formed is then subjected to the alkylation reaction with an appropriate piperidine compound of structure (5) described previously in step c to give the corresponding piperidine hydroxyethylphenylalcohol derivative of formula (I) wherein $R_3$ is protected or unprotected $-CH_2OH$. The appropriate piperidine hydroxyethylphenylalcohol derivative of formula (I) wherein $R_3$ is $-CH_2OH$ is then subjected to the oxidation reaction described previously in step d using a selective oxidizing agent such as 2,2,6,6-tetramethylpiperidinyl-1-oxy (TEMPO) or quinolinium chlorochromate to give the intermediate aldehyde followed by oxidation with silver oxide to give the corresponding piperidine carboxyphenylalcohol derivative of formula (I) wherein $R_3$ is -COOH. Reduction of an appropriate piperidine hydroxyethylphenylketone derivative of formula (II) wherein $R_3$ is protected or unprotected $-CH_2OH$ using the techniques and methods described previously in step e gives the corresponding piperidine hydroxyethylphenylalcohol derivative of formula (I) wherein $R_3$ is $-CH_2OH$ which is then subjected to the oxidation reaction described previously in step d using a selective oxidizing agent such as 2,2,6,6-tetramethylpiperidinyl-1-oxy (TEMPO) or quinolinium chlorochromate to give the intermediate aldehyde followed by oxidation with silver oxide to give the corresponding piperidine carboxyphenylalcohol derivative of formula (I) wherein $R_3$ is -COOH.

As one skilled in the art would appreciate, the benzeneacetic acid compounds of structure (1), the phenethyl alcohols of structure (2), the ω-halo hydroxyethylphenylketones of structure (4), piperidine compounds of structure (5), piperidine hydroxyethylphenylketone derivatives of formula (II) wherein $R_3$ is $-CH_2OH$, the piperidine carboxyphenylke-

tone derivatives of formula (II) wherein $R_3$ is -COOH, the piperidine carboxyphenylketone ester derivatives of formula (II) wherein $R_3$ is -COOalkyl, the piperidine hydroxyethylphenylalcohol derivatives of formula (I) wherein $R_3$ is $-CH_2OH$, the piperidine carboxyphenylalcohol derivatives of formula (I) wherein $R_3$ is -COOH or the piperidine carboxyphenylalcohol ester derivatives of formula (I) wherein $R_3$ is -COOalkyl which bear hydroxy or phenolic functionalities may be protected prior to use in the synthesis depicted in Scheme A using suitable protecting groups as described previously in step b.

As one skilled in the art would appreciate, the benzeneactic acids of structure (1), the phenethyl alcohols of structure (2), the ω-halo hydroxyethylphenylketones of structure (4), piperidine compounds of structure (5), piperidine hydroxyethylphenylketone derivatives of formula (II) wherein $R_3$ is $-CH_2OH$, the piperidine carboxyphenylketone derivatives of formula (II) wherein $R_3$ is -COOH or the piperidine carboxyphenylketone ester derivatives of formula (II) wherein $R_3$ is -COOalkyl, the piperidine hydroxyethylphenylalcohol derivatives of formula (I) wherein $R_3$ is $-CH_2OH$, the piperidine carboxyphenylalcohol derivatives of formula (I) wherein $R_3$ is -COOH or the piperidine carboxyphenylalcohol ester derivatives of formula (I) wherein $R_3$ is-COOalkyl which bear protected hydroxy or phenolic functionalities may be reacting with prior appropriate deprotecting reagents prior to use in the synthesis depicted in Scheme A. The selection and utilization of appropriate deprotecting reagents is well known by one of ordinary skill in the art and is described in "Protective Groups in Organic Syntheses", Theodora W. Greene, Wiley (1981). Examples of appropriate deprotecting reagents are mineral acids, strong organic acids, Lewis acids, aqueous mineral bases, catalytic hydrogenation and the like.

For example, cleavage of an acetate ester protecting group on the hydroxyethyl functionality of any of the ω-halo hydroxyethylphenylketones of structure (4), piperidine compounds of structure (5), piperidine hydroxyethylphenylketone derivatives of formula (II) wherein $R_3$ is $-CH_2OH$ or piperidine hydroxyethylphenylalcohol derivatives of formula (I) wherein $R_3$ is $-CH_2OH$ can be achieved by using a base, such as sodium methoxide in methanol as is known in the art. Other methods known in the art for acetate ester cleavage include potassium carbonate in methanol, methanolic ammonia, sodium hydroxide/pyridine in methanol and potassium cyanide in ethanol.

Cleavage of β-methoxyethoxymethyl (MEM) protecting groups on any of those ω-halo hydroxyethylphenylketones of structure (4), piperidine compounds of structure (5), piperidine hydroxyethylphenylketone derivative of formula (II) wherein $R_3$ is $-CH_2OH$, piperidine carboxyphenylketone derivative of formula (II) wherein $R_3$ is -COOH, piperidine carboxyphenylketone ester derivatives of formula (II) wherein $R_3$ is -COOalkyl, piperidine carboxyphenylalcohol derivatives of formula (I) wherein $R_3$ is -COOH, piperidine carboxyphenylalcohol ester derivatives of formula (I) wherein $R_3$ is -COOalkyl or piperidine hydroxyethylphenylalcohol derivatives of formula (I) wherein $R_3$ is $-CH_2OH$ wherein A is hydroxy, for example, can be achieved by using trifluoroacetic acid at room temperature or using 5 to 8 equivalents of powdered anhydrous zinc bromide in methylene chloride at about 25°C by the general procedure of E. J. Corey et al., *Tetrahedron Letters*, 11, 809-812 1976.

The following examples represent typical syntheses as described in Scheme A. These examples are understood to be illustrative only and are not intended to limit the scope of the present invention in any way. As used herein, the following terms have the indicated meanings: "g" refers to grams; "mmol" refers to millimoles; "mL" refers to milliliters; "bp" refers to boiling point; "mp" refers to melting point; "°C" refers to degrees Celsius; "mm Hg" refers to millimeters of mercury; "µL" refers to microliters; "µg" refers to micrograms; and "µM" refers to micromolar.

Example 1

4-[4-[4-(Hydroxydiphenylmethyl)-1-piperidinyl]-1-hydroxybutyl]-α,α-dimethylbenzeneacetic acid hydrochloride

METHOD A

Step a: 2,2-Dimethyl-phenethyl acetate

Dissolve α,α-dimethylphenyl acetic acid (140.0g, 0.853mol) in anhydrous tetrahydrofuran (100mL) and place under a nitrogen atmosphere. Add, by dropwise addition, a solution of lithium aluminum hydride (639mL of a 1.0M solution in tetrahydrofuran, 24.3g, 0.639mol) over a period of approximately 2 hours. Quench with deionized water (24mL), with 15% aqueous sodium hydroxide (24mL) and again with deionized water (72mL). Stir the milky white mixture for 20 minutes, filter through filter aid, dry ($MgSO_4$) and filter through filter aid once more. Evaporate the solvent *in vacuo* to give 2,2-dimethylphenethyl alcohol as a clear yellow oil.

Dissolve 2,2-dimethylphenethyl alcohol (118.0g, 0.786mol) in pyridine (700mL). Add, by dropwise addition, acetic anhydride (222mL, 240.7g, 2.358mol) and stir overnight at room temperature. Evaporate the solvent *in vacuo* and purify by distillation to give the title compound as a clear colorless oil; bp 75°C @ 0.4mmHg.

METHOD B

Dissolve 2-methyl-2-propenyl acetate (1.28mmol) in benzene (80mL) and add to a stirred solution of $AlCl_3$ (172.3g, 1.29mol) in benzene (800mL) at -10°C over 45 minutes under a stream of nitrogen. Stir at 5°C for 20 minutes, pour onto ice (800g) and stir for 10 minutes. Separate the organic phase, dry ($MgSO_4$) and evaporate the solvent *in vacuo* (30°C/60 torr). Purify by distillation to give the title compound.

Step b: 4-(4-Chloro-1-oxobutyl)-2,2-dimethylphenethyl acetate

Charge a flask with aluminum chloride (223g, 1.68mol) and methylene chloride (200mL). Place under a nitrogen atmosphere, cool to 0°-5°C and add, by dropwise addition, ω-chlorobutyryl chloride (188.6g, 1.34mol). After acid chloride addition is complete, add, by dropwise addition, 2,2-dimethylphenethyl acetate (128.0g, 0.67mol), keeping the temperature at approximately 0°C. Continue stirring at 0°C for 2 hours, quench by slowly pouring over approximately 2L of crushed ice. Add methylene chloride (500mL) and stir for 5 minutes. Separate the organic phase and extract the aqueous phase with methylene chloride (300mL). Combine the organic phases and wash with saturated aqueous sodium hydrogen carbonate (3X200mL), with deionized water (200mL) and brine (200mL). Dry ($MgSO_4$) and stir for 30 minutes before filtering. Evaporate the solvent *in vacuo* and purify by chromatography (ethyl acetate/hexane) to give the title compound as an orange/brown oil.
IR (neat) 3239, 2970, 1741, 1684, 1607, 1408, 1375, 1233, 1040, 998, 844, 823 $cm^{-1}$;
$^1H$ NMR ($CDCl_3$) δ 7.93 (d, 2H, J = 9.0Hz), 7.46 (d, 2H, J = 9.0 Hz), 4.14 (s, 2H), 3.68 (t, 2H, J = 7.5 Hz), 3.16 (t, 2H, J = 7.5 Hz), 2.2 (m, 2H), 2.00 (s, 3H), 1.38 (s, 6H); $^{13}C$ NMR ($CDCl_3$) δ 198.5, 170.9, 151.9, 134.8, 127.9, 126.2, 72.4, 44.6, 38.6, 35.2, 26.7, 25.7, 20.8;
MS (CI, $CH_4$) m/z (rel. intensity) 297 ($MH^+$, 56), 261 (59), 237 (100), 219 (52).

Step c: 4-[4-[4-(Hydroxydiphenylmethyl)-1-piperidinyl]-1-oxobutyl]-2,2-dimethylphenethyl alcohol

Mix 4-(4-chloro-1-oxobutyl)-2,2-dimethylphenethyl acetate (99.5g, 0.335mol), α,α-diphenyl-4-piperidinemethanol (101.8g, 0.335mol), potassium hydrogen carbonate (83.8g, 0.838mol), potassium iodide (1.008, 0.006mol), toluene (600mL) and water (220mL). Stir at reflux for 72 hours, add toluene (200mL) and deionized water (100mL). Filter through filter aid while at 80°C and separate the organic phase. Dry ($MgSO_4$), filter and purify by chromatography (ethyl acetate) to give 4-[4-[4-(hydroxydiphenylmethyl)-1-piperidinyl]-l-oxobutyl]-2,2-dimethylphenethyl acetate as an oily solid.
IR (KBr) 3690, 3608, 3012, 2950, 2810, 1734, 1681, 1607, 1470, 1448, 1376, 1253, 1040, 997, 704, 667 $cm^{-1}$;
$^1H$ NMR ($CDCl_3$) δ 7.90 (d, 2H, J = 8.2 Hz), 7.4 (m, 5H), 7.3 (m, 5H), 7.2 (m, 2H), 4.14 (s, 2H), 3.0 (m, 4H), 2.4 (m, 3H), 2.0 (m, 3H), 1.95 (s, 3H), 1.4 (m, 4H), 1.38 (s, 6H);
$^{13}C$ NMR ($CDCl_3$) δ 199.4, 170.9, 151.7, 145.8, 135.1, 128.1, 128.0, 126.5, 126.2, 125.7, 79.3, 72.5, 57.6, 53.7, 43.8, 38.6, 36.1, 25.7, 21.2, 20.8;
MS (CI, $CH_4$) m/z (rel. intensity) 528 ($MH^+$, 100), 510 (63), 450 (12), 293 (14).
Dissolve 4-[4-[4-(hydroxydiphenylmethyl)-1-piperidinyl]-1-oxobutyl]-2,2-dimethylphenethyl acetate (69.0g, 0.131mol) in methanol (2.5L) and add 10% aqueous sodium hydroxide (769mL, 1.92mol). Stir at reflux for 1.5 hours, cool to 68°C and evaporate the solvent *in vacuo* to a residue (700mL). Add chloroform (1L) and stir until solids are dissolved. Separate the organic phase and extract the aqueous phase with chloroform (3X300mL). Combine the organic phases, dry ($MgSO_4$) and evaporate the solvent *in vacuo* and recrystallize (toluene) to give the title compound as a cream-colored powder; mp 135-137°C.
IR (KBr) 3609, 3011, 2950, 2809, 2772, 1680, 1606, 1492, 1470, 1448, 1366, 1282, 1238, 1044, 791, 704, 668 $cm^{-1}$;
$^1H$ NMR ($CDCl_3$) δ 7.93 (d, 2H, J = 8.2 Hz), 7.4 (m, 5H), 7.3 (m, 5H), 7.2 (m, 2H), 3.64 (s, 2H), 2.9 (m, 4H), 2.4 (m, 3H), 1.9 (M, 5H), 1.38 (s, 6H), 1.3 (m, 4H);
$^{13}C$ NMR ($CDCl_3$) δ 199.6, 152.1, 145.9, 135.2, 128.2, 126.4, 125.7, 79.5, 72.7, 57.8, 53.9, 44.0, 40.4, 36.2, 26.1, 25.2, 22.2;
MS (CI, $CH_4$) m/z (rel. intensity) 486 ($MH^+$, 100), 468 (81), 408 (19), 293 (23).

METHOD A

Step d: 4-[4-[4-(Hydoxydiphenylmethyl)-1-piperidinyl]-1-oxobutyl]-α,α-dimethylbenzeneacetic acid hydrochloride

Dissolve oxalyl chloride (1.57g, 12.4mmol) in methylene chloride (17mL), cool to -55°C and place under a nitrogen atmosphere. Add, by dropwise addition, a solution of dimethylsulfoxide (1.77g, 1.61mL) in methylene chloride (4.5mL). Stir for 15 minutes and add, by dropwise addition, a solution of 4-[4-[4-(hydoxydiphenylmethyl)-1-piperidinyl]-1-oxobutyl]-2,2-dimethylphenethyl alcohol (5.0g, 10.3mol) in methylene chloride (33mL). Stir for 30 minutes and add, by dropwise addition, triethylamine (7.2mL). Stir for 15 minutes and then allow to warm to -10°C. Add a solution of oxone (12.66g) in deionized water (50mL). Stir for 15 minutes and add methylene chloride (25mL). Separate the organic phase, wash with brine, dry (MgSO4) and evaporate the solvent in vacuo. Purify by chromatography (ethyl acetate) to give 4-[4-[4-(hydoxydiphenylmethyl)-1-piperidinyl]-1-oxobutyl]-α,α-dimethylbenzeneacetaldehyde.
$^1$H NMR ($CDCl_3$) δ 9.52 (s, 1H), 7.95 (d, 2H, J = 8.2 Hz), 7.5 (m, 4H), 7.36 (d, 2H, J = 8.2 Hz), 7.3 (m, 4H), 7.2 (m, 2H), 2.9 (m, 4H), 2.4 (m, 4H), 2.0 (m, 4H), 1.50 (s, 6H), 1.4 (m, 4H);
$^{13}$C NMR ($CDCl_3$) δ 202, 199.9, 146.2, 136.2, 128.7, 128.3, 127.1, 126.6, 125.9, 79.4, 57.7, 53.8, 50.6, 43.9, 42.5, 36.2, 25.9, 22.3, 21.5;
MS (CI, $CH_4$) m/z (rel. intensity) 484 ($MH^+$, 76), 466 (100), 454 (19), 406 (16), 293 (16), 233 (19), 183 (49, 155 (54).

Dissolve 4-[4-[4-(hydroxydiphenylmethyl)-1-piperidinyl]-1-oxobutyl]-α,α-dimethylbenzeneacetaldehyde (3.40g, 7.03mmol) in acetone (30mL) and cool to 15°C. Add, by dropwise addition, IN hydrochloric acid (10.5mL). After addition of the hydrochloric acid is complete, add, by dropwise addition, a solution of potassium permanganate (1.82g, 11.51mmol) in acetone (80mL). Stir at room temperature for 6 hours, filter and wash the filter cake with acetone (60mL). Evaporate the filtrate *in vacuo*, dilute with methylene chloride (500mL), dry ($MgSO_4$) and filter. Evaporate the solvent *in vacuo* and purify by chromatography (ethyl acetate) to give the title compound as a pale yellow solid.
IR (KBr) 3420, 3057, 2964, 1677, 1604, 1569, 1470, 1448, 1406, 1363, 1249, 1189, 1099, 750, 705 $cm^{-1}$;
$^1$H NMR ($CDCl_3$) δ 7.75 (d, 2H, J = 8.2 Hz), 7.4 (m, 6H), 7.2 (m, 4H), 7.1 (m, 2H), 3.9 (br. s, 2H), 3.1 (m, 2H), 2.9 (m, 2H), 2.6 (m, 2H), 2.3 (m, 2H), 1.9 (m, 3H), 1.7 (m, 2H). 1.44 (s, 6H), 1.4 (m, 2H);
$^{13}$C NMR ($CDCl_3$) δ 199.4, 147.2, 134.5, 127.7, 127.5, 126.2, 125.7, 78.4, 57.3, 53.5, 46.6, 43.5, 35.6, 26.8, 25.9, 21.3;
MS (CI, $CH_4$) m/z (rel. intensity) 500 ($MH^+$, 79), 482 (62), 456 (100), 422 (30), 366 (42).

METHOD B

Mix 4-[4-[4-(hydoxydiphenylmethyl)-1-piperidinyl]-1-oxobutyl]-2,2-dimethylphenethyl alcohol (9.7mmol), chloroform (20mL), acetonitrile (20mL), water (30mL) and $H_5IO_6$ (5.6g, 24.3mmol). Add $RuCl_3$•$5H_2O$ (0.15g, 0.49mmol) and stir at room temperature for 2 hours. Dilute the reaction mixture with methylene chloride (200mL), wash with saturated $NaHSO_3$ (2X100ML) and dry ($MgSO_4$). Filter, evaporate the filtrate *in vacuo* (20°C/60 torr) and purify by silica gel chromatography to give the title compound.

METHOD C

Mix $K_2S_2O_8$ (2.8g, 10.2mmol), KOH (85%, 1.95g, 30mmol) and water (20mL). Add $RuCl_3$•$5H_2O$ (30mg, 0.2mmol) and stir at room temperature for 5 minutes. Dissolve 4-[4-[4-(hydoxydiphenylmethyl)-1-piperidinyl]-1-oxobutyl]-2,2-dimethylphenethyl alcohol (0.97mmol) in acetone (14mL) and acetonitrile (2mL) and add to the above solution. Stir at room temperature for 2.5 hours, filter and neutralize the filtrate with IN HCl to pH 5. Extract with methylene chloride (2X50mL), dry ($MgSO_4$), evaporate the solvent *in vacuo* (20°C/60 torr) and purify by silica gel chromatography to give the title compound.

Step e: 4-[4-[4-(Hydroxydiphenylmethyl)-1-piperidinyl]-1-hydroxybutyl]-α,α-dimethylbenzeneacetic acid hydrochloride

Add sodium borohydride (0.1058, 2.77mmol) to a solution of sodium hydroxide (0.053g, 1.33mmol) in deionized water (2mL) and add, by dropwise addition, to a solution of 4-[4-[4-(hydroxydiphenylmethyl)-1-piperidinyl]-1-oxobutyl]-α,α-dimethylbenzeneacetic acid hydrochloride (0.70g, 1.31mmol) in ethanol (30mL). Stir at room temperature for 3.5 hours at pH 7-8. Evaporate the solvent *in vacuo* and stir the residue with methylene chloride (15mL) and deionized water (15mL). Dry ($MgSO_4$), acidify to pH 3 with gaseous hydrogen chloride and evaporate the solvent. Add ether with stirring, filter the white solid and wash with additional ether. Dry to give the title compound.
IR (KBr) 3403, 3058, 2971, 1718, 1634, 1492, 1471, 1448, 1393, 1227, 1150, 1099, 1069, 839, 750, 706 $cm^{-1}$;
$^1$H NMR ($CDCl_3$) δ 7.50 (d, 4H, J = 8.2 Hz), 7.3 (m, 8H), 7.2 (m, 2H), 4.66 (t, 1H, J = 5.6 Hz), 3.5 (m, 2H), 3.0 (m, 4H), 2.8 (m, 2H), 1.7 (m, 8H), 1.53 (s, 6H);
13C NMR ($CDCl_3$) δ 181.1, 147.4, 146.1, 144.4, 129.5, 128.0, 127.4, 127.2, 79.9, 73.9, 57.0, 54.1, 42.7, 36.8, 27.1, 25.7, 21.7;
MS (CI, $CH_4$) m/z (rel. intensity) 502 ($MH^+$, 50), 485 (33), 484 (100), 458 (25), 454 (33), 424 (17).

Example 2

(R)-4-[4-[4-(Hydroxydiphenylmethyl)-1-piperidinyl]-1-hydroxybutyl]-α,α-dimethylbenzeneacetic, ethyl ester

Dissolve (+)-B-chlorodiisopinocamphenylborane (2.5g, 7.8mmol) in anhydrous tetrahydrofuran (5mL). Add a solution of 4-[4-[4-(hydroxydiphenylmethyl)-1-piperidinyl]-1-oxobutyl]-α,α-dimethylbenzeneacetic, ethyl ester (2g, 3.54mmol) in anhydrous tetrahydrofuran (5mL). Stir at room temperature for 3 days and cool to 0°C. Add water (1mL) and 30% hydrogen peroxide (2mL) and stir for 20 minutes. Add methylene chloride (30mL) and wash with brine (30mL), then aqueous sodium hydrogen carbonate (30mL), then brine (30mL). Dry ($MgSO_4$), evaporate the solvent *in vacuo* and purify by chromatography (1:19 methanol:ethyl acetate) to give the title compound as a solid; mp 87-90°C.
IR (KBr) 3436, 3058, 2932, 2813, 1725, 1632, 1599, 1470, 1448, 1255, 1147, 1097, 830, 749, 704 $cm^{-1}$;
$^1H$ NMR ($CDCl_3$) δ 7.5 (m, 4H), 7.3 (m, 8H), 7.2 (m, 2H), 4.6 (m, 1H), 4.08 (q, 2H, J = 7.5 Hz), 3.1 (m, 1H), 3.0 (m, 1H), 2.4 (m, 3H), 2.0 (m, 3H), 1.7 (m, 5H), 1.53 (s, 6H), 1.5 (m, 2H), 1.42 (s, 2H), 1.15 (t, 3H, J = 7.5 Hz); $[\alpha]^{20}_D$ +39.4° (c = 0.99, $CHCl_3$).

Example 3

(S)-4-[4-[4-(Hydroxydiphenylmethyl)-1-piperidinyl]-1-hydroxybutyl]-α,α-dimethylbenzeneacetic acid, ethyl ester

Dissolve (-)-B-chlorodiisopinocamphenylborane (2.5g, 7.8mmol) in anhydrous tetrahydrofuran (5mL). Add a solution of 4-[4-[4-(hydroxydiphenylmethyl)-1-piperidinyl]-1-oxobutyl]-α,α-dimethylbenzeneacetic acid, ethyl ester (2g, 3.54mmol) in anhydrous tetrahydrofuran (5mL). Stir at room temperature for 3 days and cool to 0°C. Add water (1mL) and 30% hydrogen peroxide (2mL) and stir for 20 minutes. Add methylene chloride (30mL) and wash with brine (30mL), then aqueous sodium hydrogen carbonate (30mL), then brine (30mL). Dry ($MgSO_4$), evaporate the solvent *in vacuo* and purify by chromatography to give the title compound.

**Claims**

**1.** A piperidine formylphenylalcohol compound of the formula

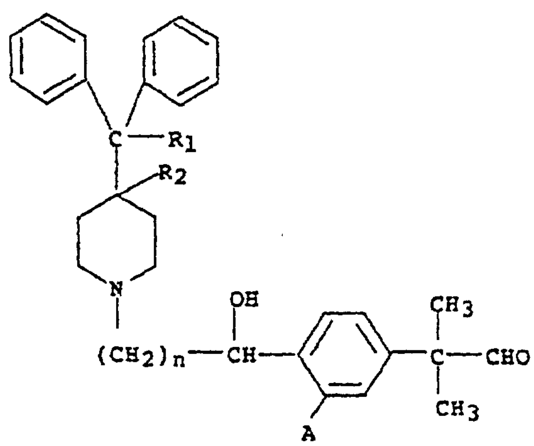

wherein
$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5; and
A is hydrogen.

**2.** A piperidine formylphenylketone compound of the formula

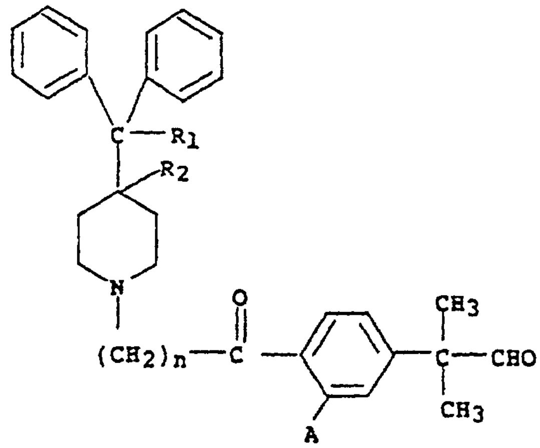

wherein

$R_1$ represents hydrogen or hydroxy;

$R_2$ represents hydrogen; or

$R_1$ and $R_2$ taken together form a second bond between the carbon atoms bearing $R_1$ and $R_2$;

n is an integer of from 1 to 5; and

A is hydrogen.

**3.** A piperidine protected hydroxyethylphenylalcohol compound of the formula

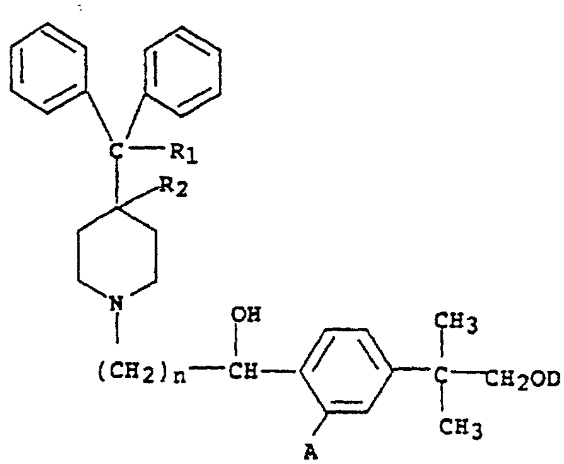

wherein

$R_1$ represents hydrogen or hydroxy;

$R_2$ represents hydrogen; or

$R_1$ and $R_2$ taken together form a second bond between th carbon atoms bearing $R_1$ and $R_2$;

n is an integer of from 1 to 5;

A is hydrogen; and

D is $C(=O)CH_3$ or $C(=O)C_6H_5$.

**4.** A piperidine protected hydroxyethylphenylketone compound of the formula

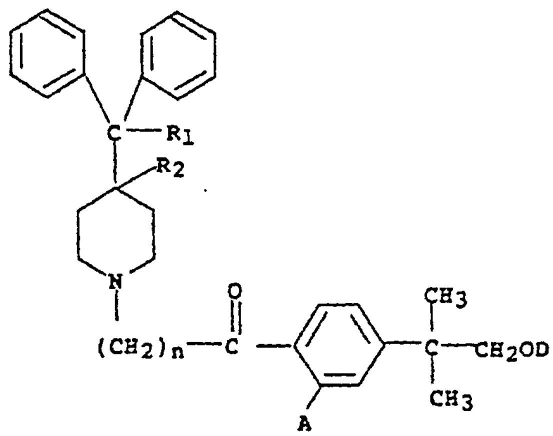

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
A is hydrogen and
D is $C(=O)CH_3$ or $C(=O)C_6H_5$.

**5.** A process for preparing a piperidine formylphenylalcohol compound of the formula

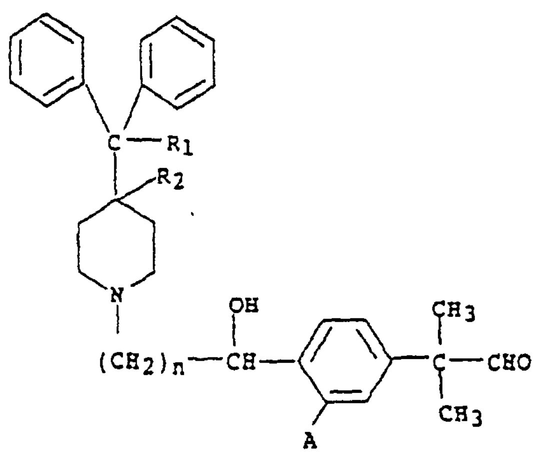

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5; and
A is hydrogen, or

comprising the steps of:

(a) reacting a benzeneacetic acid compound of the formula

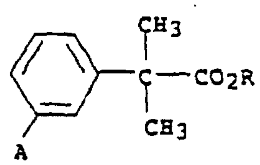

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

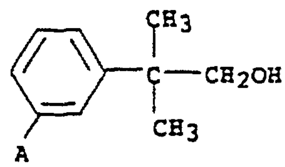

wherein A is hydrogen;

(b) protecting the hydroxyethyl functionality with a suitable protecting group to give a protected phenethyl alcohol of the formula

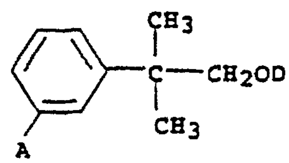

wherein A is hydrogen and D is a suitable protecting group;

(c) reacting the protected phenethyl alcohol with a ω-halo compound of the formula

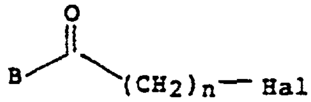

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

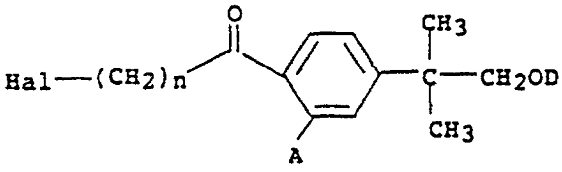

wherein Hal, n and D are defined above and A is hydrogen

(d) reacting the ω-halo protected hydroxyethylphenylketone with a piperidine compound of the formula

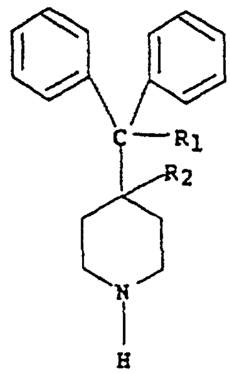

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

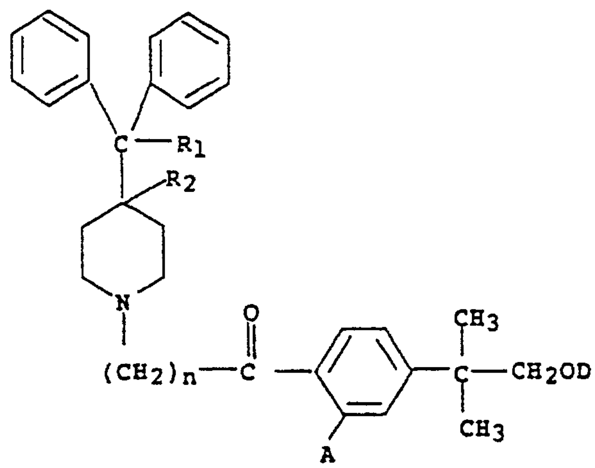

wherein $R_1$, $R_2$, n and D are defined above and A is hydrogen;

(e) reacting the piperidine protected hydroxyethylphenylketone with sodium borohydride, potassium borohydride, sodium cyanoborohydride, tetramethylammonium borohydride, lithium tri-tert-butylaluminohydride, diisobutylaluminum hydride or catalytic hydrogenation to produce a piperidine protected hydroxyethylphenylalcohol of the formula

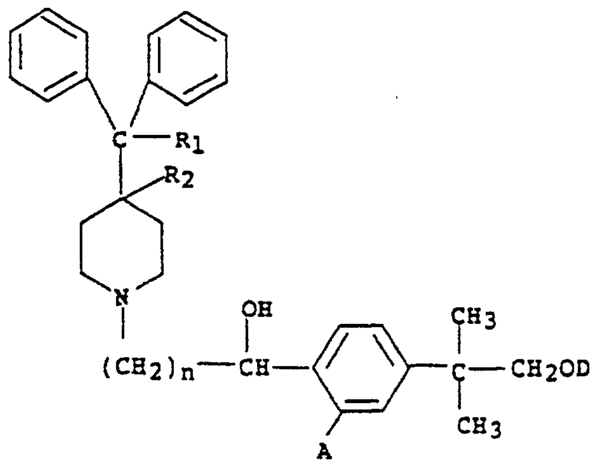

wherein $R_1$, $R_2$, D and n are defined above and A is hydrogen;

(f) deprotecting the hydroxyethyl functionality of the piperidine protected hydroxyethylphenylalcohol with an appropriate deprotecting reagent to produce a piperidine hydroxyethylphenylalcohol of the formula

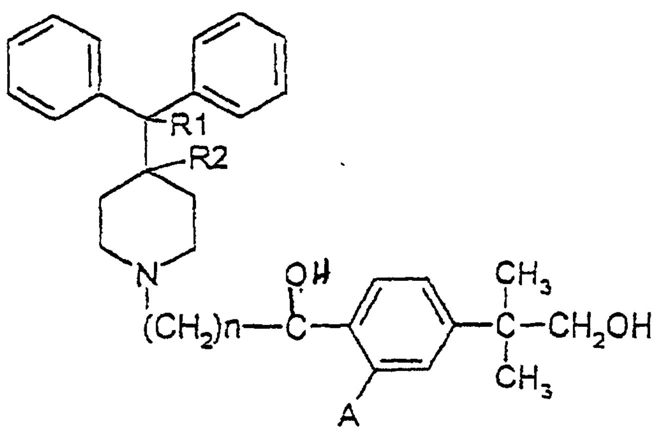

wherein $R_1$, $R_2$ and n are defined above and A is hydrogen;

(g) reacting the piperidine hydroxyethylphenylalcohol with dimethyl sulfoxide, oxyalyl chloride and triethylamine or with Dess-Martin reagent, chromium (IV) oxide, nickel peroxide, sodium dichromate, potassium dichromate, t-butyl chromate, silver oxide, argentic picolinate manganese dioxide lead tetraacetate, dicyclohexylcarbodiimide, 2,3-dichloro-5,6-dicyanoquinone, tetrachloro-1,2-benzoquinone, 2,2,6,6-tetramethylpiperidinyl-1-oxy (TEMPO) or quinolinium chlorochromate to produce a piperidine formylphenylalcohol of the formula

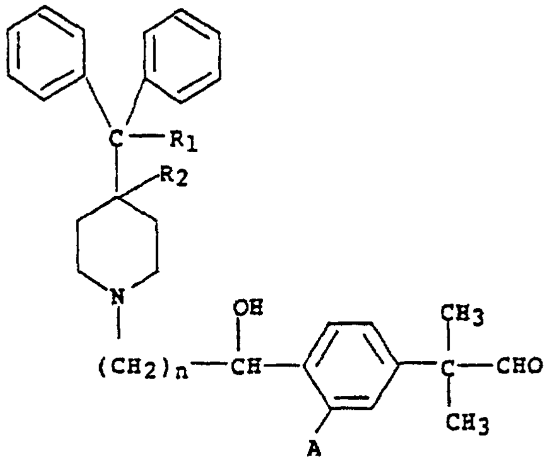

wherein $R_1$, $R_2$ and n are defined above and A is hydrogen.

**6.** A process for preparing a piperidine formylphenyl ketone compound of the formula

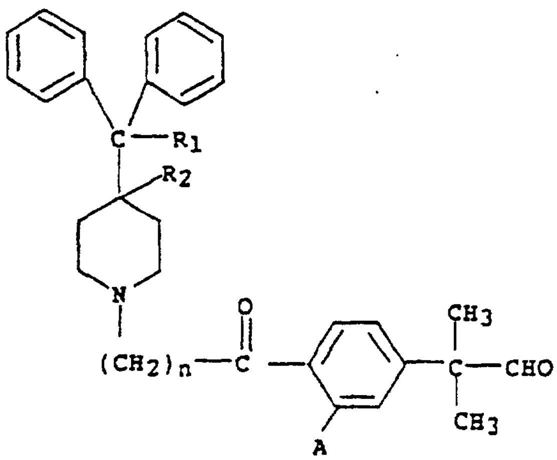

wherein

$R_1$ represents hydrogen or hydroxy;

$R_2$ represents hydrogen; or

$R_1$ and $R_2$ taken together form a second bond between the carbon atoms bearing $R_1$ and $R_2$;

n is an integer of from 1 to 5;

A is hydrogen

comprising the steps of:

(a) reacting a benzeneacetic acid compound of the formula

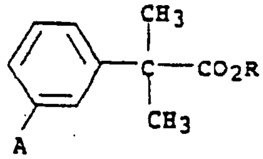

wherein A is as defined above

and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

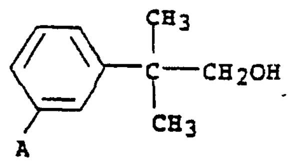

wherein A is defined above;

(b) protecting the hydroxyethyl functionality with a suitable protecting group to give a protected phenethyl alcohol of the formula

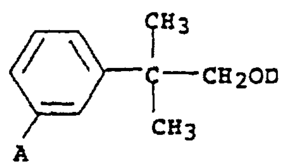

wherein A is hydrogen and D is a suitable protecting group;

(c) reacting the protected phenethyl alcohol with a ω-halo compound of the formula

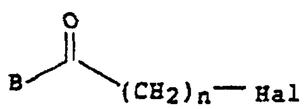

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

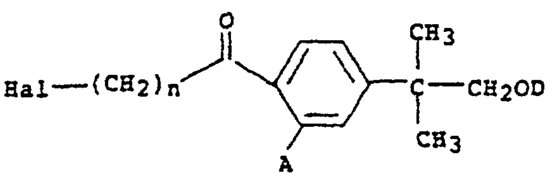

wherein Hal, n, A and D are defined above;

(d) reacting the ω-halo protected hydroxyethylphenylketone with a piperidine compound of the formula

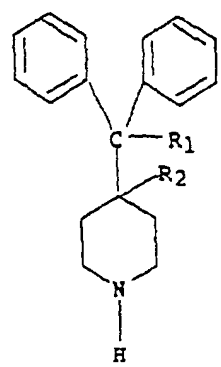

wherein $R_1$ and $R_2$ are as defined above in the presence of a sodium bicarbonate, potassium carbonate, potassium bicarbonate, triethylamine, pyridine, or an excess of the piperidine compound may be used to produce the piperidine protected hydroxyethylphenylketone compound of the formula

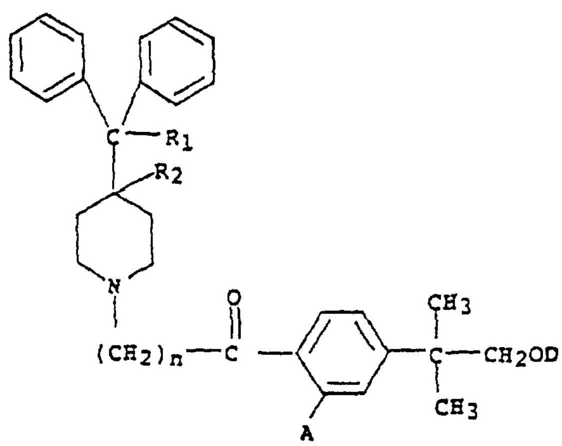

wherein $R_1$, $R_2$, n and A are defined above and D is $-C(=O)CH_3$ or $-C(=O)C_6H_5$;

(e) deprotecting the hydroxyethyl functionality of the piperidine protected hydroxyethylphenylketone with an appropriate deprotecting reagent to produce a piperidine hydroxyethylphenylketone of the formula

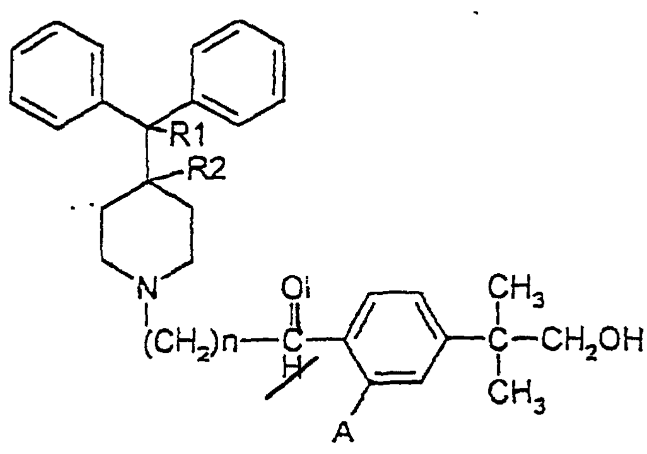

wherein $R_1$, $R_2$, n and A are defined above; and

(f) reacting the piperidine hydroxyethylphenylketone compound with dimethyl sulfoxide, oxyalyl chloride and triethylamine or with Dess-Martin reagent, chromium (IV) oxide, nickel peroxide, sodium dichromate, potassium dichromate, t-butyl chromate, silver oxide, argentic picolinate manganese dioxide lead tetraacetate, dicyclohexylcarbodiimide, 2,3-dichloro-5,6-dicyanoquinone, tetrachloro-1,2-benzoquinone, 2,2,6,6-tetramethylpiperidinyl-1-oxy (TEMPO) or quinolinium chlorochromate to produce the piperidine Eormylphenyl ketone.

**7.** A process for preparing a piperidine protected hydroxyphenylalcohol compound of the formula

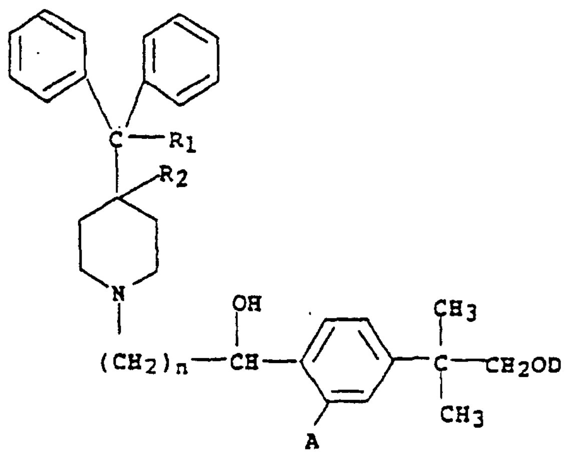

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
A is hydrogen; and
D is $-C(=O)CH_3$ or $-C(=O)C_6H_5$,

comprising the steps of:

(a) reacting a benzeneacetic acid compound of the formula

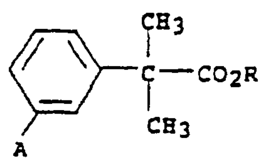

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

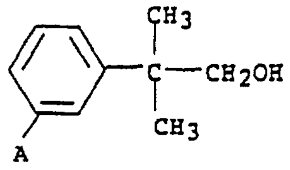

wherein A is hydrogen;

(b) protecting the hydroxyethyl functionality with a suitable protecting group to give a protected phenethyl alcohol of the formula

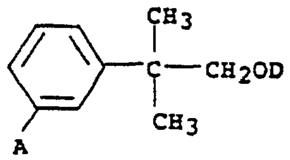

wherein A is hydrogen and D is a suitable protecting group;

(c) reacting the protected phenethyl alcohol with a ω-halo compound of the formula

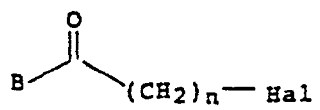

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

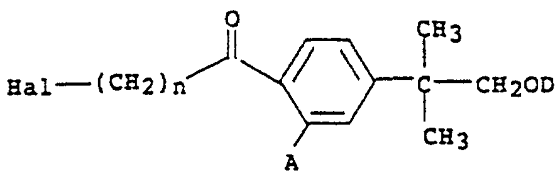

wherein Hal, n and D are defined above and A is hydrogen

(d) reacting the ω-halo protected hydroxyethylphenylketone with a piperidine compound of the formula

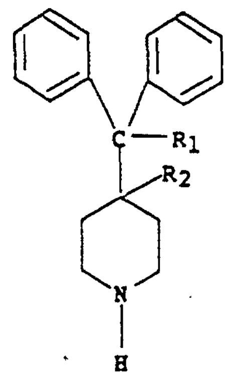

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

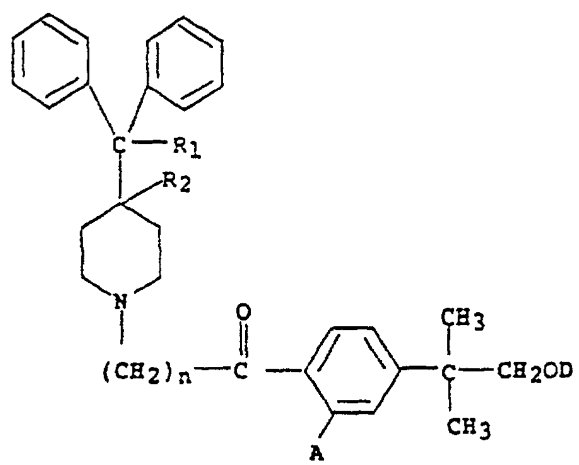

wherein $R_1$, $R_2$, n and D are defined above and A is hydrogen;

(e) reacting the piperidine protected hydroxyethylphenylketone with sodium borohydride, potassium borohydride, sodium cyanoborohydride, tetramethylammonium borohydride, lithium tri-tert-butylaluminohydride, diisobutylaluminum hydride or catalyitc hydrogenation to produce a piperidine protected hydroxyethylphenylalcohol of the formula

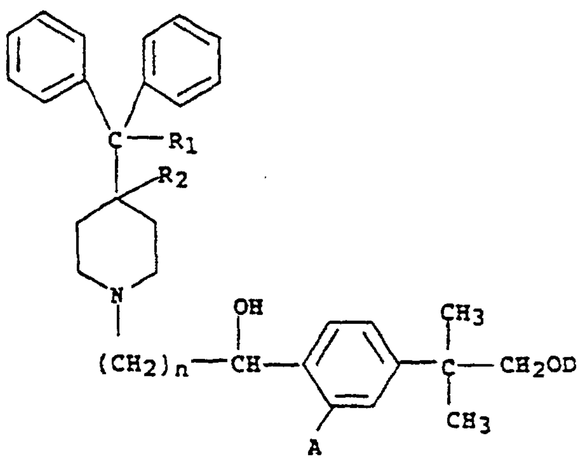

wherein $R_1$, $R_2$, D and n are defined above and A is hydrogen.

**8.** A process for preparing a piperidine protected hydroxyethylphenylketone compound of the formula

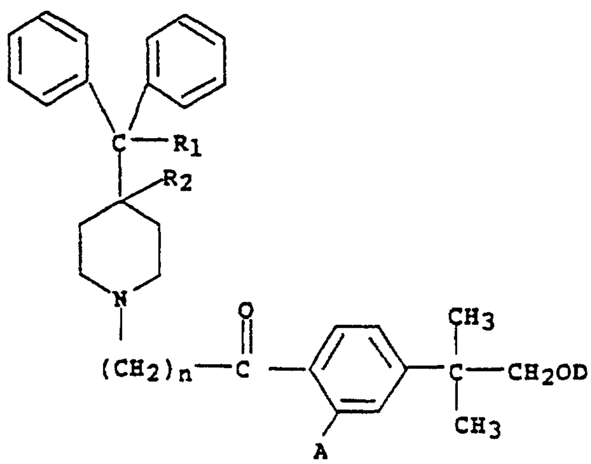

wherein
$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
A is hydrogen; and
D is $-C(=O)CH_3$ or $-C(=O)C_6H_5$,
comprising the steps of:

(a) reacting a benzeneacetic acid compound of the formula

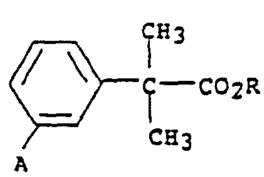

wherein A is as defined above
and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

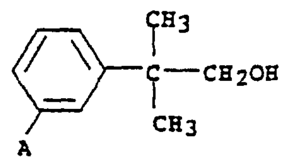

wherein A is defined above;

(b) protecting the hydroxyethyl functionality with a suitable protecting group to give a protected phenethyl alcohol of the formula

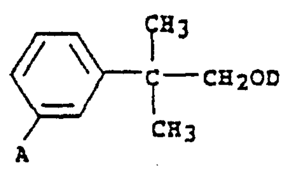

wherein A is hydrogen and D is a suitable protecting group;

(c) reacting the protected phenethyl alcohol with a ω-halo compound of the formula

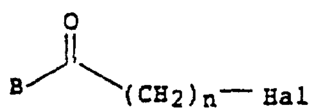

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

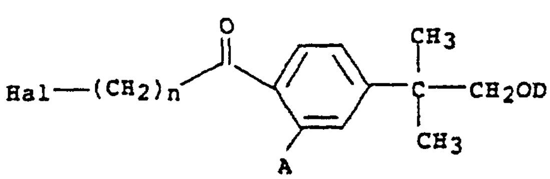

wherein Hal, n, A and D are defined above;

(d) reacting the ω-halo protected hydroxyethylphenylketone with a piperidine compound of the formula

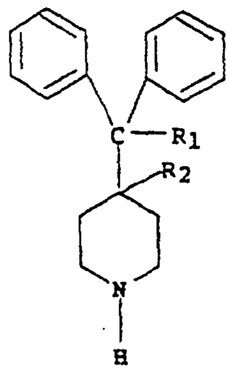

wherein $R_1$ and $R_2$ are as defined above in the presence of a sodium bicarbonate, potassium carbonate, potassium bicarbonate, triethylamine, pyridine, or an excess of the piperidine compound may be used to produce the piperidine protected hydroxyethylphenylketone.

**9.** A process for preparing a compound of the formula

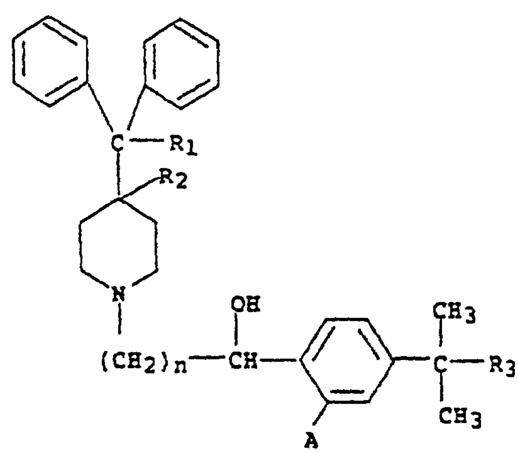

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is -COOH or -COOalkyl wherein the alkyl moiety has from 1 to 6 carbon atoms and is straight or branched;
A is hydrogen; and
pharmaceutically acceptable salts thereof comprising the steps of:

(a) reacting a benzeneacetic acid compound of the formula

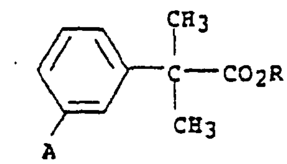

wherein A is hydrogen and R is hydrogen or $C_1$-$C_6$ alkyl with sodium bis(2-methoxyethoxy)aluminium hydride,

lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

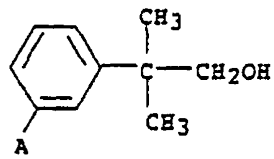

wherein A is hydrogen;

(b) protecting the hydroxyethyl functionality with a suitable protecting group to give a protected phenethyl alcohol of the formula

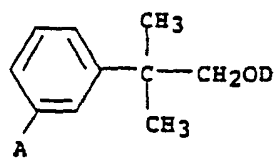

wherein A is hydrogen and D is a suitable protecting group;

(c) reacting the protected phenethyl alcohol with a ω-halo compound of the formula

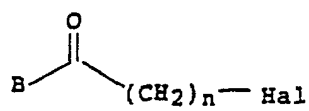

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula.

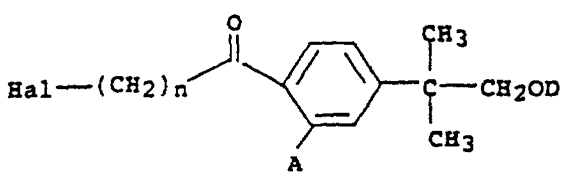

wherein Hal, n and D are defined above and A is hydrogen;

(d) reacting the ω-halo protected hydroxyethylphenylketone with a piperidine compound of the formula

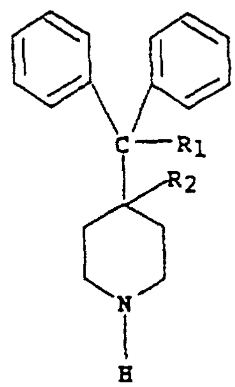

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

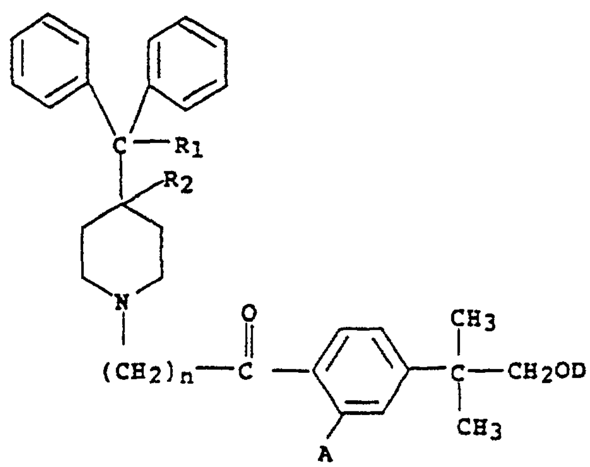

wherein $R_1$, $R_2$, n and D are defined above and A is hydrogen;

(e) deprotecting the hydroxyethyl functionality of the piperidine protected hydroxyethylphenylketone with an appropriate deprotecting reagent to produce a piperidine hydroxyethylphenylketone of the formula

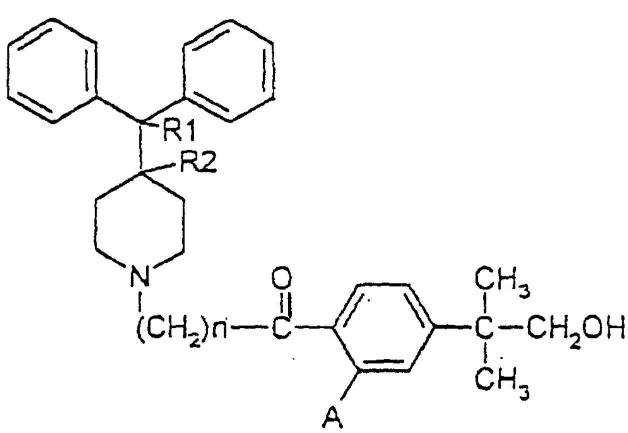

wherein $R_1$, $R_2$, n and A are defined above; and

(f) reacting the piperidine hydroxyethylphenylketone with dimethyl sulfoxide, oxyalyl chloride and triethylamine or with Dess-Martin reagent, chromium (IV) oxide, nickel peroxide, sodium dichromate, potassium dichromate, t-butyl chromate, silver oxide, argentic picolinate manganese dioxide lead tetraacetate, dicyclohexylcarbodiimide, 2,3-dichloro-5,6-dicyanoquinone, tetrachloro-1,2-benzoquinone, 2,2,6,6-tetramethylpiperidinyl-1-oxy (TEMPO) or quinolinium chlorochromate to produce a piperidine formylphenylketone of the formula

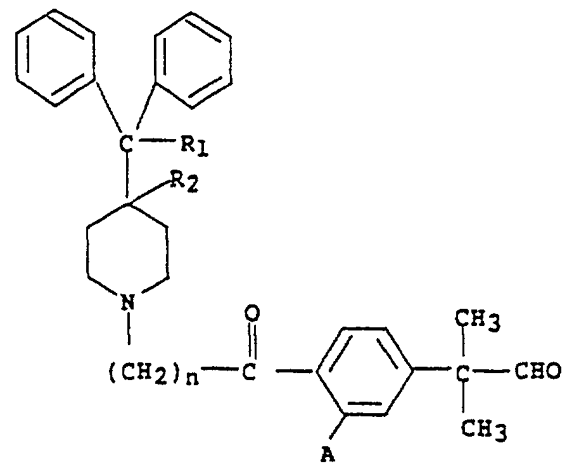

wherein $R_1$, $R_2$ and n are defined above and A is hydrogen;

(g) reacting the piperidine formylphenylketone with potassium permanganate, chromium (IV) oxide, silver (I) oxide, silver oxide, argentic picolinate, peroxide, nitric acid, m-chloroperbenzoic acid and peracetic acid to produce a piperidine carboxyphenylketone of the formula

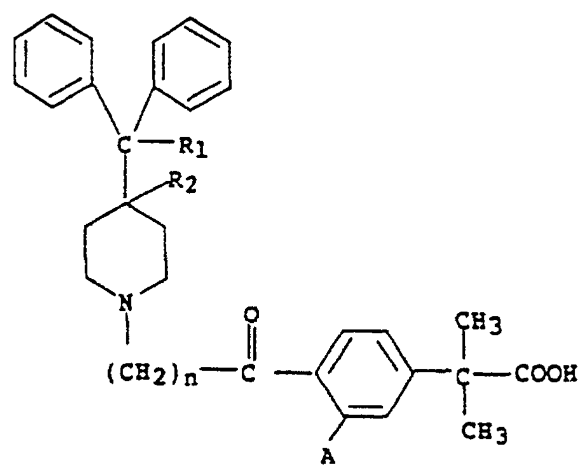

wherein $R_1$, $R_2$ and n are defined above and A is hydrogen;

(h) reacting the piperidine carboxyphenylketone with sodium borohydride, potassium borohydride, sodium cyanoborohydride, tetramethylammonium borohydride, lithium tri-tert-butylaluminohydride, diisobutylaluminum hydride or catalytic hydrogenation to produce a piperidine carboxyphenylalcohol of the formula

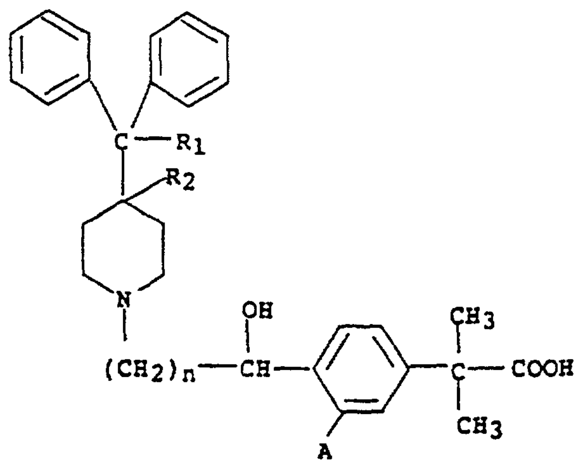

wherein $R_1$, $R_2$ and n are defined above and A is hydrogen;

(i) optionally reacting the piperidine carboxyphenylalcohol with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylalcohol ester of the formula

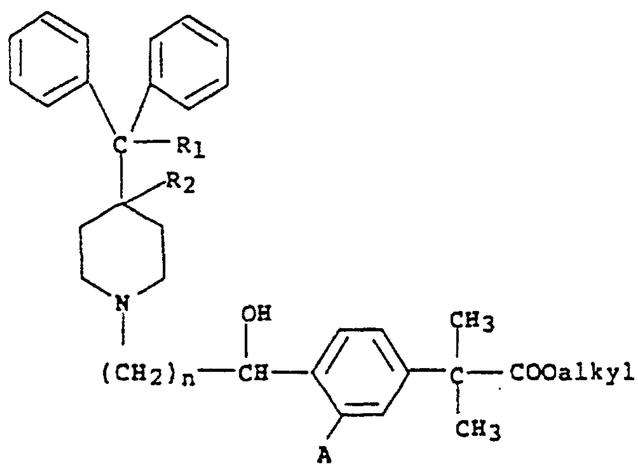

wherein $R_1$, $R_2$, n and alkyl are defined above and A is hydrogen.

**10.** A process for preparing a compound of the formula

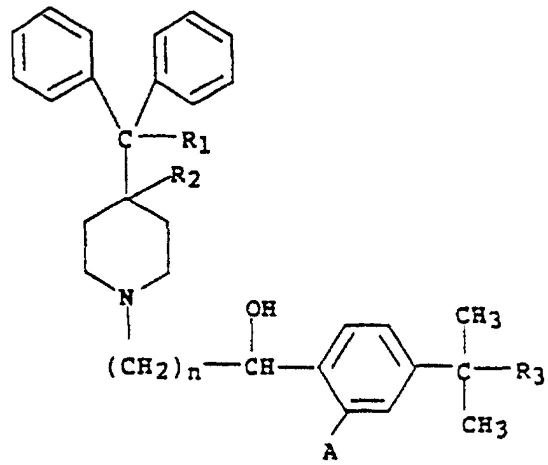

wherein

$R_1$ represents hydrogen or hydroxy;

$R_2$ represents hydrogen; or

$R_1$ and $R_2$ taken together form a second bond between the carbon atoms bearing $R_1$ and $R_2$;

n is an integer of from 1 to 5;

$R_3$ is -CODE or -COOalkyl wherein the alkyl moiety has from 1 to 6 carbon atoms and is straight or branched;

A is hydrogen and

pharmaceutically acceptable salts thereof comprising the steps of:

(a) reacting a benzeneacetic acid compound of the formula

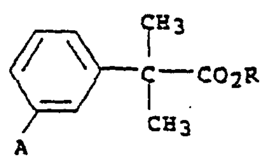

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

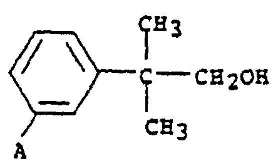

wherein A is hydrogen

(b) protecting the hydroxyethyl functionality with a suitable protecting group to give a protected phenethyl alcohol of the formula

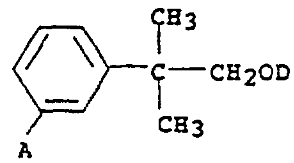

wherein A is hydrogen and D is a suitable protecting group;

(c) reacting the protected phenethyl alcohol with a ω-halo compound of the formula

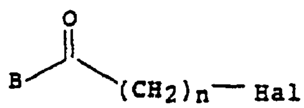

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

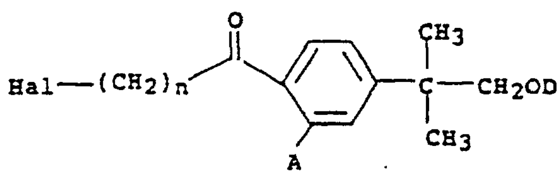

wherein Hal, n and D are defined above and A is hydrogen;

(d) reacting the ω-halo protected hydroxyethylphenylketone with a piperidine compound of the formula

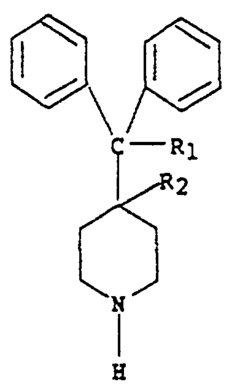

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

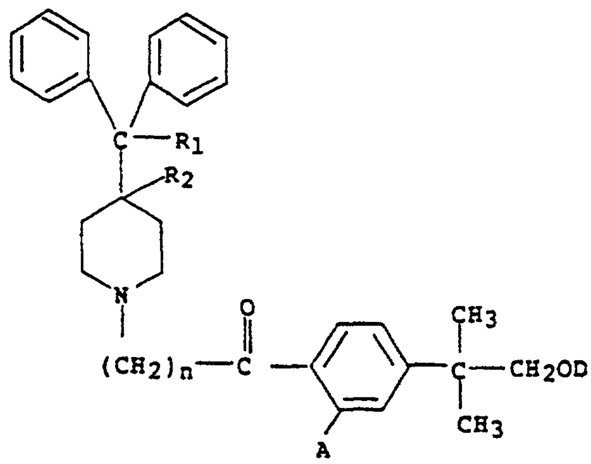

wherein $R_1$, $R_2$, n and D are defined above and A is hydrogen

(e) deprotecting the hydroxyethyl functionality of the piperidine protected hydroxyethylphenylketone with an appropriate deprotecting reagent to produce a piperidine hydroxyethylphenylketone of the formula

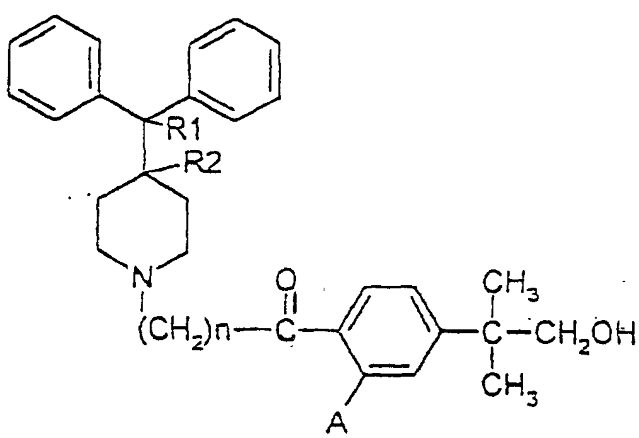

wherein $R_1$, $R_2$, n and A are defined above; and

(f) reacting the piperidine hydroxyethylphenylketone with chromium (IV) oxide, potassium permanganate, nitric acid, nitrogen dioxide, ruthenium (VIII) oxide, nickel peroxide, silver oxide, t-butyl chromate or xenic acid to produce a piperidine carboxyphenylketone of the formula

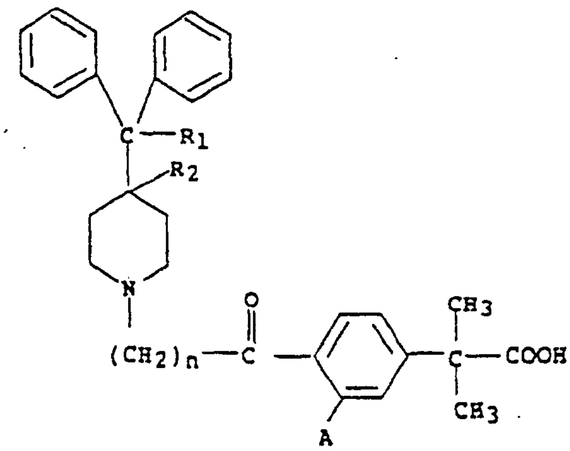

wherein $R_1$, $R_2$ and n are defined above and A is hydrogen;

(g) reacting the piperidine carboxyphenylketone with sodium borohydride, potassium borohydride, sodium cyanoborohydride, tetramethylammonium borohydride, lithium tri-tert-butylaluminohydride, diisobutylaluminum hydride or catalytic hydrogenation to produce a piperidine carboxyphenylalcohol of the formula

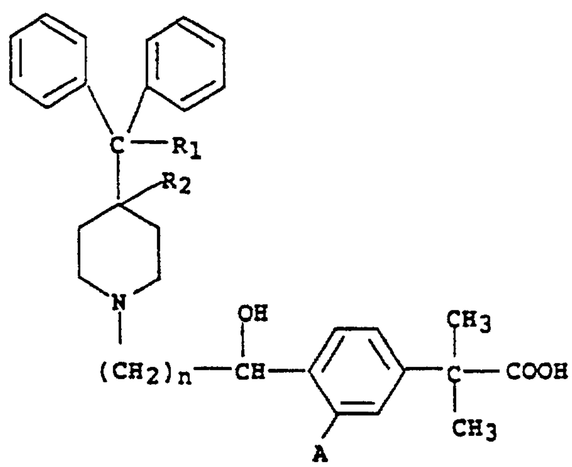

wherein $R_1$, $R_2$ and n are defined above and A is hydrogen

(h) optionally reacting the piperidine carboxyphenylalcohol with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylalcohol ester of the formula

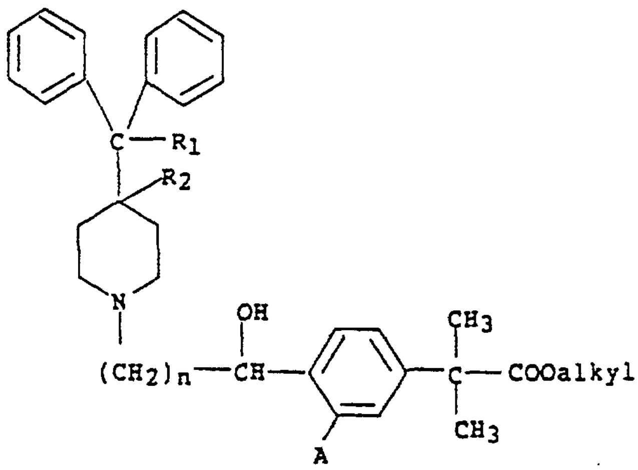

wherein $R_1$, $R_2$, n and alkyl are defined above and A is hydrogen; and

**11.** A process according to Claim 10 wherein the piperidine hydroxyethylphenylketone produced in step e is reacted with ruthenium(VIII) oxide to produce the piperidine carboxyphenylketone of step f.

**12.** A process for preparing a compound of the formula

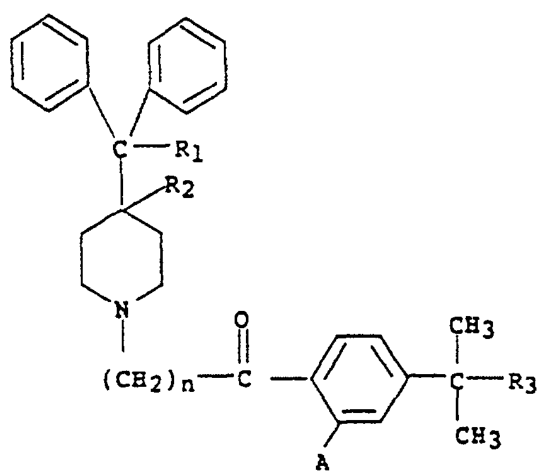

wherein

$R_1$ represents hydrogen or hydroxy;

$R_2$ represents hydrogen; or

$R_1$ and $R_2$ taken together form a second bond between the carbon atoms bearing $R_1$ and $R_2$;

n is an integer of from 1 to 5;

$R_3$ is -COOH or -COOalkyl wherein the alkyl moiety has from 1 to 6 carbon atoms and is straight or branched;

A is hydrogen; and

pharmaceutically acceptable salts thereof comprising the steps of:

(a) reacting a benzeneacetic acid compound of the formula

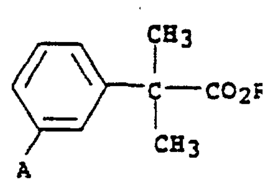

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

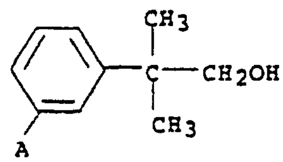

wherein A is hydrogen

(b) protecting the hydroxyethyl functionality with a suitable protecting group to give a protected phenethyl alcohol of the formula

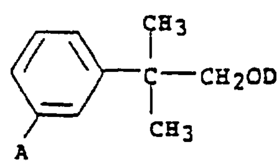

wherein A is hydrogen and D is a suitable protecting group;

(c) reacting the protected phenethyl alcohol with a ω-halo compound of the formula

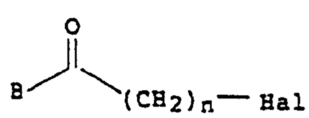

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

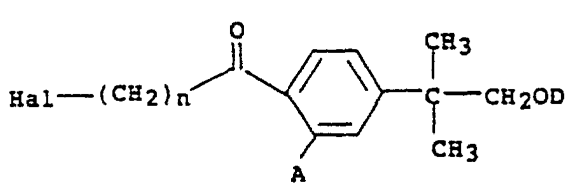

wherein Hal, n and D are defined above and A is hydrogen

(d) reacting the ω-halo protected hydroxyethylphenylketone with a piperidine compound of the formula

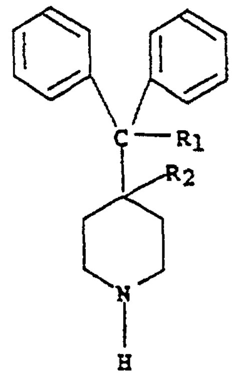

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

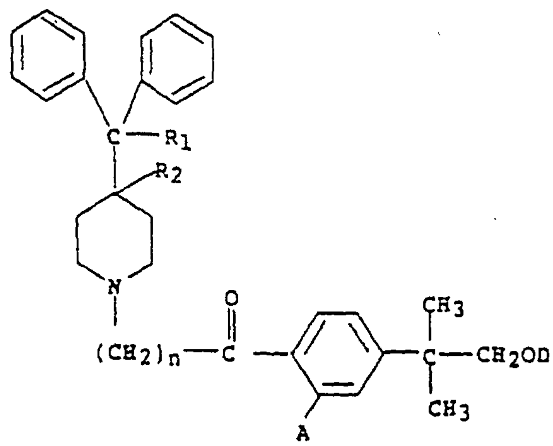

wherein $R_1$, $R_2$, n and D are defined above and A is hydrogen;

(e) deprotecting the hydroxyethyl functionality of the piperidine protected hydroxyethylphenylketone with an appropriate deprotecting reagent to produce a piperidine hydroxyethylphenylketone of the formula

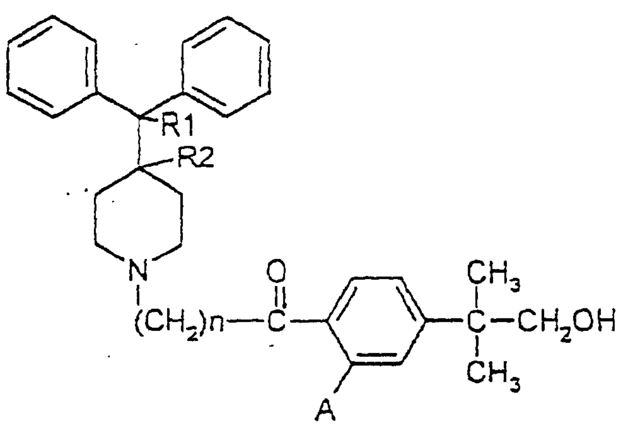

wherein $R_1$, $R_2$, n and A are defined above; and
wherein $R_1$, $R_2$ and n are defined above and A is hydrogen;

(f) reacting the piperidine hydroxyethylphenylketone with dimethyl sulfoxide, oxyalyl chloride and triethylamine or with Dess-Martin reagent, chromium (IV) oxide, nickel peroxide, sodium dichromate, potassium dichromate, t-butyl chromate, silver oxide, argentic picolinate manganese dioxide lead tetraacetate, dicyclohexylcarbodiimide, 2,3-dichloro-5,6-dicyanoquinone, tetrachloro-1,2-benzoquinone, 2,2,6,6-tetramethylpiperidinyl-1-oxy (TEMPO) or quinolinium chlorochromate to produce a piperidine formylphenylketone of the formula

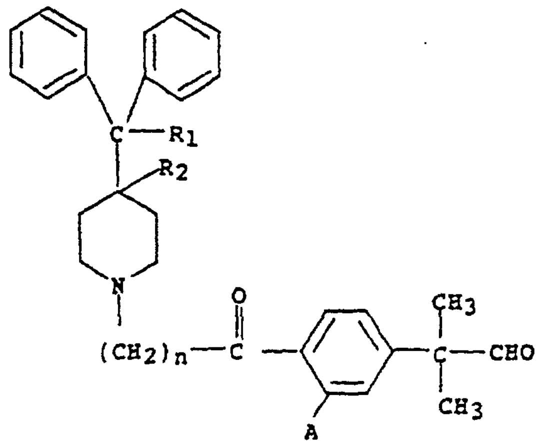

wherein $R_1$, $R_2$ and n are defined above and A is hydrogen;

(g) reacting the piperidine formylphenylketone with potassium permanganate, chromium (IV) oxide, silver (I) oxide, silver oxide, argentic picolinate, peroxide, nitric acid, m-chloroperbenzoic acid and peracetic acid to produce a piperidine carboxyphenylketone of the formula

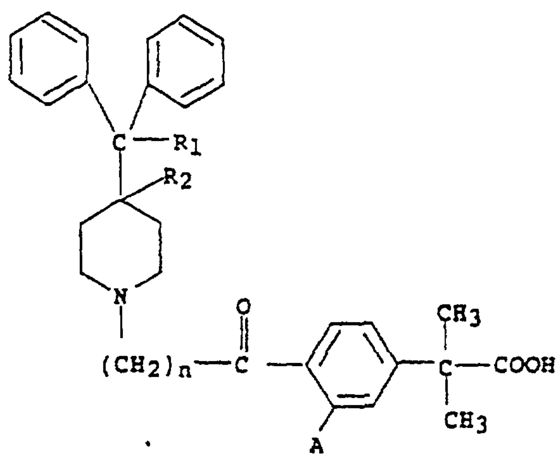

wherein $R_1$, $R_2$ and n are defined above and A is hydrogen;

(h) optionally reacting the piperidine carboxyphenylketone with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylketone ester of the formula

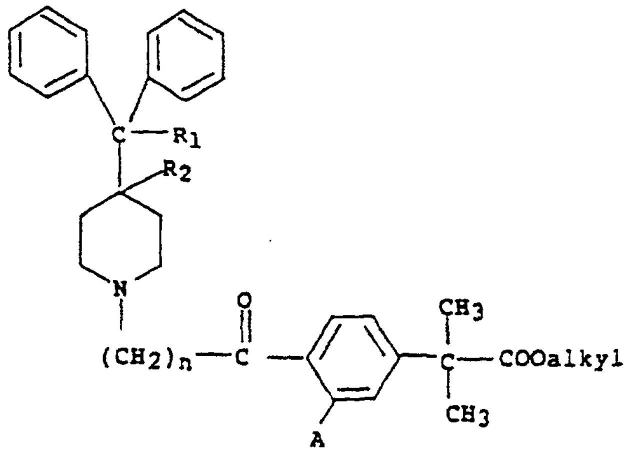

wherein $R_1$, $R_2$, n and alkyl are defined above and A is hydrogen.

**13.** A process for preparing a compound of the formula

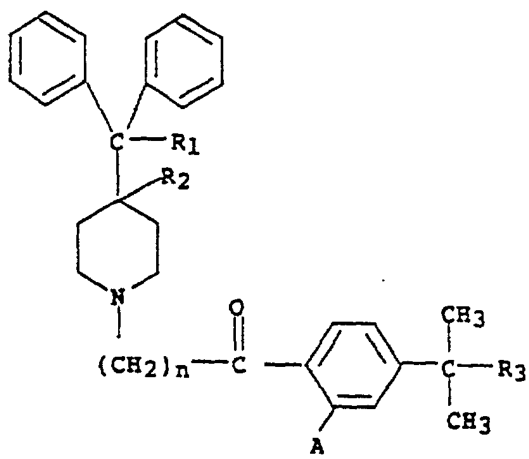

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is -COOH or -COOalkyl wherein the alkyl moiety has from 1 to 6 carbon atoms and is straight or branched;
A is hydrogen; and
pharmaceutically acceptable salts thereof comprising the steps of:

(a) reacting a benzeneacetic acid compound of the formula

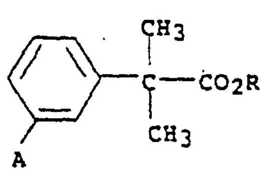

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

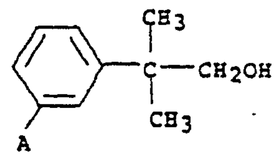

wherein A is hydrogen;

(b) protecting the hydroxyethyl functionality with a suitable protecting group to give a protected phenethyl alcohol of the formula

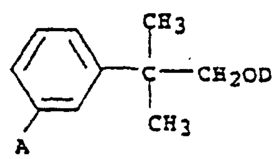

wherein A is hydrogen and D is a suitable protecting group;

(c) reacting the protected phenethyl alcohol with a ω-halo compound of the formula

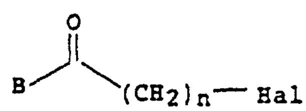

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

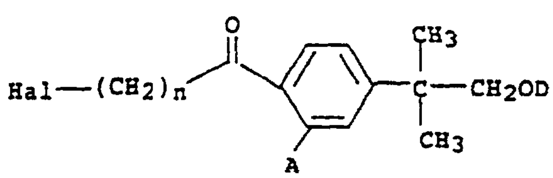

wherein Hal, n and D are defined above and A is hydrogen

(d) reacting the ω-halo protected hydroxyethylphenylketone with a piperidine compound of the formula

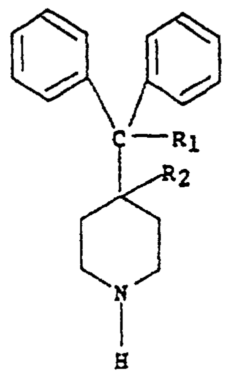

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

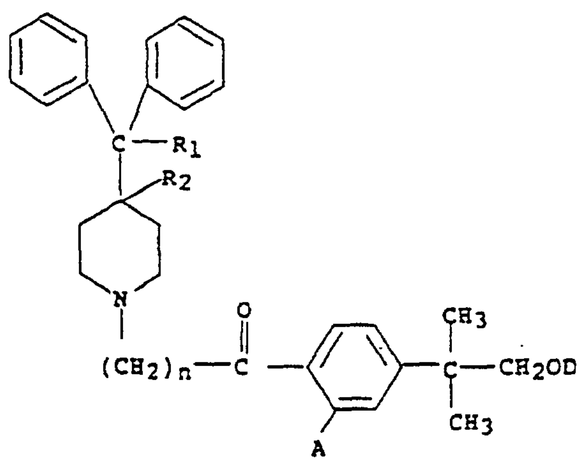

wherein $R_1$, $R_2$, n and D are defined above and A is hydrogen;

(e) deprotecting the hydroxyethyl functionality of the piperidine protected hydroxyethylphenylketone with an appropriate deprotecting reagent to produce a piperidine hydroxyethylphenylketone of the formula

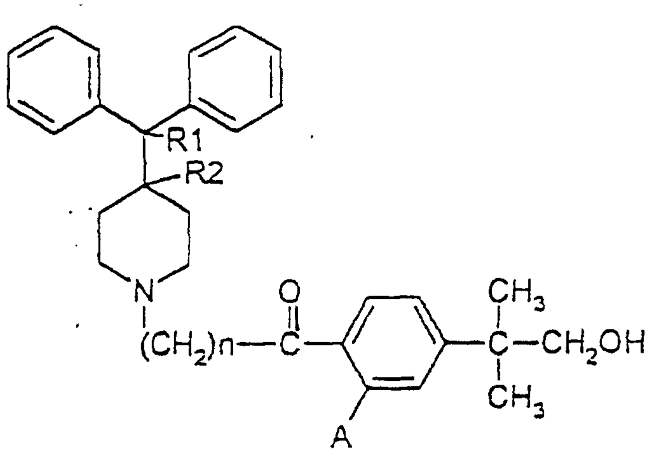

wherein $R_1$, $R_2$, n and A are defined above; and

(f) reacting the piperidine hydroxyethylphenylketone with chromium (IV) oxide, potassium permanganate, nitric acid, nitrogen dioxide, ruthenium (VIII) oxide, nickel peroxide, silver oxide, t-butyl chromate or xenic acid to produce the piperidine carboxyphenylketone of formula

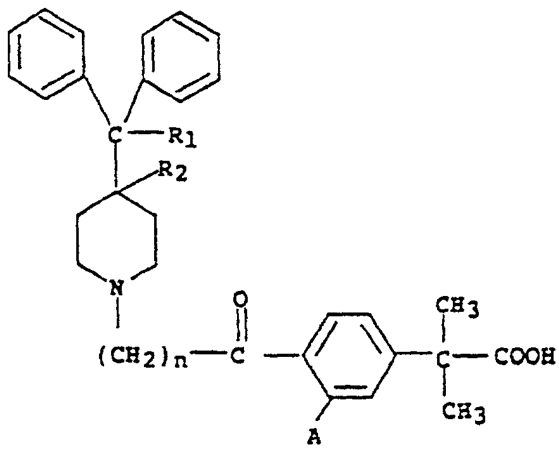

wherein $R_1$, $R_2$ and n are defined above and A is hydrogen;

(g) optionally reacting the piperidine carboxyphenylketone with diazomethane or 2,2-dimethoxypropane or with thionyl chloride followed by an alcohol of the formula HOalkyl wherein the alkyl moiety has from 1 to 6 carbon atoms and is straight or branched to form a piperidine carboxyphenylketone ester of the formula

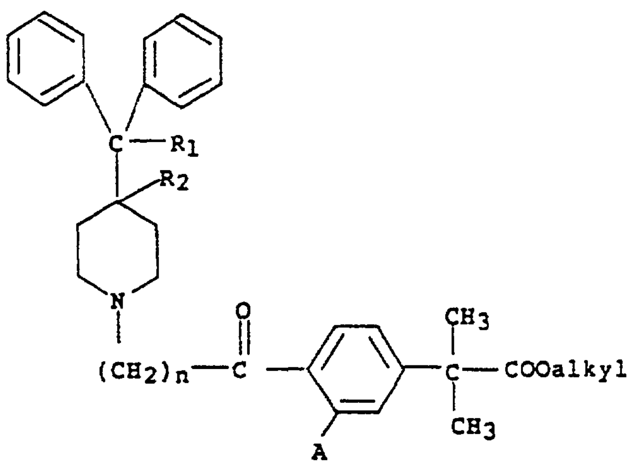

wherein $R_1$, $R_2$, n and alkyl are defined above and A is hydrogen.

**14.** A process according to Claim 13 wherein the piperidine hydroxyethylphenylketone produced in step e is reacted with ruthenium(VIII) oxide to produce the piperidine carboxyphenylketone of step f.

**15.** A process for preparing a piperidine hydroxyethylphenylketone compound of the formula

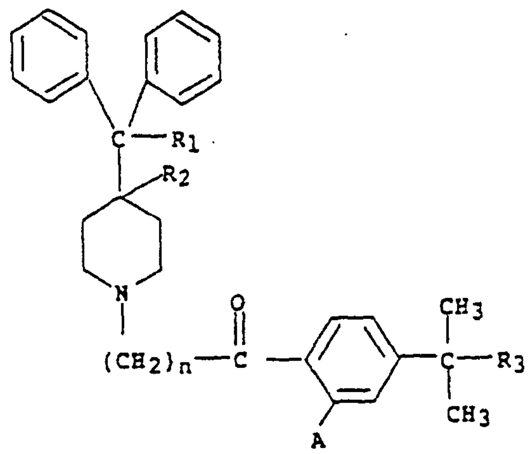

wherein
$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is $-CH_2OH$;
A is hydrogen; and
pharmaceutically acceptable salts thereof comprising the steps of:

(a) reacting a benzeneacetic acid compound of the formula

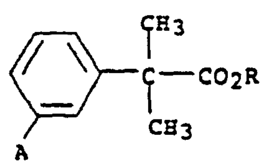

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

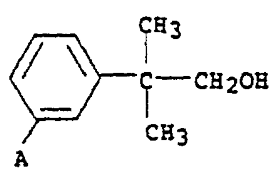

wherein A is hydrogen;

(b) protecting the hydroxyethyl functionality with a suitable protecting group to give a protected phenethyl alcohol of the formula

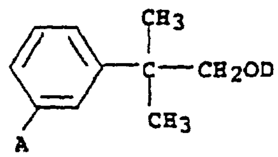

wherein A is hydrogen and D is a suitable protecting group;

(c) reacting the protected phenethyl alcohol with a ω-halo compound of the formula

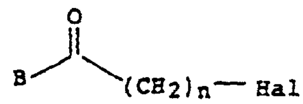

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

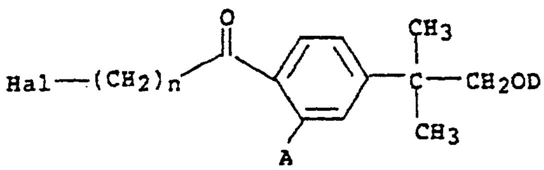

wherein Hal, n and D are defined above and A is hydrogen;

(d) reacting the ω-halo protected hydroxyethylphenylketone with a piperidine compound of the formula

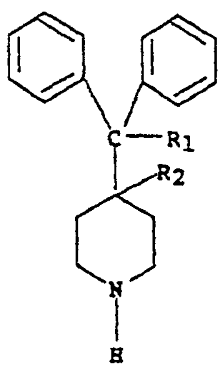

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

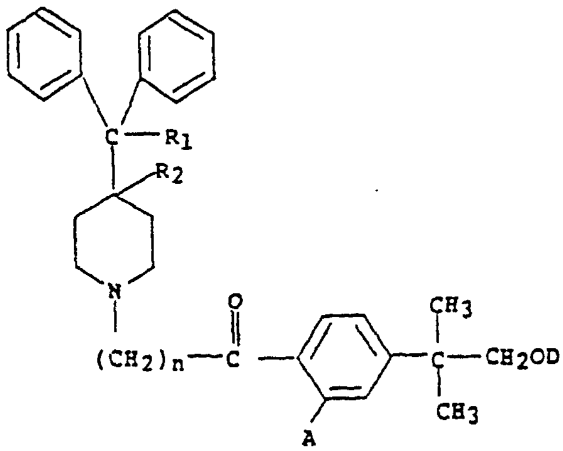

wherein $R_1$, $R_2$, n and D are defined above and A is hydrogen;

(e) deprotecting the hydroxyethyl functionality of the piperidine protected hydroxyethylphenylketone with an appropriate deprotecting reagent to produce a piperidine hydroxyethylphenylketone of the formula

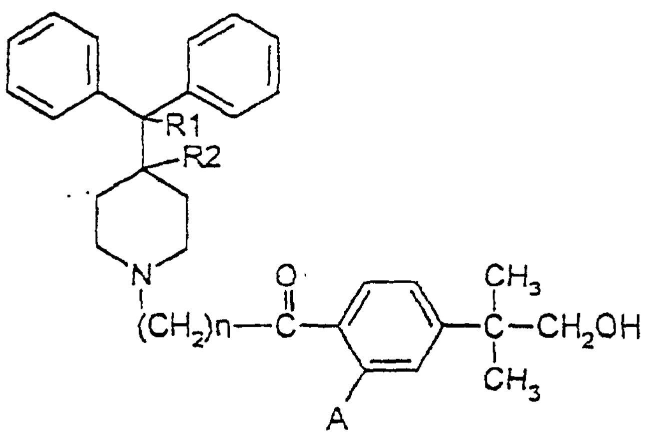

wherein $R_1$, $R_2$, n and A are defined above.

**16.** A process for preparing a compound of the formula

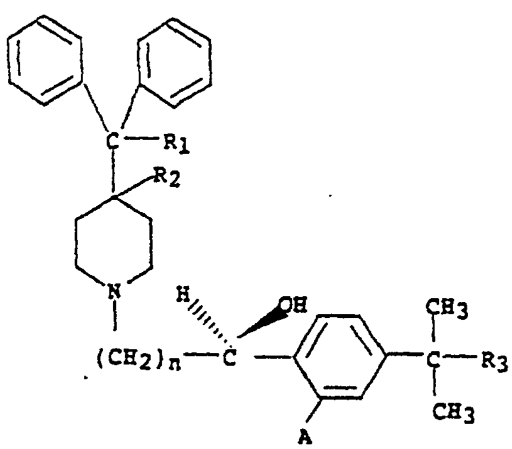

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is -COOH or -COOalkyl wherein the alkyl moiety has from 1 to 6 carbon atoms and is straight or branched;
A is hydrogen; and
pharmaceutically acceptable salts thereof comprising the steps of:

(a) reacting a benzeneacetic acid compound of the formula

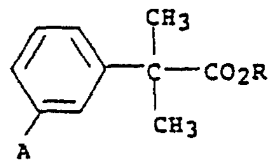

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

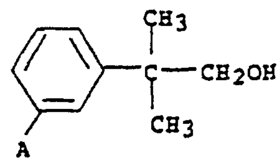

wherein A is hydrogen;

(b) protecting the hydroxyethyl functionality with a suitable protecting group to give a protected phenethyl alcohol of the formula

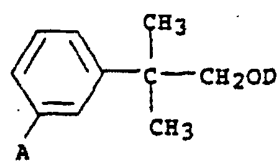

wherein A is hydrogen and D is a suitable protecting group;

(c) reacting the protected phenethyl alcohol with a ω-halo compound of the formula

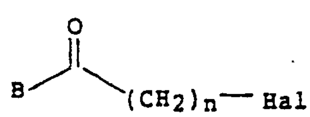

wherein B is halo or hydroxy, Hal represents Cl, Br or I and n is as defined above, in the presence of trichloride, aluminum chloride, titanium tetrachloride, boron trifluoride, tin tetrachloride and zinc chloride to produce a ω-halo protected hydroxyethylphenylketone of the formula

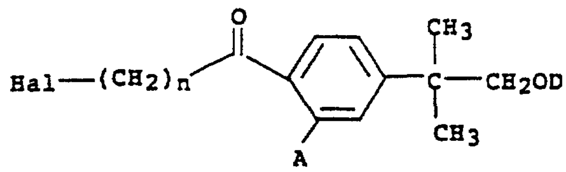

wherein Hal, n and D are defined above and A is hydrogen;

(d) reacting the ω-halo protected hydroxyethylphenylketone with a piperidine compound of the formula

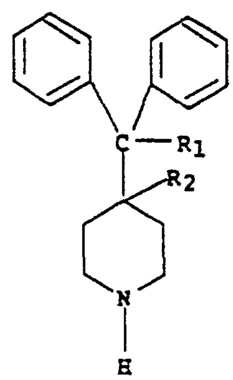

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

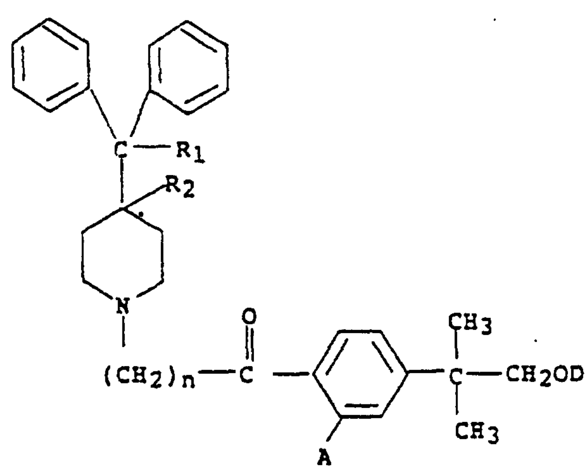

wherein $R_1$, $R_2$, n and D are defined above and A is hydrogen;

(e) deprotecting the hydroxyethyl functionality of the piperidine protected hydroxyethylphenylketone with an appropriate deprotecting reagent to produce a piperidine hydroxyethylphenylphenylketone of the formula

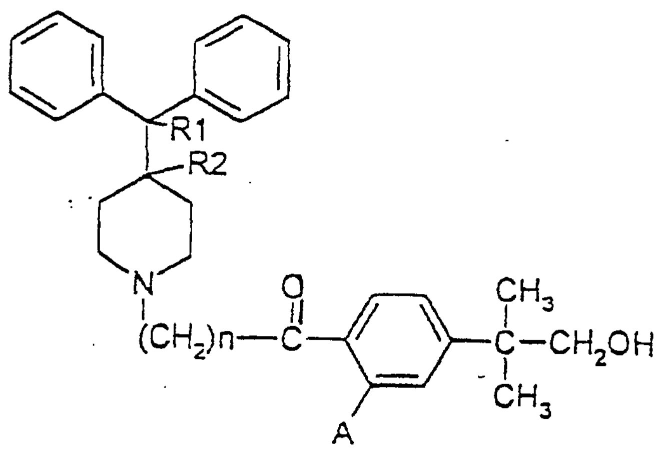

wherein $R_1$, $R_2$, n and A are defined above; and

(f) reacting the piperidine hydroxyethylphenylketone with dimethyl sulfoxide, oxyalyl chloride and triethylamine or with Dess-Martin reagent, chromium (IV) oxide, nickel peroxide, sodium dichromate, potassium dichromate, t-butyl chromate, silver oxide, argentic picolinate manganese dioxide lead tetraacetate, dicyclohexylcarbodiimide, 2,3-dichloro-5,6-dicyanoquinone, tetrachloro-1,2-benzoquinone, 2,2,6,6-tetramethylpiperidinyl-1-oxy (TEMPO) or quinolinium chlorochromate to produce a piperidine formylphenylketone of the formula

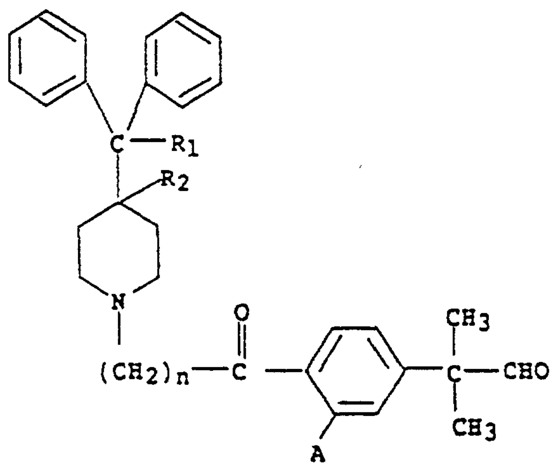

wherein $R_1$, $R_2$ and n are defined above and A is hydrogen;

(g) reacting the piperidine formylphenylketone with potassium permanganate, chromium (IV) oxide, silver (I) oxide, silver oxide, argentic picolinate, peroxide, nitric acid, m-chloroperbenzoic acid and peracetic acid to produce a piperidine carboxyphenylketone of the formula

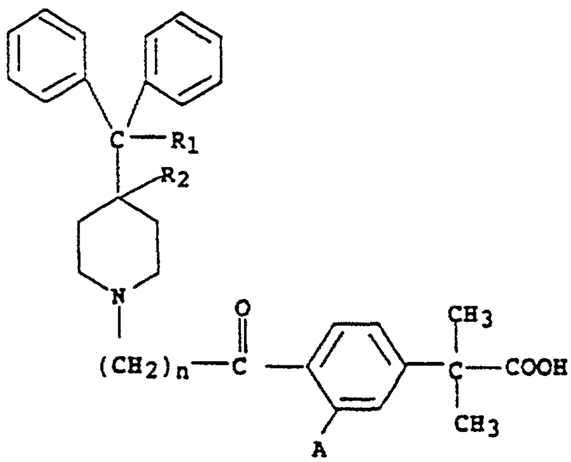

wherein $R_1$, $R_2$ and n are defined above and A is hydrogen

(h) reacting the piperidine carboxyphenylketone with (+)-B-chlorodiisopinocamphenylborane to produce a piperidine carboxyphenylalcohol of the formula

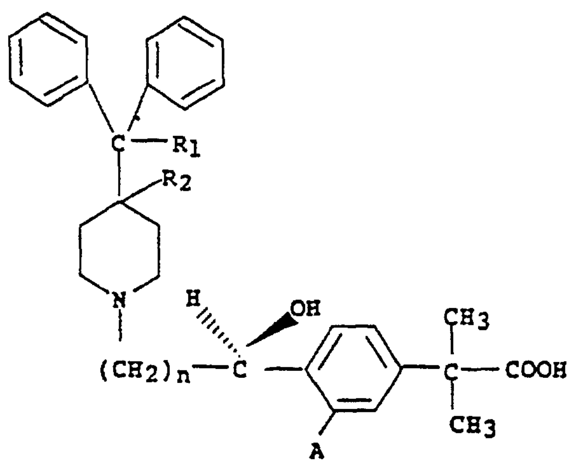

wherein $R_1$, $R_2$ and n are defined above and A is hydrogen

(i) optionally reacting the piperidine carboxyphenylalcohol with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylalcohol ester of the formula

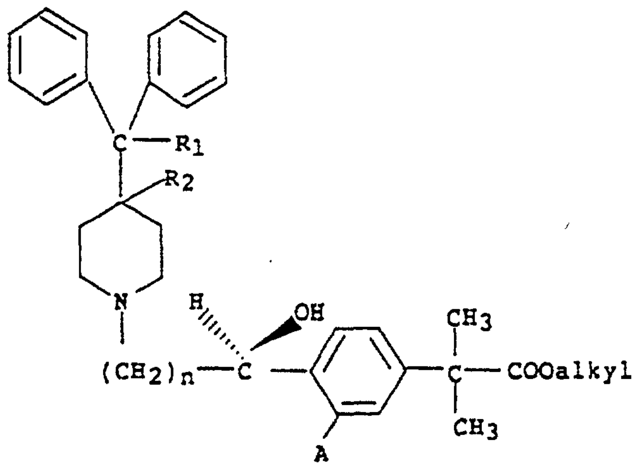

wherein $R_1$, $R_2$, n and alkyl are defined above and A is hydrogen.

**17.** A process for preparing a compound of the formula

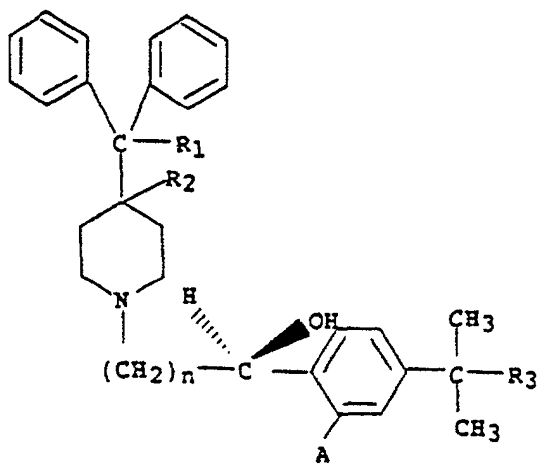

wherein

$R_1$ represents hydrogen or hydroxy;

$R_2$ represents hydrogen; or

$R_1$ and $R_2$ taken together form a second bond between the carbon atoms bearing $R_1$ and $R_2$;

n is an integer of from 1 to 5;

$R_3$ is -COOH or -COOalkyl wherein the alkyl moiety has from 1 to 6 carbon atoms and is straight or branched;

A is hydrogen; and

pharmaceutically acceptable salts thereof comprising the steps of:

(a) reacting a benzeneacetic acid compound of the formula

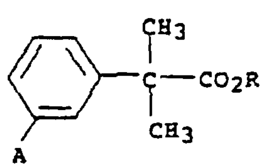

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

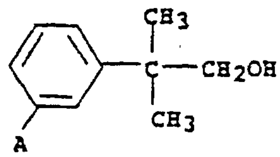

wherein A is hydrogen;

(b) protecting the hydroxyethyl functionality with a suitable protecting group to give a protected phenethyl alcohol of the formula

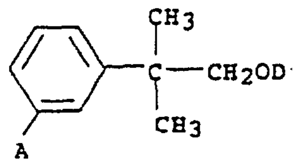

wherein A is hydrogen and D is a suitable protecting group;

(c) reacting the protected phenethyl alcohol with a ω-halo compound of the formula

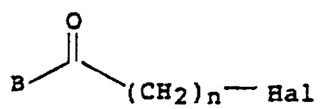

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

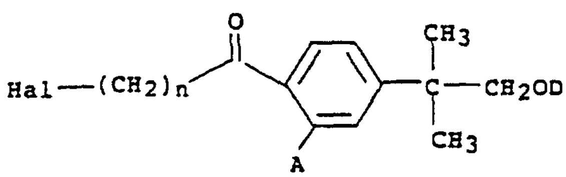

wherein Hal, n and D are defined above and A is hydrogen

(d) reacting the ω-halo protected hydroxyethylphenylketone with a piperidine compound of the formula

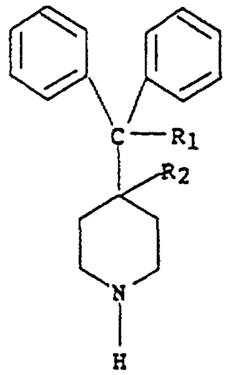

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

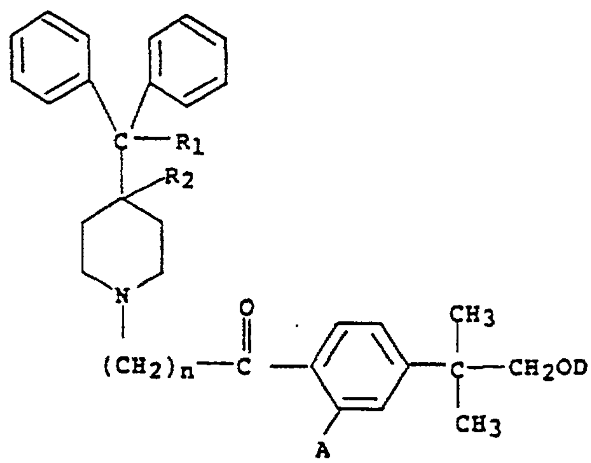

wherein $R_1$, $R_2$, n and D are defined above and A is hydrogen

(e) deprotecting the hydroxyethyl functionality of the piperidine protected hydroxyethylphenylketone with an appropriate deprotecting reagent to produce a piperidine hydroxyethylphenylketone of the formula

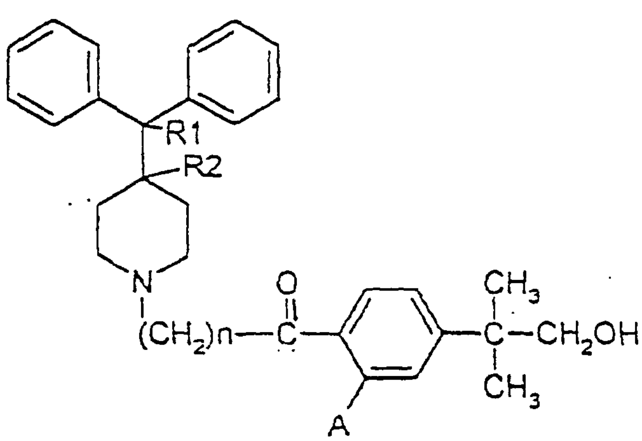

wherein $R_1$, $R_2$, n and A are defined above; and

(f) reacting the piperidine hydroxyethylphenylketone with chromium (IV) oxide, potassium permanganate, nitric acid, nitrogen dioxide, ruthenium (VIII) oxide, nickel peroxide, silver oxide, t-butyl chromate or xenic acid to produce a piperidine carboxyphenylketone of the formula

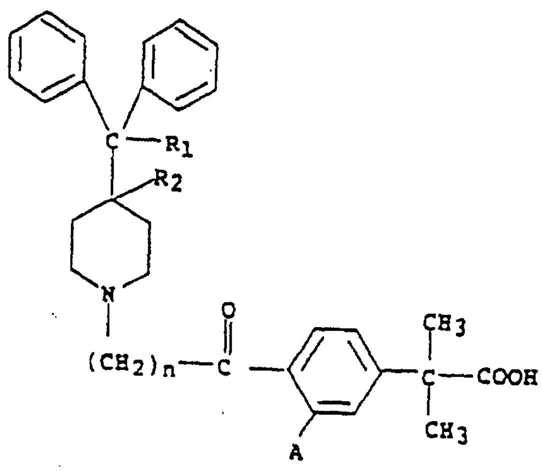

wherein $R_1$, $R_2$ and n are defined above and A is hydrogen

(g) reacting the piperidine carboxyphenylketone with (+)-B--chlorodiisopinocamphenylborane to produce a piperidine carboxyphenylalcohol of the formula

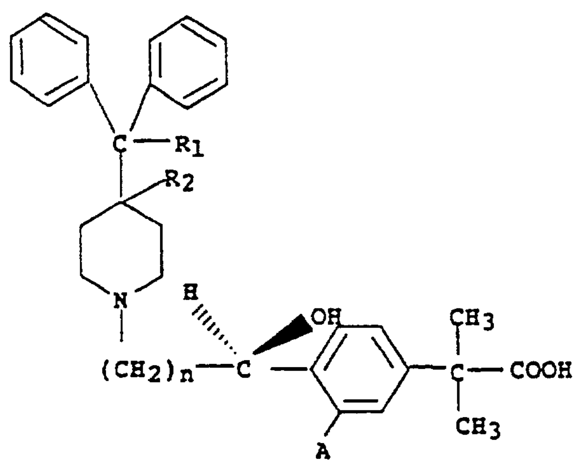

wherein $R_1$, $R_2$ and n are defined above and A is hydrogen;

(h) optionally reacting the piperidine carboxyphenylalcohol with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylalcohol ester of the formula

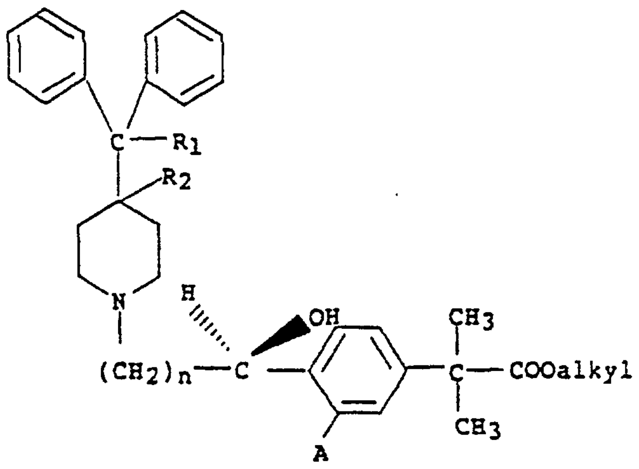

wherein $R_1$, $R_2$, n and alkyl are defined above and A is hydrogen.

**18.** A process for preparing a compound of the formula

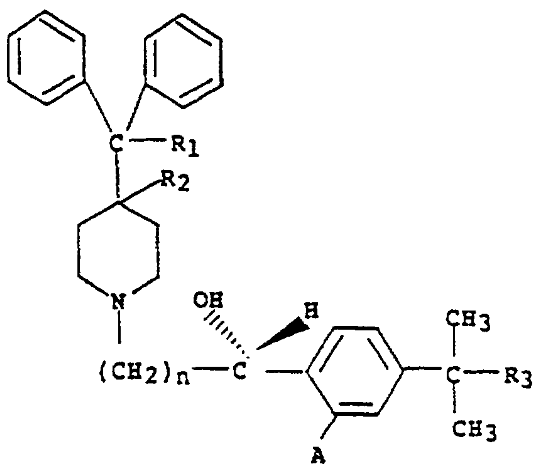

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is -COOH or -COOalkyl wherein the alkyl moiety has from 1 to 6 carbon atoms and is straight or branched;
A is hydrogen; and
pharmaceutically acceptable salts thereof comprising the steps of:

(a) reacting a benzeneacetic acid compound of the formula

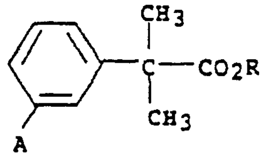

wherein A is hydrogen and R is hydrogen or $C_1$-$C_6$ alkyl with sodium bis(2-methoxyethoxy)aluminium hydride,

lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

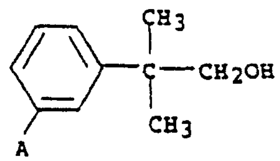

wherein A is hydrogen

(b) protecting the hydroxyethyl functionality with a suitable protecting group to give a protected phenethyl alcohol of the formula

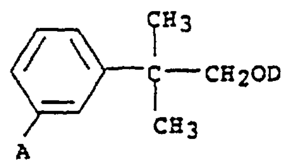

wherein A is hydrogen and D is a suitable protecting group;

(c) reacting the protected phenethyl alcohol with a ω-halo compound of the formula

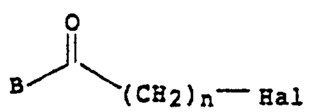

wherein B is halo or hydroxy, Hal represents Cl, Br or I and n is as defined above, in the presence of trichloride, aluminum chloride, titanium tetrachloride, boron trifluoride, tin tetrachloride and zinc chloride to produce a ω-halo protected hydroxyethylphenylketone of the formula

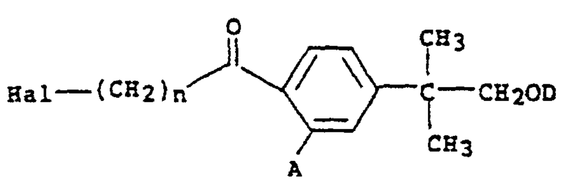

wherein Hal, n and D are defined above and A is hydrogen;

(d) reacting the ω-halo protected hydroxyethylphenylketone with a piperidine compound of the formula

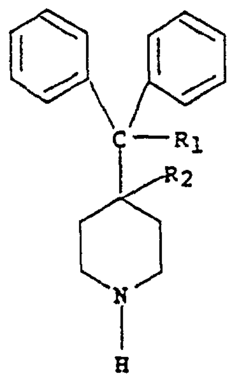

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

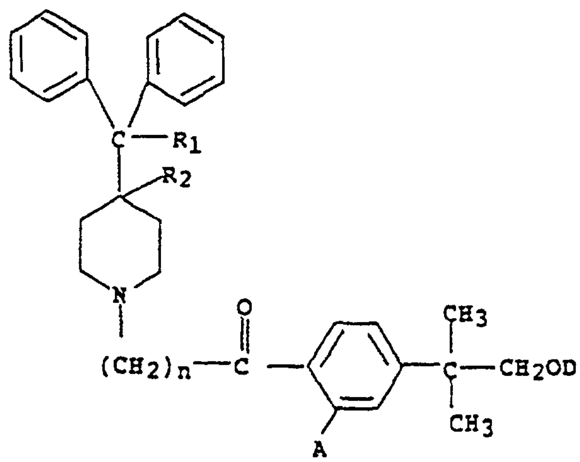

wherein $R_1$, $R_2$, n and D are defined above and A is hydrogen;

(e) deprotecting the hydroxyethyl functionality of the piperidine protected hydroxyethylphenylketone with an appropriate deprotecting reagent to produce a piperidine hydroxyethylphenylketone of the formula

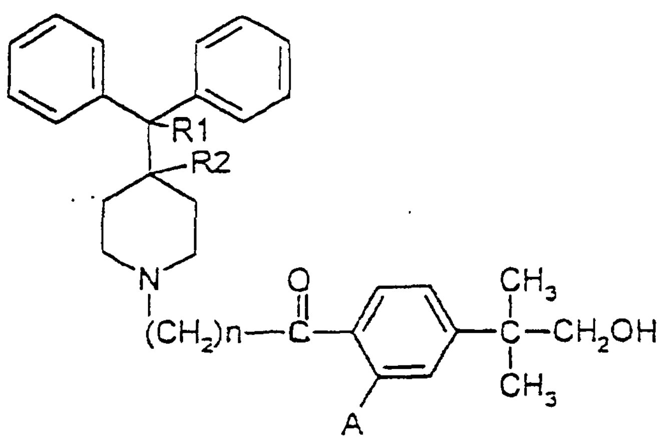

wherein $R_1$, $R_2$, n and A are defined above; and

(f) reacting the piperidine hydroxyethylphenylketone with dimethyl sulfoxide, oxyalyl chloride and triethylamine or with Dess-Martin reagent, chromium (IV) oxide, nickel peroxide, sodium dichromate, potassium dichromate, t-butyl chromate, silver oxide, argentic picolinate manganese dioxide lead tetraacetate, dicyclohexylcarbodiimide, 2,3-dichloro-5,6-dicyanoquinone, tetrachloro-1,2-benzoquinone, 2,2,6,6-tetramethylpiperidinyl-1-oxy (TEMPO) or quinolinium chlorochromate to produce a piperidine formylphenylketone of the formula

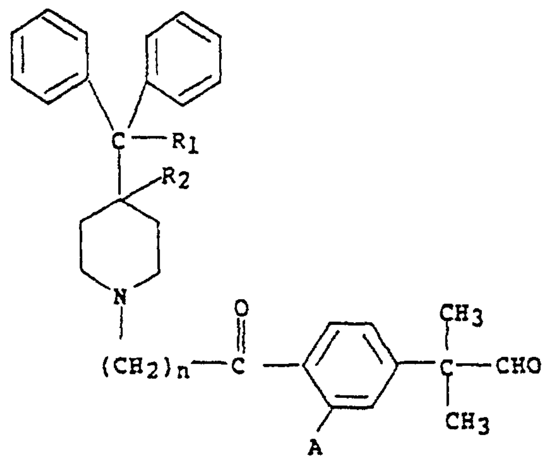

wherein $R_1$, $R_2$ and n are defined above and A is hydrogen;

(g) reacting the piperidine formylphenylketone with potassium permanganate, chromium (IV) aoxide, silver (I) oxide, silver oxide, argentic picolinate, peroxide, nitric acid, m-chloroperbenzoic acid and peracetic acid to produce a piperidine carboxyphenylketone of the formula

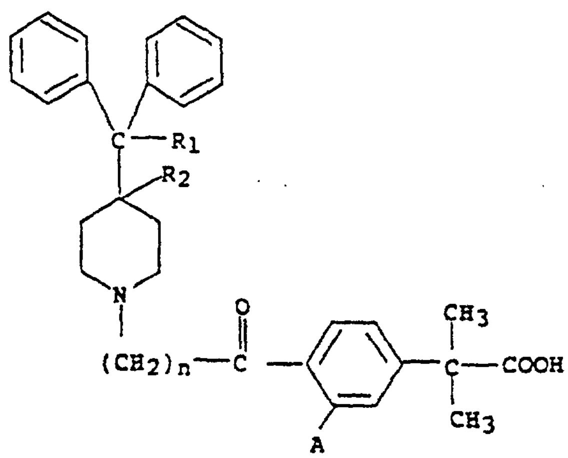

wherein $R_1$, $R_2$ and n are defined above and A is hydrogen;

(h) reacting the piperidine carboxyphenylketone with (-)-B-chlorodiisopinocamphenylborane to produce a piperidine carboxyphenylalcohol of the formula

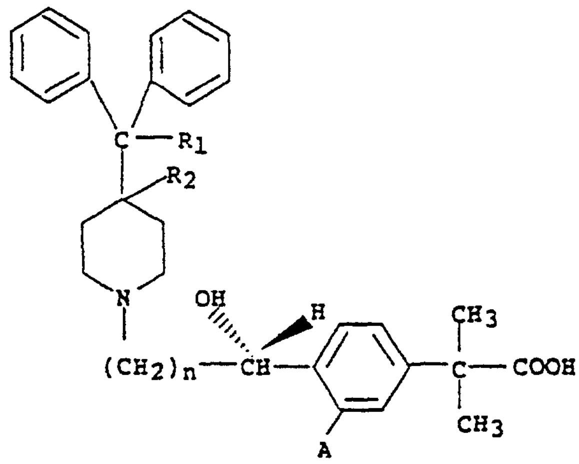

wherein $R_1$, $R_2$ and n are defined above and A is hydrogen;

(i) optionally reacting the piperidine carboxyphenylalcohol with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylalcohol ester of the formula

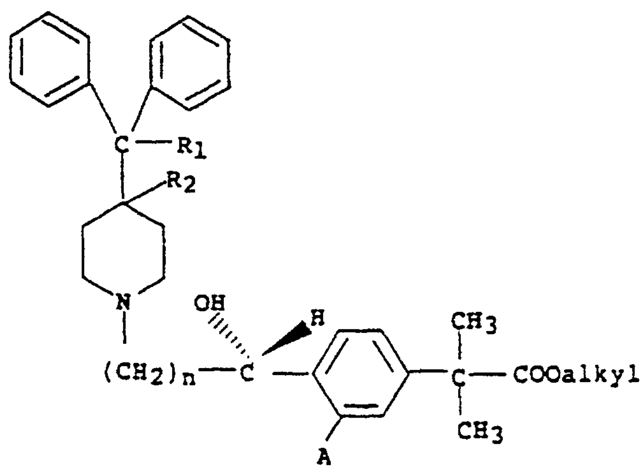

wherein $R_1$, $R_2$, n and alkyl are defined above and A is hydrogen.

**19.** A process for preparing a compound of the formula

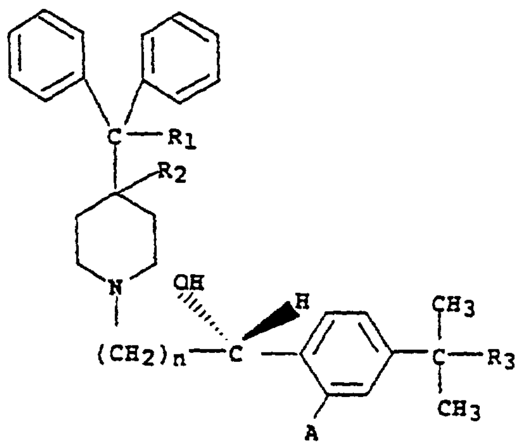

wherein

$R_1$ represents hydrogen or hydroxy;

$R_2$ represents hydrogen; or

$R_1$ and $R_2$ taken together form a second bond between the carbon atoms bearing $R_1$ and $R_2$;

n is an integer of from 1 to 5;

$R_3$ is -COOH or -COOalkyl wherein the alkyl moiety has from 1 to 6 carbon atoms and is straight or branched;

A is hydrogen and

pharmaceutically acceptable salts thereof comprising the steps of:

(a) reacting a benzeneacetic acid compound of the formula

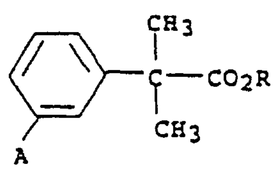

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

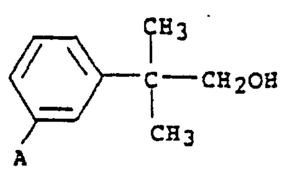

wherein A is hydrogen;

(b) protecting the hydroxyethyl functionality with a suitable protecting group to give a protected phenethyl alcohol of the formula

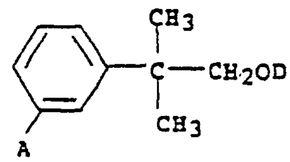

wherein A is hydrogen and D is a suitable protecting group;

(c) reacting the protected phenethyl alcohol with a ω-halo compound of the formula

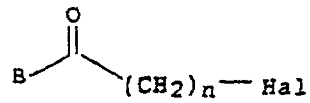

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

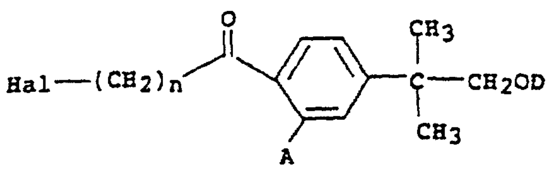

wherein Hal, n and D are defined above and A is hydrogen;

(d) reacting the ω-halo protected hydroxyethylphenylketone with a piperidine compound of the formula

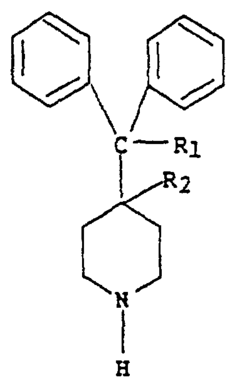

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

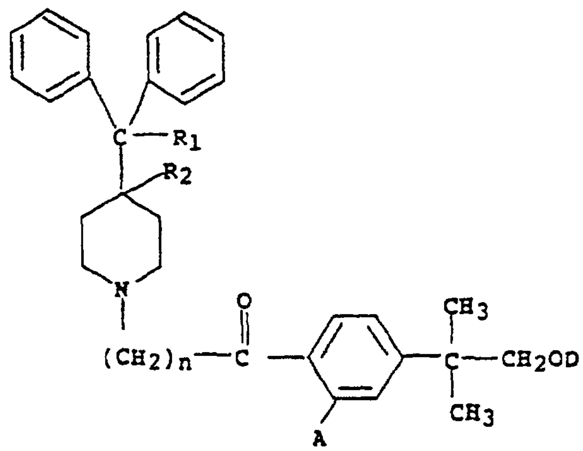

wherein $R_1$, $R_2$, n and D are defined above and A is hydrogen

(e) deprotecting the hydroxyethyl functionality of the piperidine protected hydroxyethylphenylketone with an appropriate deprotecting reagent to produce a piperidine hydroxyethylphenylketone of the formula

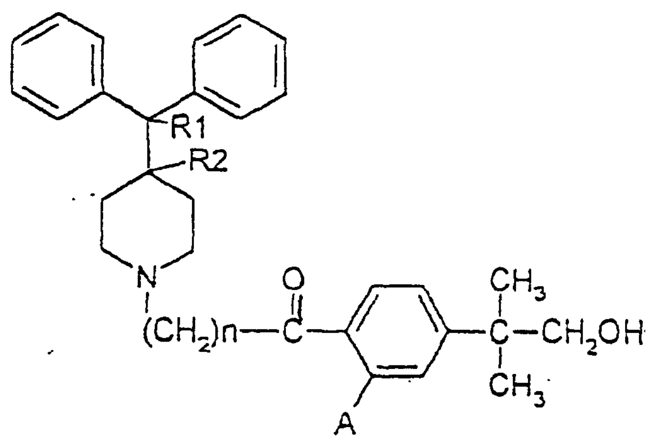

wherein $R_1$, $R_2$, n and A are defined above; and

(f) reacting the piperidine hydroxyethylphenylketone with chromium (IV) oxide, potassium permanganate, nitric acid, nitrogen dioxide, ruthenium (VIII) oxide, nickel peroxide, silver oxide, t-butyl chromate or xenic acid to produce a piperidine carboxyphenylketone of the formula

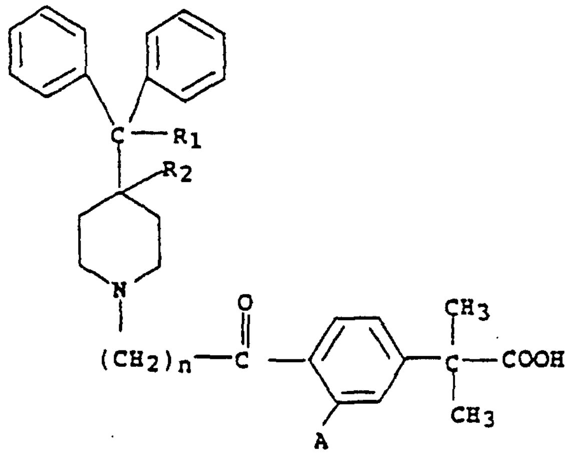

wherein $R_1$, $R_2$ and n are defined above and A is hydrogen;

(g) reacting the piperidine carboxyphenylketone with (-)-B-chlorodiisopinocamphenylborane to produce a piperidine carboxyphenylalcohol of the formula

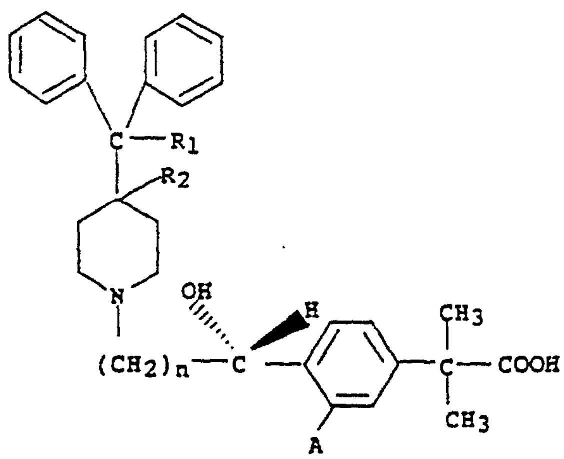

wherein $R_1$, $R_2$ and n are defined above and A is hydrogen

(h) optionally reacting the piperidine carboxyphenylalcohol with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylalcohol ester of the formula

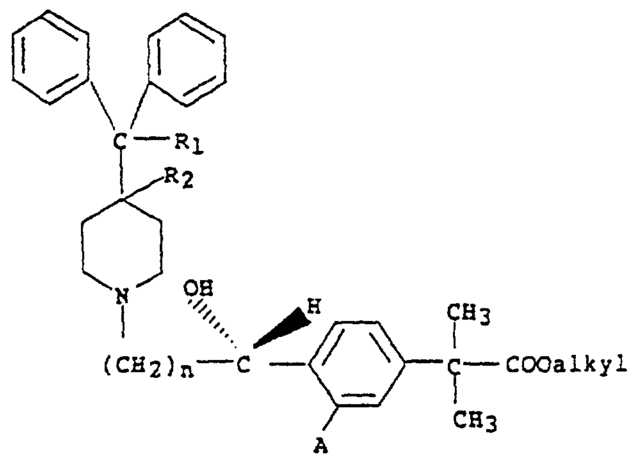

wherein $R_1$, $R_2$, n and alkyl are defined above and A is hydrogen.

**20.** A process for preparing a compound of the formula

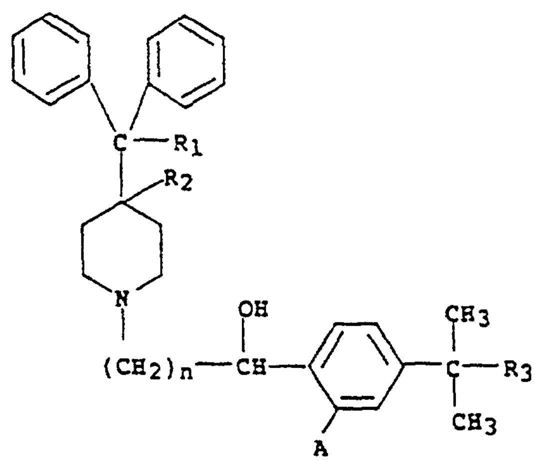

wherein
$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is -COOH or -COOalkyl wherein the alkyl moiety has from 1 to 6 carbon atoms and is straight or branched;
A is hydrogen; and
pharmaceutically acceptable salts thereof comprising the steps of:

(a) reacting a benzeneacetic acid compound of the formula

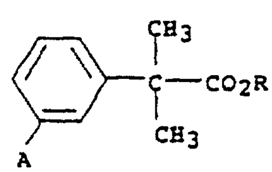

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

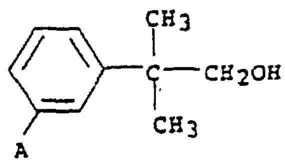

wherein A is hydrogen;

(b) protecting the hydroxyethyl functionality with a suitable protecting group to give a protected phenethyl alcohol of the formula

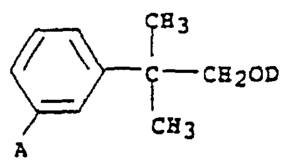

wherein A is hydrogen and D is a suitable protecting group;

(c) reacting the protected phenethyl alcohol with a ω-halo compound of the formula

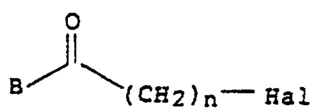

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

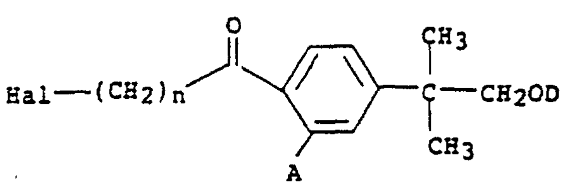

wherein Hal, n and D are defined above and A is hydrogen;

(d) reacting the ω-halo protected hydroxyethylphenylketone with sodium borohydride, potassium borohydride, sodium cyanoborohydride, tetramethylammonium borohydride, lithium tri-tert-butylaluminohydride, diisobutylaluminum hydride or catalytic hydrogenation to produce a ω-halo protected hydroxyethylphenylalcohol of the formula

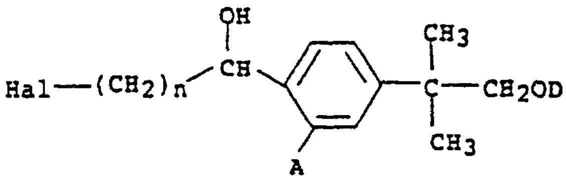

wherein Hal, n and D are defined above and A is hydrogen;

(e) reacting the ω-halo protected hydroxyethylphenylalcohol with a piperidine compound of the formula

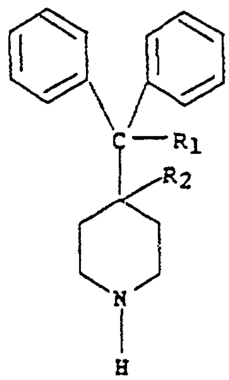

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylalcohol of the formula

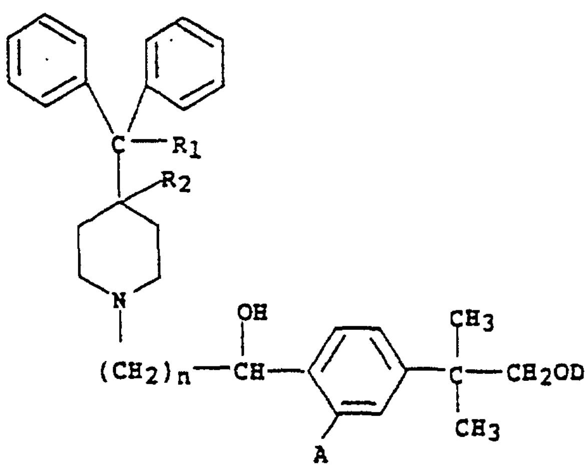

wherein $R_1$, $R_2$, n and D are defined above and A is hydrogen;

(f) deprotecting the hydroxyethyl functionality of the piperidine protected hydroxyethylphenylalcohol with an appropriate deprotecting reagent to produce a piperidine hydroxyethylphenylalcohol of the formula

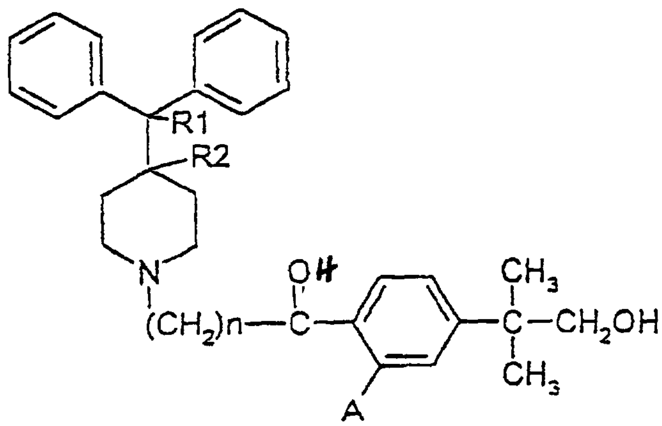

wherein $R_1$, $R_2$ and n are defined above and A is hydrogen;

(g) reacting the piperidine hydroxyethylphenylalcohol with dimethyl sulfoxide, oxyalyl chloride and triethylamine or with Dess-Martin reagent, chromium (IV) oxide, nickel peroxide, sodium dichromate, potassium dichromate, t-butyl chromate, silver oxide, argentic picolinate manganese dioxide lead tetraacetate, dicyclohexylcarbodiimide, 2,3-dichloro-5,6-dicyanoquinone, tetrachloro-1,2-benzoquinone, 2,2,6,6-tetramethylpiperidinyl-1-oxy (TEMPO) or quinolinium chlorochromate to produce a piperidine formylphenylalcohol of the formula

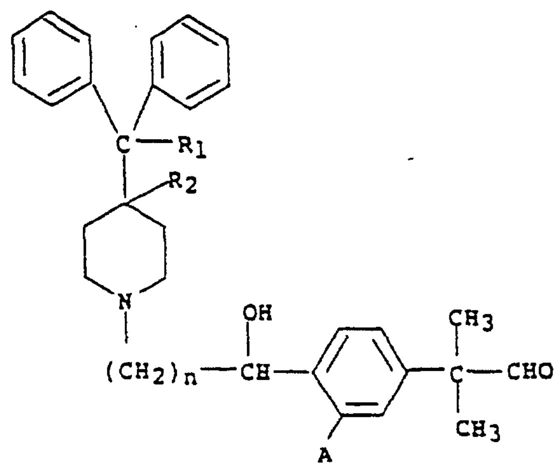

wherein $R_1$, $R_2$ and n are defined above and A is hydrogen;

(h) reacting the piperidine formylphenylalcohol with potassium permanganate, chromium (IV) oxide, silver (I) oxide, silver oxide, argentic picolinate, peroxide, nitric acid, m-chloroperbenzoic acid and peracetic acid to produce a piperidine carboxyphenylalcohol of the formula

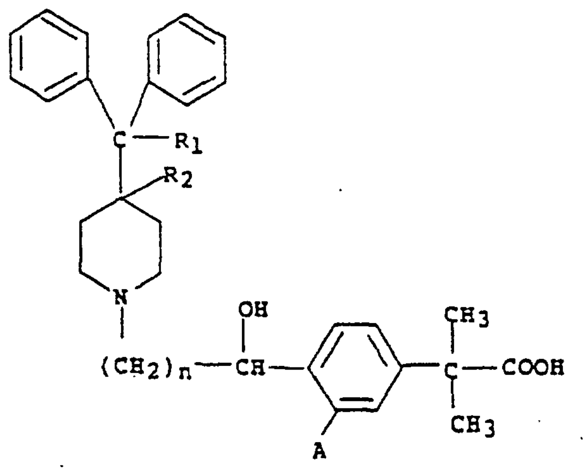

wherein $R_1$, $R_2$ and n are defined above and A is hydrogen;

(i) optionally reacting the piperidine carboxyphenylalcohol with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylalcohol ester of the formula

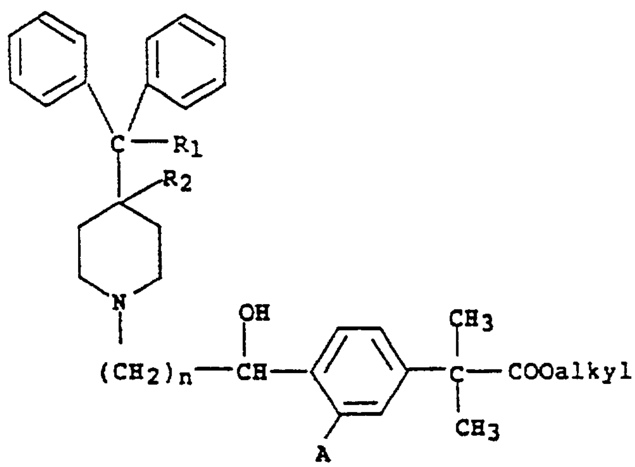

wherein $R_1$, $R_2$, n and alkyl are defined above and A is hydrogen.

**21.** A process for preparing a compound of the formula

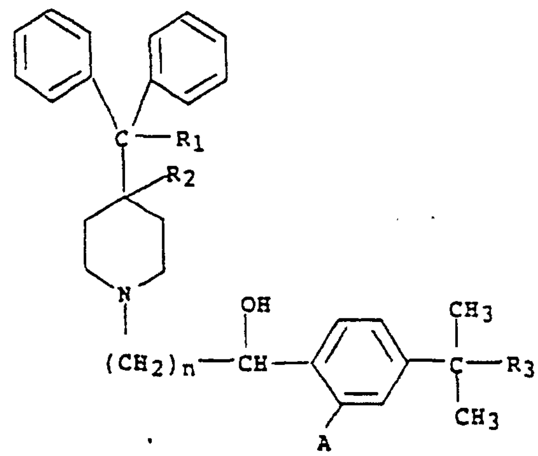

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is -COOH or -COOalkyl wherein the alkyl moiety has from 1 to 6 carbon atoms and is straight or branched;
A is hydrogen; and
pharmaceutically acceptable salts thereof comprising the steps of:

(a) reacting a benzeneacetic acid compound of the formula

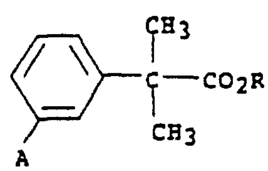

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

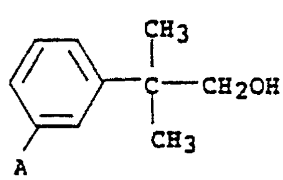

wherein A is hydrogen;

(b) protecting the hydroxyethyl functionality with a suitable protecting group to give a protected phenethyl alcohol of the formula

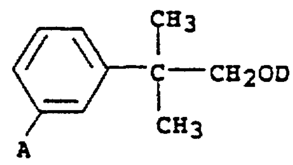

wherein A is hydrogen and D is a suitable protecting group;

(c) reacting the protected phenethyl alcohol with a ω-halo compound of the formula

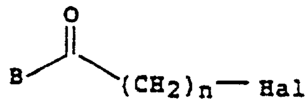

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

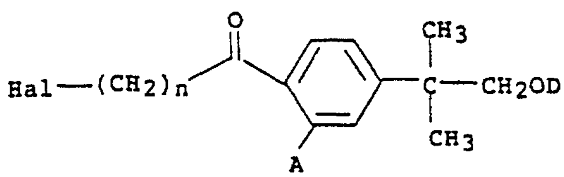

wherein Hal, n and D are defined above and A is hydrogen;

(d) reacting the ω-halo protected hydroxyethylphenylketone with sodium borohydride, potassium borohydride, sodium cyanoborohydride, tetramethylammonium borohydride, lithium tri-tert-butylaluminohydride, diisobutylaluminum hydride or catalytic hydrogenation to produce a ω-halo protected hydroxyethylphenylalcohol of the formula

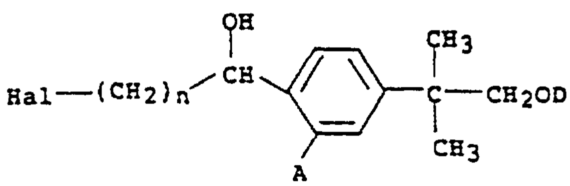

wherein Hal, n and D are defined above and A is hydrogen;

(e) reacting the ω-halo protected hydroxyethylphenylalcohol with a piperidine compound of the formula

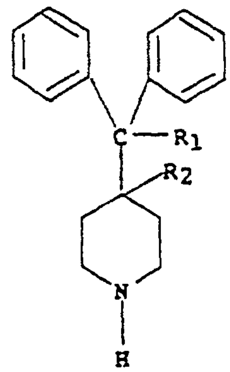

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylalcohol of the formula

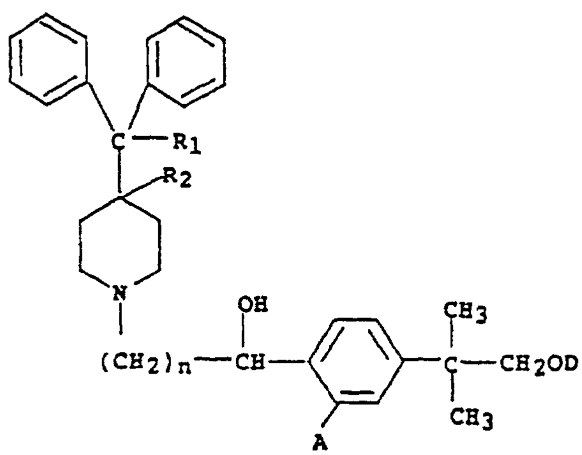

wherein $R_1$, $R_2$, n and D are defined above and A is hydrogen;

(f) deprotecting the hydroxyethyl functionality of the piperidine protected hydroxyethylphenylalcohol with an appropriate deprotecting reagent to produce a piperidine hydroxyethylphenylalcohol of the formula

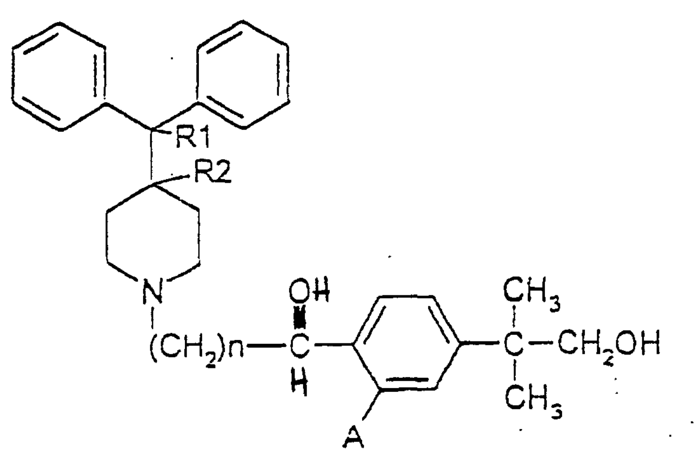

wherein $R_1$, $R_2$ and n are defined above and A is hydrogen;

(g) reacting the piperidine hydroxyethylphenylalcohol with chromium (IV) oxide, potassium permanganate, nitric acid, nitrogen dioxide, ruthenium (VIII) oxide, nickel peroxide, silver oxide, t-butyl chromate or xenic acid to produce a piperidine carboxyphenylalcohol of the formula

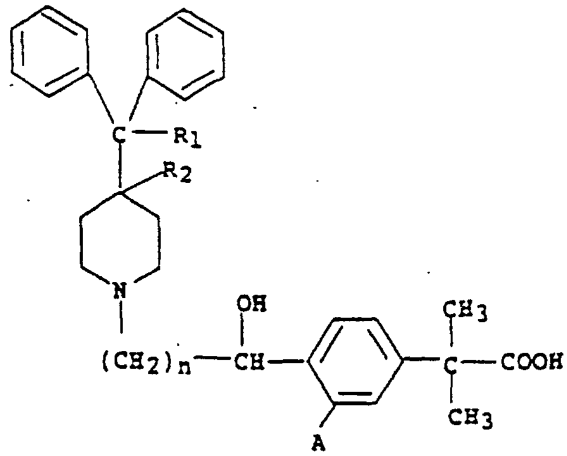

wherein $R_1$, $R_2$ and n are defined above and A is hydrogen;

(h) optionally reacting the piperidine carboxyphenylalcohol with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylalcohol ester of the formula

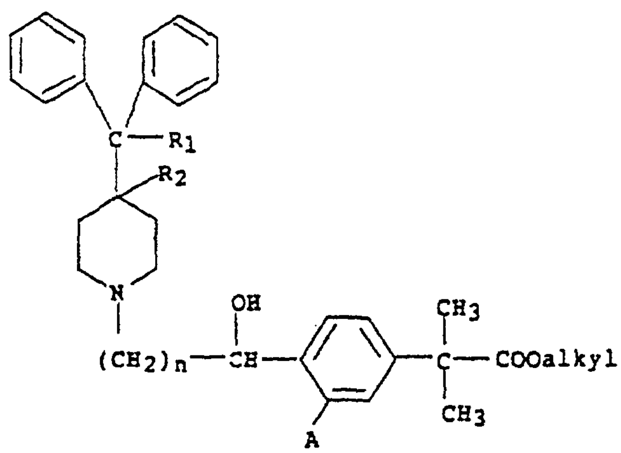

wherein $R_1$, $R_2$, n and alkyl are defined above and A is hydrogen.

**22.** A process according to Claim 21 wherein the piperidine hydroxyphenylalcohol produced in step f is reacted with a ruthenium (VIII) oxide to produce a piperidine carboxyphenylalcohol of step g.

**23.** A process for preparing a compound of the formula wherein

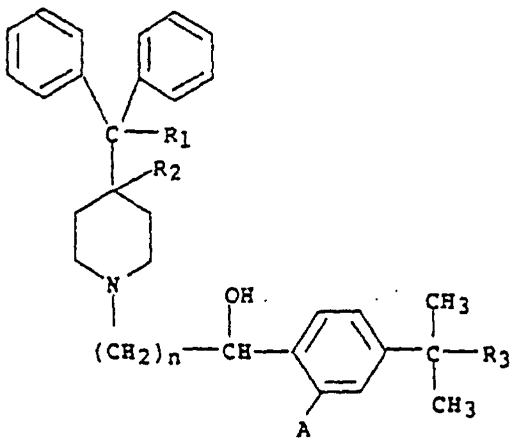

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is $-CH_2OH$;
A is hydrogen; and
pharmaceutically acceptable salts thereof comprising the steps of:

(a) reacting a benzeneacetic acid compound of the formula

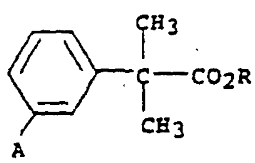

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

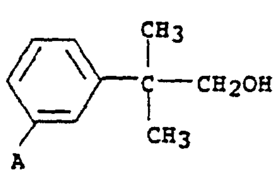

wherein A is hydrogen;

(b) protecting the hydroxyethyl functionality with a suitable protecting group to give a protected phenethyl alcohol of the formula

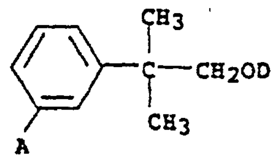

wherein A is hydrogen and D is a suitable protecting group;

(c) reacting the protected phenethyl alcohol with a ω-halo compound of the formula

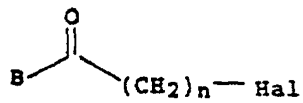

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

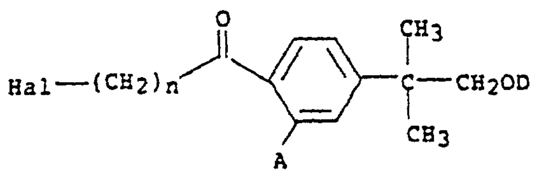

wherein Hal, n and D are defined above and A is hydrogen;

(d) reacting the ω-halo protected hydroxyethylphenylketone with sodium borohydride, potassium borohydride, sodium cyanoborohydride, tetramethylammonium borohydride, lithium tri-tert-butylaluminohydride, diisobutylaluminum hydride or catalyitc hydrogenation to produce a ω-halo protected hydroxyethylphenylalcohol of the formula

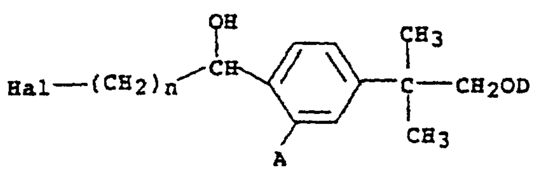

wherein Hal, n and D are defined above and A is hydrogen;

(e) reacting the ω-halo protected hydroxyethylphenylalcohol with a piperidine compound of the formula

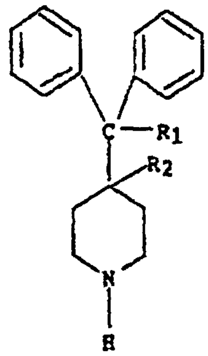

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylalcohol of the formula

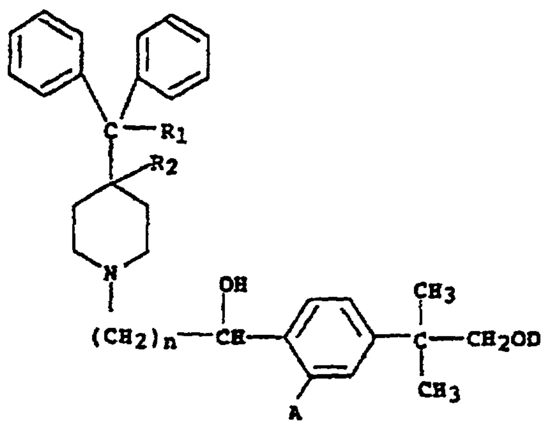

wherein $R_1$, $R_2$, n and D are defined above and A is hydrogen

(f) deprotecting the hydroxyethyl functionality of the piperidine protected hydroxyethylphenylalcohol with an appropriate deprotecting reagent to produce a piperidine hydroxyethylphenylalcohol of the formula

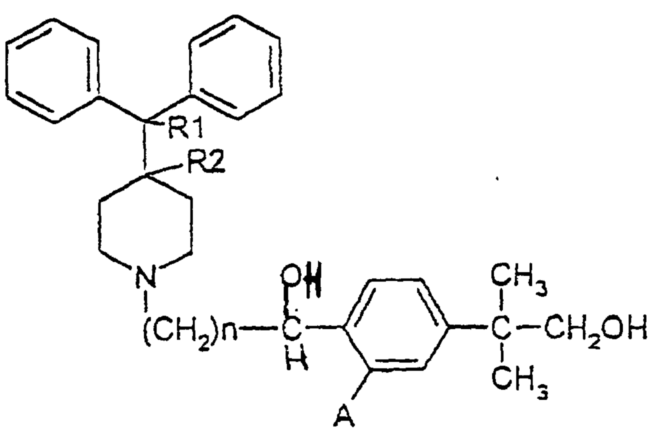

wherein $R_1$, $R_2$ and n are defined above and A is hydrogen.

**24.** A process for preparing a compound of the formula

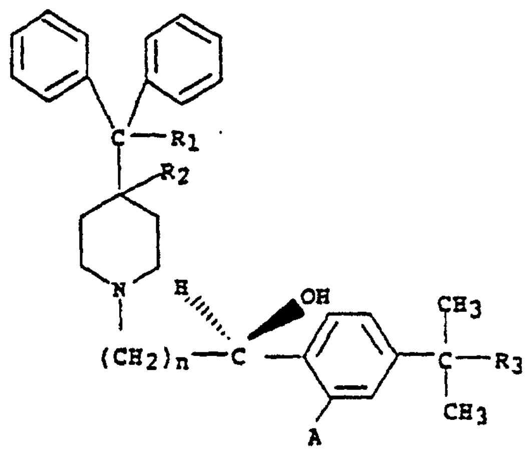

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is -COOH or -COOalkyl wherein the alkyl moiety has from 1 to 6 carbon atoms and is straight or branched;
A is hydrogen; and
pharmaceutically acceptable salts thereof comprising the steps of:

(a) reacting a benzeneacetic acid compound of the formula

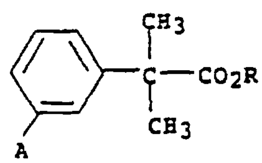

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

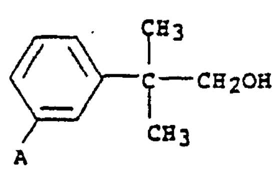

wherein A is hydrogen;

(b) protecting the hydroxyethyl functionality with a suitable protecting group to give a protected phenethyl alcohol of the formula

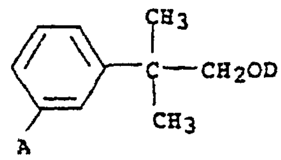

wherein A is hydrogen and D is a suitable protecting group;

(c) reacting the protected phenethyl alcohol with a ω-halo compound of the formula

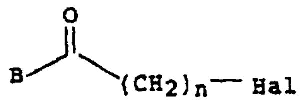

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

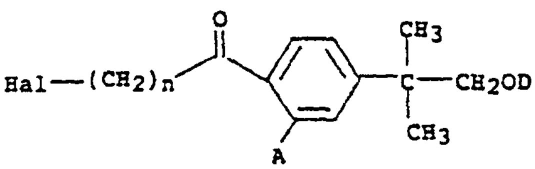

wherein Hal, n and D are defined above and A is hydrogen

(d) reacting the ω-halo protected hydroxyethylphenylketone with (+)-B-chlorodiisopinocamphenylborane to produce a ω-halo protected hydroxyethylphenylalcohol of the formula

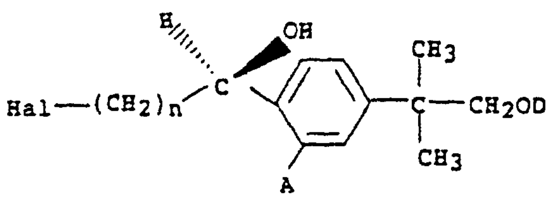

wherein Hal, n and D are defined above and A is hydrogen;

(e) reacting the ω-halo protected hydroxyethylphenylalcohol with a piperidine compound of the formula

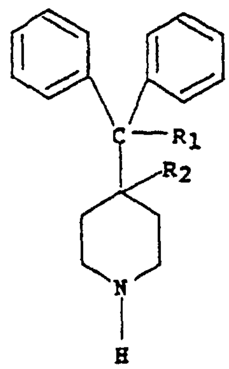

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylalcohol of the formula

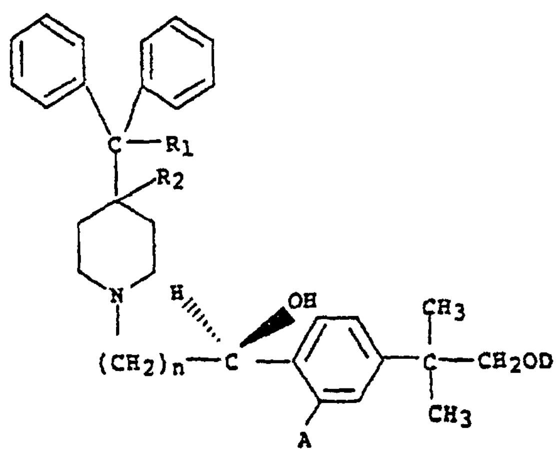

wherein $R_1$, $R_2$, n and D are defined above and A is hydrogen;

(f) deprotecting the hydroxyethyl functionality of the piperidine protected hydroxyethylphenylalcohol with an appropriate deprotecting reagent to produce a piperidine hydroxyethylphenylalcohol of the formula

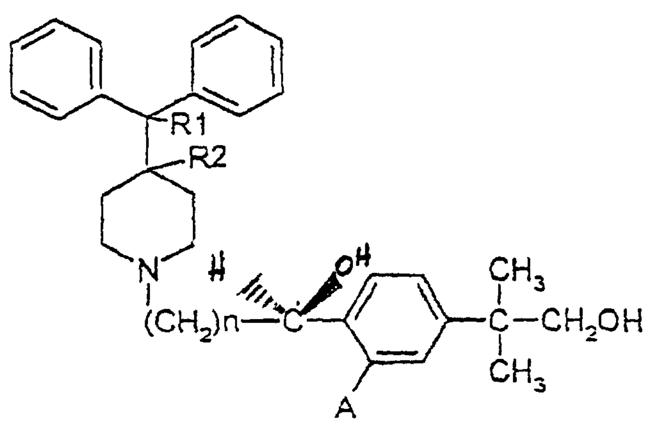

wherein $R_1$, $R_2$ and n are defined above and A is hydrogen;

(g) reacting the piperidine hydroxyethylphenylalcohol with dimethyl sulfoxide, oxyalyl chloride and triethylamine or with Dess-Martin reagent, chromium (IV) oxide, nickel peroxide, sodium dichromate, potassium dichromate, t-butyl chromate, silver oxide, argentic picolinate manganese dioxide lead tetraacetate, dicyclohexylcarbodiimide, 2,3-dichloro-5,6-dicyanoquinone, tetrachloro-1,2-benzoquinone, 2,2,6,6-tetramethylpiperidinyl-l-oxy (TEMPO) or quinolinium chlorochromate to produce a piperidine formylphenylalcohol of the formula

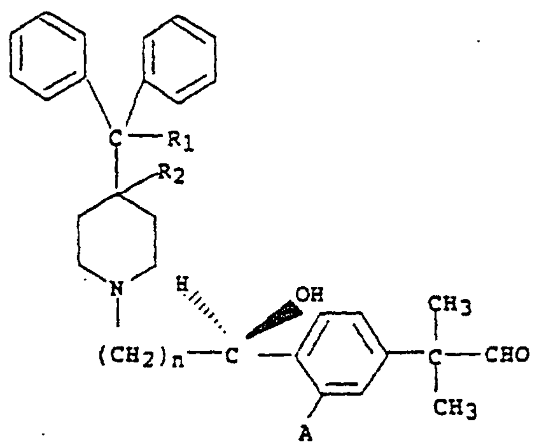

wherein $R_1$, $R_2$ and n are defined above and A is hydrogen;

(h) reacting the piperidine formylphenylalcohol with potassium permanganate, chromium (IV) oxide, silver (I) oxide, silver oxide, argentic picolinate, peroxide, nitric acid, m-chloroperbenzoic acid and peracetic acid to produce a piperidine carboxyphenylalcohol of the formula

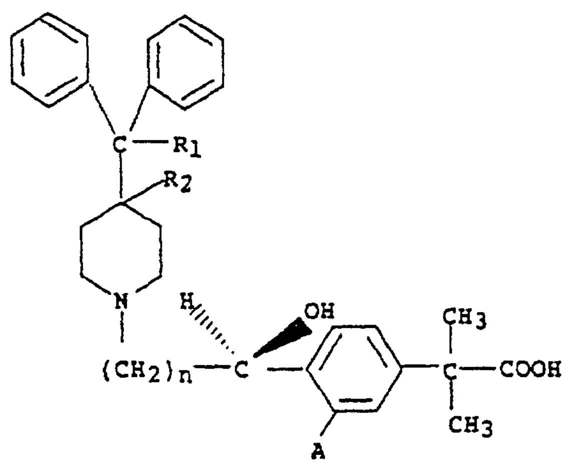

wherein $R_1$, $R_2$ and n are defined above and A is hydrogen;

(i) optionally reacting the piperidine carboxyphenylalcohol with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylalcohol ester of the formula

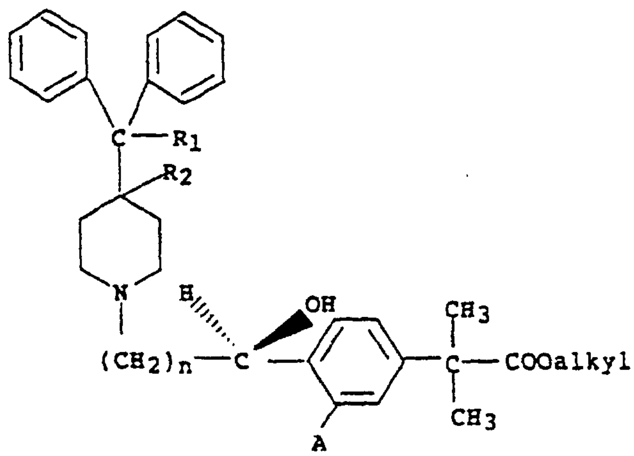

wherein $R_1$, $R_2$, n and alkyl are defined above and A is hydrogen.

**25.** A process for preparing a compound of the formula

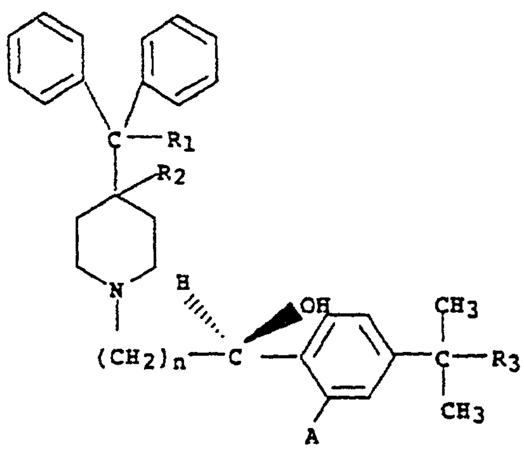

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is -COOH or -COOalkyl wherein the alkyl moiety has from 1 to 6 carbon atoms and is straight or branched;
A is hydrogen; and
pharmaceutically acceptable salts thereof comprising the steps of:

(a) reacting a benzeneacetic acid compound of the formula

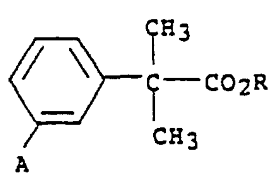

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

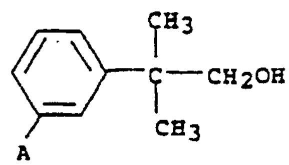

wherein A is hydrogen;

(b) protecting the hydroxyethyl functionality with a suitable protecting group to give a protected phenethyl alcohol of the formula

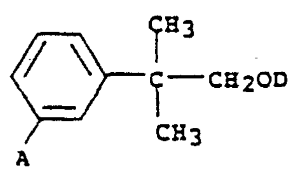

wherein A is hydrogen and D is a suitable protecting group;

(c) reacting the protected phenethyl alcohol with a ω-halo compound of the formula

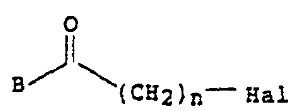

wherein B is halo or hydroxy, Hal represents Cl, Br or I and n is as defined above, in the presence of trichloride, aluminum chloride, titanium tetrachloride, boron trifluoride, tin tetrachloride and zinc chloride to produce a ω-halo protected hydroxyethylphenylketone of the formula

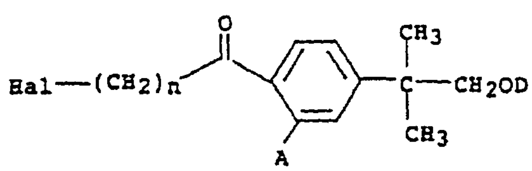

wherein Hal, n and D are defined above and A is hydrogen

(d) reacting the ω-halo protected hydroxyethylphenylketone with (+)-B-chlorodiisopinocamphenylborane to produce a ω-halo protected hydroxyethylphenylalcohol of the formula

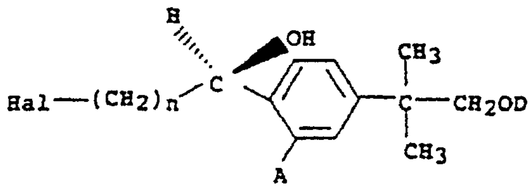

wherein Hal, n and D are defined above and A is hydrogen;

(e) reacting the ω-halo protected hydroxyethylphenylalcohol with a piperidine compound of the formula

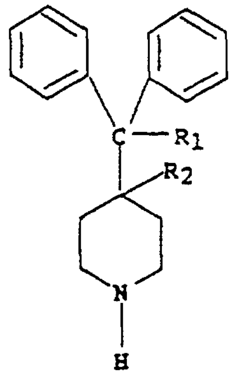

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylalcohol of the formula

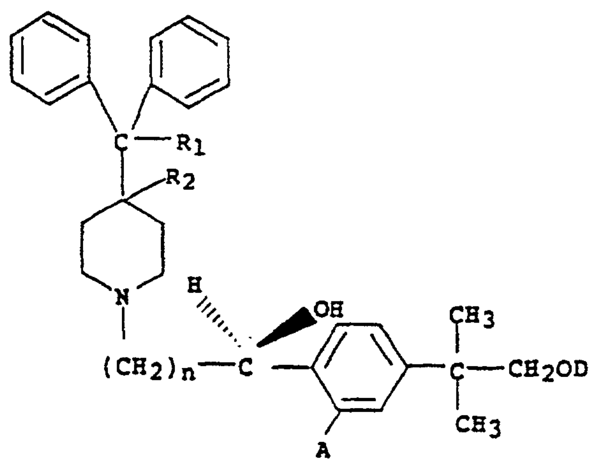

wherein $R_1$, $R_2$, n and D are defined above and A is hydrogen;

(f) deprotecting the hydroxyethyl functionality of the piperidine protected hydroxyethylphenylalcohol with an appropriate deprotecting reagent to produce a piperidine hydroxyethylphenylalcohol of the formula

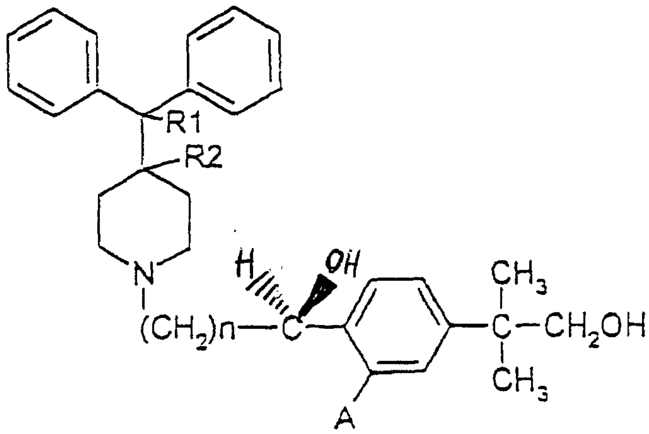

wherein $R_1$, $R_2$ and n are defined above and A is hydrogen;

(g) reacting the piperidine hydroxyethylphenylalcohol with chromium (IV) oxide, potassium permanganate, nitric acid, nitrogen dioxide, ruthenium (VIII) oxide, nickel peroxide, silver oxide, t-butyl chromate or xenic acid to produce a piperidine carboxyphenylalcohol of the formula

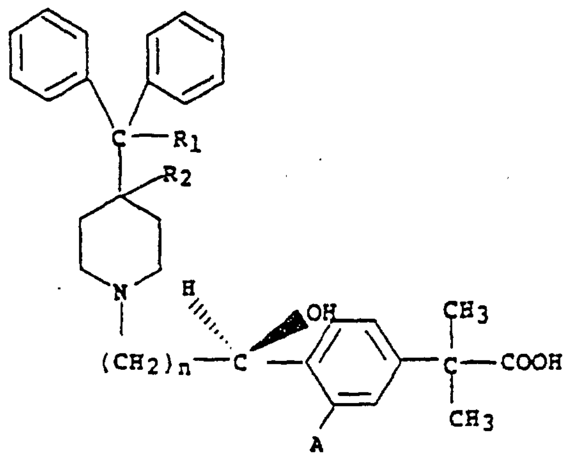

wherein $R_1$, $R_2$ and n are defined above and A is hydrogen;

(h) optionally reacting the piperidine carboxyphenylalcohol with an excess of diazomethane or an excess of 2,2-dimethoxypropane or with thionyl chloride followed by an alcohol of the formula Hoalkyl wherein the alkyl moiety has from 1 to 6 carbon atoms and is straight or branched to form a piperidine carboxyphenylalcohol ester of the formula

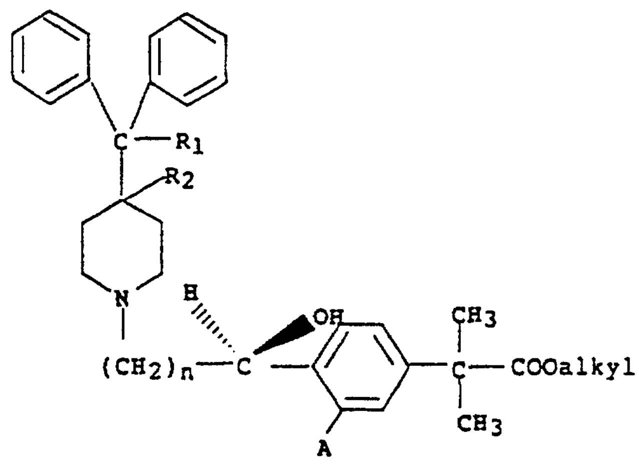

wherein $R_1$, $R_2$, n and alkyl are defined above and A is hydrogen.

**26.** A process for preparing a compound of the formula

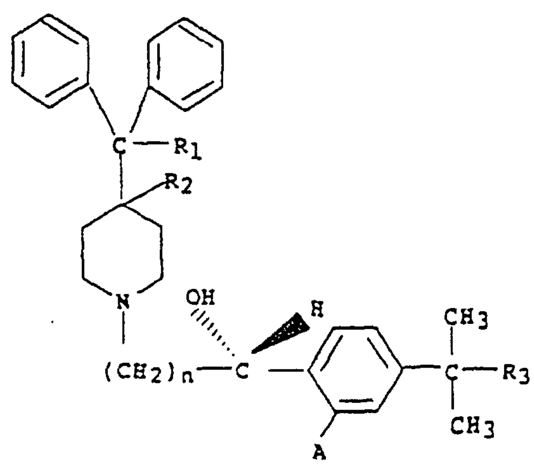

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is -COOH or -COOalkyl wherein the alkyl moiety has from 1 to 6 carbon atoms and is straight or branched;
A is hydrogen; and
pharmaceutically acceptable salts thereof comprising the steps of:

(a) reacting a benzeneacetic acid compound of the formula

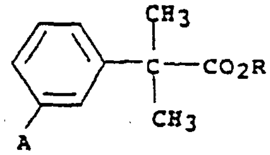

wherein A is hydrogen and R is hydrogen or $C_1$-$C_6$ alkyl with sodium bis(2-methoxyethoxy)aluminium hydride,

lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

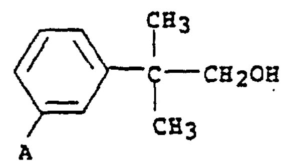

wherein A is hydrogen;

(b) protecting the hydroxyethyl functionality with a suitable protecting group to give a protected phenethyl alcohol of the formula

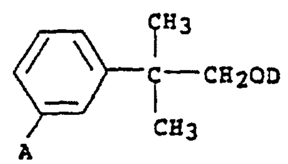

wherein A is hydrogen and D is a suitable protecting group;

(c) reacting the protected phenethyl alcohol with a ω-halo compound of the formula

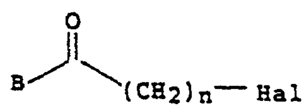

wherein B is halo or hydroxy, Hal represents Cl, Br or I and n is as defined above, in the presence of trichloride, aluminum chloride, titanium tetrachloride, boron trifluoride, tin tetrachloride and zinc chloride to produce a ω-halo protected hydroxyethylphenylketone of the formula

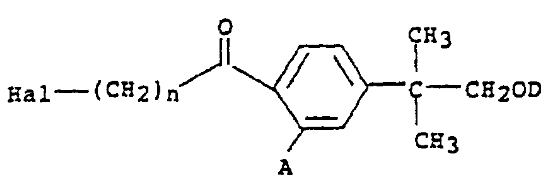

wherein Hal, n and D are defined above and A is hydrogen;

(d) reacting the ω-halo protected hydroxyethylphenylketone with (-)-B-chlorodiisopinocamphenylborane to produce a ω-halo protected hydroxyethylphenylalcohol of the formula

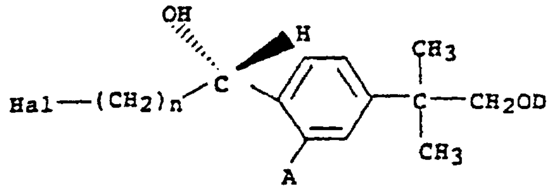

wherein Hal, n and D are defined above and A is hydrogen;

(e) reacting the ω-halo protected hydroxyethylphenylalcohol with a piperidine compound of the formula

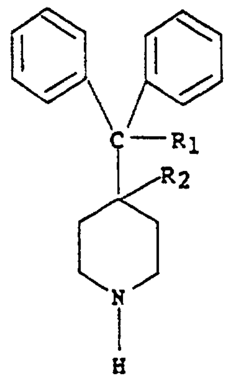

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylalcohol of the formula

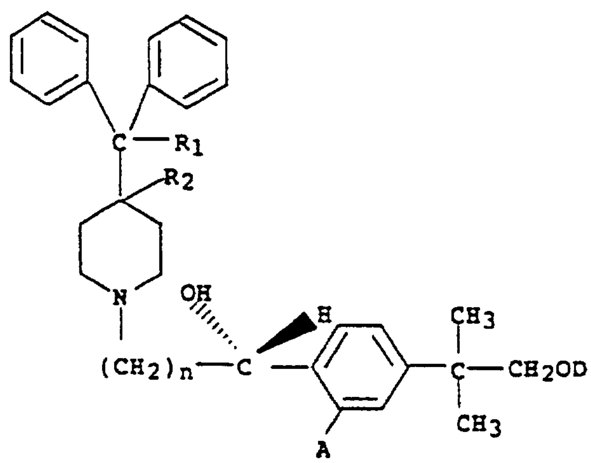

wherein $R_1$, $R_2$, n and D are defined above and A is be hydrogen;

(f) deprotecting the hydroxyethyl functionality of the piperidine protected hydroxyethylphenylalcohol with an appropriate deprotecting reagent to produce a piperidine hydroxyethylphenylalcohol of the formula

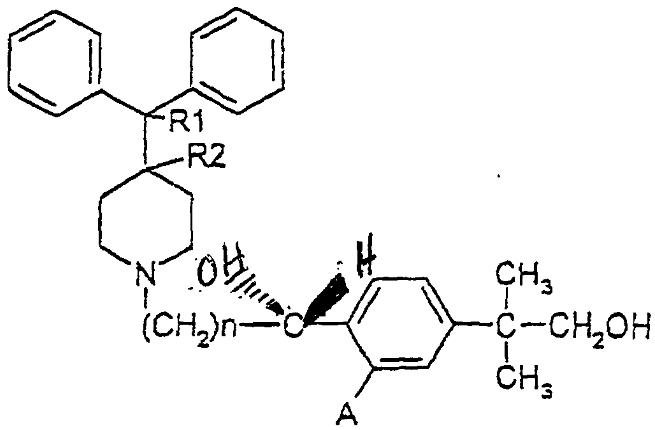

wherein $R_1$, $R_2$ and n are defined above and A is hydrogen;

(g) reacting the piperidine hydroxyethylphenylalcohol with dimethyl sulfoxide, oxyalyl chloride and triethylamine or with Dess-Martin reagent, chromium (IV) oxide, nickel peroxide, sodium dichromate, potassium dichromate, t-butyl chromate, silver oxide, argentic picolinate manganese dioxide lead tetraacetate, dicyclohexylcarbodiimide, 2,3-dichloro-5,6-dicyanoquinone, tetrachloro-1,2-benzoquinone, 2,2,6,6-tetramethylpiperidinyl-1-oxy (TEMPO) or quinolinium chlorochromate to produce a piperidine formylphenylalcohol of the formula

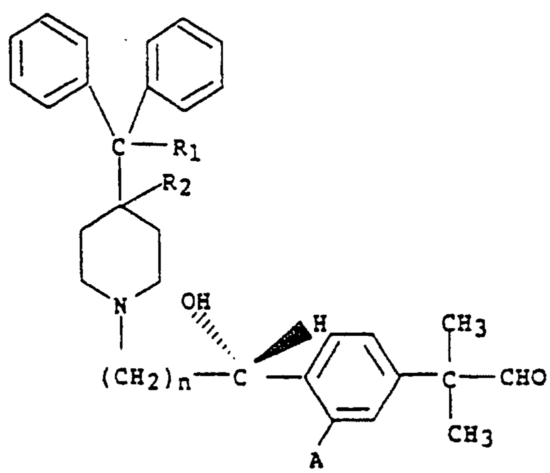

wherein $R_1$, $R_2$ and n are defined above and A is hydrogen;

(h) reacting the piperidine formylphenylalcohol with potassium permanganate, chromium (IV) oxide, silver (I) oxide, silver oxide, argentic picolinate, peroxide, nitric acid, m-chloroperbenzoic acid and peracetic acid to produce a piperidine carboxyphenylalcohol of the formula

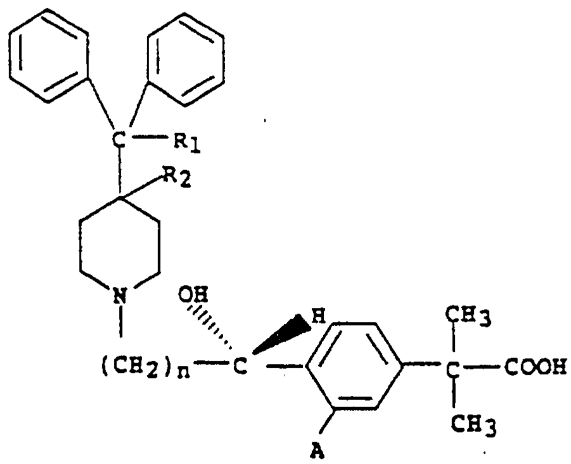

wherein $R_1$, $R_2$ and n are defined above and A is hydrogen;

(i) optionally reacting the piperidine carboxyphenylalcohol with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylalcohol ester of the formula

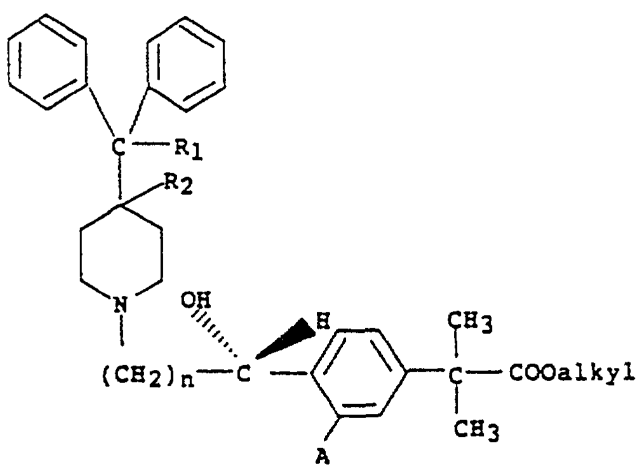

wherein $R_1$, $R_2$, n and alkyl are defined above and A is hydrogen.

**27.** A process for preparing a compound of the formula

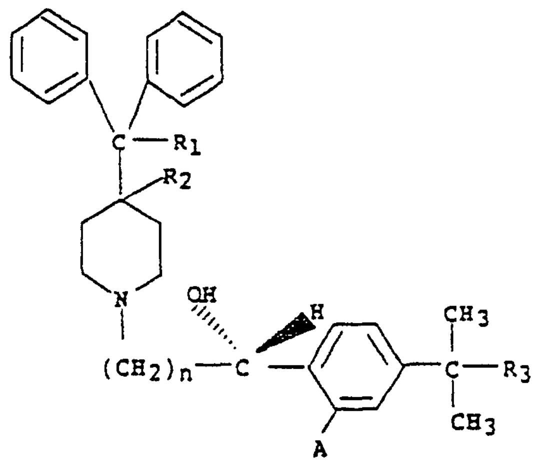

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is -COOH or -COOalkyl wherein the alkyl moiety has from 1 to 6 carbon atoms and is straight or branched;
A is hydrogen; and
pharmaceutically acceptable salts thereof comprising the steps of:

(a) reacting a benzeneacetic acid compound of the formula

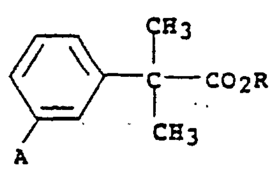

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

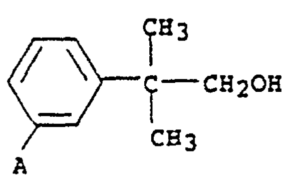

wherein A is hydrogen;

(b) protecting the hydroxyethyl functionality with a suitable protecting group to give a protected phenethyl alcohol of the formula

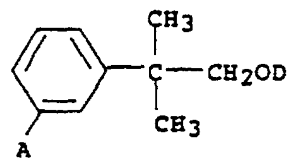

wherein A is hydrogen and D is a suitable protecting group;

(c) reacting the protected phenethyl alcohol with a ω-halo compound of the formula

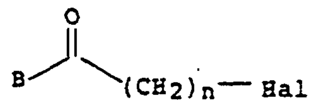

wherein B is halo or hydroxy, Hal represents Cl, Br or I and n is as defined above, in the presence of trichloride, aluminum chloride, titanium tetrachloride, boron trifluoride, tin tetrachloride and zinc chloride to produce a ω-halo protected hydroxyethylphenylketone of the formula

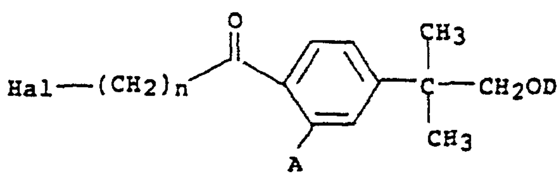

wherein Hal, n and D are defined above and A is hydrogen;

(d) reacting the ω-halo protected hydroxyethylphenylketone with (-)-B-chlorodiisopinocamphenylborane to produce a ω-halo protected hydroxyethylphenylalcohol of the formula

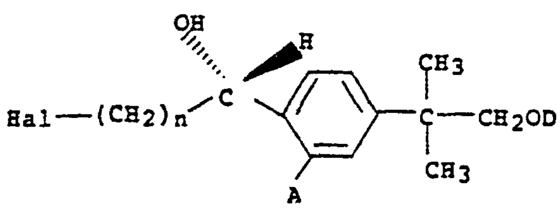

wherein Hal, n and D are defined above and A is hydrogen;

(e) reacting the ω-halo protected hydroxyethylphenylalcohol with a piperidine compound of the formula

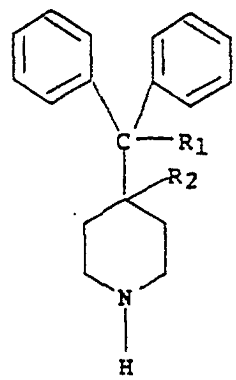

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylalcohol of the formula

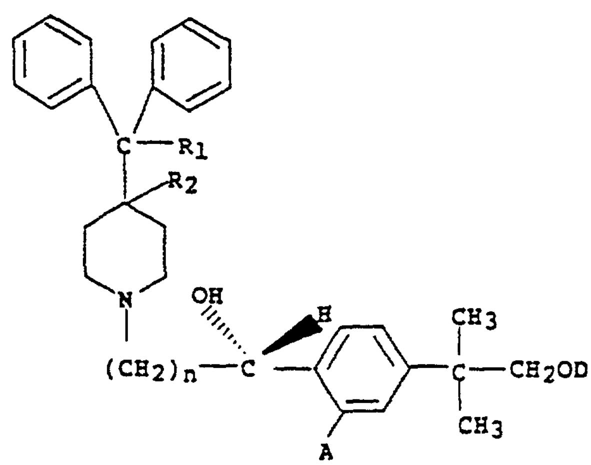

wherein $R_1$, $R_2$, n and D are defined above and A is

(f) deprotecting the hydroxyethyl functionality of the piperidine protected hydroxyethylphenylalcohol with an appropriate deprotecting reagent to produce a piperidine hydroxyethylphenylalcohol of the formula

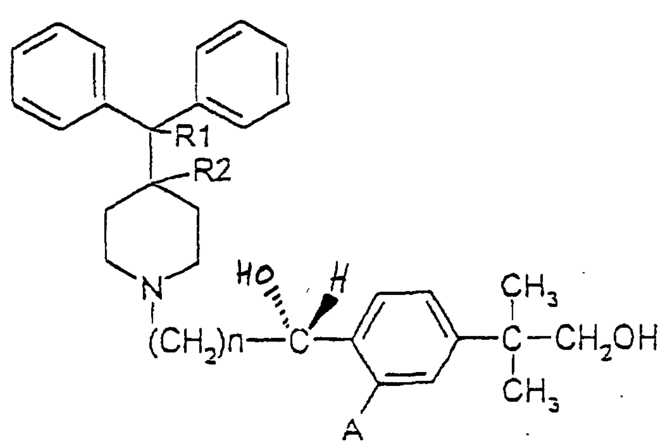

wherein $R_1$, $R_2$ and n are defined above and A is hydrogen;

(g) reacting the piperidine hydroxyethylphenylalcohol with chromium (IV) oxide, potassium permanganate, nitric acid, nitrogen dioxide, ruthenium (VIII) oxide, nickel peroxide, silver oxide, t-butyl chromate or xenic acid to produce a piperidine carboxyphenylalcohol of the formula

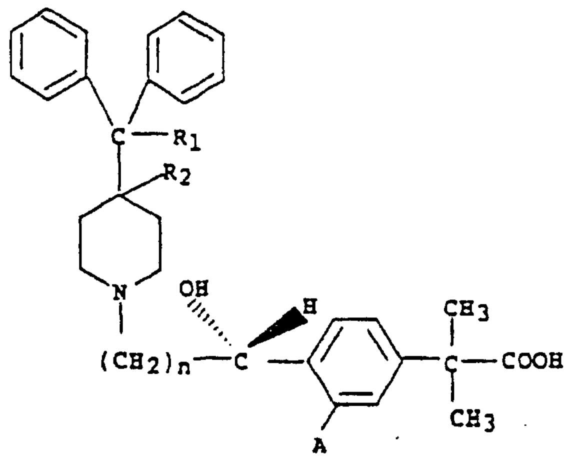

wherein $R_1$, $R_2$ and n are defined above and A is hydrogen;

(h) optionally reacting the piperidine carboxyphenylalcohol with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylalcohol ester of the formula

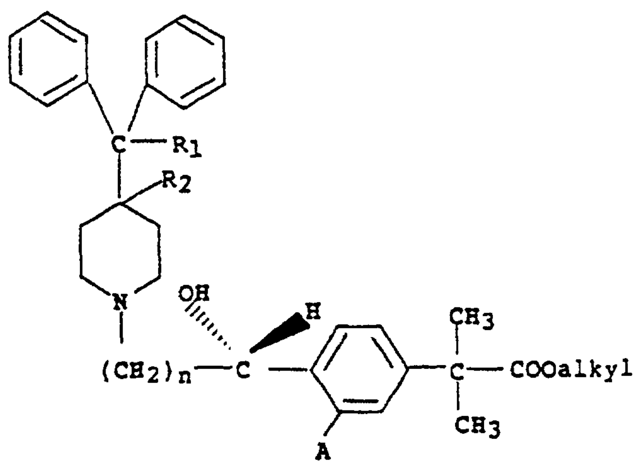

wherein $R_1$, $R_2$, n and alkyl are defined above and A is hydrogen.

**28.** A process for preparing a compound of the formula

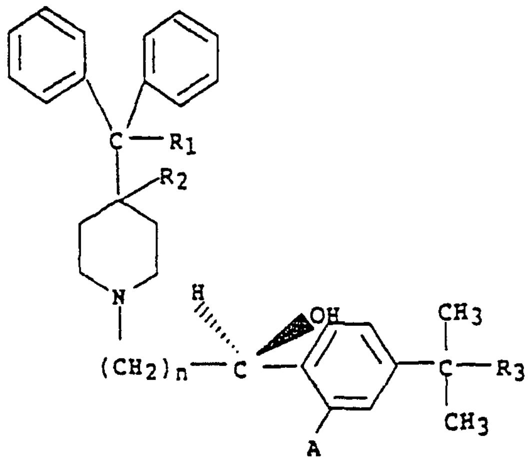

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is $-CH_2OH$;
A is hydrogen; and
pharmaceutically acceptable salts thereof comprising the steps of:

(a) reacting a benzeneacetic acid compound of the formula

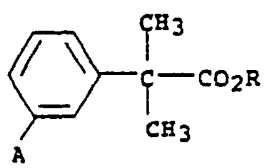

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

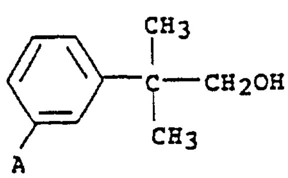

wherein A is hydrogen;

(b) protecting the hydroxyethyl functionality with a suitable protecting group to give a protected phenethyl alcohol of the formula

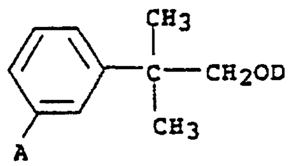

wherein A is hydrogen and D is a suitable protecting group;

(c) reacting the protected phenethyl alcohol with a ω-halo compound of the formula

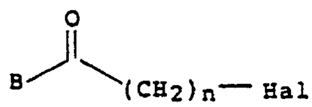

wherein B is halo or hydroxy, Hal represents Cl, Br or I and n is as defined above, in the presence of trichloride, aluminum chloride, titanium tetrachloride, boron trifluoride, tin tetrachloride and zinc chloride to produce a ω-halo protected hydroxyethylphenylketone of the formula

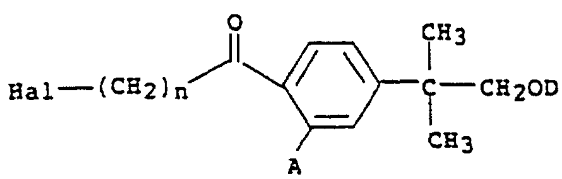

wherein Hal, n and D are defined above and A is hydrogen;

(d) reacting the ω-halo protected hydroxyethylphenylketone with (+)-B-chlorodiisopinocamphenylborane a ω-halo protected hydroxyethylphenylalcohol of the formula

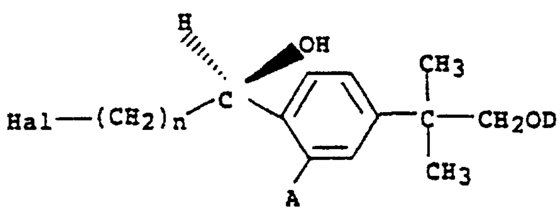

wherein Hal, n and D are defined above and A is hydrogen;

(e) reacting the ω-halo protected hydroxyethylphenylalcohol with a piperidine compound of the formula

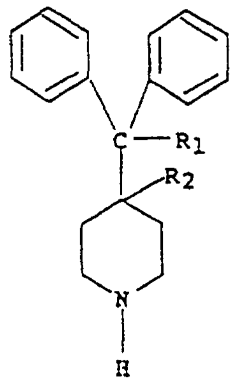

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylalcohol of the formula

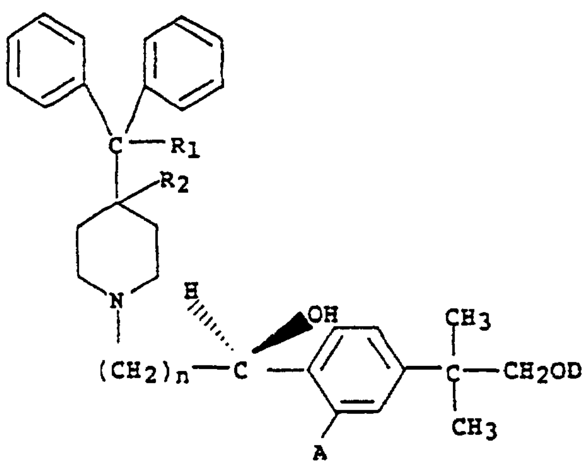

wherein $R_1$, $R_2$, n and D are defined above and A is hydrogen;

(f) deprotecting the hydroxyethyl functionality of the piperidine protected hydroxyethylphenylalcohol with an appropriate deprotecting reagent to produce a piperidine hydroxyethylphenylalcohol of the formula

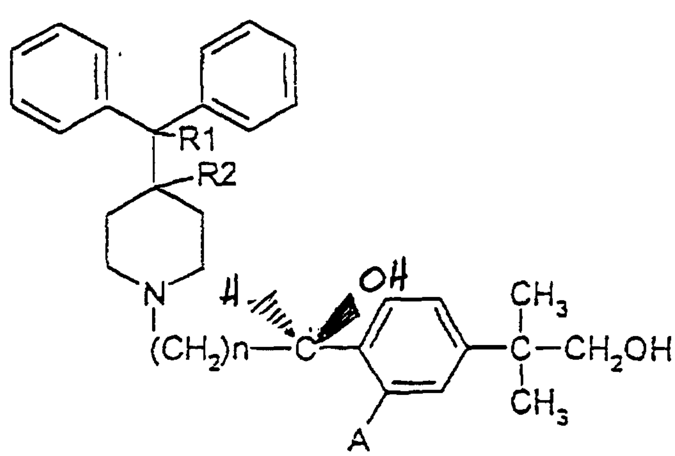

wherein $R_1$, $R_2$ and n are defined above and A is hydrogen;

**29.** A process for preparing a compound of the formula

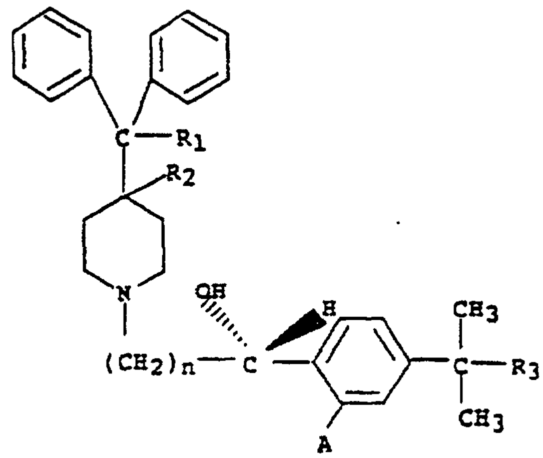

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is $-CH_2OH$;
A is hydrogen; and
pharmaceutically acceptable salts thereof comprising the steps of:

(a) reacting a benzeneacetic acid compound of the formula

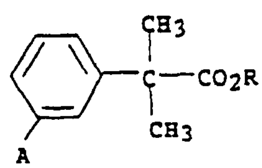

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

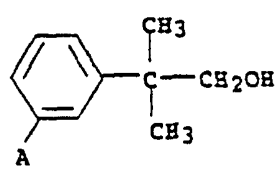

wherein A is hydrogen;

(b) protecting the hydroxyethyl functionality with a suitable protecting group to give a protected phenethyl alcohol of the formula

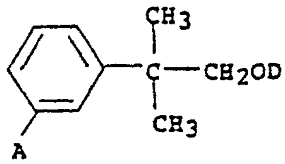

wherein A is hydrogen and D is a suitable protecting group;

(c) reacting the protected phenethyl alcohol with a ω-halo compound of the formula

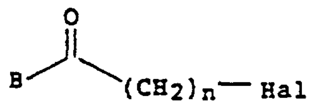

wherein B is halo or hydroxy, Hal represents Cl, Br or I and n is as defined above, in the presence of trichloride, aluminum chloride, titanium tetrachloride, boron trifluoride, tin tetrachloride and zinc chloride to produce a ω-halo protected hydroxyethylphenylketone of the formula

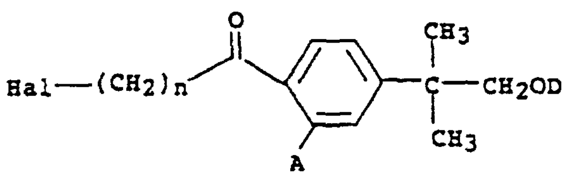

wherein Hal, n and D are defined above and A is hydrogen;

(d) reacting the ω-halo protected hydroxyethylphenylketone with (-)-B-chlorodiisopinocamphenylborane to produce a ω-halo protected hydroxyethylphenylalcohol of the formula

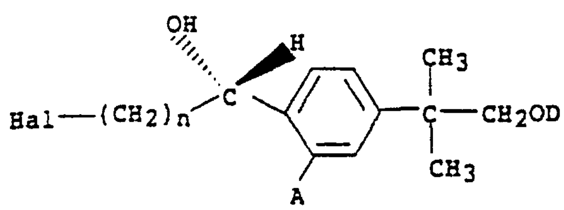

wherein Hal, n and D are defined above and A is hydrogen;

(e) reacting the ω-halo protected hydroxyethylphenylalcohol with a piperidine compound of the formula

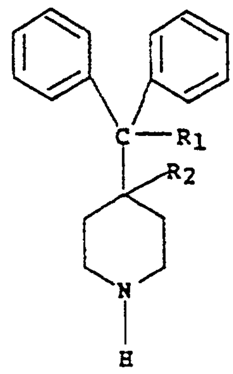

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylalcohol of the formula

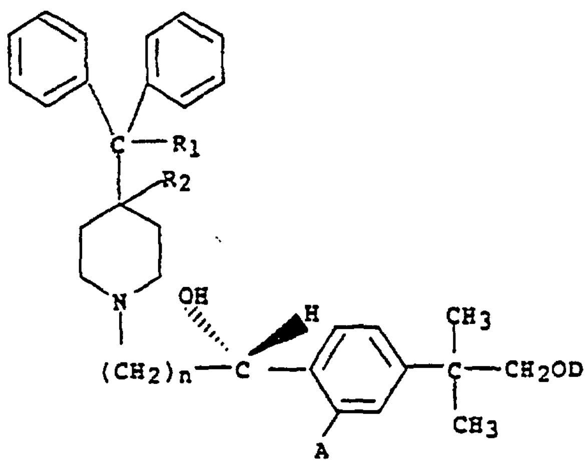

wherein $R_1$, $R_2$, *n* and D are defined above and A is hydrogen;

(f) deprotecting the hydroxyethyl functionality of the piperidine protected hydroxyethylphenylalcohol with an appropriate deprotecting reagent to produce a piperidine hydroxyethylphenylalcohol of the formula

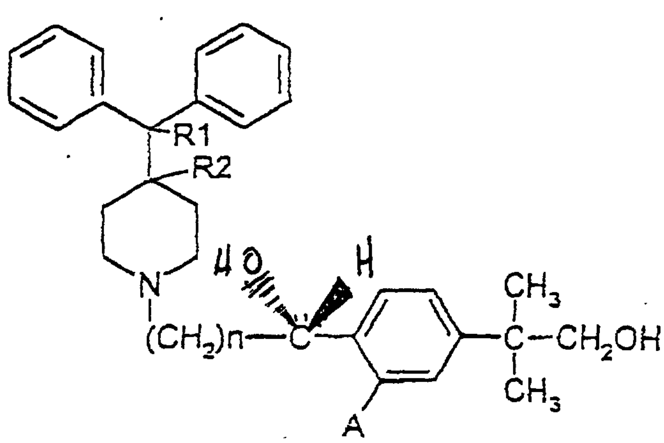

wherein $R_1$, $R_2$ and n are defined above and A is hydrogen.

**30.** A process for preparing a compound of the formula

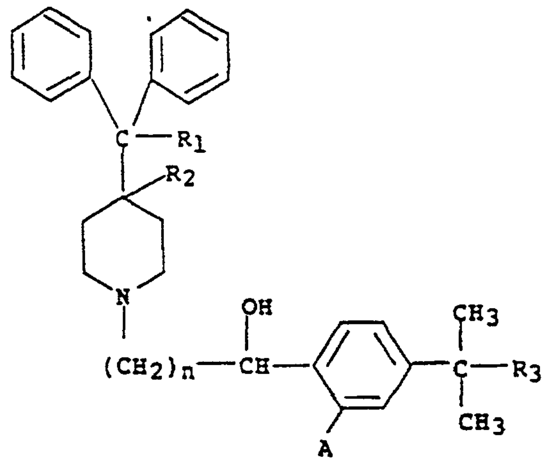

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is -COOH or -COOalkyl wherein the alkyl moiety has from 1 to 6 carbon atoms and is straight or branched;
A is hydrogen; and
pharmaceutically acceptable salts thereof comprising the steps of:

(a) reacting a benzeneacetic acid compound of the formula

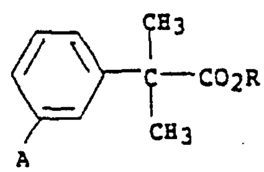

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

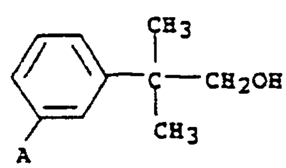

wherein A is hydrogen;

(b) protecting the hydroxyethyl functionality with a suitable protecting group to give a protected phenethyl alcohol of the formula

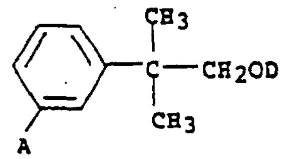

wherein A is hydrogen; and D is a suitable protecting group;

(c) reacting the protected phenethyl alcohol with a ω-halo compound of the formula

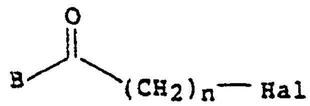

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

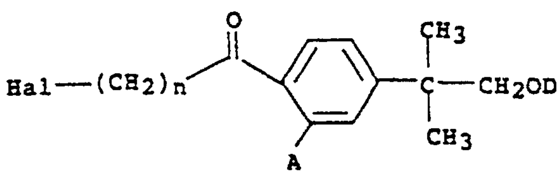

wherein Hal, n and D are defined above and A is hydrogen;

(d) reacting the ω-halo protected hydroxyethylphenylketone with a piperidine compound of the formula

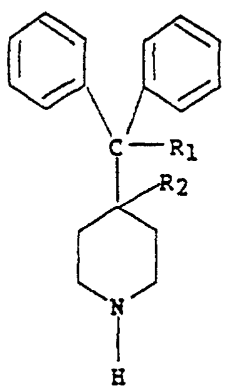

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

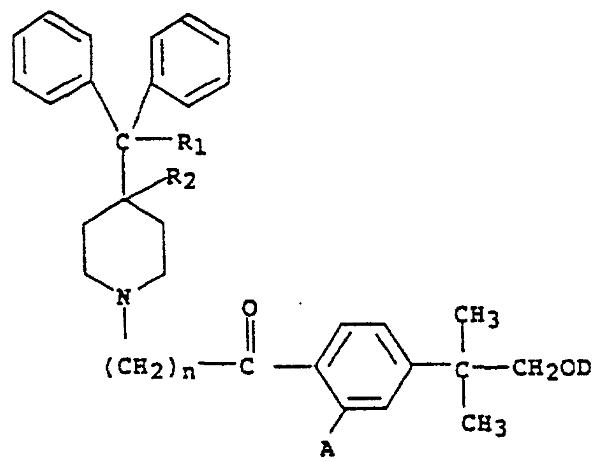

wherein $R_1$, $R_2$, n and D are defined above and A is hydrogen;

(e) reacting the piperidine protected hydroxyethylphenylketone with sodium. borohydride, potassium borohydride, sodium cyanoborohydride, tetramethylammonium borohydride, lithium tri-tert-butylaluminohydride, diisobutylaluminum hydride or catalyitc hydrogenation to produce a piperidine protected hydroxyethylphenylalcohol of the formula

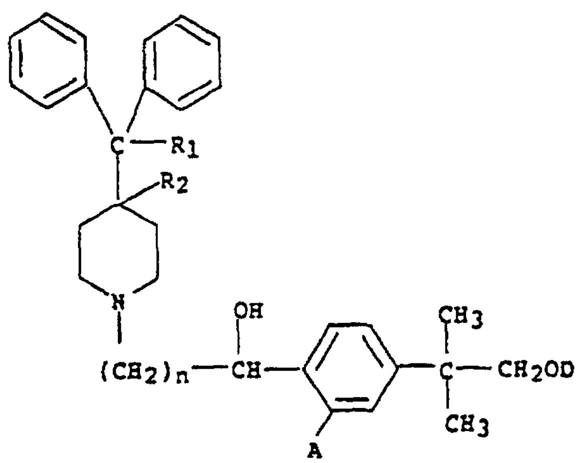

wherein $R_1$, $R_2$, D and n are defined above and A is hydrogen;

(f) deprotecting the hydroxyethyl functionality of the piperidine protected hydroxyethylphenylalcohol with an appropriate deprotecting reagent to produce a piperidine hydroxyethylphenylalcohol of the formula

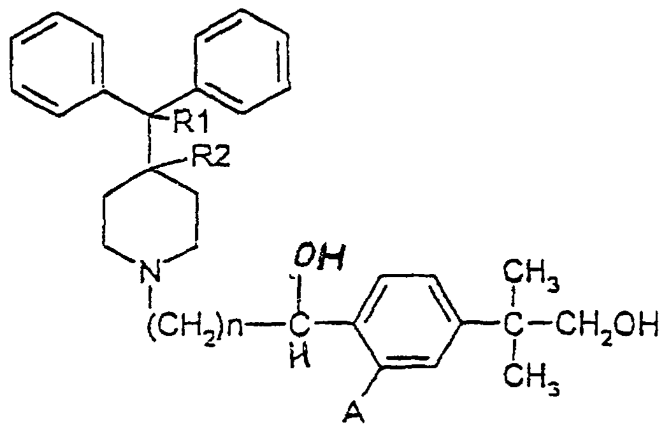

wherein $R_1$, $R_2$ and n are defined above and A is hydrogen;

(g) reacting the piperidine hydroxyethylphenylalcohol with dimethyl sulfoxide, oxyalyl chloride and triethylamine or with Dess-Martin reagent, chromium (IV) oxide, nickel peroxide, sodium dichromate, potassium dichromate, t-butyl chromate, silver oxide, argentic picolinate manganese dioxide lead tetraacetate, dicyclohexylcarbodiimide, 2,3-dichloro-5,6-dicyanoquinone, tetrachloro-1,2-benzoquinone, 2,2,6,6-tetramethylpiperidinyl-1-oxy (TEMPO) or quinolinium chlorochromate to produce a piperidine formylphenylalcohol of the formula

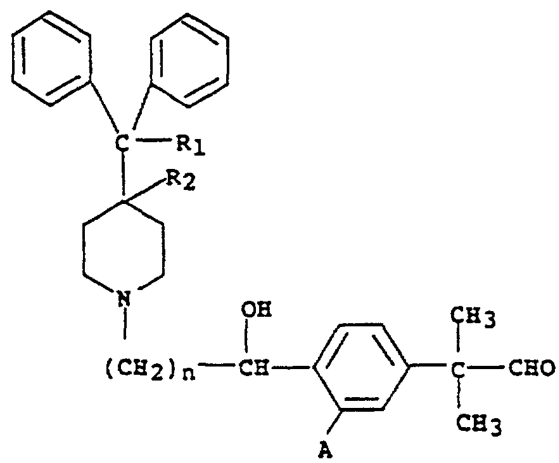

wherein $R_1$, $R_2$ and n are defined above and A is hydrogen;

(h) reacting the piperidine formylphenylalcohol with potassium permanganate, chromium (IV) oxide, silver (I) oxide, silver oxide, argentic picolinate, peroxide, nitric acid, m-chloroperbenzoic acid and peracetic acid to produce a piperidine carboxyphenylalcohol of the formula

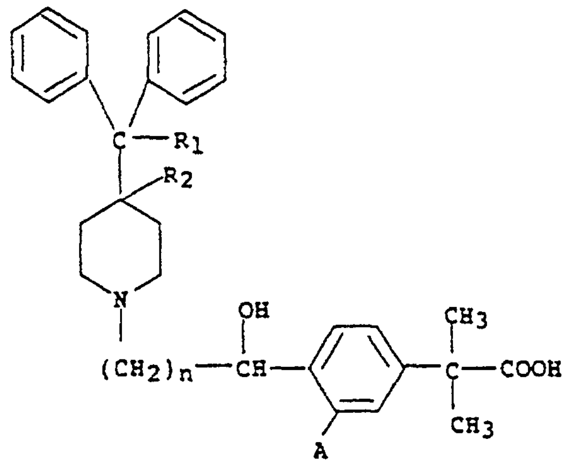

wherein $R_1$, $R_2$ and n are defined above and A is hydrogen;

(i) optionally reacting the piperidine carboxyphenylalcohol with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylalcohol ester of the formula

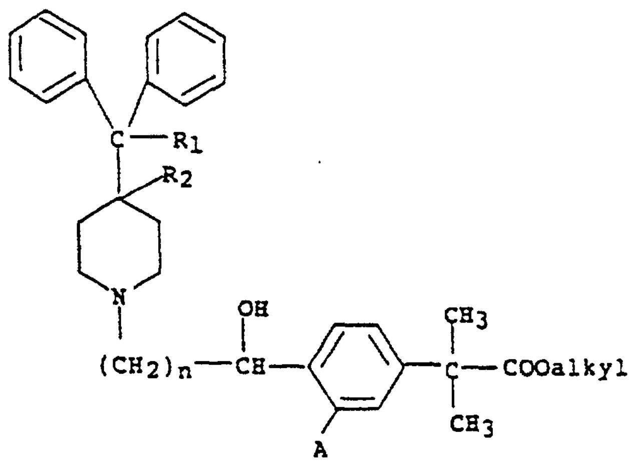

wherein $R_1$, $R_2$, n and alkyl are defined above and A is hydrogen.

**31.** A process for preparing a compound of the formula

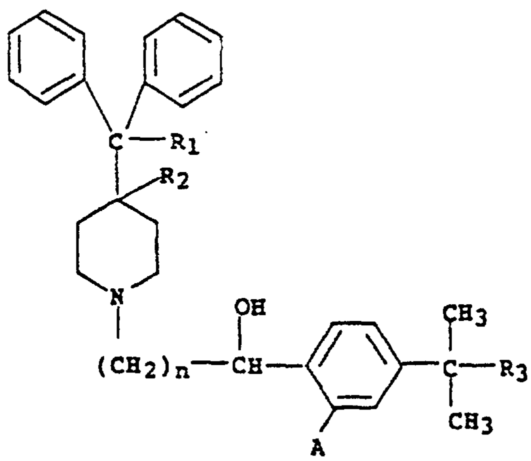

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is -COOH or -COOalkyl wherein the alkyl moiety has from 1 to 6 carbon atoms and is straight or branched;
A is hydrogen; and
pharmaceutically acceptable salts thereof comprising the steps of:

(a) reacting a benzeneacetic acid compound of the formula

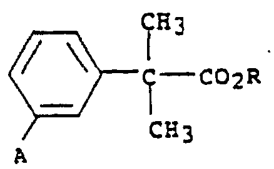

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

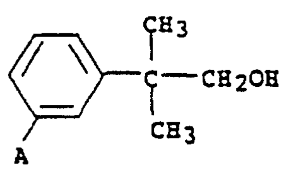

wherein A is hydrogen;

(b) protecting the hydroxyethyl functionality with a suitable protecting group to give a protected phenethyl alcohol of the formula

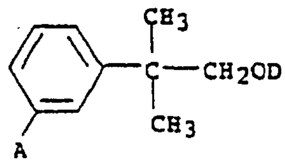

wherein A is hydrogen and D is a suitable protecting group;

(c) reacting the protected phenethyl alcohol with a ω-halo compound of the formula

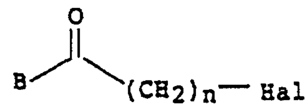

wherein B is halo or hydroxy, Hal represents Cl, Br or I and n is as defined above, in the presence of trichloride, aluminum chloride, titanium tetrachloride, boron trifluoride, tin tetrachloride and zinc chloride to produce a ω-halo protected hydroxyethylphenylketone of the formula

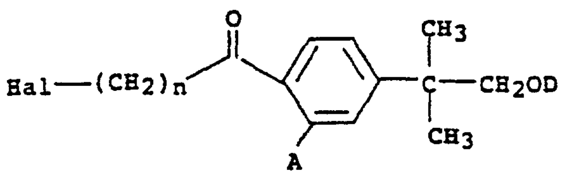

wherein Hal, n and D are defined above and A is hydrogen;

(d) reacting the ω-halo protected hydroxyethylphenylketone with a piperidine compound of the formula

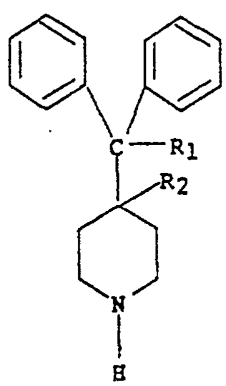

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

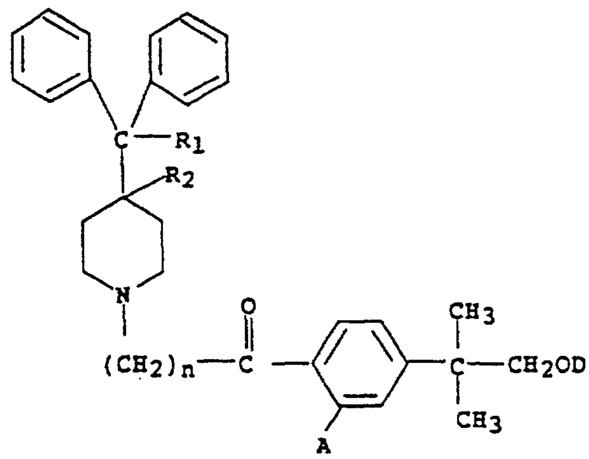

wherein $R_1$, $R_2$, n and D are defined above and A is hydrogen;

(e) reacting the piperidine protected hydroxyethylphenylketone with sodium borohydride, potassium borohydride, sodium cyanoborohydride, tetramethylammonium borohydride, lithium tri-tert-butylaluminohydride, diisobutylaluminum hydride or catalyitc hydrogenation to produce a piperidine protected hydroxyethylphenylalcohol of the formula

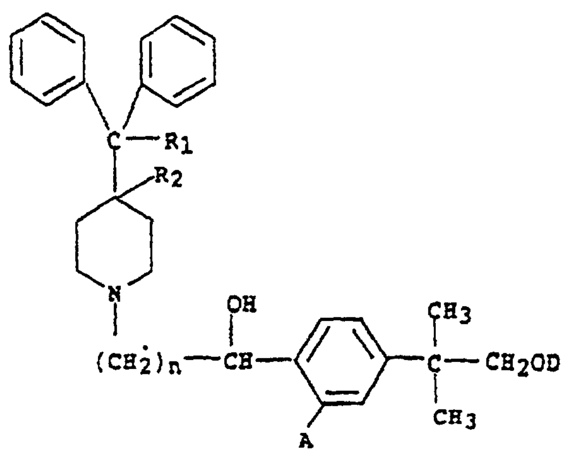

wherein $R_1$, $R_2$, D and n are defined above and A is hydrogen;

(f) deprotecting the hydroxyethyl functionality of the piperidine protected hydroxyethylphenylalcohol with an appropriate deprotecting reagent to produce a piperidine hydroxyethylphenylalcohol of the formula

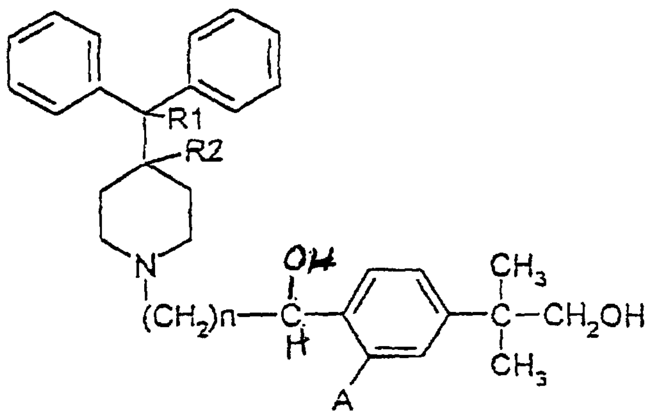

wherein $R_1$, $R_2$ and n are defined above and A is hydrogen;

(g) reacting the piperidine hydroxyethylphenylalcohol with chromium (IV) oxide, potassium permanganate, nitric acid, nitrogen dioxide, ruthenium (VIII) oxide, nickel peroxide, silver oxide, t-butyl chromate or xenic acid to produce a piperidine carboxyphenylalcohol of the formula

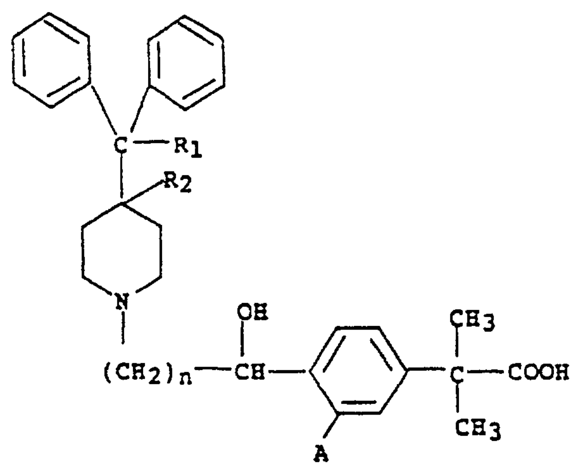

wherein $R_1$, $R_2$ and n are defined above and A is hydrogen;

(h) optionally reacting the piperidine carboxyphenylalcohol with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylalcohol ester of the formula

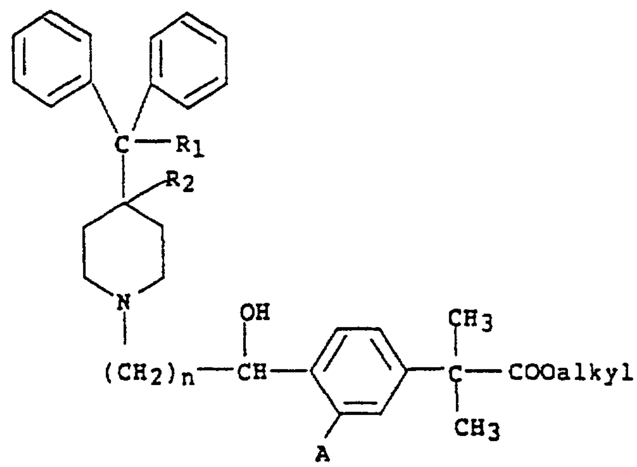

wherein $R_1$, $R_2$, n and alkyl are defined above and A is hydrogen.

**32.** A process according to Claim 31 wherein the piperidine hydroxyethylphenylalcohol produced in step f is reacted with ruthenium (VIII) oxide to produce the piperidine carboxyphenylketone of step g.

**33.** A process for preparing a compound of the formula

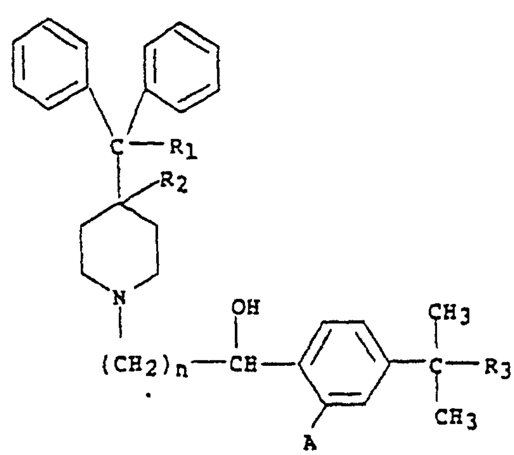

wherein

$R_1$ represents hydrogen or hydroxy;

$R_2$ represents hydrogen; or

$R_1$ and $R_2$ taken together form a second bond between the carbon atoms bearing $R_1$ and $R_2$;

n is an integer of from 1 to 5;

A is hydrogen;

$R_3$ is $-CH_2OH$; and

pharmaceutically acceptable salts thereof comprising the steps of:

(a) reacting a benzeneacetic acid compound of the formula

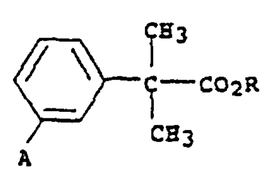

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

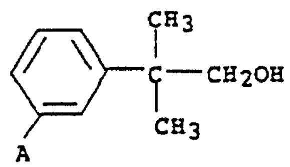

(b) protecting the hydroxyethyl functionality with a suitable protecting group to give a protected phenethyl alcohol of the formula

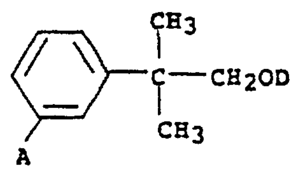

wherein A is hydrogen and D is a suitable protecting group;

(c) reacting the protected phenethyl alcohol with a ω-halo compound of the formula

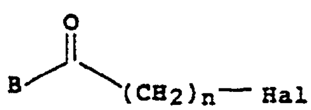

wherein B is halo or hydroxy, Hal represents Cl, Br or I and n is as defined above, in the presence of trichloride, aluminum chloride, titanium tetrachloride, boron trifluoride, tin tetrachloride and zinc chloride to produce a ω-halo protected hydroxyethylphenylketone of the formula

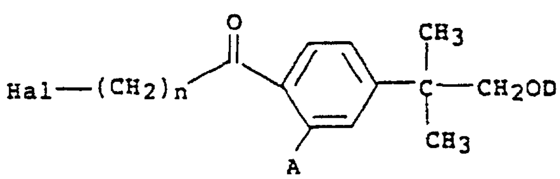

wherein Hal, n and D are defined above and A is hydrogen

(d) reacting the ω-halo protected hydroxyethylphenylketone with a piperidine compound of the formula

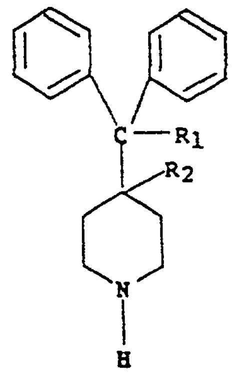

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

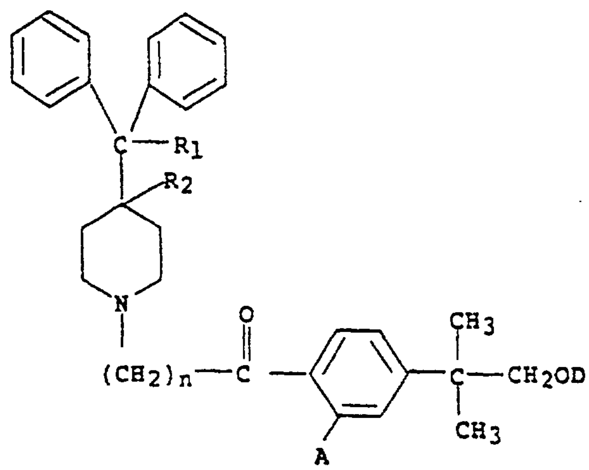

wherein $R_1$, $R_2$, n and D are defined above and A is hydrogen

(e) reacting the piperidine protected hydroxyethylphenylketone with sodium borohydride, potassium borohydride, sodium cyanoborohydride, tetramethylammonium borohydride, lithium tri-tert-butylaluminohydride, diisobutylaluminum hydride or catalyitc hydrogenation to produce a piperidine protected hydroxyethylphenylalcohol of the formula

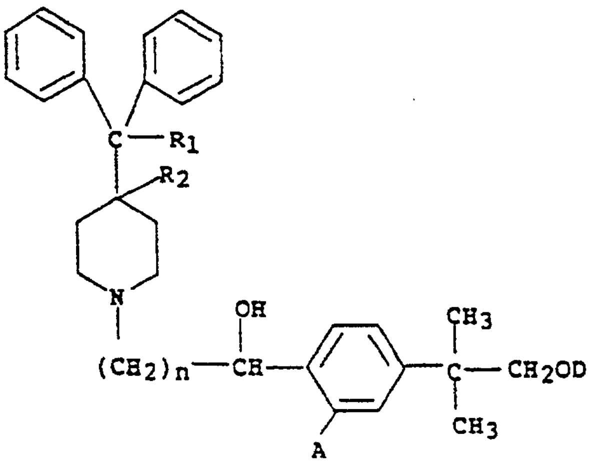

wherein $R_1$, $R_2$, n and D are defined above and A is hydrogen;

(f) deprotecting the hydroxyethyl functionality of the piperidine protected hydroxyethylphenylalcohol with an appropriate deprotecting reagent to produce a piperidine hydroxyethylphenylalcohol of the formula

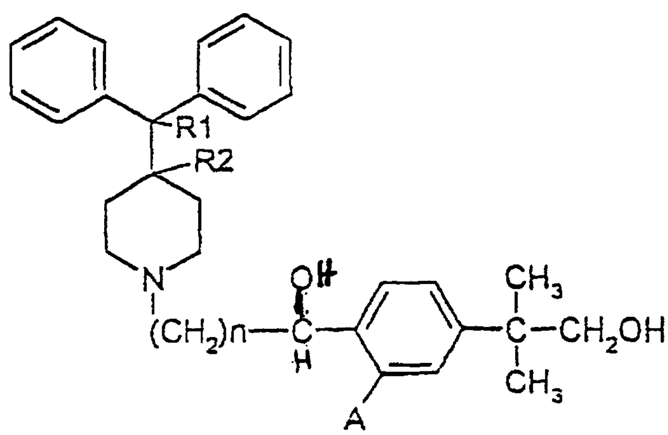

wherein $R_1$, $R_2$ and n are defined above and A is hydrogen.

**34.** A process for preparing a compound of the formula wherein
$R_1$ represents hydrogen or hydroxy;
$R_2$ represents hydrogen; or

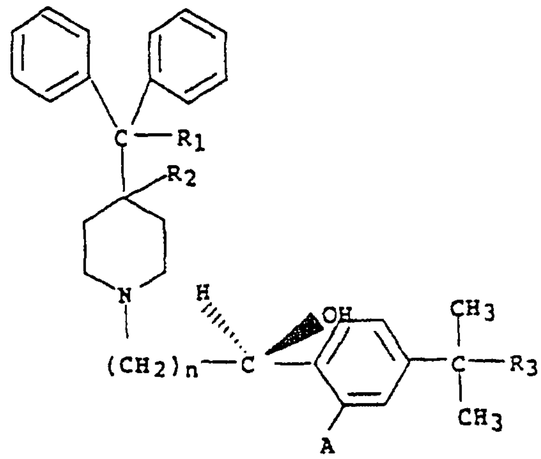

$R_1$ and $R_2$ taken together form a second bond between the carbon atoms bearing $R_1$ and $R_2$;
n is an integer of from 1 to 5;
$R_3$ is -COOH or -COOalkyl wherein the alkyl moiety has from 1 to 6 carbon atoms and is straight or branched;
A is hydrogen; and
pharmaceutically acceptable salts thereof comprising the steps of:

(a) reacting a benzeneacetic acid compound of the formula

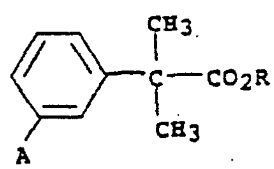

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

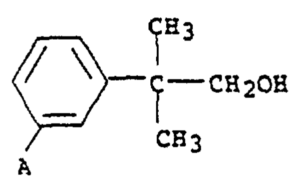

wherein A is hydrogen;

(b) protecting the hydroxyethyl functionality with a suitable protecting group to give a protected phenethyl alcohol of the formula

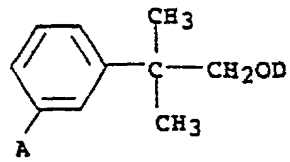

wherein A is hydrogen and D is a suitable protecting group;

(c) reacting the protected phenethyl alcohol with a ω-halo compound of the formula

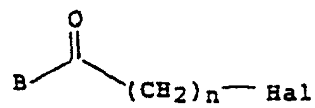

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

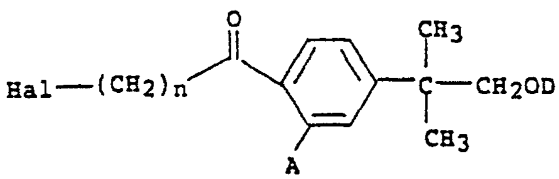

wherein Hal, n and D are defined above and A is hydrogen

(d) reacting the ω-halo protected hydroxyethylphenylketone with a piperidine compound of the formula

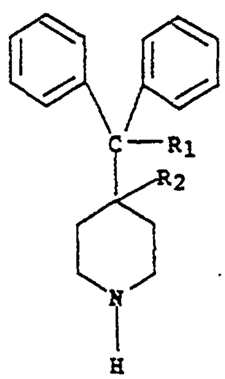

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

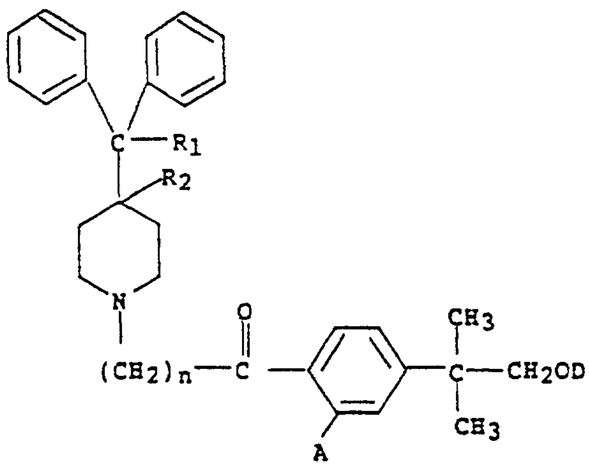

wherein $R_1$, $R_2$, n and D are defined above and A is hydrogen;

(e) reacting the piperidine protected hydroxyethylphenylketone with (+)-B-chlorodiisopinocamphenylborane to produce a piperidine protected hydroxyethylphenylalcohol of the formula

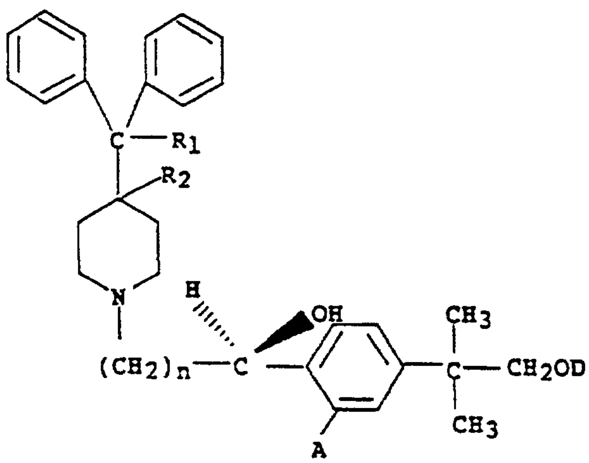

wherein $R_1$, $R_2$, D and n are defined above and A is hydrogen

(f) deprotecting the hydroxyethyl functionality of the piperidine protected hydroxyethylphenylalcohol with an appropriate deprotecting reagent to produce a piperidine hydroxyethylphenylalcohol of the formula

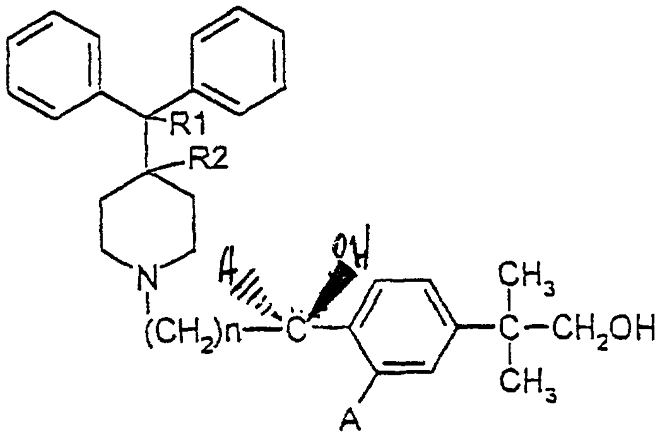

wherein $R_1$, $R_2$ and n are defined above and A is hydrogen;

(g) reacting the piperidine hydroxyethylphenylalcohol with dimethyl sulfoxide, oxyalyl chloride and triethylamine or with Dess-Martin reagent, chromium (IV) oxide, nickel peroxide, sodium dichromate, potassium dichromate, t-butyl chromate, silver oxide, argentic picolinate manganese dioxide lead tetraacetate, dicyclohexylcarbodiimide, 2,3-dichloro-5,6-dicyanoquinone, tetrachloro-1,2-benzoquinone, 2,2,6,6-tetramethylpiperidinyl-1-oxy (TEMPO) or quinolinium chlorochromate to produce a piperidine formylphenylalcohol of the formula

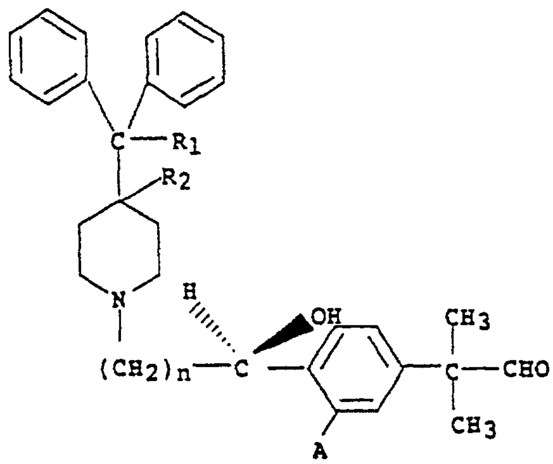

wherein $R_1$, $R_2$ and n are defined above and A is hydrogen;

(h) reacting the piperidine formylphenylalcohol with potassium permanganate, chromium (IV) oxide, silver (I) oxide, silver oxide, argentic picolinate, peroxide, nitric acid, m-chloroperbenzoic acid and peracetic acid to produce a piperidine carboxyphenylalcohol of the formula

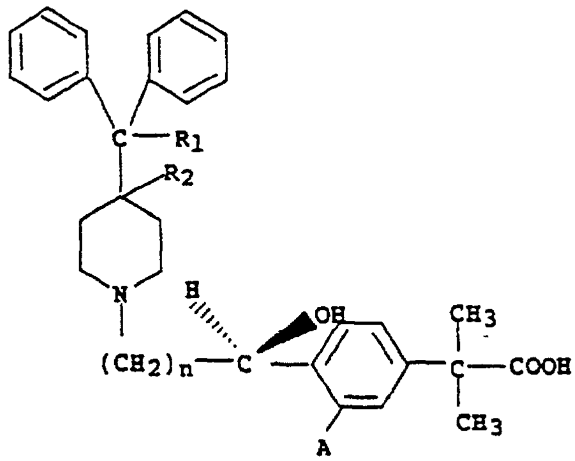

wherein $R_1$, $R_2$ and n are defined above and A is hydrogen

(i) optionally reacting the piperidine carboxyphenylalcohol with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylalcohol ester of the formula

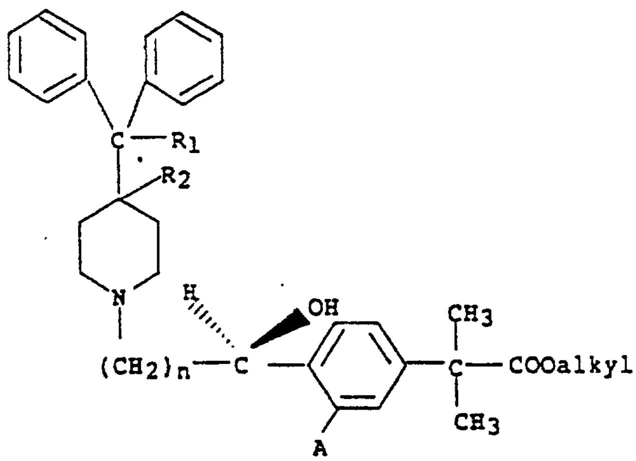

wherein $R_1$, $R_2$, n and alkyl are defined above and A is hydrogen.

**35.** A process for preparing a compound of the formula

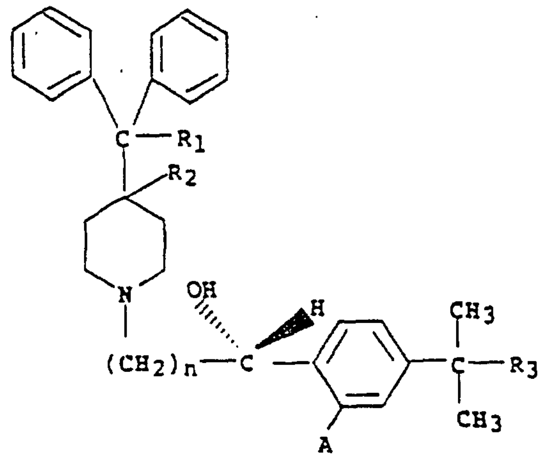

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
$R_3$ is -COOR or -COOalkyl wherein the alkyl moiety has from 1 to 6 carbon atoms and is straight or branched;
A is hydrogen; and
pharmaceutically acceptable salts thereof comprising the steps of:

(a) reacting a benzeneacetic acid compound of the formula

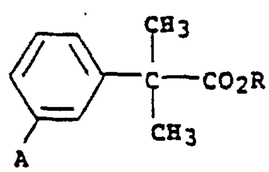

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

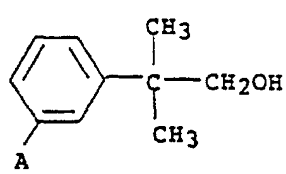

wherein A is hydrogen;

(b) protecting the hydroxyethyl functionality with a suitable protecting group to give a protected phenethyl alcohol of the formula

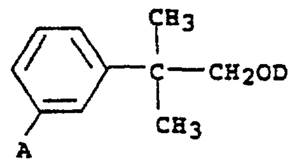

wherein A is hydrogen and D is a suitable protecting group;

(c) reacting the protected phenethyl alcohol with a ω-halo compound of the formula

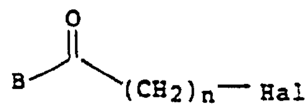

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

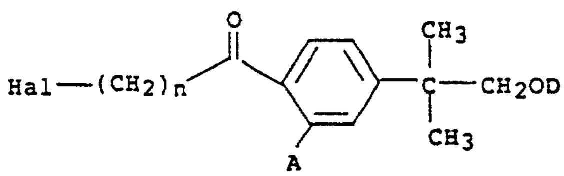

wherein Hal, n and D are defined above and A is hydrogen

(d) reacting the ω-halo protected hydroxyethylphenylketone with a piperidine compound of the formula

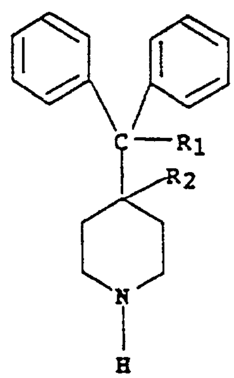

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

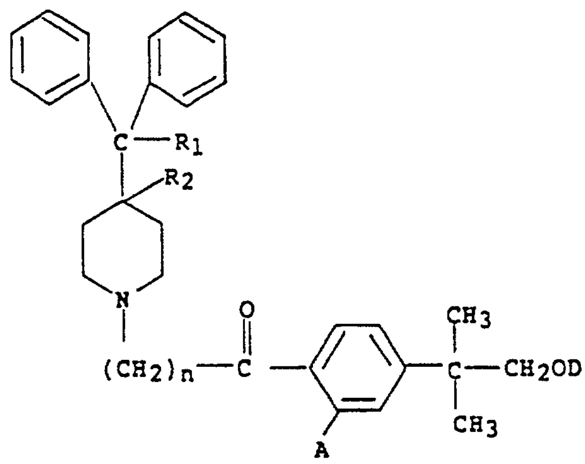

wherein $R_1$, $R_2$, n and D are defined above and A is hydrogen;

(e) reacting the piperidine protected hydroxyethylphenylketone with (-)-B-chlorodiisopinocamphenylborane to produce a piperidine protected hydroxyethylphenylalcohol of the formula

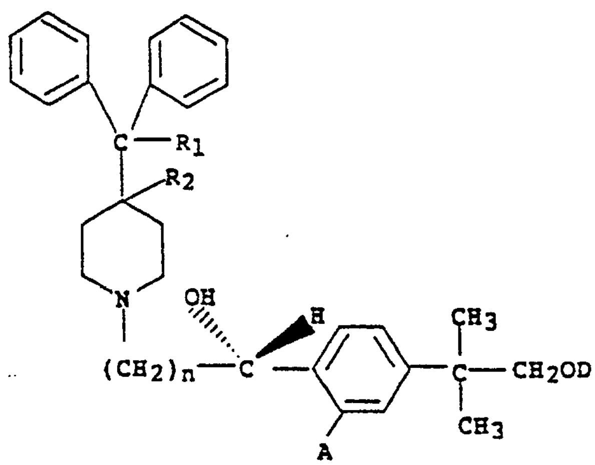

wherein $R_1$, $R_2$, D and n are defined above and A is hydrogen;

(f) deprotecting the hydroxyethyl functionality of the piperidine protected hydroxyethylphenylalcohol with an appropriate deprotecting reagent to produce a piperidine hydroxyethylphenylalcohol of the formula

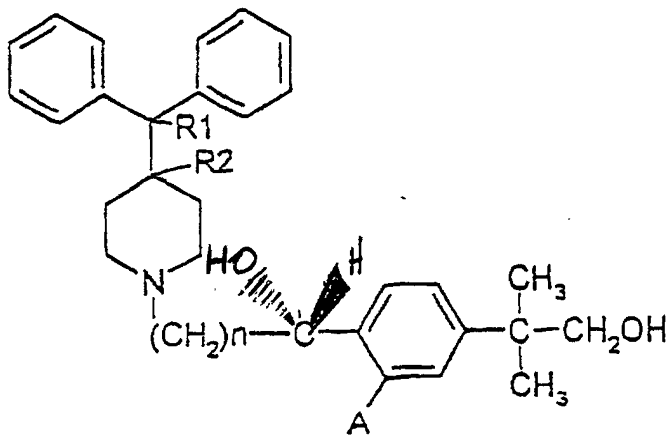

wherein $R_1$, $R_2$ and n are defined above and A is hydrogen;

(g) reacting the piperidine hydroxyethylphenylalcohol with dimethyl sulfoxide, oxyalyl chloride and triethylamine or with Dess-Martin reagent, chromium (IV) oxide, nickel peroxide, sodium dichromate, potassium dichromate, t-butyl chromate, silver oxide, argentic picolinate manganese dioxide lead tetraacetate, dicyclohexylcarbodiimide, 2,3-dichloro-5,6-dicyanoquinone, tetrachloro-1,2-benzoquinone, 2,2,6,6-tetramethylpiperidinyl-1-oxy (TEMPO) or quinolinium chlorochromate to produce a piperidine formylphenylalcohol of the formula

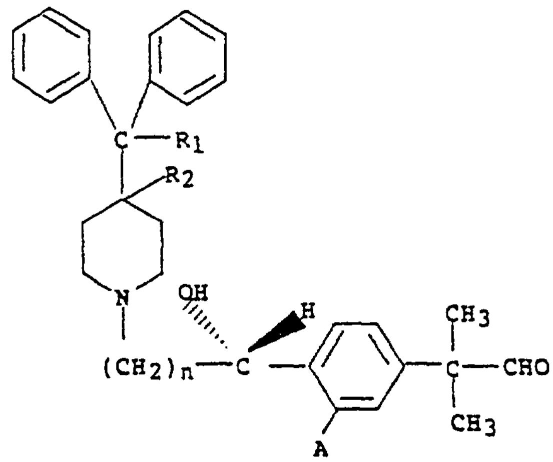

wherein $R_1$, $R_2$ and n are defined above and A is hydrogen;

(h) reacting the piperidine formylphenylalcohol with potassium permanganate, chromium (IV) oxide, silver (I) oxide, silver oxide, argentic picolinate, peroxide, nitric acid, m-chloroperbenzoic acid and peracetic acid to produce a piperidine carboxyphenylalcohol of the formula

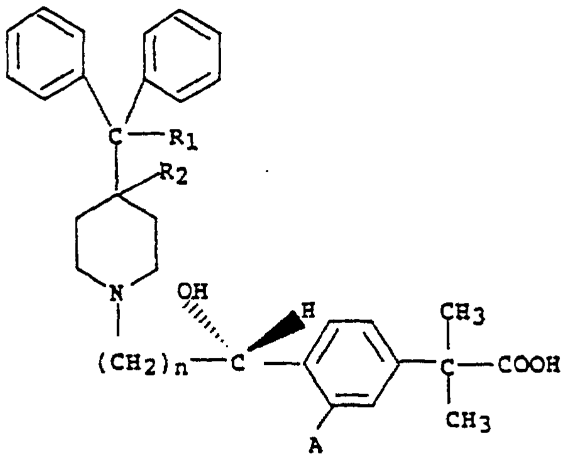

wherein $R_1$, $R_2$ and n are defined above and A is hydrogen

(i) optionally reacting the piperidine carboxyphenylalcohol with an excess of diazomethane or an excess of 2,2-dimethoxypropane or with thionyl chloride followed by an alcohol of the formula HOalkyl wherein the alkyl moiety has from 1 to 6 carbon atoms and is straight or branched to form a piperidine carboxyphenylalcohol ester of the formula

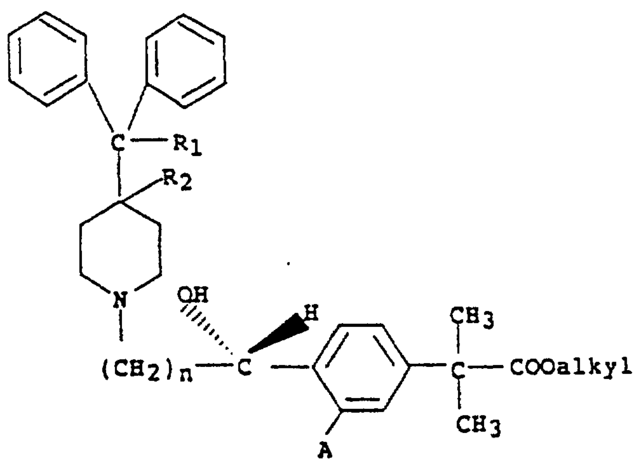

wherein $R_1$, $R_2$, n and alkyl are defined above and A is hydrogen.

**36.** A process for preparing a compound of the formula

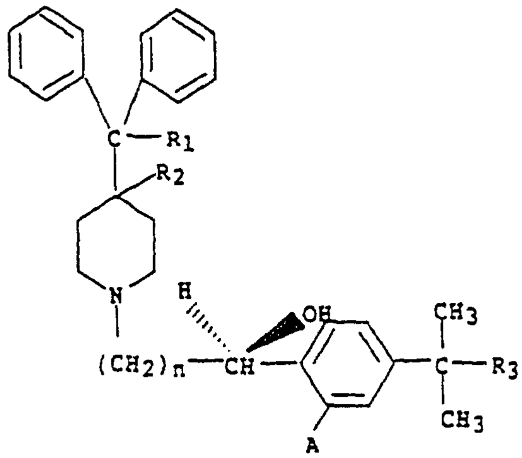

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
A is hydrogen;
$R_3$ is $-CH_2OH$; and
pharmaceutically acceptable salts thereof comprising the steps of:

(a) reacting a benzeneacetic acid compound of the formula

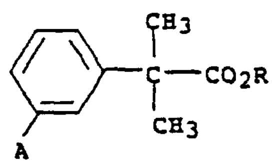

wherein A is hydrogen and R is hydrogen or $C_1$-$C_6$ alkyl with sodium bis(2-methoxyethoxy)aluminium hydride, lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

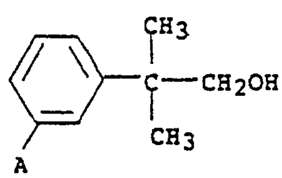

(b) protecting the hydroxyethyl functionality with a suitable protecting group to give a protected phenethyl alcohol of the formula

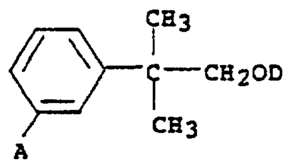

wherein A is hydrogen and D is a suitable protecting group;

(c) reacting the protected phenethyl alcohol with a ω-halo compound of the formula

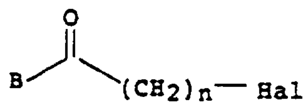

wherein B is halo or hydroxy, Hal represents Cl, Br or I and n is as defined above, in the presence of trichloride, aluminum chloride, titanium tetrachloride, boron trifluoride, tin tetrachloride and zinc chloride to produce a ω-halo protected hydroxyethylphenylketone of the formula

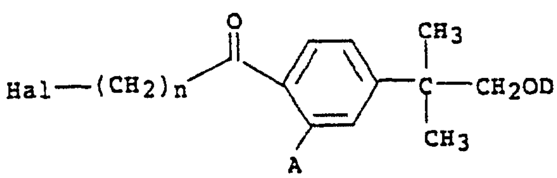

wherein Hal, n and D are defined above and A is hydrogen;

(d) reacting the ω-halo protected hydroxyethylphenylketone with a piperidine compound of the formula

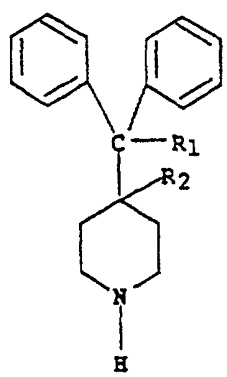

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

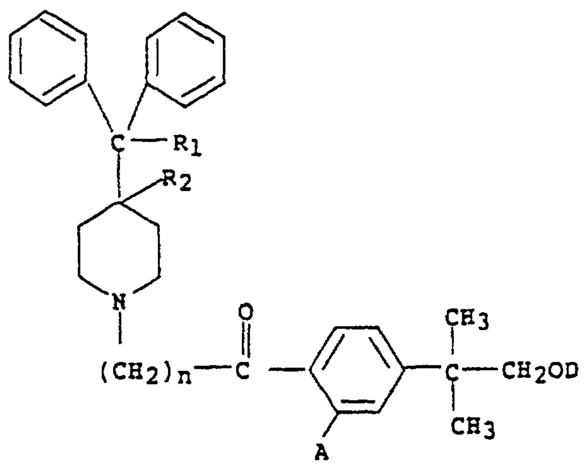

wherein $R_1$, $R_2$, n and D are defined above and A is hydrogen;

(e) reacting the piperidine protected hydroxyethylphenylketone with (+)-B-chlorodiisopinocamphenylborane to produce a piperidine protected hydroxyethylphenylalcohol of the formula

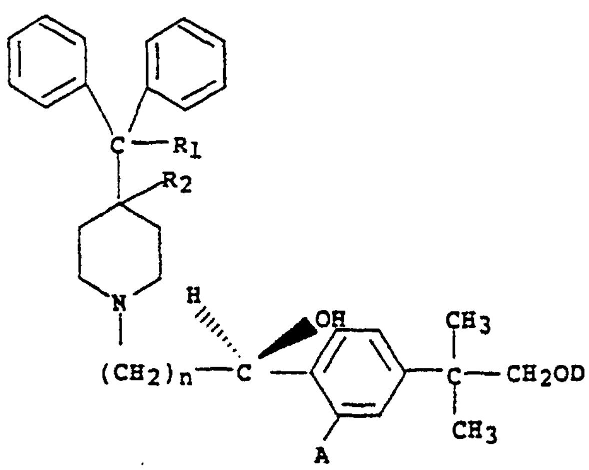

wherein $R_1$, $R_2$, n and D are defined above and A is hydrogen

(f) deprotecting the hydroxyethyl functionality of the piperidine protected hydroxyethylphenylalcohol with an appropriate deprotecting reagent to produce a piperidine hydroxyethylphenylalcohol of the formula

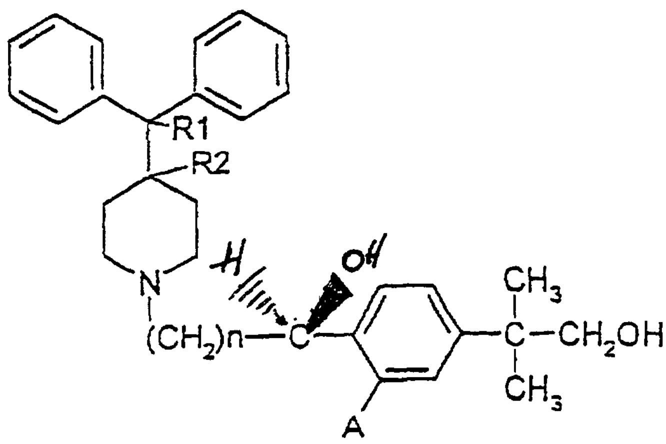

wherein $R_1$, $R_2$ and n are defined above and A is hydrogen.

**37.** A process for preparing a compound of the formula

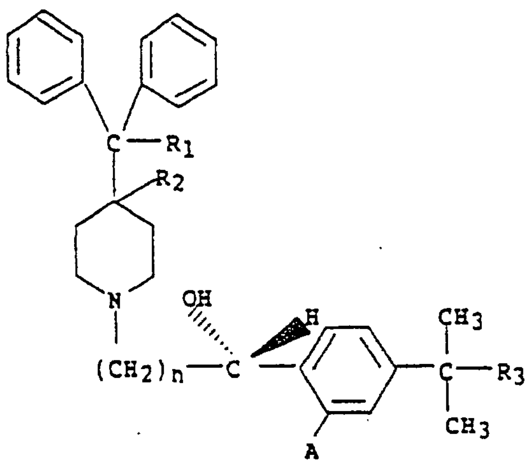

wherein

$R_1$ represents hydrogen or hydroxy;
$R_2$ represents hydrogen; or
$R_1$ and $R_2$ taken together form a second bond between the carbon atoms bearing $R_1$ and $R_2$;
n is an integer of from 1 to 5;
A is hydrogen;
$R_3$ is $-CH_2OH$; and
pharmaceutically acceptable salts thereof comprising the steps of:

(a) reacting a benzeneacetic acid compound of the formula

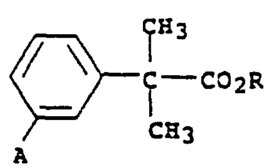

wherein A is hydrogen and R is hydrogen or $C_1$-$C_6$ alkyl with sodium bis(2-methoxyethoxy)aluminium hydride,

lithium aluminium hydride, diborane, or aluminium hydride when R is hydrogen and with sodium bis(2-methoxyethoxy)aluminium hydride, lithium aluminium hydride, lithium borohydride, lithium treiethylborohydride, lithium tri-sec-butylborohydride, or aluminium hydride when R is $C_1$-$C_6$ alkyl to give a phenethyl alcohol of the formula

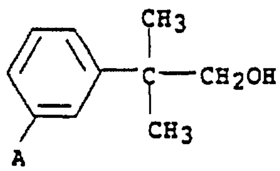

(b) protecting the hydroxyethyl functionality with a suitable protecting group to give a protected phenethyl alcohol of the formula

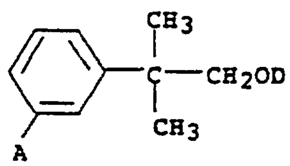

wherein A is hydrogen and D is a suitable protecting group;

(c) reacting the protected phenethyl alcohol with a ω-halo compound of the formula

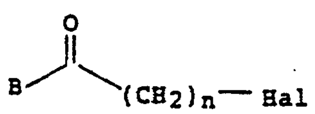

wherein B is halo or hydroxy, Hal represents Cl, Br or I and n is as defined above, in the presence of boron trichloride, aluminum chloride, titanium tetrachloride, boron trifluoride, tin tetrachloride and zinc chloride to produce a ω-halo protected hydroxyethylphenylketone of the formula

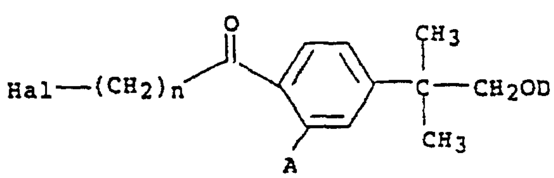

wherein Hal, n and D are defined above and A is hydrogen;

(d) reacting the ω-halo protected hydroxyethylphenylketone with a piperidine compound of the formula

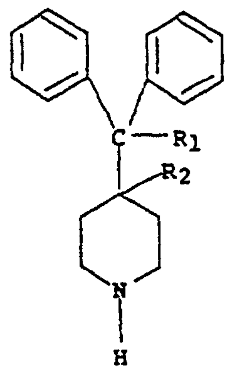

wherein $R_1$ and $R_2$ are as defined above in the presence of a sodium bicarbonate, potassium carbonate, potassium bicarbonate, triethylamine, pyridine, or an excess of the piperidine compound may be used to produce a piperidine protected hydroxyethylphenylketone of the formula

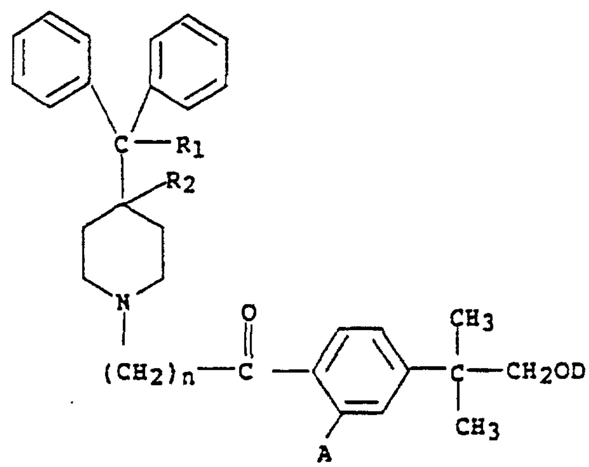

wherein $R_1$, $R_2$, n and D are defined above and A is hydrogen;

(e) reacting the piperidine protected hydroxyethylphenylketone with (-)-B-chlorodiisopinocamphenylborane to produce a piperidine protected hydroxyethylphenylalcohol of the formula

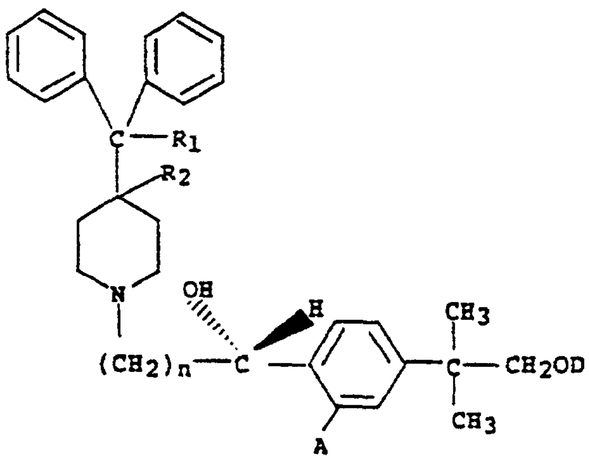

wherein $R_1$, $R_2$, n and D are defined above and A is hydrogen

(f) deprotecting the hydroxyethyl functionality of the piperidine protected hydroxyethylphenylalcohol with an appropriate deprotecting reagent to produce a piperidine hydroxyethylphenylalcohol of the formula

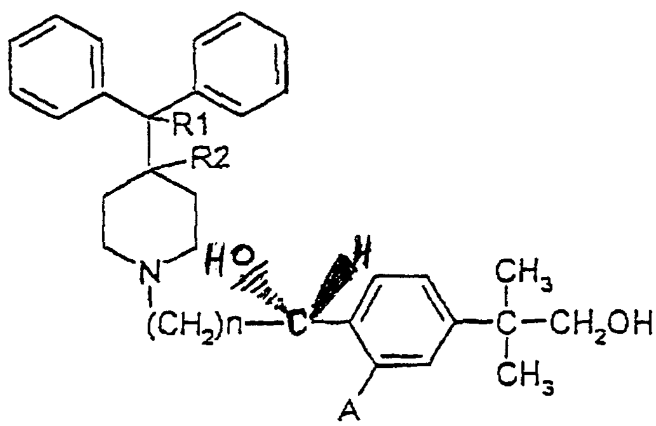

wherein $R_1$, $R_2$ and n are defined above and A is hydrogen.

**Patentansprüche**

1. Piperidinformylphenylalkoholverbindung der Formel

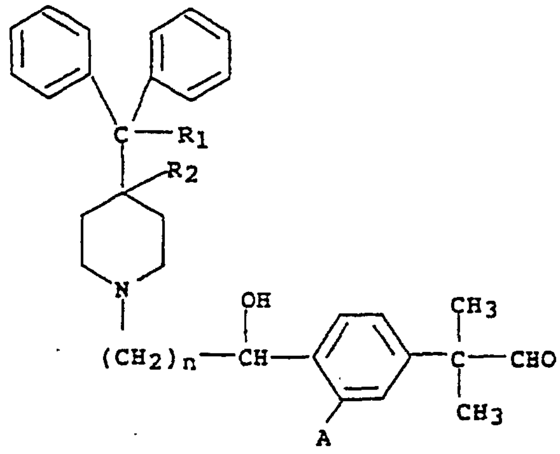

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist und
A ein Wasserstoffatom ist.

**2.** Piperidinformylphenylketonverbindung der Formel

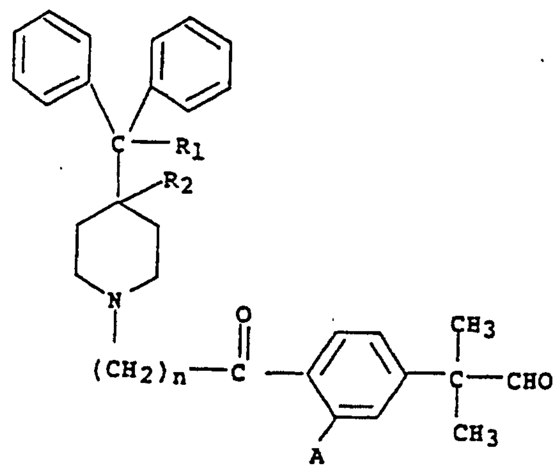

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist und
A ein Wasserstoffatom ist.

**3.** Geschützte Piperidinhydroxyethylphenylalkoholverbindung der Formel

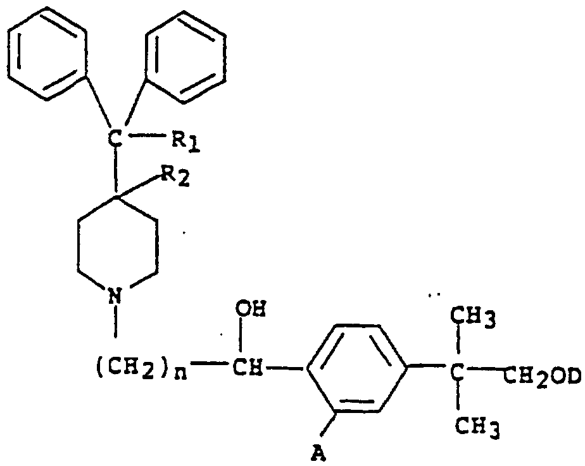

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
A ein Wasserstoffatom ist und
D die Gruppe $C(=O)CH_3$ oder $C(=O)C_6H_5$ ist.

**4.** Geschützte Piperidinhydroxyethylphenylketonverbindung der Formel

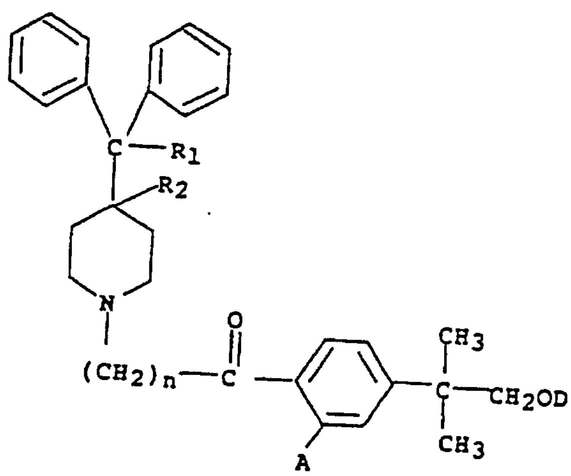

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
A ein Wasserstoffatom ist und
D die Gruppe $C(=O)CH_3$ oder $C(=O)C_6H_5$ ist.

**5.** Verfahren zur Herstellung einer Piperidinformylphenylalkoholverbindung der Formel

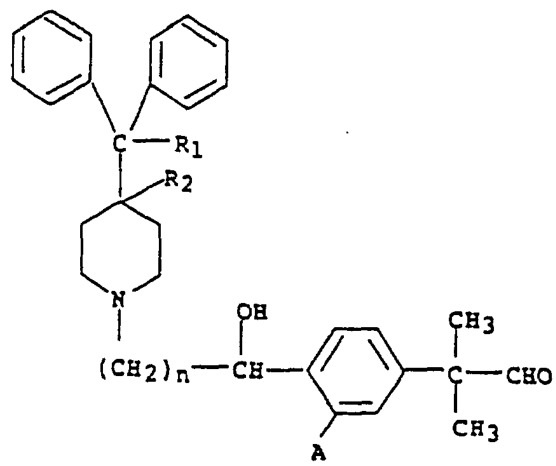

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist und
A ein Wasserstoffatom ist,
umfassend die Schritte:

(a) Umsetzen einer Benzolessigsäureverbindung der Formel

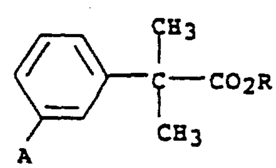

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

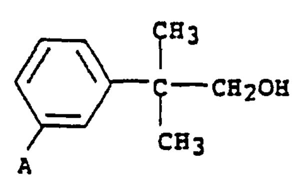

wobei A ein Wasserstoffatom ist, zu erhalten,

(b) Schützen der Hydroxyethylfunktionalität mit einer geeigneten Schutzgruppe, um einen geschützten Phenethylalkohol der Formel

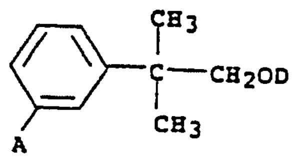

wobei A ein Wasserstoffatom ist und D eine geeignete Schutzgruppe ist, zu erhalten,

(c) Umsetzen des geschützten Phenethylalkohols mit einer ω-Halogenverbindung der Formel

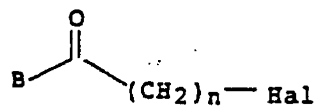

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

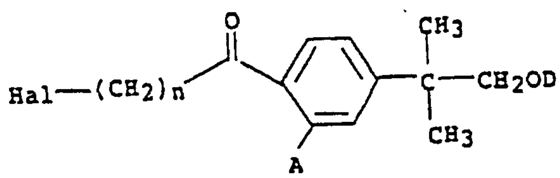

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

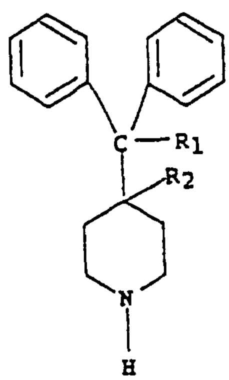

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

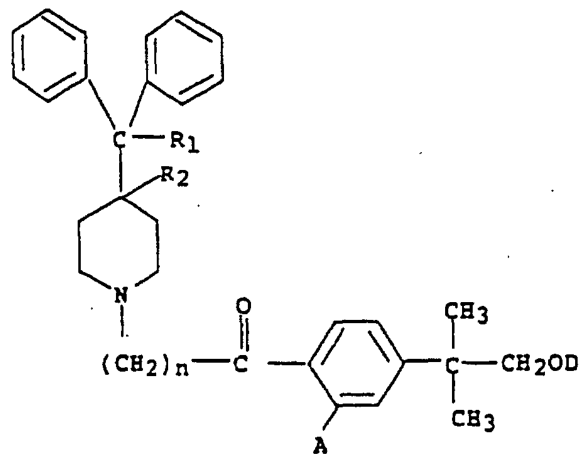

wobei $R_1$, $R_2$, n und D wie vorstehend definiert sind und A ein Wasserstoffatom ist, herzustellen,

(e) Umsetzen des geschützten Piperidinhydroxyethylphenylketons mit Natriumborhydrid, Kaliumborhydrid, Natriumcyanoborhydrid, Tetramethylammoniumborhydrid, Lithiumtri-tert-butylaluminohydrid, Diisobutylaluminiumhydrid oder katalytische Hydrierung, um einen geschützten Piperidinhydroxyethylphenylalkohol der Formel

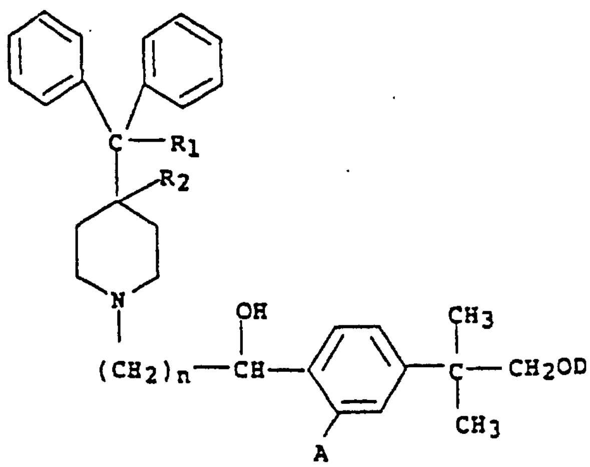

wobei $R_1$, $R_2$, D und n wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

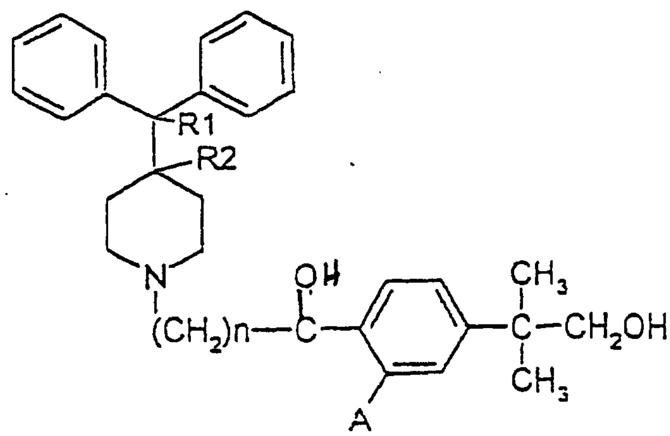

wobei $R_1$, $R_2$ und n wie vorstehend definiert sind und A ein Wasserstoffatom ist, herzustellen,

(g) Umsetzen des Piperidinhydroxyethylphenylalkohols mit Dimethylsulfoxid, Oxyalylchlorid und Triethylamin oder mit Dess-Martin-Reagenz, Chrom(IV)oxid, Nickelperoxid, Natriumdichromat, Kaliumdichromat, t-Butylchromat, Silberoxid, Silberpicolinatmangandioxidbleitetraacetat, Dicyclohexylcarbodiimid, 2,3-Dichlor-5,6-dicyanochinon, Tetrachlor-1,2-benzochinon, 2,2,6,6-Tetramethylpiperidinyl-1-oxy (TEMPO) oder Chinoliniumchlorchromat, um einen Piperidinformylphenylalkohol der Formel

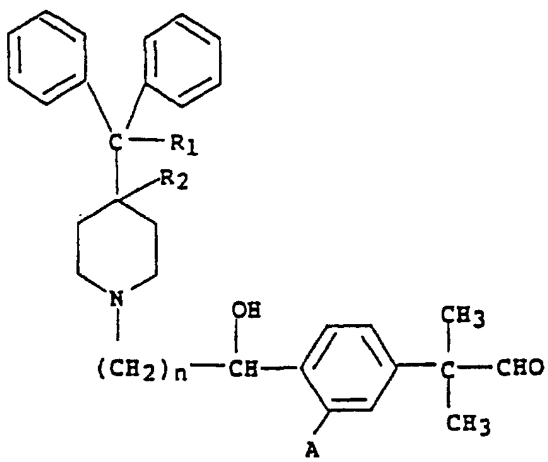

wobei $R_1$, $R_2$ und n wie vorstehend definiert sind und A ein Wasserstoffatom ist, herzustellen.

**6.** Verfahren zur Herstellung einer Piperidinformylphenylketonverbindung der Formel

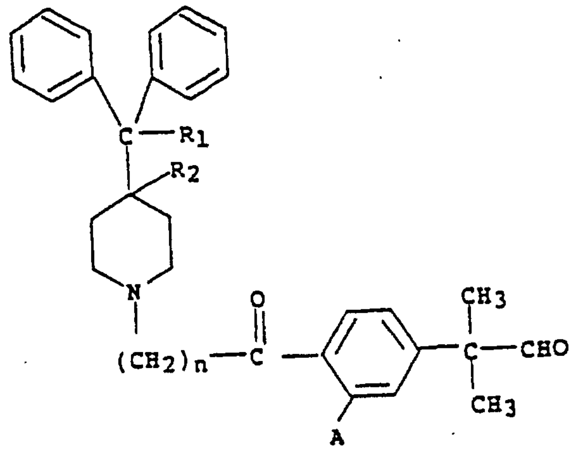

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist und
A ein Wasserstoffatom ist,
umfassend die Schritte:

(a) Umsetzen einer Benzolessigsäureverbindung der Formel

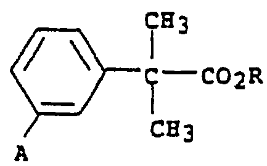

wobei A wie vorstehend definiert ist und R ein Wasserstoffatom oder ein $C_1$-$C_6$-Alkylrest ist, mit Natriumbis (2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

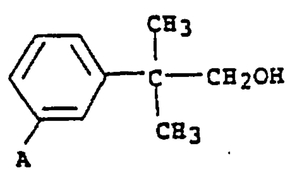

wobei A wie vorstehend definiert ist, zu erhalten,

(b) Schützen der Hydroxyethylfunktionalität mit einer geeigneten Schutzgruppe, um einen geschützten Phenethylalkohol der Formel

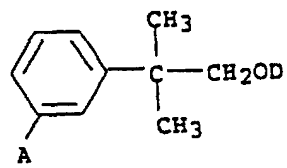

wobei A ein Wasserstoffatom ist und D eine geeignete Schutzgruppe ist, zu erhalten,

(c) Umsetzen des geschützten Phenethylalkohols mit einer ω-Halogenverbindung der Formel

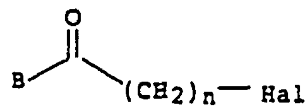

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

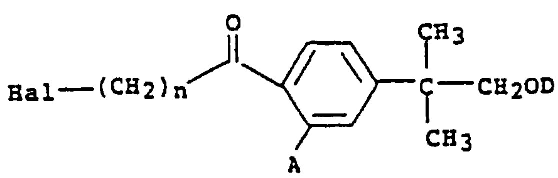

wobei Hal, n, A und D wie vorstehend definiert sind, herzustellen,

(d) Umsetzen des geschützten ω-Halogenhydroxyethylphenylketons mit einer Piperidinverbindung der Formel

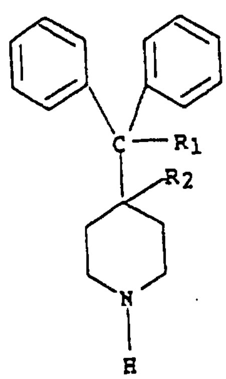

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um eine geschützte Piperidinhydroxyethylphenylketonverbindung der Formel

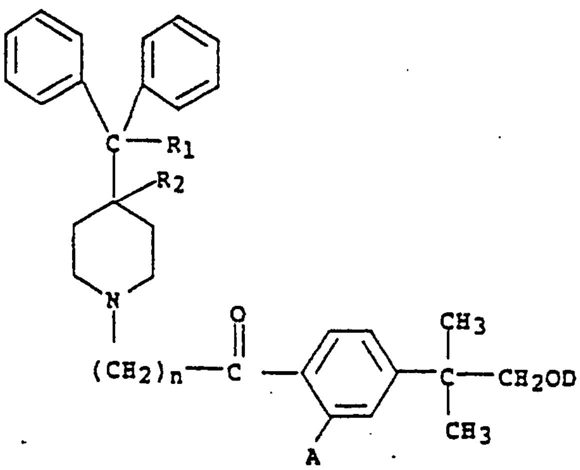

wobei $R_1$, $R_2$, n und A wie vorstehend definiert sind und D die Gruppe $-C(=O)CH_3$ oder $-C(=O)C_6H_5$ ist, herzustellen,

(e) Schutzgruppenabspaltung der Hydroxyethylfunktionalität des geschützten Piperidinhydroxyethylphenylketons mit einem geeigneten Reagenz zur Schutzgruppenabspaltung, um ein Piperidinhydroxyethylphenylketon der Formel

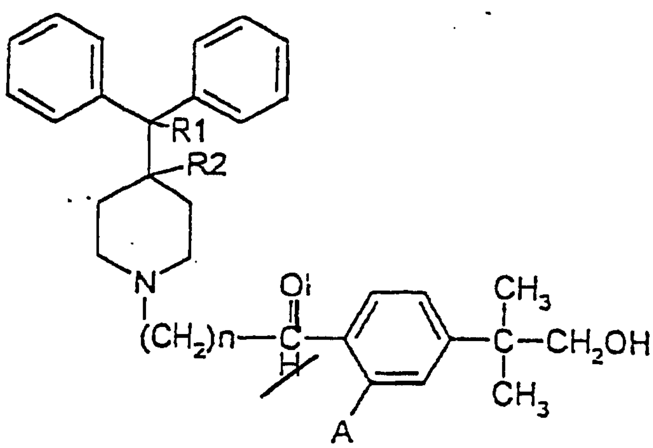

wobei $R_1$, $R_2$, n und A wie vorstehend definiert sind, herzustellen, und

(f) Umsetzen der Piperidinhydroxyethylphenylketonverbindung mit Dimethylsulfoxid, Oxyalylchlorid und Triethylamin oder mit Dess-Martin-Reagenz, Chrom(IV)oxid, Nickelperoxid, Natriumdichromat, Kaliumdichromat, t-Butylchromat, Silberoxid, Silberpicolinatmangandioxidbleitetraacetat, Dicyclohexylcarbodiimid, 2,3-Dichlor-5,6-dicyanochinon, Tetrachlor-1,2-benzochinon, 2,2,6,6-Tetramethylpiperidinyl-1-oxy (TEMPO) oder Chinoliniumchlorchromat, um ein Piperidinformylphenylketon herzustellen.

**7.** Verfahren zur Herstellung einer geschützten Piperidinhydroxyethylphenylalkoholverbindung der Formel

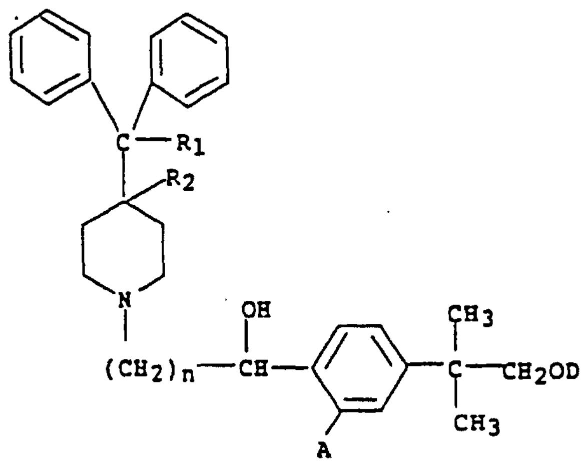

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
A ein Wasserstoffatom ist und
D die Gruppe $-C(=O)CH_3$ oder $-C(=O)C_6H_5$ ist,
umfassend die Schritte:

(a) Umsetzen einer Benzolessigsäureverbindung der Formel

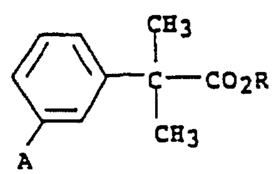

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

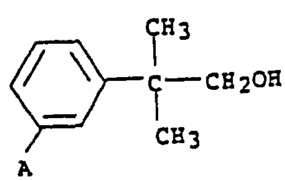

wobei A ein Wasserstoffatom ist, zu erhalten,

(b) Schützen der Hydroxyethylfunktionalität mit einer geeigneten Schutzgruppe, um einen geschützten Phenethylalkohol der Formel

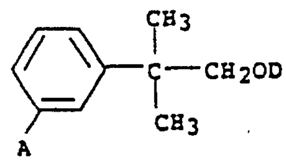

wobei A ein Wasserstoffatom ist und D eine geeignete Schutzgruppe ist, zu erhalten,

(c) Umsetzen des geschützten Phenethylalkohols mit einer ω-Halogenverbindung der Formel

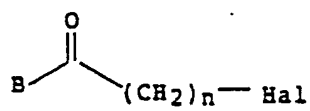

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

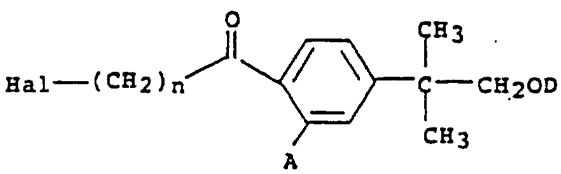

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

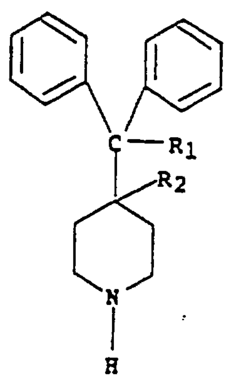

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

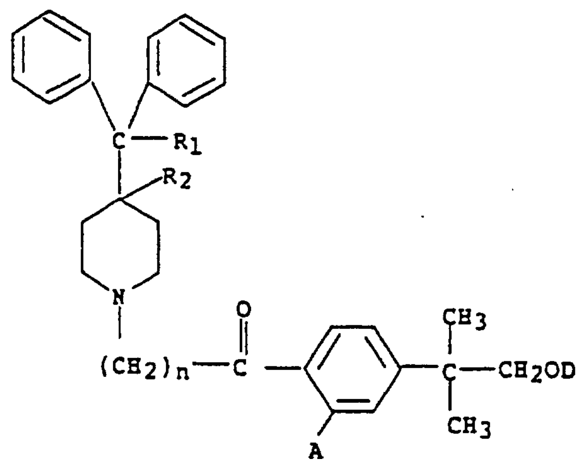

wobei $R_1$, $R_2$, n und D wie vorstehend definiert sind und A ein Wasserstoffatom ist, herzustellen,

(e) Umsetzen des geschützten Piperidinhydroxyethylphenylketons mit Natriumborhydrid, Kaliumborhydrid, Natriumcyanoborhydrid, Tetramethylammoniumborhydrid, Lithiumtri-tert-butylaluminohydrid, Diisobutylaluminiumhydrid oder katalytische Hydrierung, um einen geschützten Piperidinhydroxyethylphenylalkohol der Formel

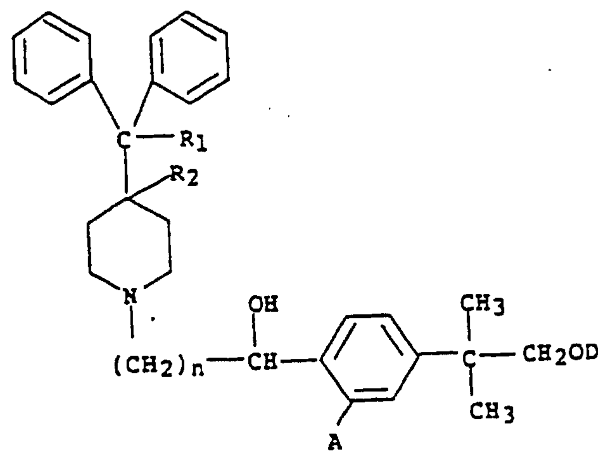

wobei $R_1$, $R_2$, D und n wie vorstehend definiert sind und A ein Wasserstoffatom ist, herzustellen.

**8.** Verfahren zur Herstellung einer geschützten Piperidinhydroxyethylphenylketonverbindung der Formel

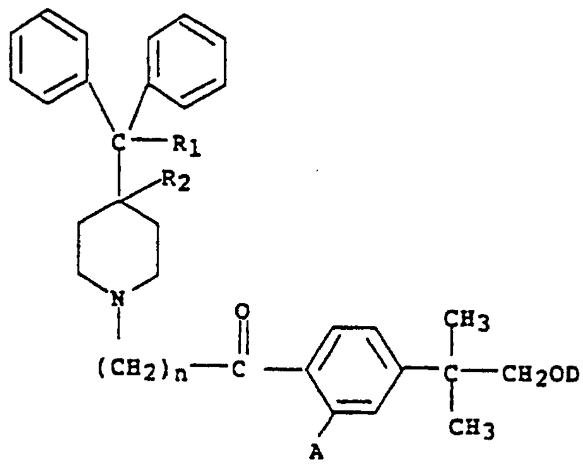

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
A ein Wasserstoffatom ist und
D die Gruppe $-C(=O)CH_3$ oder $-C(=O)C_6H_5$ ist,
umfassend die Schritte:

(a) Umsetzen einer Benzolessigsäureverbindung der Formel

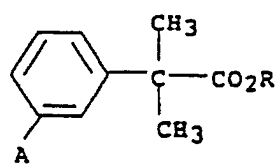

wobei A wie vorstehend definiert ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

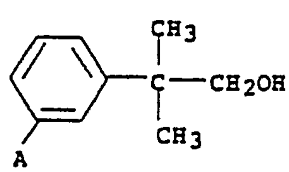

wobei A wie vorstehend definiert ist, zu erhalten,

(b) Schützen der Hydroxyethylfunktionalität mit einer geeigneten Schutzgruppe, um einen geschützten Phenethylalkohol der Formel

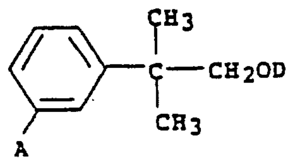

wobei A ein Wasserstoffatom ist und D eine geeignete Schutzgruppe ist, zu erhalten,

(c) Umsetzen des geschützten Phenethylalkohols mit einer ω-Halogenverbindung der Formel

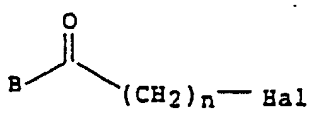

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

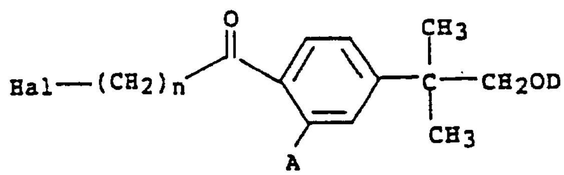

wobei Hal, n, A und D wie vorstehend definiert sind, herzustellen,

(d) Umsetzen des geschützten ω-Halogenhydroxyethylphenylketons mit einer Piperidinverbindung der Formel

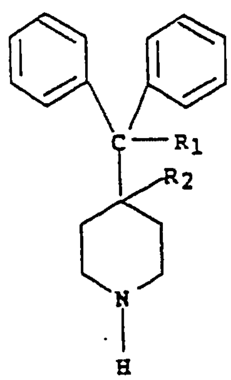

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon herzustellen.

**9.** Verfahren zur Herstellung einer Verbindung der Formel

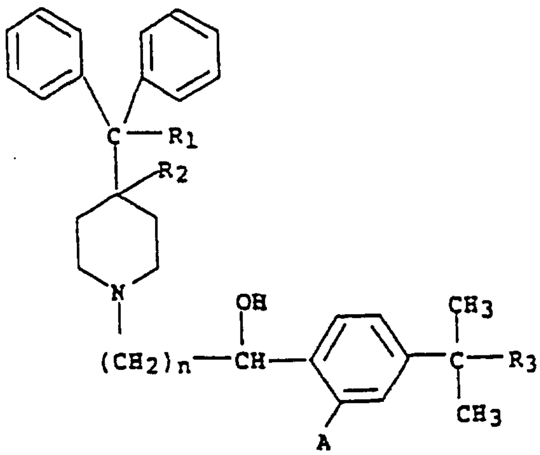

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

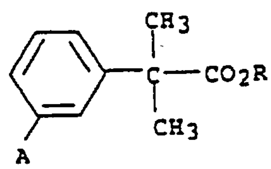

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

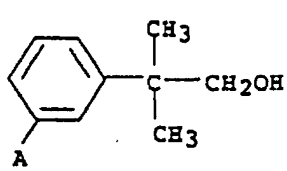

wobei A ein Wasserstoffatom ist, zu erhalten,

(b) Schützen der Hydroxyethylfunktionalität mit einer geeigneten Schutzgruppe, um einen geschützten Phenethylalkohol der Formel

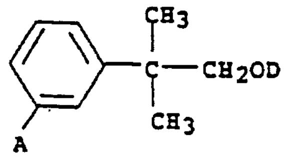

wobei A ein Wasserstoffatom ist und D eine geeignete Schutzgruppe ist, zu erhalten,

(c) Umsetzen des geschützten Phenethylalkohols mit einer ω-Halogenverbindung der Formel

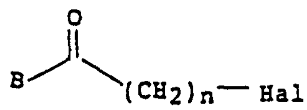

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

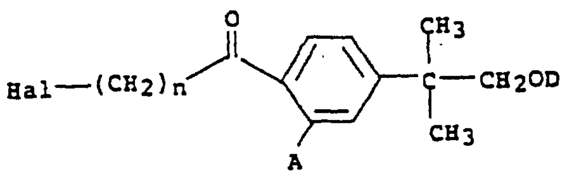

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

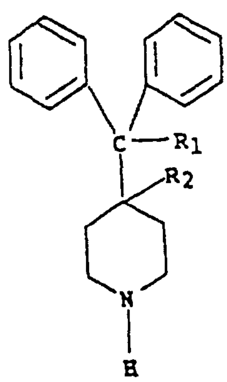

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

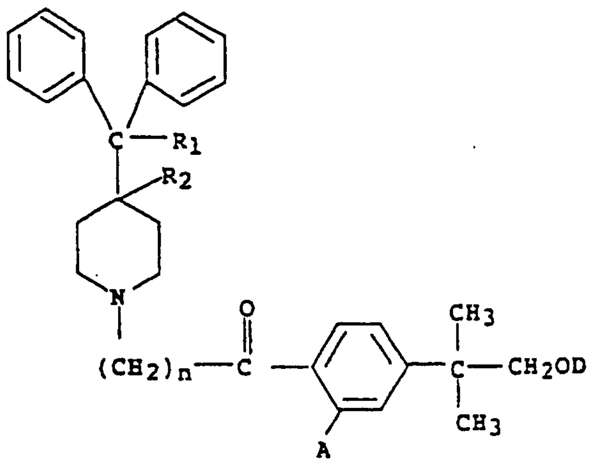

wobei $R_1$, $R_2$, n und D wie vorstehend definiert sind und A ein Wasserstoffatom ist, herzustellen,

(e) Schutzgruppenabspaltung der Hydroxyethylfunktionalität des geschützten Piperidinhydroxyethylphenylketons mit einem geeigneten Reagenz zur Schutzgruppenabspaltung, um ein Piperidinhydroxyethylphenylketon der Formel

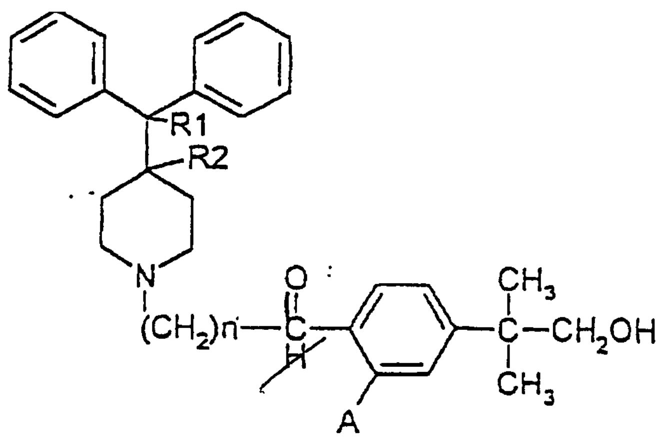

wobei $R_1$, $R_2$, n und A wie vorstehend definiert sind, herzustellen,

(f) Umsetzen des Piperidinhydroxyethylphenylketons mit Dimethylsulfoxid, Oxyalylchlorid und Triethylamin oder mit Dess-Martin-Reagenz, Chrom(IV)oxid, Nickelperoxid, Natriumdichromat, Kaliumdichromat, t-Butylchromat, Silberoxid, Silberpicolinatmangandioxidbleitetraacetat, Dicyclohexylcarbodiimid, 2,3-Dichlor-5,6-dicyanochinon, Tetrachlor-1,2-benzochinon, 2,2,6,6-Tetramethylpiperidinyl-1-oxy (TEMPO) oder Chinoliniumchlorchromat, um ein Piperidinformylphenylketon der Formel

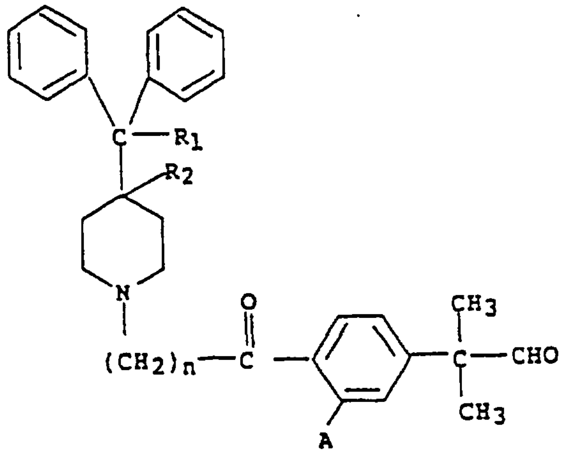

wobei $R_1$, $R_2$ und n wie vorstehend definiert sind und A ein Wasserstoffatom ist, herzustellen,

(g) Umsetzen des Piperidinformylphenylketons mit Kaliumpermanganat, Chrom(IV)oxid, Silber(I)oxid, Silberoxid, Silberpicolinat, Peroxid, Salpetersäure, m-Chlorbenzoesäure und Peressigsäure, um ein Piperidincarboxyphenylketon der Formel

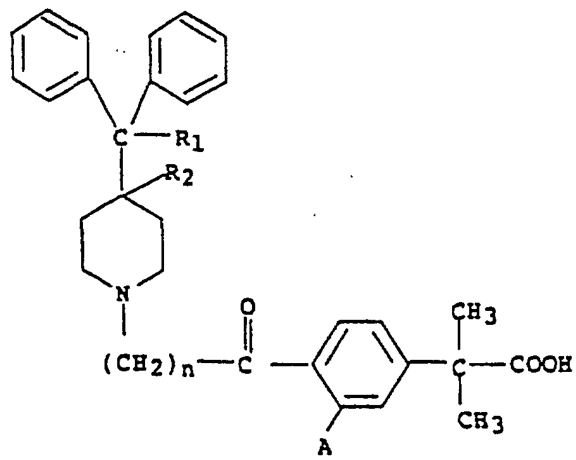

wobei $R_1$, $R_2$ und n wie vorstehend definiert sind und A ein Wasserstoffatom ist, herzustellen,

(h) Umsetzen des Piperidincarboxyphenylketons mit Natriumborhydrid, Kaliumborhydrid, Natriumcyanoborhydrid, Tetramethylammoniumborhydrid, Lithiumtri-tert-butylaluminohydrid, Diisobutylaluminiumhydrid oder katalytische Hydrierung, um einen Piperidincarboxyphenylalkohol der Formel

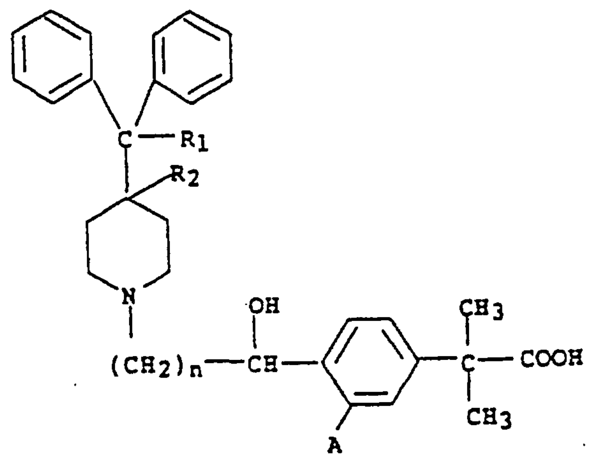

wobei $R_1$, $R_2$ und n wie vorstehend definiert sind und A ein Wasserstoffatom ist, herzustellen,

(i) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

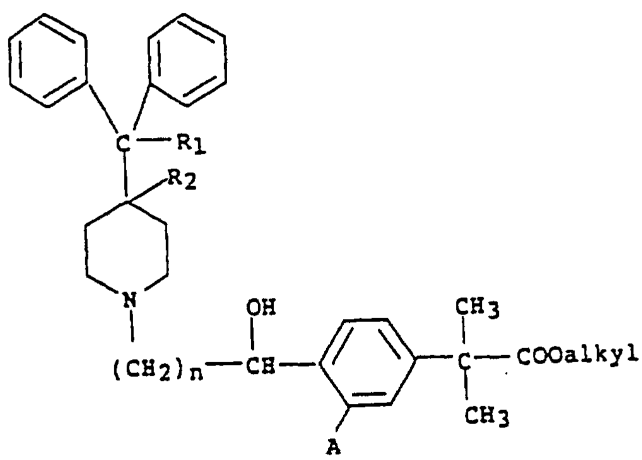

wobei $R_1$, $R_2$, n und Alkyl wie vorstehend definiert sind und A ein Wasserstoffatom ist, zu bilden.

**10.** Verfahren zur Herstellung einer Verbindung der Formel

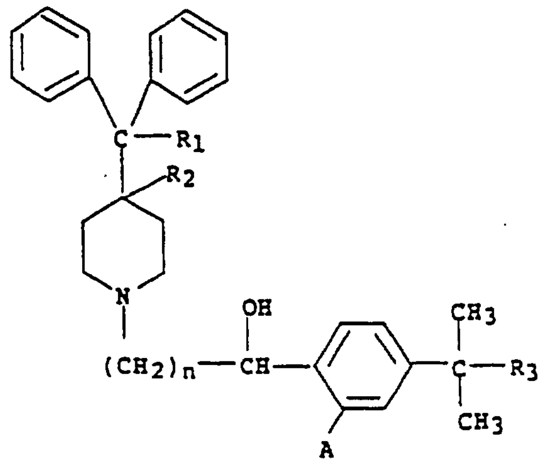

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

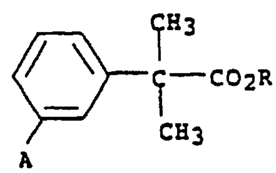

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

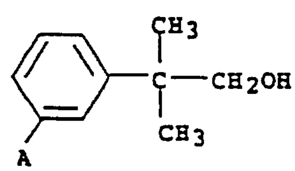

wobei A ein Wasserstoffatom ist, zu erhalten,

(b) Schützen der Hydroxyethylfunktionalität mit einer geeigneten Schutzgruppe, um einen geschützten Phenethylalkohol der Formel

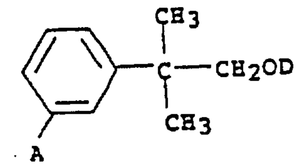

wobei A ein Wasserstoffatom ist und D eine geeignete Schutzgruppe ist, zu erhalten,

(c) Umsetzen des geschützten Phenethylalkohols mit einer ω-Halogenverbindung der Formel

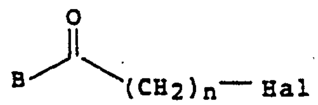

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

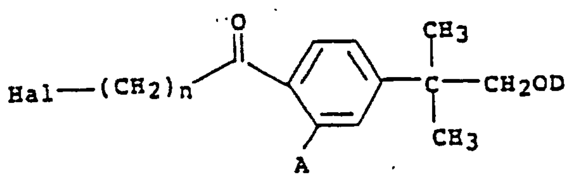

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

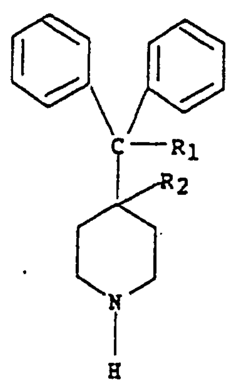

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

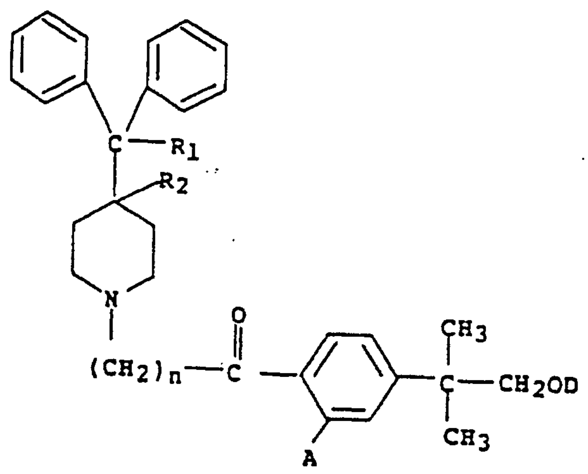

wobei $R_1$, $R_2$, n und D wie vorstehend definiert sind und A ein Wasserstoffatom ist, herzustellen,

(e) Schutzgruppenabspaltung der Hydroxyethylfunktionalität des geschützten Piperidinhydroxyethylphenylketons mit einem geeigneten Reagenz zur Schutzgruppenabspaltung, um ein Piperidinhydroxyethylphenylketon der Formel

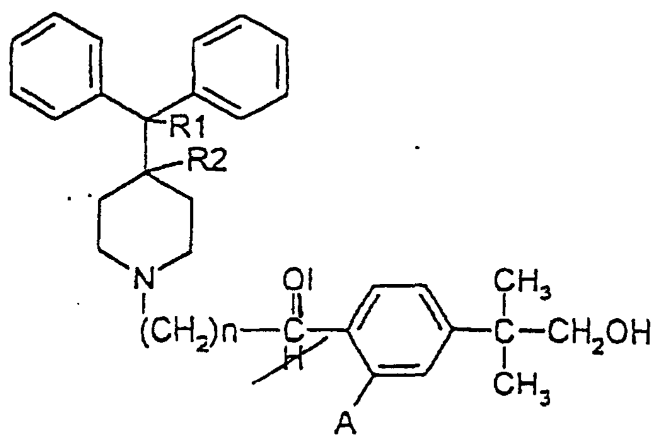

wobei $R_1$, $R_2$, n und A wie vorstehend definiert sind, herzustellen,

(f) Umsetzen des Piperidinhydroxyethoxyphenylketons mit Chrom(IV)oxid, Kaliumpermanganat, Salpetersäure, Stickstoffdioxid, Ruthenium(VIII)oxid, Nickelperoxid, Silberoxid, t-Butylchromat oder Xenonsäure, um ein Piperidincarboxyphenylketon der Formel

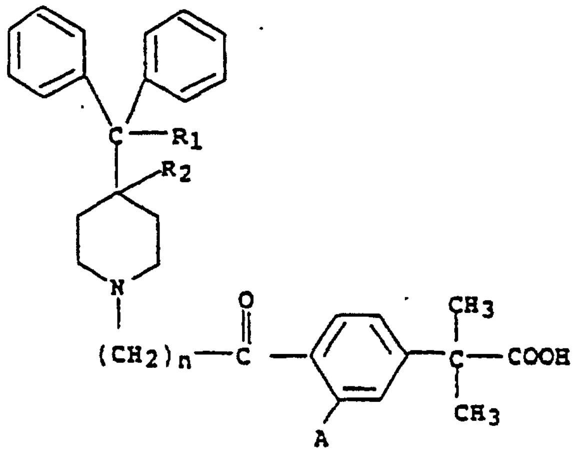

wobei $R_1$, $R_2$ und n wie vorstehend definiert sind und A ein Wasserstoffatom ist, herzustellen, und

(g) Umsetzen des Piperidincarboxyphenylketons mit Natriumborhydrid, Kaliumborhydrid, Natriumcyanoborhydrid, Tetramethylammoniumborhydrid, Lithiumtri-tert-butylaluminohydrid, Diisobutylaluminiumhydrid oder katalytische Hydrierung, um einen Piperidincarboxyphenylalkohol der Formel

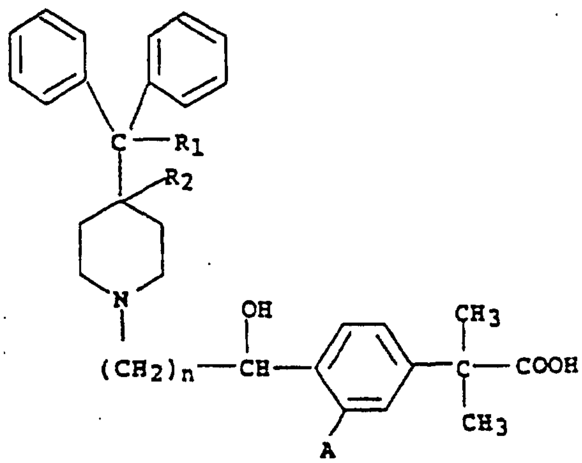

wobei $R_1$, $R_2$ und n wie vorstehend definiert sind und A ein Wasserstoffatom ist, herzustellen,

(h) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

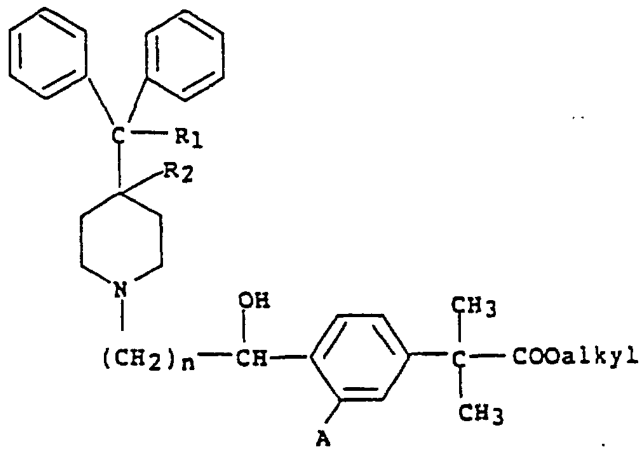

wobei $R_1$, $R_2$, n und Alkyl wie vorstehend definiert sind und A ein Wasserstoffatom ist, zu bilden.

**11.** Verfahren gemäß Anspruch 10, wobei das in Schritt e hergestellte Piperidinhydroxyethylphenylketon mit Ruthenium(VIII)oxid umgesetzt wird, um das Piperidincarboxyphenylketon von Schritt f zu erhalten.

**12.** Verfahren zur Herstellung einer Verbindung der Formel

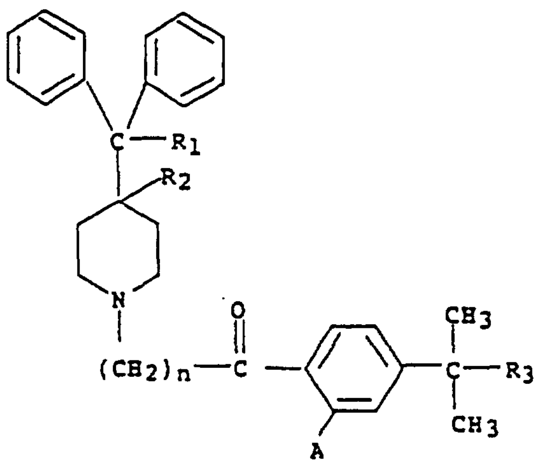

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

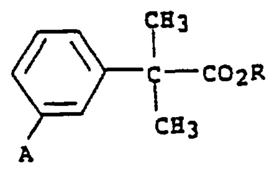

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

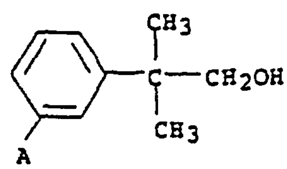

wobei A ein Wasserstoffatom ist, zu erhalten,

(b) Schützen der Hydroxyethylfunktionalität mit einer geeigneten Schutzgruppe, um einen geschützten Phenethylalkohol der Formel

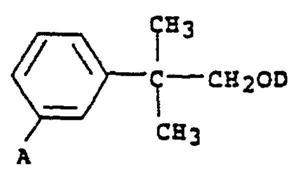

wobei A ein Wasserstoffatom ist und D eine geeignete Schutzgruppe ist, zu erhalten,

(c) Umsetzen des geschützten Phenethylalkohols mit einer ω-Halogenverbindung der Formel

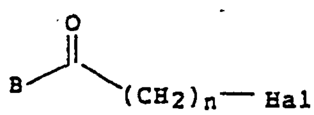

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

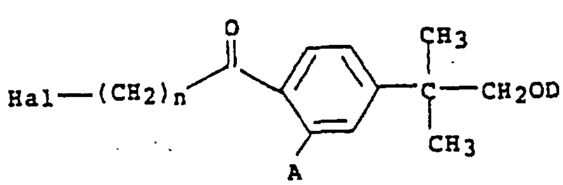

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

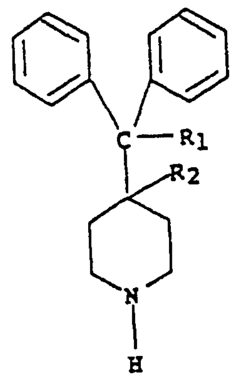

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

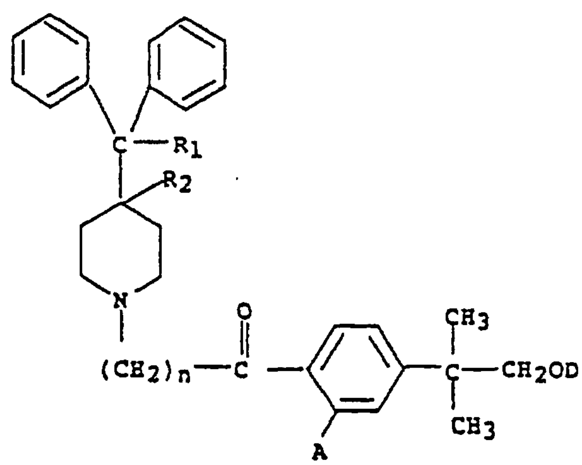

wobei $R_1$, $R_2$, n und D wie vorstehend definiert sind und A ein Wasserstoffatom ist, herzustellen,

(e) Schutzgruppenabspaltung der Hydroxyethylfunktionalität des geschützten Piperidinhydroxyethylphenylketons mit einem geeigneten Reagenz zur Schutzgruppenabspaltung, um ein Piperidinhydroxyethylphenylketon der Formel

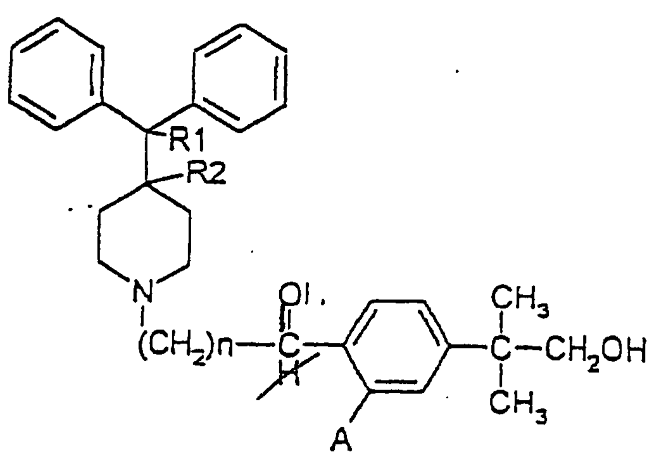

wobei $R_1$, $R_2$ und n wie vorstehend definiert sind und A ein Wasserstoffatom ist, herzustellen,

(f) Umsetzen des Piperidinhydroxyethylphenylketons mit Dimethylsulfoxid, Oxyalylchlorid und Triethylamin oder mit Dess-Martin-Reagenz, Chrom(IV)oxid, Nickelperoxid, Natriumdichromat, Kaliumdichromat, t-Butylchromat, Silberoxid, Silberpicolinatmangandioxidbleitetraacetat, Dicyclohexylcarbodiimid, 2,3-Dichlor-5,6-dicyanochinon, Tetrachlor-1,2-benzochinon, 2,2,6,6-Tetramethylpiperidinyl-1-oxy (TEMPO) oder Chinoliniumchlorchromat, um ein Piperidinformylphenylketon der Formel

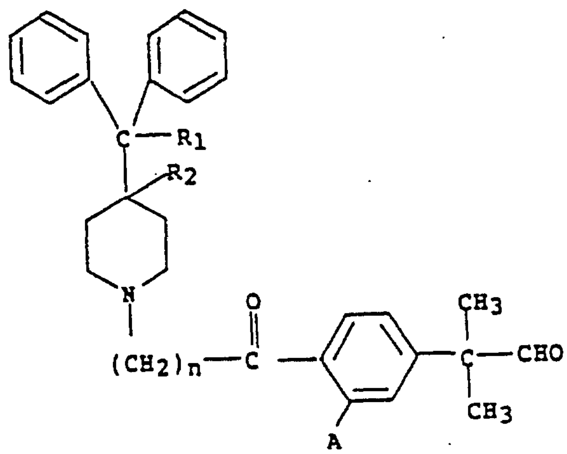

wobei $R_1$, $R_2$ und n wie vorstehend definiert sind und A ein Wasserstoffatom ist, herzustellen,

(g) Umsetzen des Piperidinformylphenylketons mit Kaliumpermanganat, Chrom(IV)oxid, Silber(I)oxid, Silberoxid, Silberpicolinat, Peroxid, Salpetersäure, m-Chlorperbenzoesäure und Peressigsäure, um ein Piperidincarboxyphenylketon der Formel

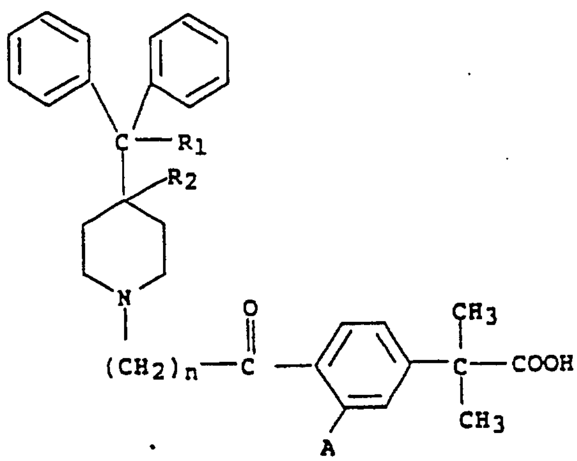

wobei $R_1$, $R_2$ und n wie vorstehend definiert sind und A ein Wasserstoffatom ist, herzustellen,

(h) gegebenenfalls Umsetzen des Piperidincarboxyphenylketons mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylketonester der Formel

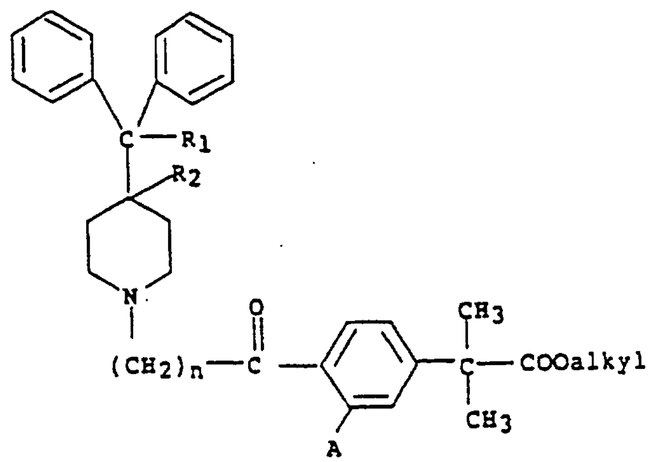

wobei $R_1$, $R_2$, n und Alkyl wie vorstehend definiert sind und A ein Wasserstoffatom ist, zu bilden.

**13.** Verfahren zur Herstellung einer Verbindung der Formel

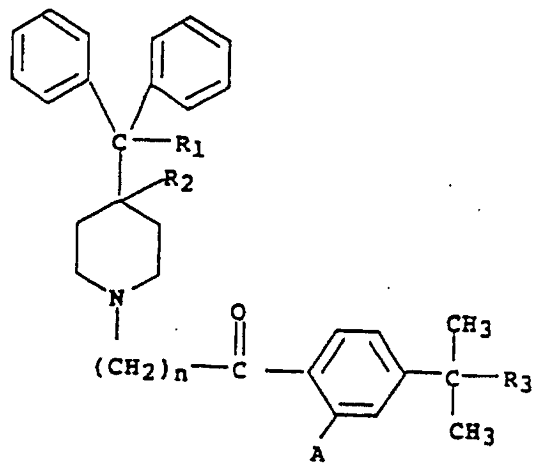

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

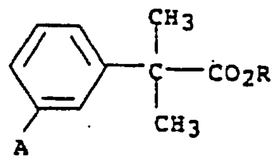

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

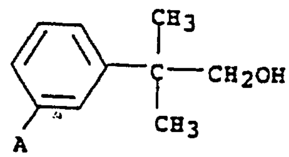

wobei A ein Wasserstoffatom ist, zu erhalten,

(b) Schützen der Hydroxyethylfunktionalität mit einer geeigneten Schutzgruppe, um einen geschützten Phenethylalkohol der Formel

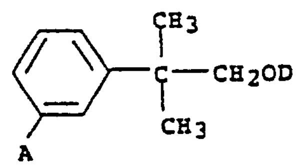

wobei A ein Wasserstoffatom ist und D eine geeignete Schutzgruppe ist, zu erhalten,

(c) Umsetzen des geschützten Phenethylalkohols mit einer ω-Halogenverbindung der Formel

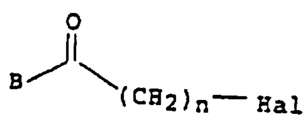

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

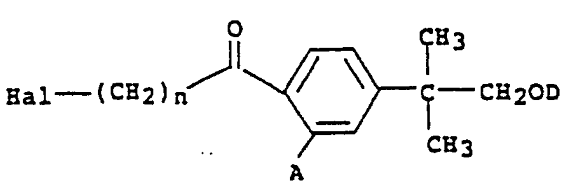

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

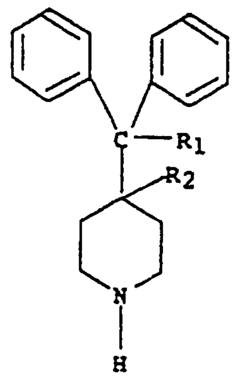

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

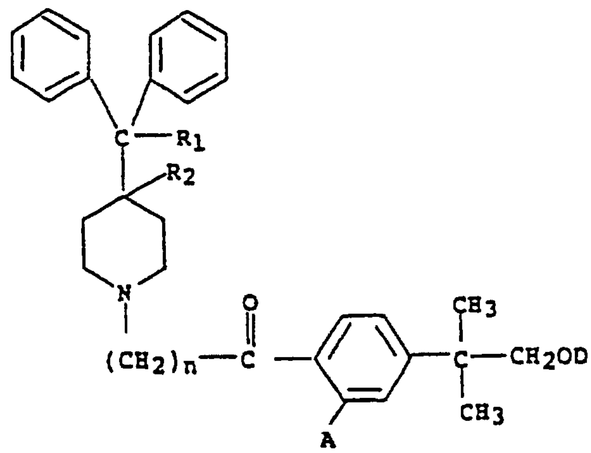

wobei $R_1$, $R_2$, n und D wie vorstehend definiert sind und A ein Wasserstoffatom ist, herzustellen,

(e) Schutzgruppenabspaltung der Hydroxyethylfunktionalität des geschützten Piperidinhydroxyethylphenylketons mit einem geeigneten Reagenz zur Schutzgruppenabspaltung, um ein Piperidinhydroxyethylphenylketon der Formel

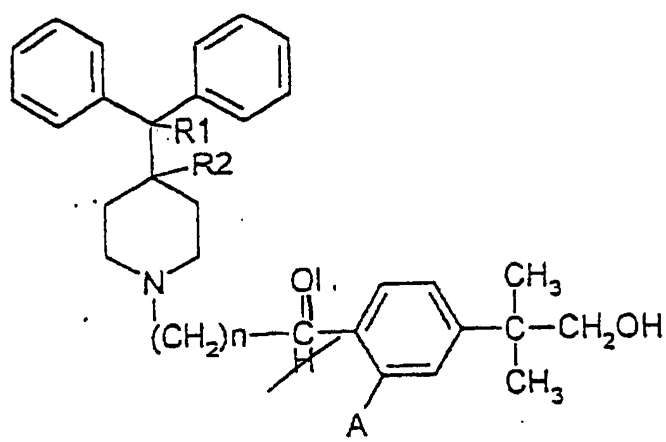

wobei $R_1$, $R_2$, n und A wie vorstehend definiert sind, herzustellen, und

(f) Umsetzen des Piperidinhydroxyethylphenylketons mit Chrom(IV)oxid, Kaliumpermanganat, Salpetersäure, Stickstoffdioxid, Ruthenium(VIII)oxid, Nickelperoxid, Silberoxid, t-Butylchromat oder Xenonsäure, um das Piperidincarboxyphenylketon der Formel

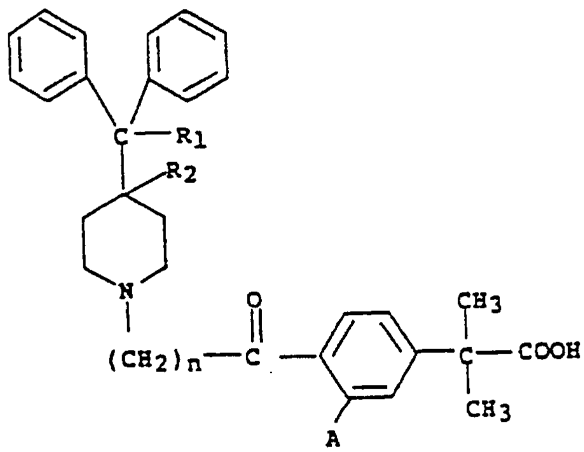

wobei $R_1$, $R_2$ und n wie vorstehend definiert sind und A ein Wasserstoffatom ist, herzustellen,

(g) gegebenenfalls Umsetzen des Piperidincarboxyphenylketons mit Diazomethan oder 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylketonester der Formel

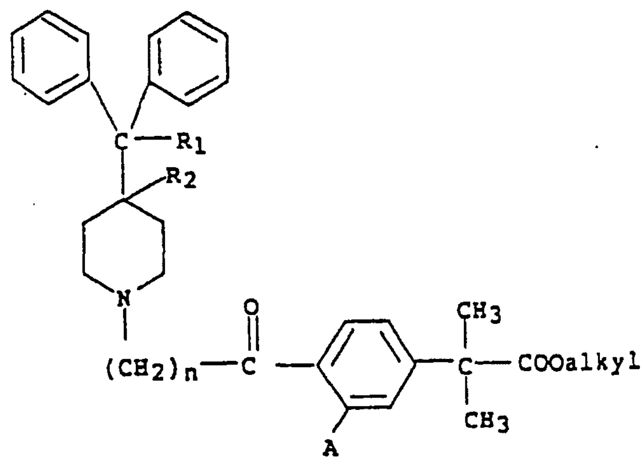

wobei $R_1$, $R_2$, n und Alkyl wie vorstehend definiert sind und A ein Wasserstoffatom ist, zu bilden.

**14.** Verfahren gemäß Anspruch 13, wobei das in Schritt e hergestellte Piperidinhydroxyethylphenylketon mit Ruthenium(VIII)oxid umgesetzt wird, um das Piperidincarboxyphenylketon von Schritt f zu erhalten.

**15.** Verfahren zur Herstellung einer Piperidinhydroxyethylphenylketonverbindung der Formel

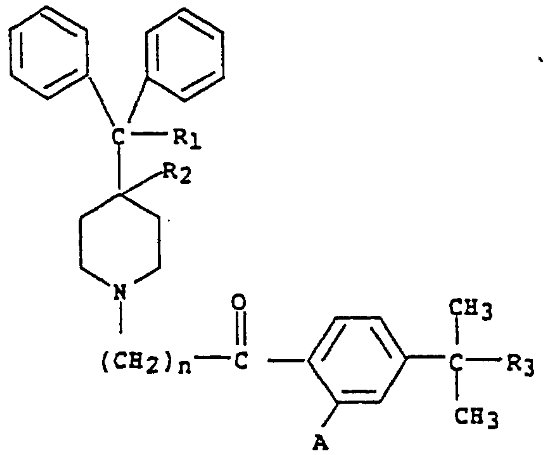

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe $-CH_2OH$ ist,
A ein Wasserstoffatom ist und
pharmazeutisch verträgliche Salze davon,
umfassend die Schritte:

(a) Umsetzen einer Benzolessigsäureverbindung der Formel

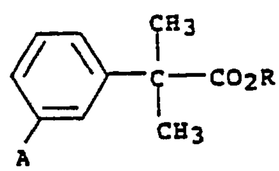

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

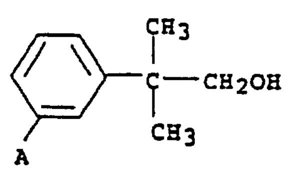

wobei A ein Wasserstoffatom ist, zu erhalten,

(b) Schützen der Hydroxyethylfunktionalität mit einer geeigneten Schutzgruppe, um einen geschützten Phenethylalkohol der Formel

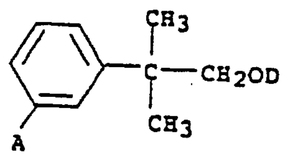

wobei A ein Wasserstoffatom ist und D eine geeignete Schutzgruppe ist, zu erhalten,

(c) Umsetzen des geschützten Phenethylalkohols mit einer ω-Halogenverbindung der Formel

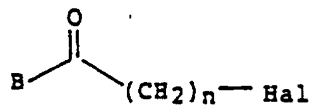

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

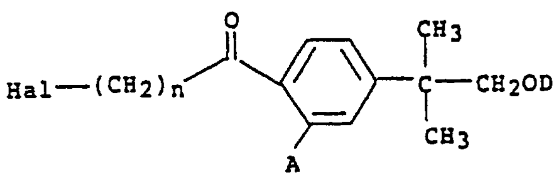

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

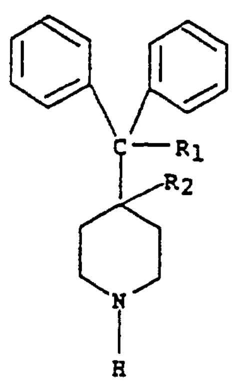

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

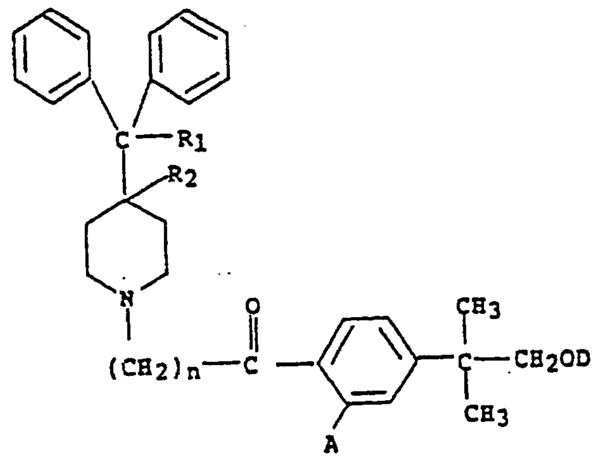

wobei $R_1$, $R_2$, n und D wie vorstehend definiert sind und A ein Wasserstoffatom ist, herzustellen,

(e) Schutzgruppenabspaltung der Hydroxyethylfunktionalität des geschützten Piperidinhydroxyethylphenylketons mit einem geeigneten Reagenz zur Schutzgruppenabspaltung, um ein Piperidinhydroxyethylphenylketon der Formel

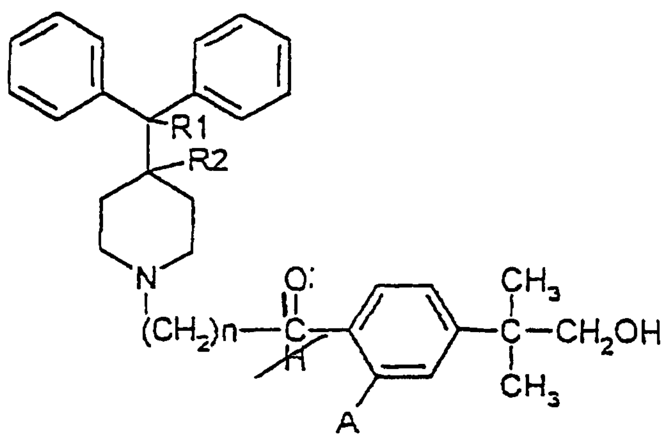

wobei $R_1$, $R_2$, n und A wie vorstehend definiert sind, herzustellen.

**16.** Verfahren zur Herstellung einer Verbindung der Formel

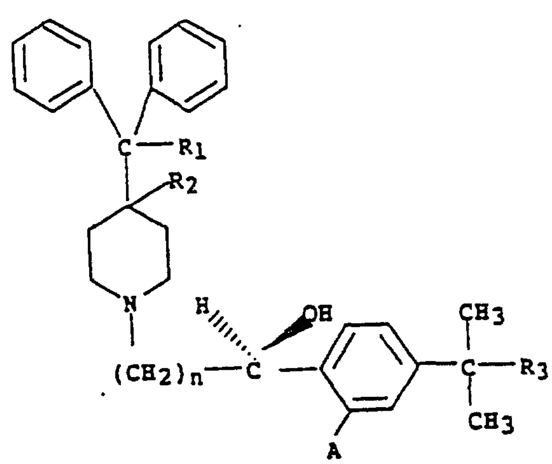

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

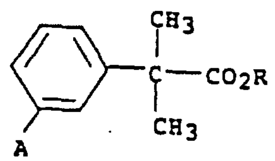

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

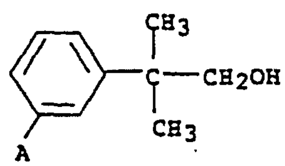

wobei A ein Wasserstoffatom ist, zu erhalten,

(b) Schützen der Hydroxyethylfunktionalität mit einer geeigneten Schutzgruppe, um einen geschützten Phenethylalkohol der Formel

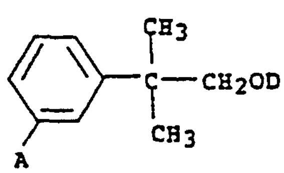

wobei A ein Wasserstoffatom ist und D eine geeignete Schutzgruppe ist, zu erhalten,

(c) Umsetzen des geschützten Phenethylalkohols mit einer ω-Halogenverbindung der Formel

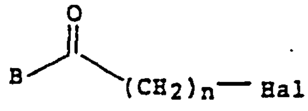

wobei B ein Halogenatom oder eine Hydroxygruppe ist, Hal Cl, Br oder I darstellt und n wie vorstehend definiert

ist, in Anwesenheit von Trichlorid, Aluminiumchlorid, Titantetrachlorid, Bortrifluorid, Zinntetrachlorid und Zinkchlorid, um ein geschütztes ω-Halogenhydroxyethylphenylketon der Formel

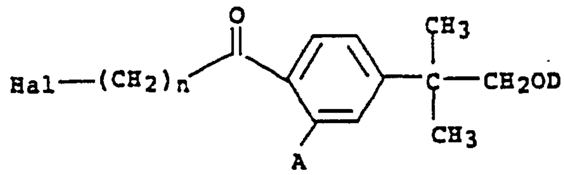

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

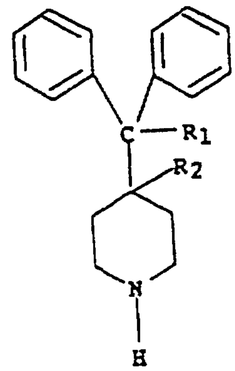

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

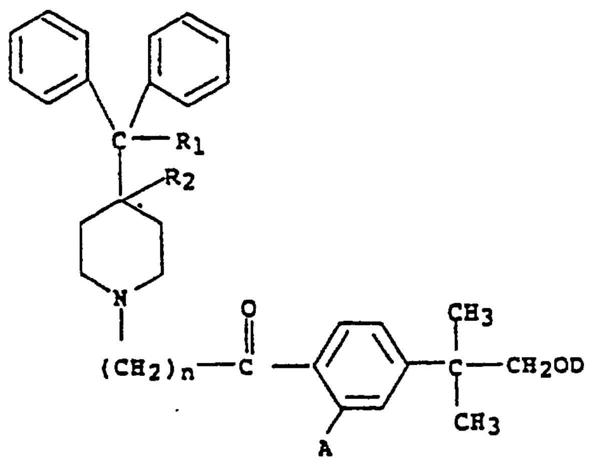

wobei $R_1$, $R_2$, n und D wie vorstehend definiert sind und A ein Wasserstoffatom ist, herzustellen,

(e) Schutzgruppenabspaltung der Hydroxyethylfunktionalität des geschützten Piperidinhydroxyethylphenylketons mit einem geeigneten Reagenz zur Schutzgruppenabspaltung, um ein Piperidinhydroxyethylphenylketon der Formel

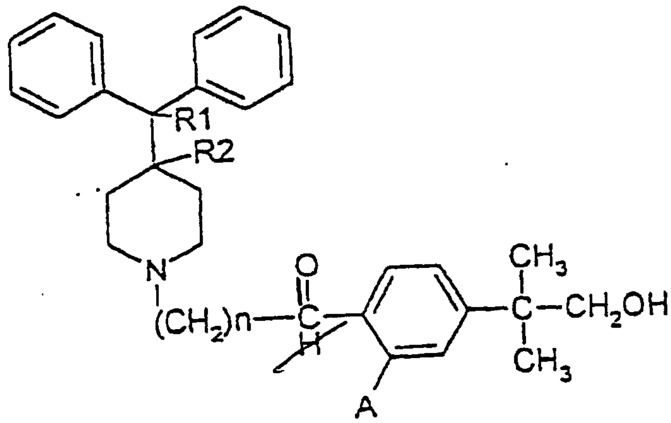

wobei $R_1$, $R_2$, n und A wie vorstehend definiert sind, herzustellen, und

(f) Umsetzen des Piperidinhydroxyethylphenylketons mit Dimethylsulfoxid, Oxyalylchlorid und Triethylamin oder mit Dess-Martin-Reagenz, Chrom(IV)oxid, Nickelperoxid, Natriumdichromat, Kaliumdichromat, t-Butylchromat, Silberoxid, Silberpicolinatmangandioxidbleitetraacetat, Dicyclohexylcarbodiimid, 2,3-Dichlor-5,6-dicyanochinon, Tetrachlor-1,2-benzochinon, 2,2,6,6-Tetramethylpiperidinyl-1-oxy (TEMPO) oder Chinoliniumchlorchromat, um ein Piperidinformylphenylketon der Formel

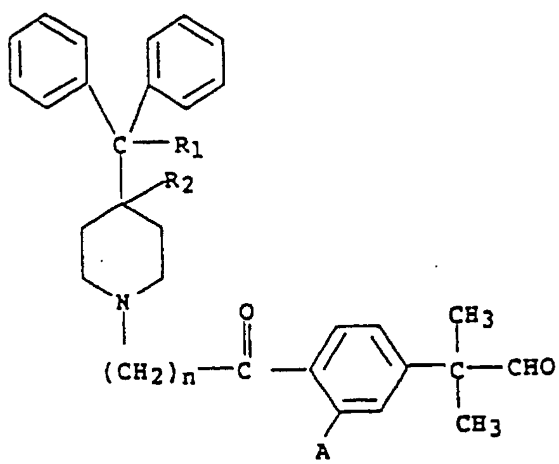

wobei $R_1$, $R_2$ und n wie vorstehend definiert sind und A ein Wasserstoffatom ist, herzustellen,

(g) Umsetzen des Piperidinformylphenylketons mit Kaliumpermanganat, Chrom(IV)oxid, Silber(I)oxid, Silberoxid, Silberpicolinat, Peroxid, Salpetersäure, m-Chlorperbenzoesäure und Peressigsäure, um ein Piperidincarboxyphenylketon der Formel

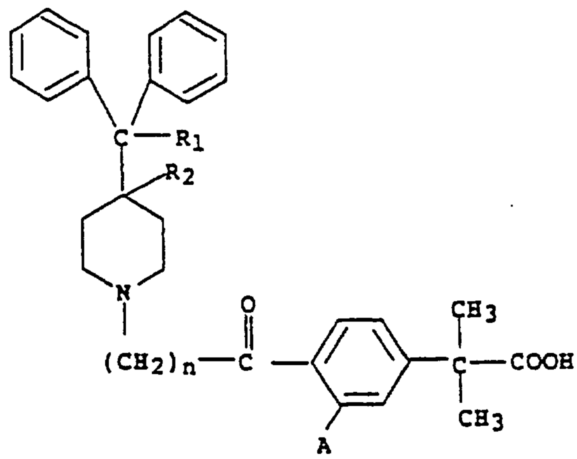

wobei $R_1$, $R_2$ und n wie vorstehend definiert sind und A ein Wasserstoffatom ist, herzustellen,

(h) Umsetzen des Piperidincarboxyphenylketons mit (+)-B-Chlordiisopinocamphenylboran, um einen Piperidincarboxyphenylalkohol der Formel

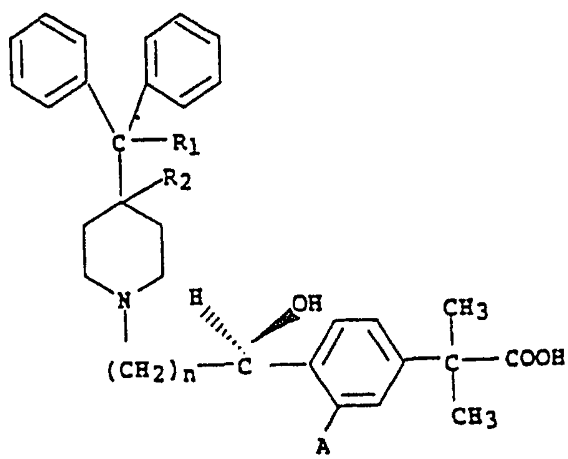

wobei $R_1$, $R_2$ und n wie vorstehend definiert sind und A ein Wasserstoffatom ist, herzustellen,

(i) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

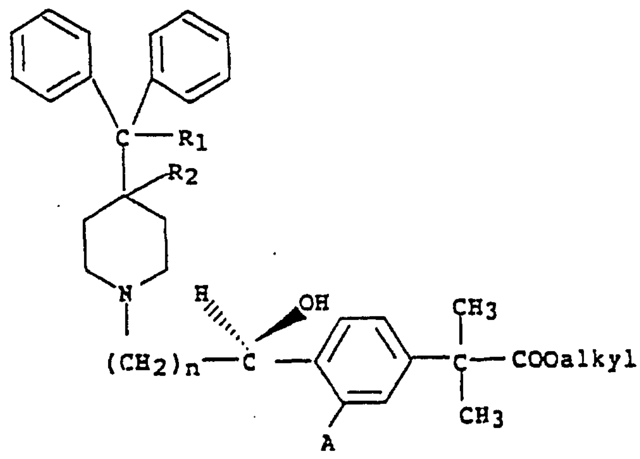

wobei $R_1$, $R_2$, n und Alkyl wie vorstehend definiert sind und A ein Wasserstoffatom ist, zu bilden.

**17.** Verfahren zur Herstellung einer Verbindung der Formel

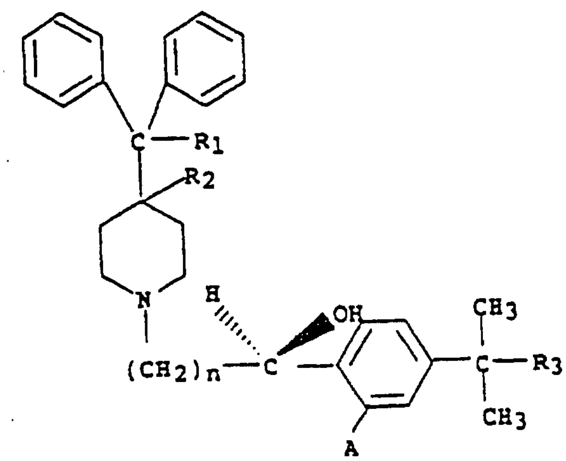

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

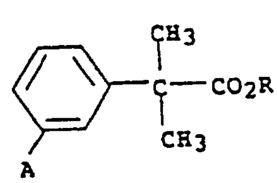

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

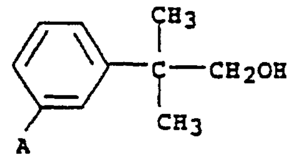

wobei A ein Wasserstoffatom ist, zu erhalten,

(b) Schützen der Hydroxyethylfunktionalität mit einer geeigneten Schutzgruppe, um einen geschützten Phenethylalkohol der Formel

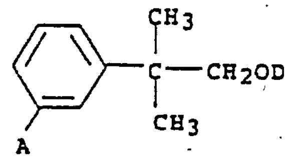

wobei A ein Wasserstoffatom ist und D eine geeignete Schutzgruppe ist, zu erhalten,

(c) Umsetzen des geschützten Phenethylalkohols mit einer ω-Halogenverbindung der Formel

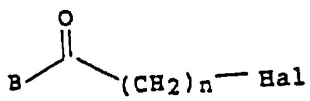

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

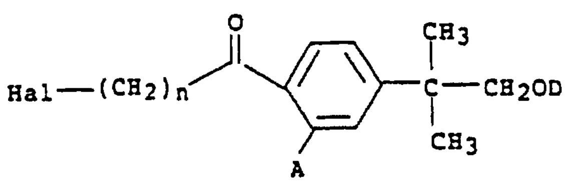

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

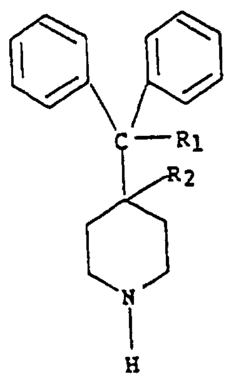

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

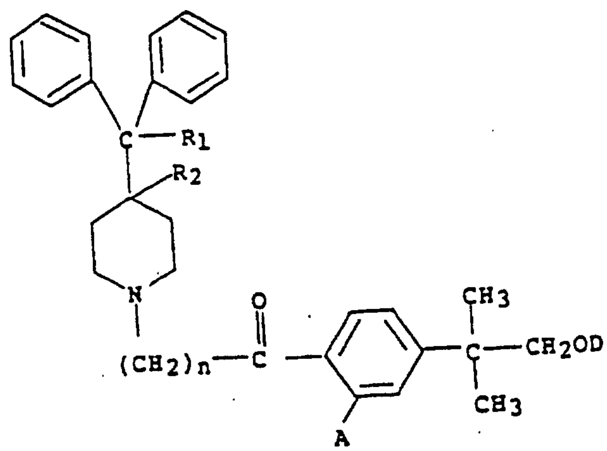

wobei $R_1$, $R_2$, n und D wie vorstehend definiert sind und A ein Wasserstoffatom ist, herzustellen,

(e) Schutzgruppenabspaltung der Hydroxyethylfunktionalität des geschützten Piperidinhydroxyethylphenylketons mit einem geeigneten Reagenz zur Schutzgruppenabspaltung, um ein Piperidinhydroxyethylphenylketon der Formel

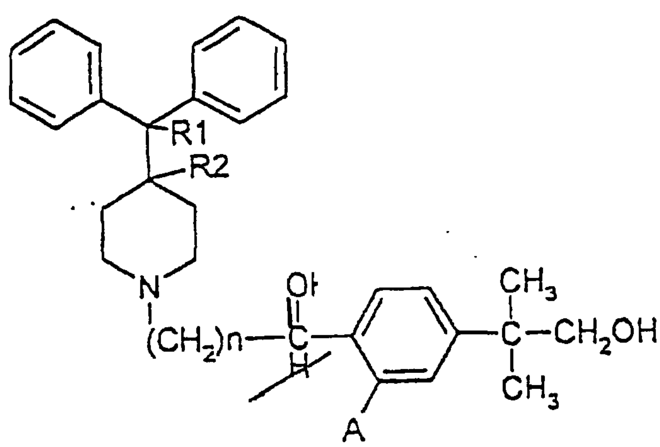

wobei $R_1$, $R_2$, n und A wie vorstehend definiert sind, herzustellen, und

(f) Umsetzen des Piperidinhydroxyethylphenylketons mit Chrom(IV)oxid, Kaliumpermanganat, Salpetersäure, Stickstoffdioxid, Ruthenium(VIII)oxid, Nickelperoxid, Silberoxid, t-Butylchromat oder Xenonsäure, um ein Piperidincarboxyphenylketon der Formel

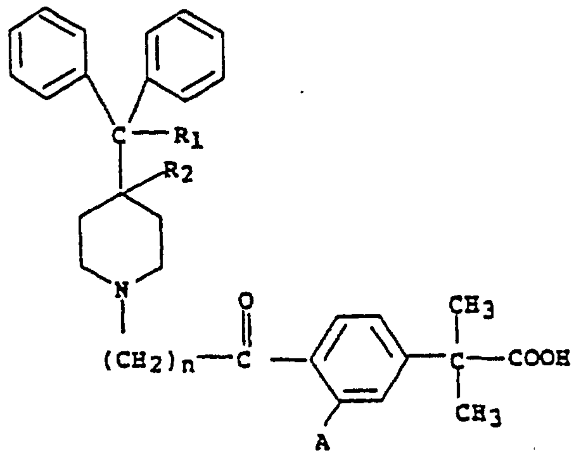

wobei $R_1$, $R_2$ und n wie vorstehend definiert sind und A ein Wasserstoffatom ist, herzustellen,

(g) Umsetzen des Piperidincarboxyphenylketons mit (+)-B-Chlordiisopinocamphenylboran, um einen Piperidincarboxyphenylalkohol der Formel

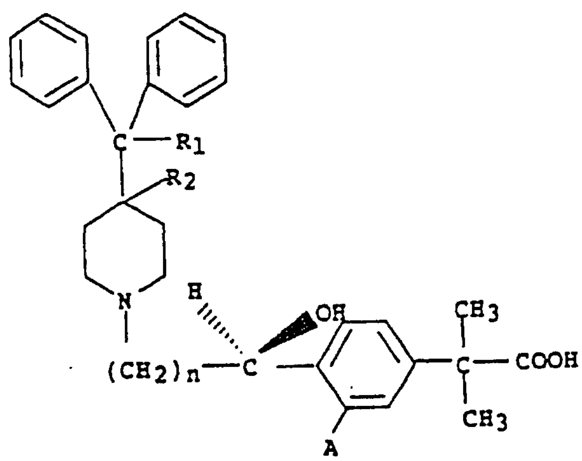

wobei $R_1$, $R_2$ und n wie vorstehend definiert sind und A ein Wasserstoffatom ist, herzustellen,

(h) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

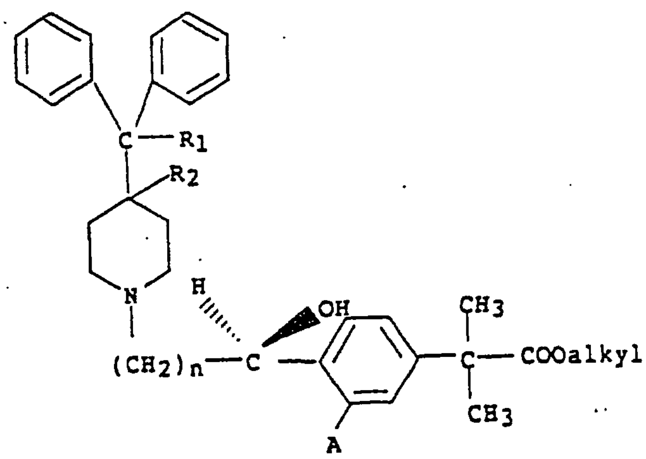

wobei $R_1$, $R_2$, n und Alkyl wie vorstehend definiert sind und A ein Wasserstoffatom ist, zu bilden.

**18.** Verfahren zur Herstellung einer Verbindung der Formel

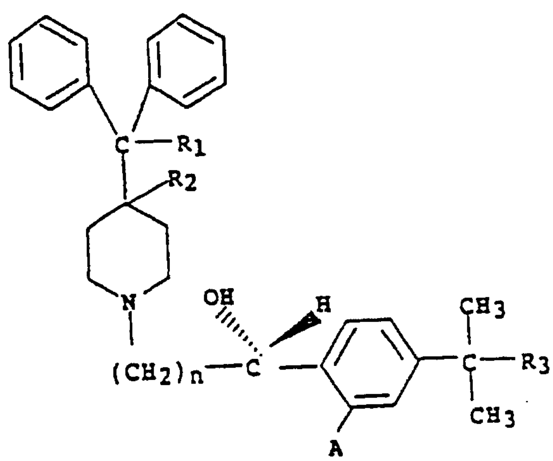

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

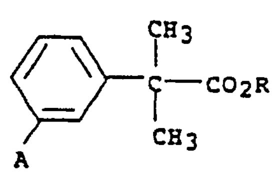

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

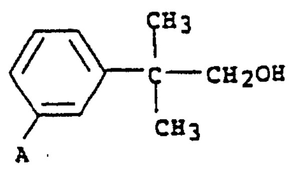

wobei A ein Wasserstoffatom ist, zu erhalten,

(b) Schützen der Hydroxyethylfunktionalität mit einer geeigneten Schutzgruppe, um einen geschützten Phenethylalkohol der Formel

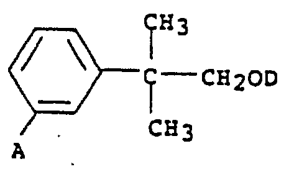

wobei A ein Wasserstoffatom ist und D eine geeignete Schutzgruppe ist, zu erhalten,

(c) Umsetzen des geschützten Phenethylalkohols mit einer ω-Halogenverbindung der Formel

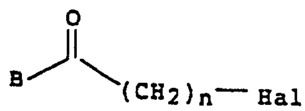

wobei B ein Halogenatom oder eine Hydroxygruppe ist, Hal Cl, Br oder I darstellt und n wie vorstehend definiert ist, in Anwesenheit von Trichlorid, Aluminiumchlorid, Titantetrachlorid, Bortrifluorid, Zinntetrachlorid und Zinkchlorid, um ein geschütztes ω-Halogenhydroxyethylphenylketon der Formel

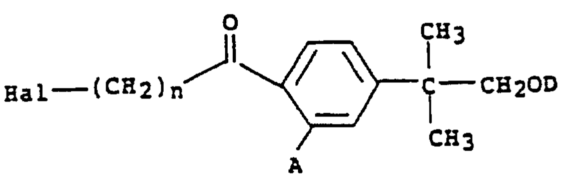

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

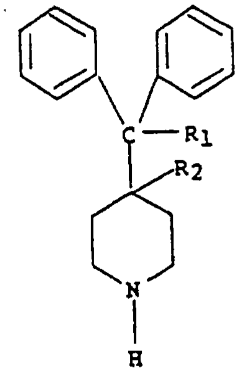

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

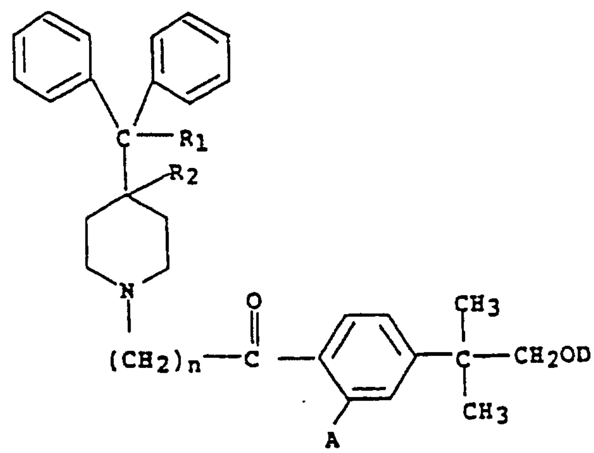

wobei $R_1$, $R_2$, n und D wie vorstehend definiert sind und A ein Wasserstoffatom ist, herzustellen,

(e) Schutzgruppenabspaltung der Hydroxyethylfunktionalität des geschützten Piperidinhydroxyethylphenylketons mit einem geeigneten Reagenz zur Schutzgruppenabspaltung, um ein Piperidinhydroxyethylphenylketon der Formel

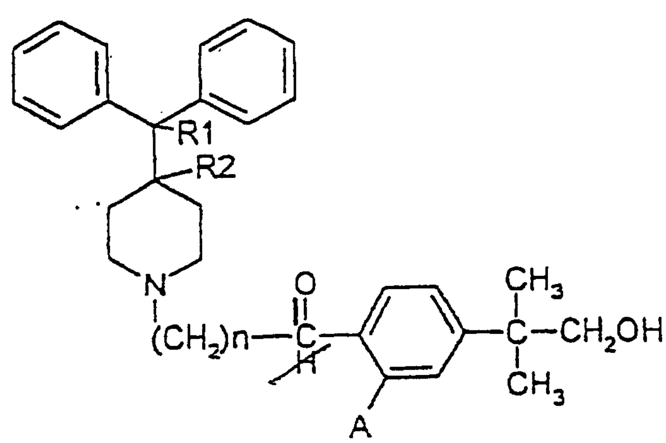

wobei $R_1$, $R_2$, n und A wie vorstehend definiert sind, herzustellen, und

(f) Umsetzen des Piperidinhydroxyethylphenylketons mit Dimethylsulfoxid, Oxyalylchlorid und Triethylamin oder mit Dess-Martin-Reagenz, Chrom(IV)oxid, Nickelperoxid, Natriumdichromat, Kaliumdichromat, t-Butylchromat, Silberoxid, Silberpicolinatmangandioxidbleitetraacetat, Dicyclohexylcarbodiimid, 2,3-Dichlor-5,6-dicyanochinon, Tetrachlor-1,2-benzochinon, 2,2,6,6-Tetramethylpiperidinyl-1-oxy (TEMPO) oder Chinoliniumchlorchromat, um ein Piperidinformylphenylketon der Formel

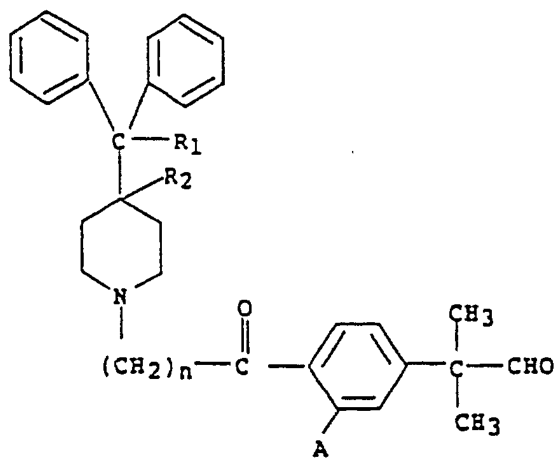

wobei $R_1$, $R_2$ und n wie vorstehend definiert sind und A ein Wasserstoffatom ist, herzustellen,

(g) Umsetzen des Piperidinformylphenylketons mit Kaliumpermanganat, Chrom(IV)oxid, Silber(I)oxid, Silberoxid, Silberpicolinat, Peroxid, Salpetersäure, m-Chlorperbenzoesäure und Peressigsäure, um ein Piperidincarboxyphenylketon der Formel

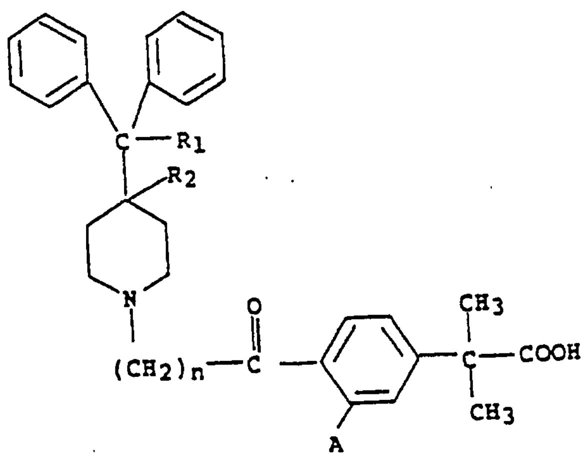

wobei $R_1$, $R_2$ und n wie vorstehend definiert sind und A ein Wasserstoffatom ist, herzustellen,

(h) Umsetzen des Piperidincarboxyphenylketons mit (-)-B-Chlordiisopinocamphenylboran, um einen Piperidincarboxyphenylalkohol der Formel

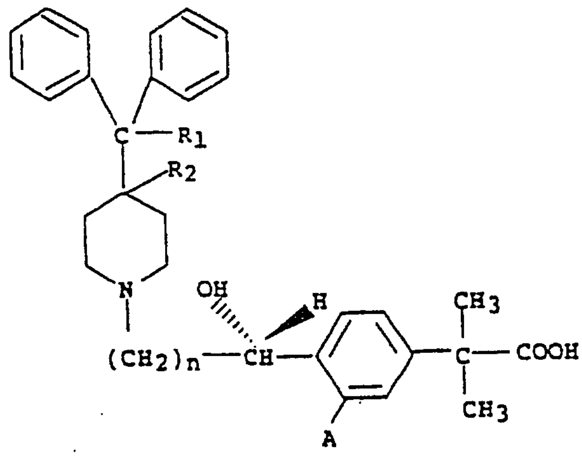

wobei $R_1$, $R_2$ und n wie vorstehend definiert sind und A ein Wasserstoffatom ist, herzustellen,

(i) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

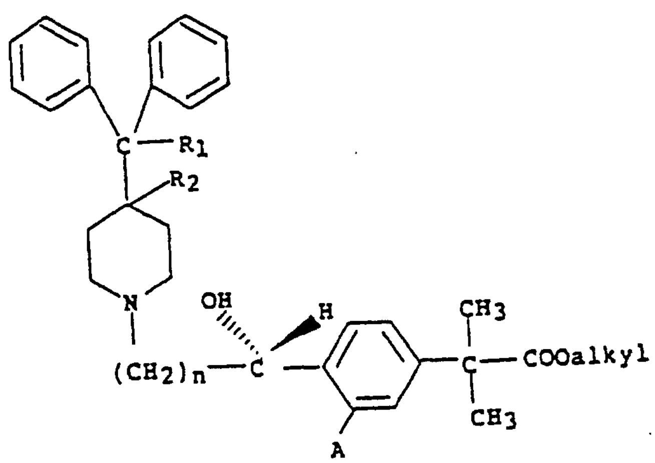

wobei $R_1$, $R_2$, n und Alkyl wie vorstehend definiert sind und A ein Wasserstoffatom ist, zu bilden.

**19.** Verfahren zur Herstellung einer Verbindung der Formel

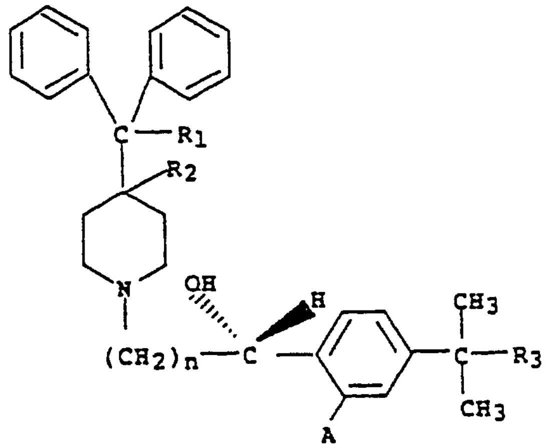

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

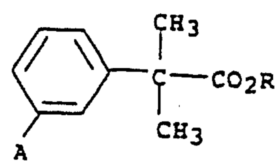

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

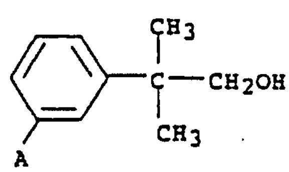

wobei A ein Wasserstoffatom ist, zu erhalten,

(b) Schützen der Hydroxyethylfunktionalität mit einer geeigneten Schutzgruppe, um einen geschützten Phenethylalkohol der Formel

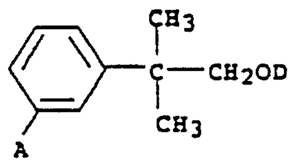

wobei A ein Wasserstoffatom ist und D eine geeignete Schutzgruppe ist, zu erhalten,

(c) Umsetzen des geschützten Phenethylalkohols mit einer ω-Halogenverbindung der Formel

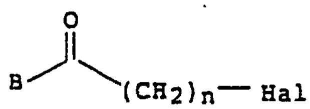

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

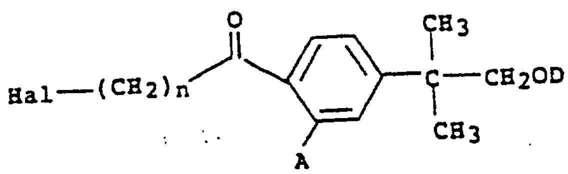

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

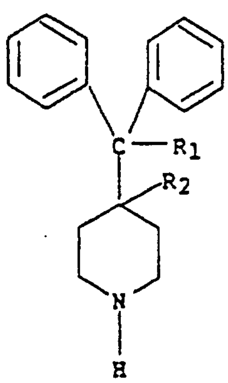

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

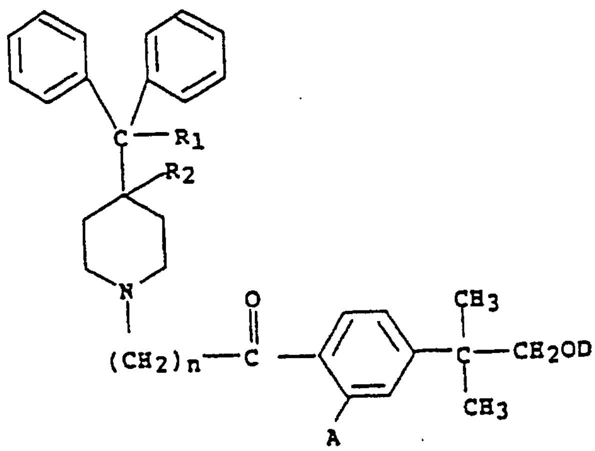

wobei $R_1$, $R_2$, n und D wie vorstehend definiert sind und A ein Wasserstoffatom ist, herzustellen,

(e) Schutzgruppenabspaltung der Hydroxyethylfunktionalität des geschützten Piperidinhydroxyethylphenylketons mit einem geeigneten Reagenz zur Schutzgruppenabspaltung, um ein Piperidinhydroxyethylphenylketon der Formel

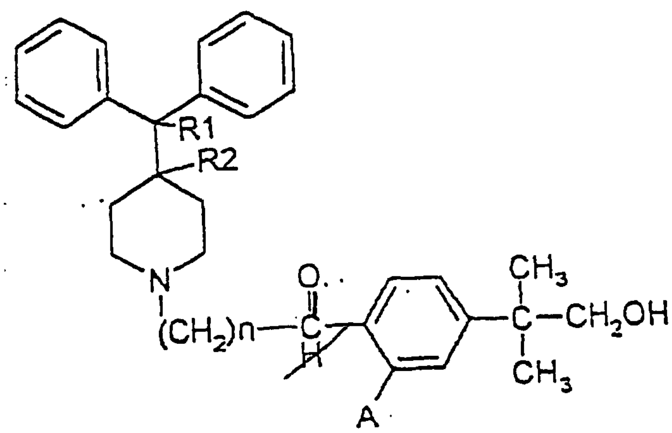

wobei $R_1$, $R_2$, n und A wie vorstehend definiert sind, herzustellen, und

(f) Umsetzen des Piperidinhydroxyethylphenylketons mit Chrom(IV)oxid, Kaliumpermanganat, Salpetersäure, Stickstoffdioxid, Ruthenium(VIII)oxid, Nickelperoxid, Silberoxid, t-Butylchromat oder Xenonsäure, um ein Piperidincarboxyphenylketon der Formel

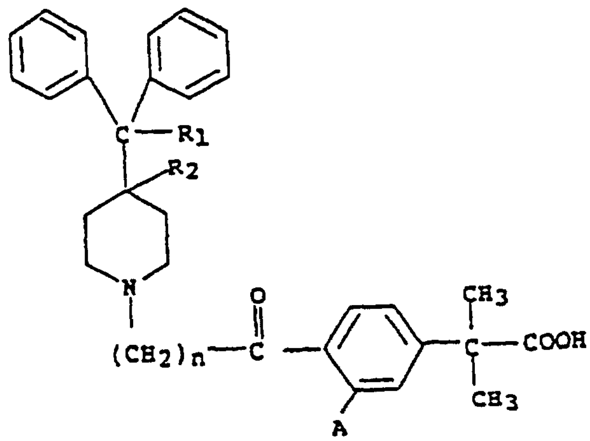

wobei $R_1$, $R_2$ und n wie vorstehend definiert sind und A ein Wasserstoffatom ist, herzustellen,

(g) Umsetzen des Piperidincarboxyphenylketons mit (-)-B-Chlordiisopinocamphenylboran, um einen Piperidincarboxyphenylalkohol der Formel

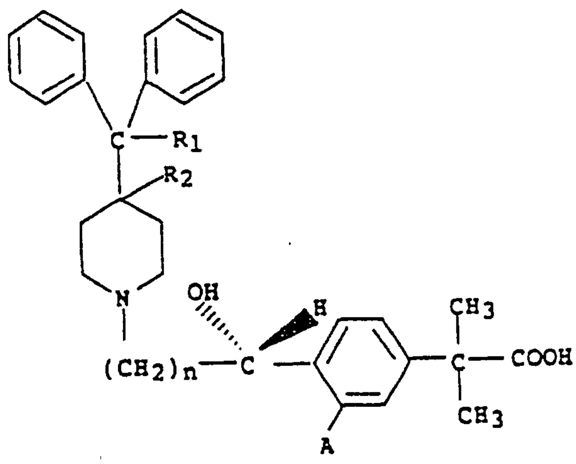

wobei $R_1$, $R_2$ und n wie vorstehend definiert sind und A ein Wasserstoffatom ist, herzustellen,

(h) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

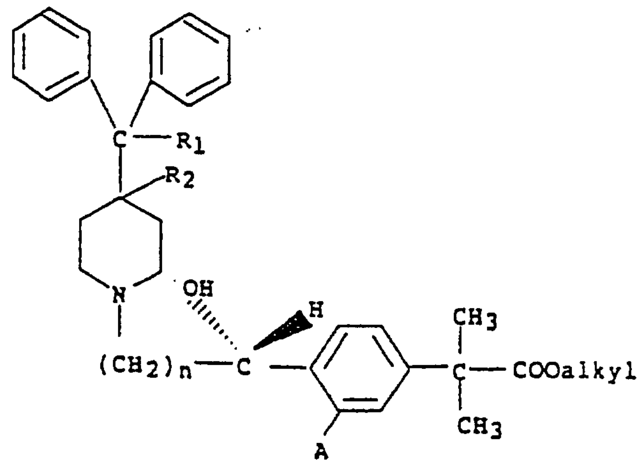

wobei $R_1$, $R_2$, n und Alkyl wie vorstehend definiert sind und A ein Wasserstoffatom ist, zu bilden.

**20.** Verfahren zur Herstellung einer Verbindung der Formel

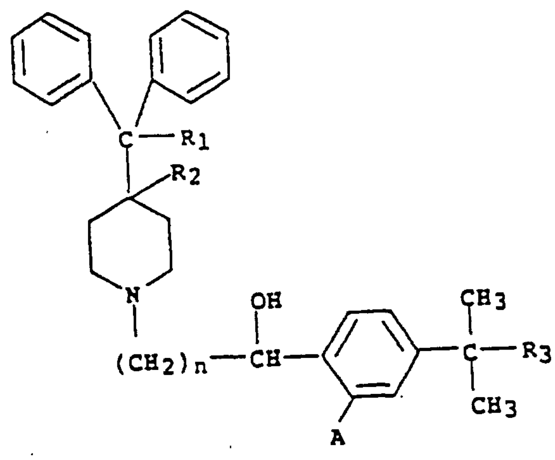

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

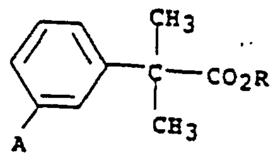

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

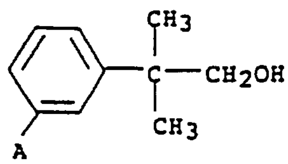

wobei A ein Wasserstoffatom ist, zu erhalten,

(b) Schützen der Hydroxyethylfunktionalität mit einer geeigneten Schutzgruppe, um einen geschützten Phenethylalkohol der Formel

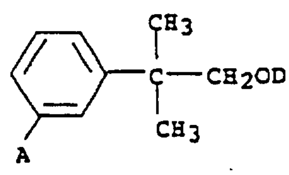

wobei A ein Wasserstoffatom ist und D eine geeignete Schutzgruppe ist, zu erhalten,

(c) Umsetzen des geschützten Phenethylalkohols mit einer ω-Halogenverbindung der Formel

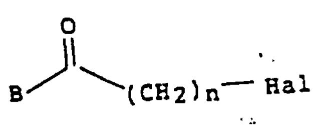

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titanteträchlorid, Bortrifluorid, Zinntetrachlorid und Zinkchlorid, um ein geschütztes ω-Halogenhydroxyethylphenylketon der Formel

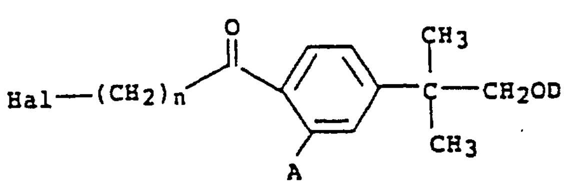

wobei Hal, n und D wie vorstehend definiert sind und A ein Wasserstoffatom ist, herzustellen,

(d) Umsetzen des geschützten ω-Halogenhydroxyethylphenylketons mit Natriumborhydrid, Kaliumborhydrid, Natriumcyanoborhydrid, Tetramethylammoniumborhydrid, Lithiumtri-tert-butylaluminohydrid, Diisobutylaluminiumhydrid oder katalytische Hydrierung, um einen geschützten ω-Halogenhydroxyethylphenylalkohol der Formel

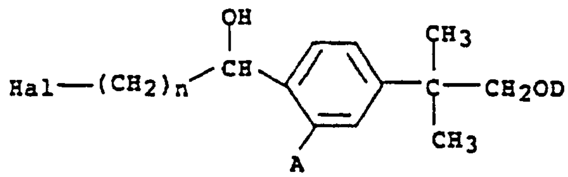

wobei Hal, n und D wie vorstehend definiert sind und A ein Wasserstoffatom ist, herzustellen,

(e) Umsetzen des geschützten ω-Halogenhydroxyethylphenylalkohols mit einer Piperidinverbindung der Formel

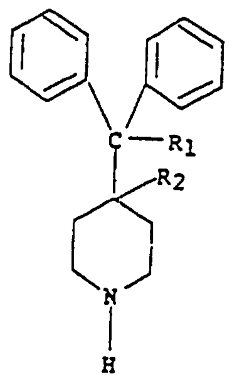

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um einen geschützten Piperidinhydroxyethylphenylalkohol der Formel

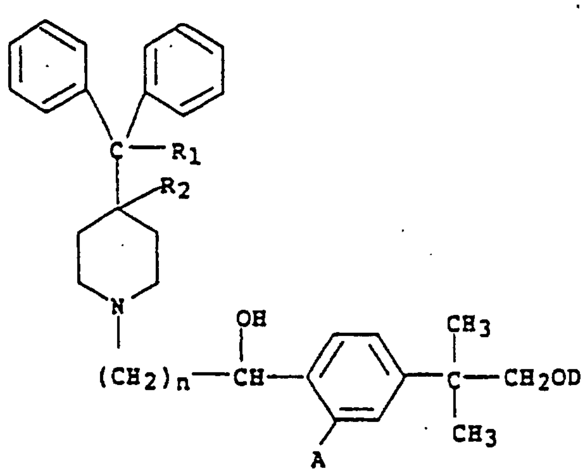

wobei $R_1$, $R_2$, n und D wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

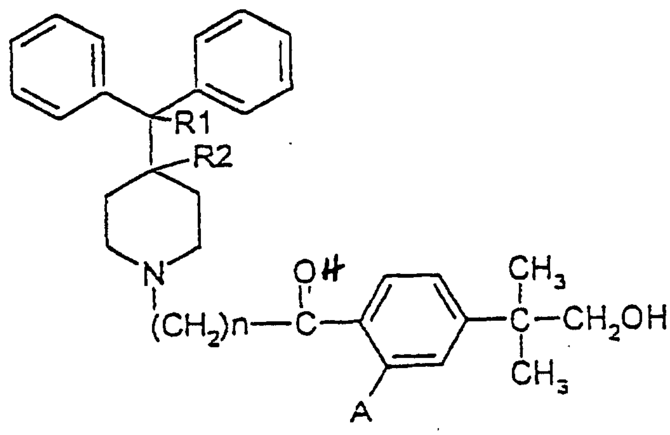

wobei $R_1$, $R_2$ und n wie vorstehend definiert sind und A ein Wasserstoffatom ist, herzustellen,

(g) Umsetzen des Piperidinhydroxyethylphenylalkohols mit Dimethylsulfoxid, Oxyalylchlorid und Triethylamin oder mit Dess-Martin-Reagenz, Chrom(IV)oxid, Nickelperoxid, Natriumdichromat, Kaliumdichromat, t-Butylchromat, Silberoxid, Silberpicolinatmangandioxidbleitetraacetat, Dicyclohexylcarbodiimid, 2,3-Dichlor-5,6-dicyanochinon, Tetrachlor-1,2-benzochinon, 2,2,6,6-Tetramethylpiperidinyl-1-oxy (TEMPO) oder Chinoliniumchlorchromat, um einen Piperidinformylphenylalkohol der Formel

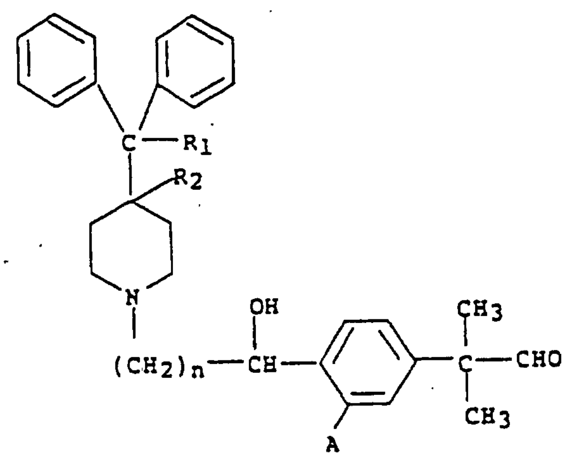

wobei $R_1$, $R_2$ und n wie vorstehend definiert sind und A ein Wasserstoffatom ist, herzustellen,

(h) Umsetzen des Piperidinformylphenylalkohols mit Kaliumpermanganat, Chrom(IV)oxid, Silber(I)oxid, Silberoxid, Silberpicolinat, Peroxid, Salpetersäure, m-Chlorperbenzoesäure und Peressigsäure, um einen Piperidincarboxyphenylalkohol der Formel

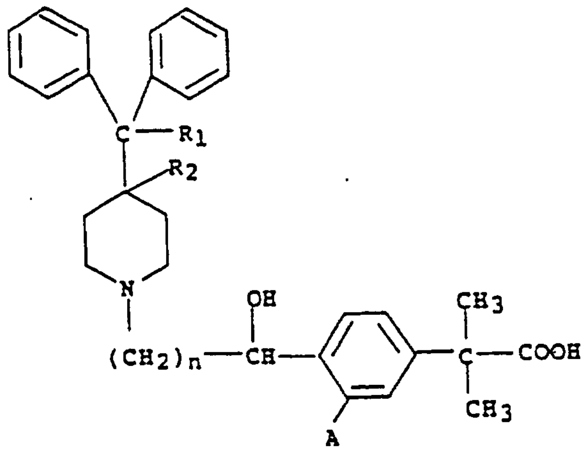

wobei $R_1$, $R_2$ und n wie vorstehend definiert sind und A ein Wasserstoffatom ist, herzustellen,

(i) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

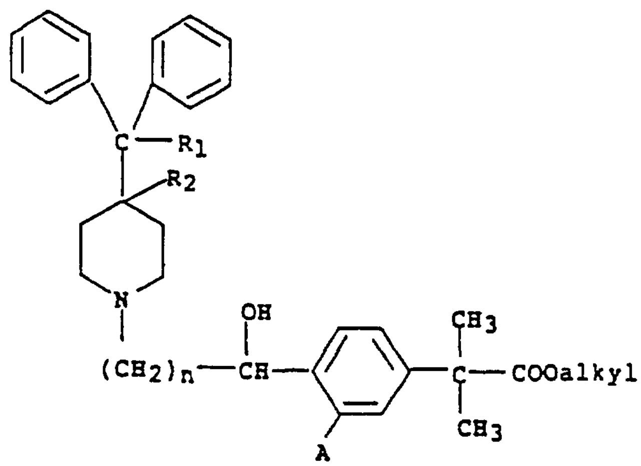

wobei $R_1$, $R_2$, n und Alkyl wie vorstehend definiert sind und A ein Wasserstoffatom ist, zu bilden.

**21.** Verfahren zur Herstellung einer Verbindung der Formel

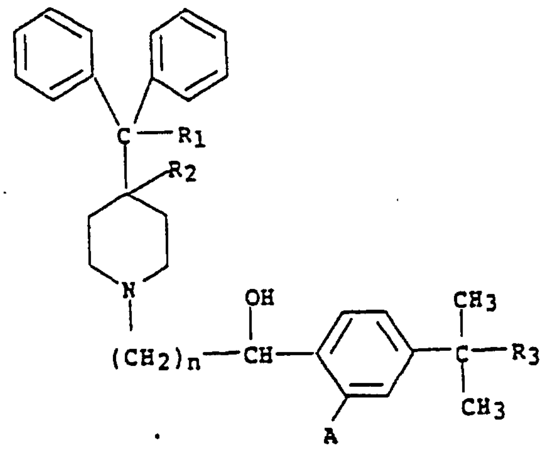

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

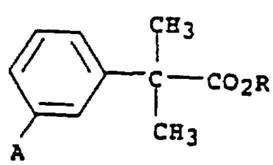

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

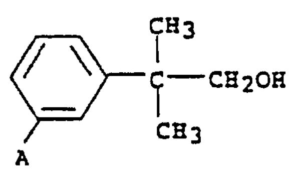

wobei A ein Wasserstoffatom ist, zu erhalten,

(b) Schützen der Hydroxyethylfunktionalität mit einer geeigneten Schutzgruppe, um einen geschützten Phenethylalkohol der Formel

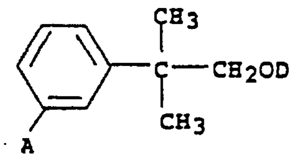

wobei A ein Wasserstoffatom ist und D eine geeignete Schutzgruppe ist, zu erhalten,

(c) Umsetzen des geschützten Phenethylalkohols mit einer ω-Halogenverbindung der Formel

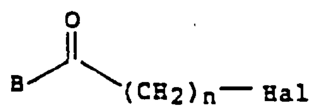

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

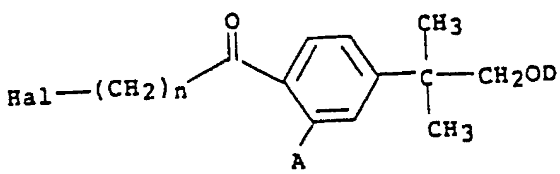

wobei Hal, n und D wie vorstehend definiert sind und A ein Wasserstoffatom ist, herzustellen,

(d) Umsetzen des geschützten ω-Halogenhydroxyethylphenylketons mit Natriumborhydrid, Kaliumborhydrid, Natriumcyanoborhydrid, Tetramethylammoniumborhydrid, Lithiumtri-tert-butylaluminohydrid, Diisobutylaluminiumhydrid oder katalytische Hydrierung, um einen geschützten ω-Halogenhydroxyethylphenylalkohol der Formel

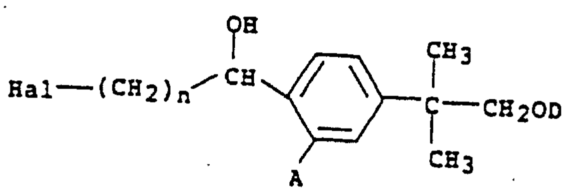

wobei Hal, n und D wie vorstehend definiert sind und A ein Wasserstoffatom ist, herzustellen,

(e) Umsetzen des geschützten ω-Halogenhydroxyethylphenylalkohols mit einer Piperidinverbindung der Formel

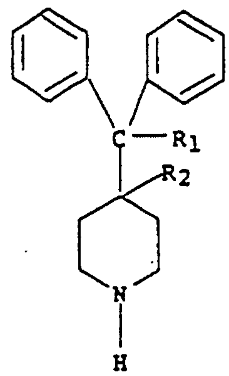

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um einen geschützten Piperidinhydroxyethylphenylalkohol der Formel

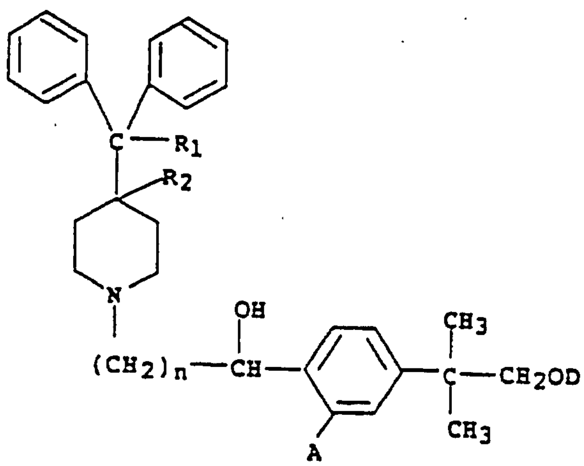

wobei $R_1$, $R_2$, n und D wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

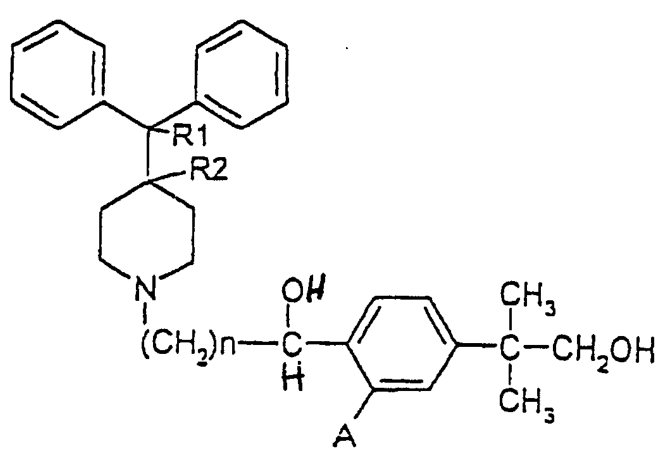

wobei $R_1$, $R_2$ und n wie vorstehend definiert sind und A ein Wasserstoffatom ist, herzustellen,

(g) Umsetzen des Piperidinhydroxyethylphenylalkohols mit Chrom(IV)oxid, Kaliumpermanganat, Salpetersäure, Stickstoffdioxid, Ruthenium(VIII)oxid, Nickelperoxid, Silberoxid, t-Butylchromat oder Xenonsäure, um einen Piperidinhydroxyethylphenylalkohol der Formel

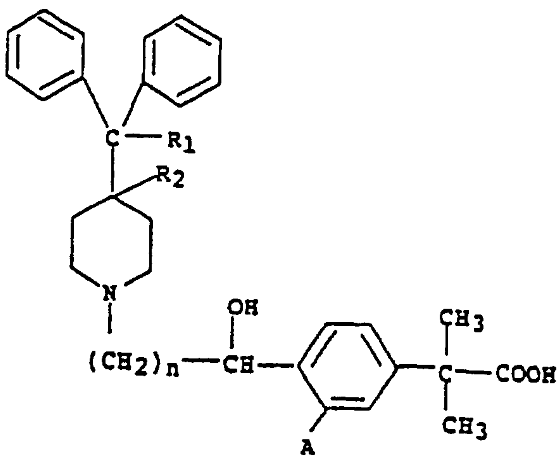

wobei $R_1$, $R_2$ und n wie vorstehend definiert sind und A ein Wasserstoffatom ist, herzustellen,

(h) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

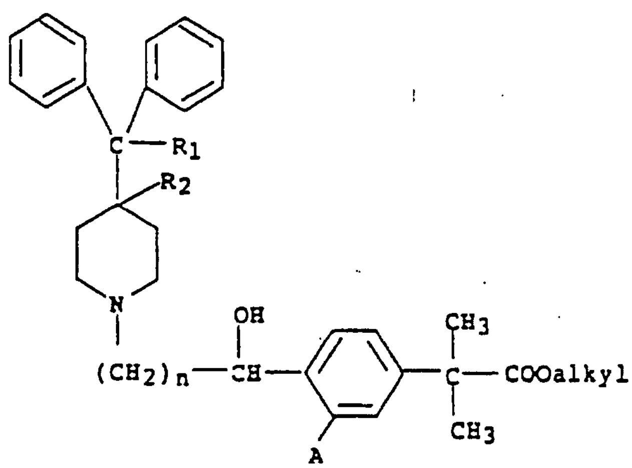

wobei $R_1$, $R_2$, n und Alkyl wie vorstehend definiert sind und A ein Wasserstoffatom ist, zu bilden.

**22.** Verfahren gemäß Anspruch 21, wobei der in Schritt f hergestellte Piperidinhydroxyethylphenylalkohol mit Ruthenium(VIII)oxid umgesetzt wird, um einen Piperidincarboxyphenylalkohol von Schritt g zu erhalten.

**23.** Verfahren zur Herstellung einer Verbindung der Formel

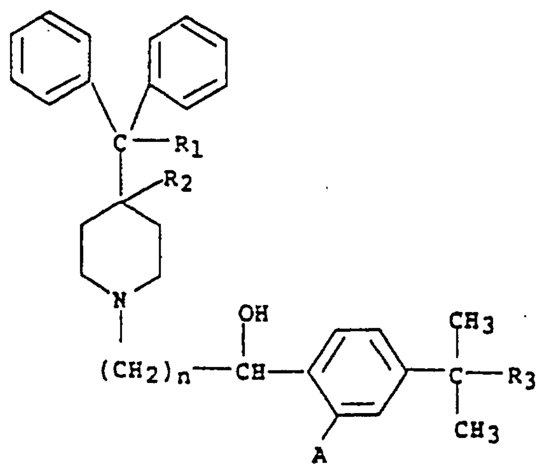

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe $-CH_2OH$ ist,
A ein Wasserstoffatom ist und
pharmazeutisch verträgliche Salze davon,
umfassend die Schritte:

(a) Umsetzen einer Benzolessigsäureverbindung der Formel

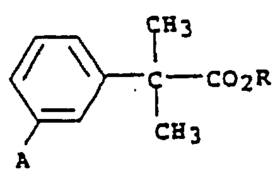

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

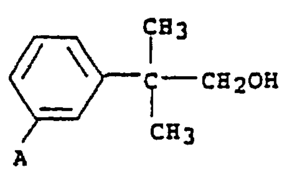

wobei A ein Wasserstoffatom ist, zu erhalten,

(b) Schützen der Hydroxyethylfunktionalität mit einer geeigneten Schutzgruppe, um einen geschützten Phenethylalkohol der Formel

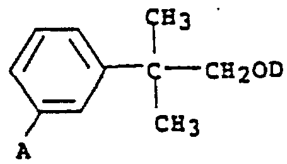

wobei A ein Wasserstoffatom ist und D eine geeignete Schutzgruppe ist, zu erhalten,

(c) Umsetzen des geschützten Phenethylalkohols mit einer ω-Halogenverbindung der Formel

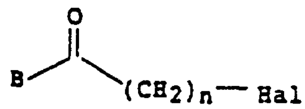

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

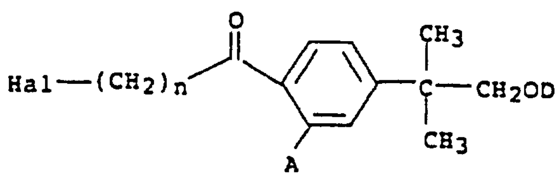

wobei Hal, n und D wie vorstehend definiert sind und A ein Wasserstoffatom ist, herzustellen,

(d) Umsetzen des geschützten ω-Halogenhydroxyethylphenylketons mit Natriumborhydrid, Kaliumborhydrid, Natriumcyanoborhydrid, Tetramethylammoniumborhydrid, Lithiumtri-tert-butylaluminohydrid, Diisobutylaluminiumhydrid oder katalytische Hydrierung, um einen geschützten Piperidinhydroxyethylphenylalkohol der Formel

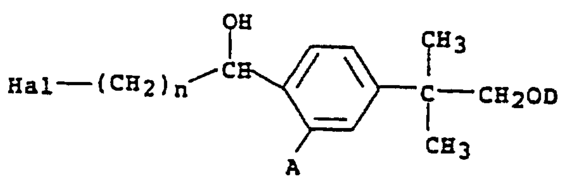

wobei Hal, n und D wie vorstehend definiert sind und A ein Wasserstoffatom ist, herzustellen,

(e) Umsetzen des geschützten ω-Halogenhydroxyethylphenylalkohols mit einer Piperidinverbindung der Formel

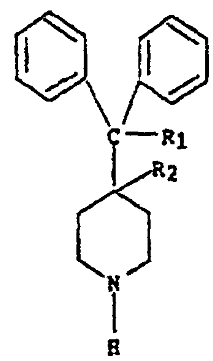

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um einen geschützten Piperidinhydroxyethylphenylalkohol der Formel

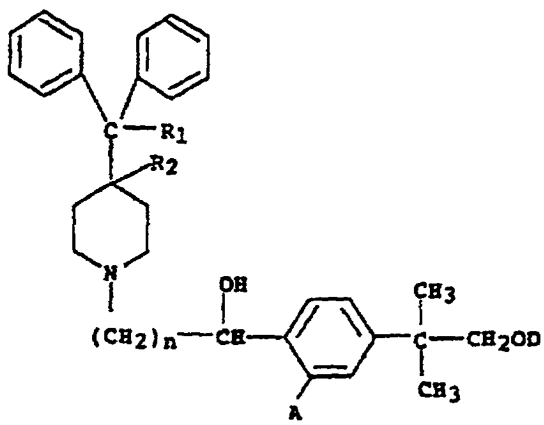

wobei $R_1$, $R_2$, n und D wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

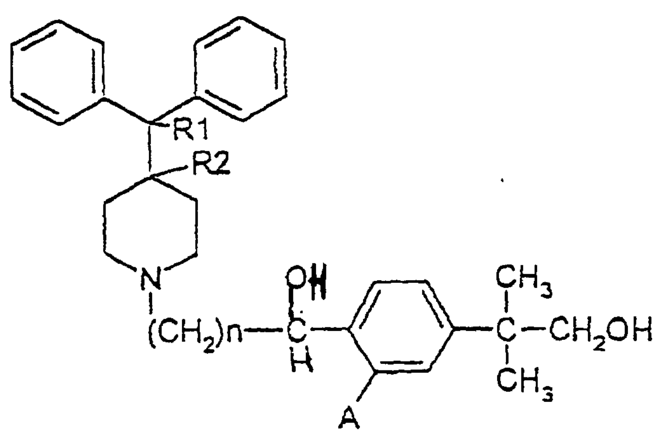

wobei $R_1$, $R_2$ und n wie vorstehend definiert sind und A ein Wasserstoffatom ist, herzustellen.

**24.** Verfahren zur Herstellung einer Verbindung der Formel

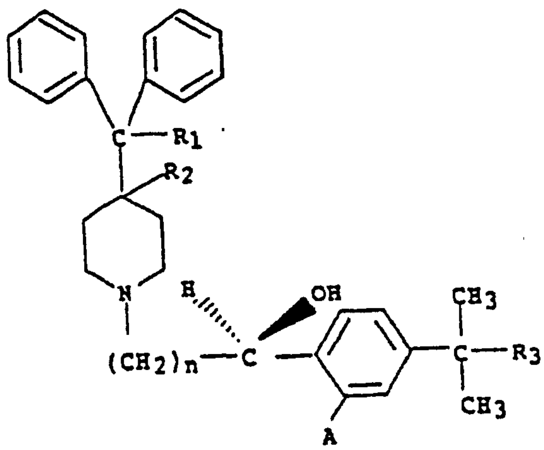

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

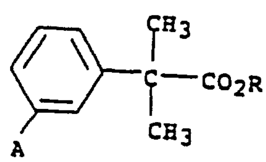

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

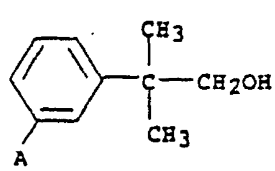

wobei A ein Wasserstoffatom ist, zu erhalten,

(b) Schützen der Hydroxyethylfunktionalität mit einer geeigneten Schutzgruppe, um einen geschützten Phenethyalkohol der Formel

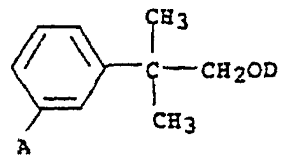

wobei A ein Wasserstoffatom ist und D eine geeignete Schutzgruppe ist, zu erhalten,

(c) Umsetzen des geschützten Phenethylalkohols mit einer ω-Halogenverbindung der Formel

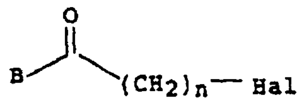

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

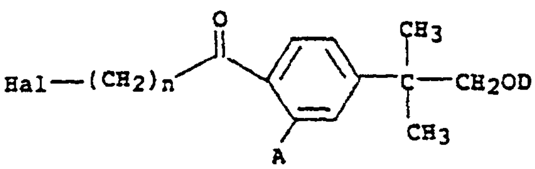

wobei Hal, n und D wie vorstehend definiert sind und A ein Wasserstoffatom ist, herzustellen,

(d) Umsetzen des geschützten ω-Halogenhydroxyethylphenylketons mit (+)-B-Chlordiisopinocamphenylboran, um einen geschützten Piperidinhydroxyethylphenylalkohol der Formel

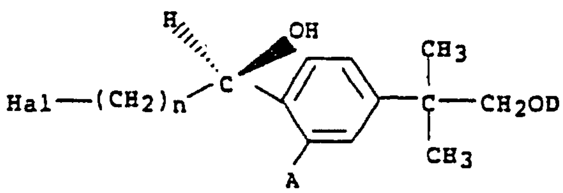

wobei Hal, n und D wie vorstehend definiert sind und A ein Wasserstoffatom ist, herzustellen,

(e) Umsetzen des geschützten ω-Halogenhydroxyethylphenylalkohols mit einer Piperidinverbindung der Formel

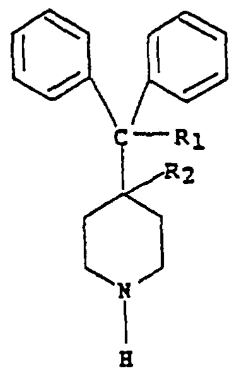

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um einen geschützten Piperidinhydroxyethylphenylalkohol der Formel

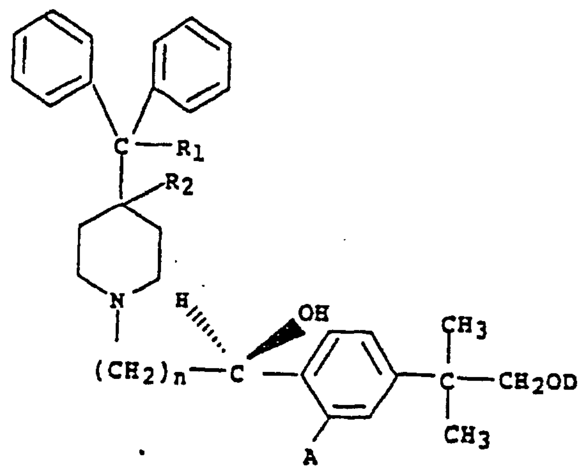

wobei $R_1$, $R_2$, n und D wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

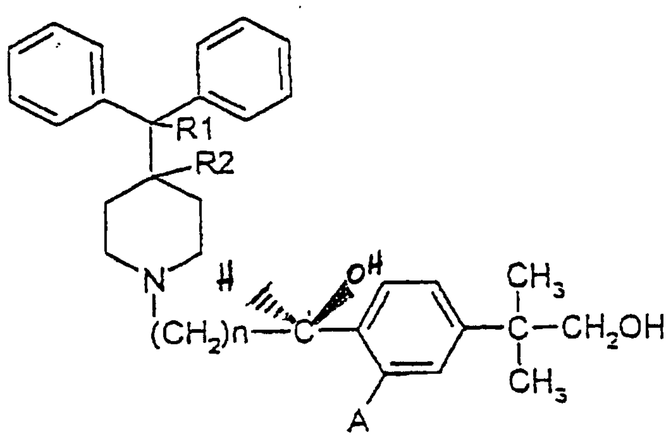

wobei $R_1$, $R_2$ und n wie vorstehend definiert sind und A ein Wasserstoffatom ist, herzustellen,

(g) Umsetzen des Piperidinhydroxyethylphenylalkohols mit Dimethylsulfoxid, Oxyalylchlorid und Triethylamin oder mit Dess-Martin-Reagenz, Chrom(IV)oxid, Nickelperoxid, Natriumdichromat, Kaliumdichromat, t-Butylchromat, Silberoxid, Silberpicolinatmangandioxidbleitetraacetat, Dicyclohexylcarbodiimid, 2,3-Dichlor-5,6-dicyanochinon, Tetrachlor-1,2-benzochinon, 2,2,6,6-Tetramethylpiperidinyl-1-oxy (TEMPO) oder Chinoliniumchlorchromat, um einen Piperidinformylphenylalkohol der Formel

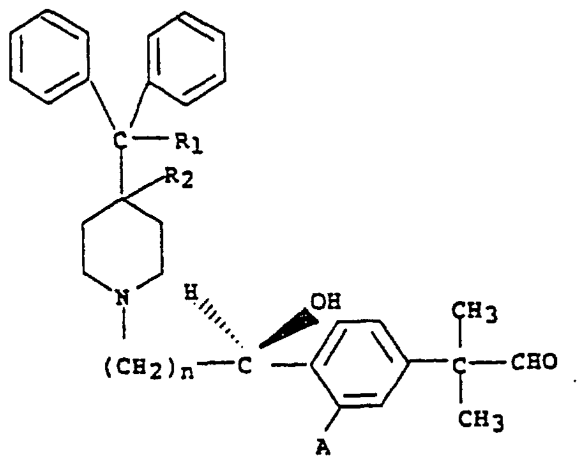

wobei $R_1$, $R_2$ und n wie vorstehend definiert sind und A ein Wasserstoffatom ist, herzustellen,

(h) Umsetzen des Piperidinformylphenylalkohols mit Kaliumpermanganat, Chrom(IV)oxid, Silber(I)oxid, Silberoxid, Silberpicolinat, Peroxid, Salpetersäure, m-Chlorperbenzoesäure und Peressigsäure, um einen Piperidincarboxyphenylalkohol der Formel

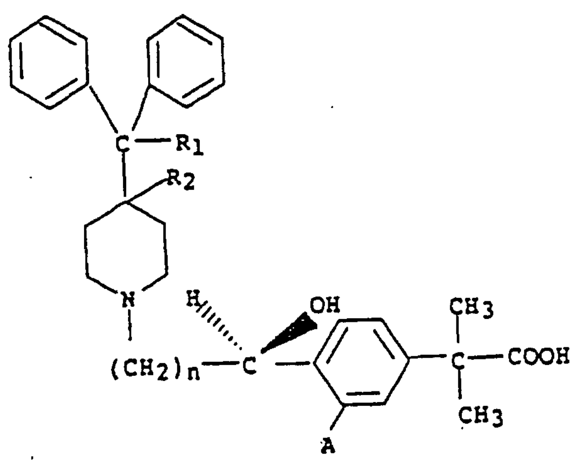

wobei $R_1$, $R_2$ und n wie vorstehend definiert sind und A ein Wasserstoffatom ist, herzustellen,

(i) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

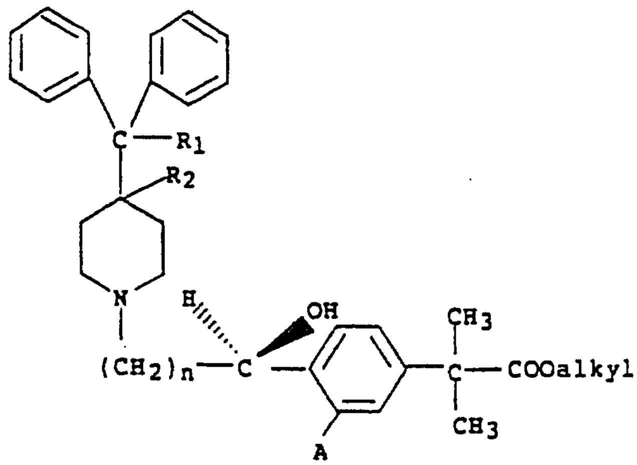

wobei $R_1$, $R_2$, n und Alkyl wie vorstehend definiert sind und A ein Wasserstoffatom ist, zu bilden.

**25.** Verfahren zur Herstellung einer Verbindung der Formel

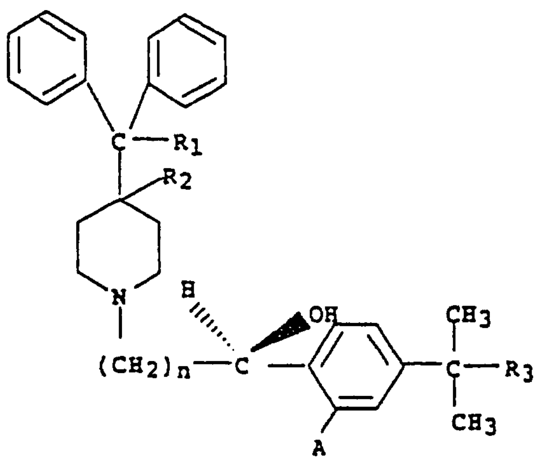

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

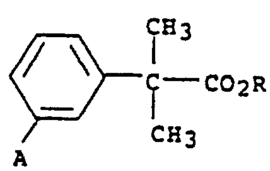

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

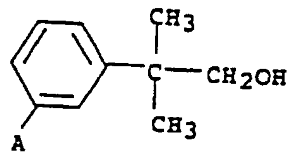

wobei A ein Wasserstoffatom ist, zu erhalten,

(b) Schützen der Hydroxyethylfunktionalität mit einer geeigneten Schutzgruppe, um einen geschützten Phenethylalkohol der Formel

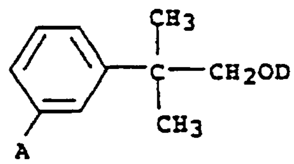

wobei A ein Wasserstoffatom ist und D eine geeignete Schutzgruppe ist, zu erhalten,

(c) Umsetzen des geschützten Phenethylalkohols mit einer ω-Halogenverbindung der Formel

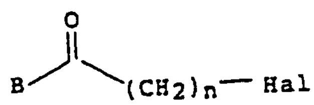

wobei B ein Halogenatom oder eine Hydroxygruppe ist, Hal Cl, Br oder I darstellt und n wie vorstehend definiert ist, in Anwesenheit von Trichlorid, Aluminiumchlorid, Titantetrachlorid, Bortrifluorid, Zinntetrachlorid und Zinkchlorid, um ein geschütztes ω-Halogenhydroxyethylphenylketon der Formel

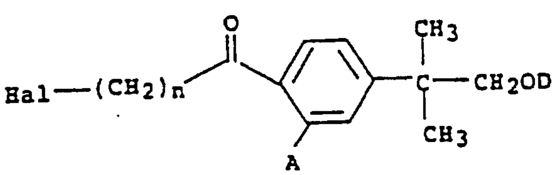

wobei Hal, n und D wie vorstehend definiert sind und A ein Wasserstoffatom ist, herzustellen,

(d) Umsetzen des geschützten ω-Halogenhydroxyethylphenylketons mit (+)-B-Chlordiisopinocamphenylboran, um einen geschützten ω-Halogenhydroxyethylphenylalkohol der Formel

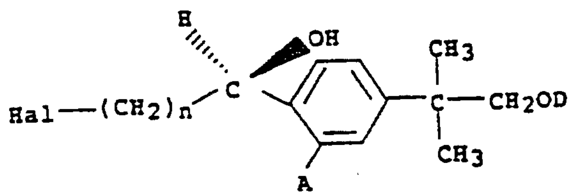

wobei Hal, n und D wie vorstehend definiert sind und A ein Wasserstoffatom ist, herzustellen,

(e) Umsetzen des geschützten ω-Halogenhydroxyethylphenylalkohols mit einer Piperidinverbindung der Formel

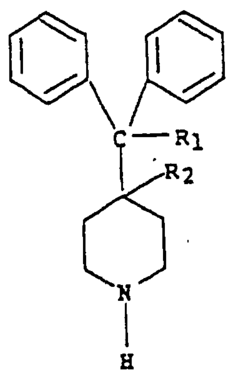

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um einen geschützten Piperidinhydroxyethylphenylalkohol der Formel

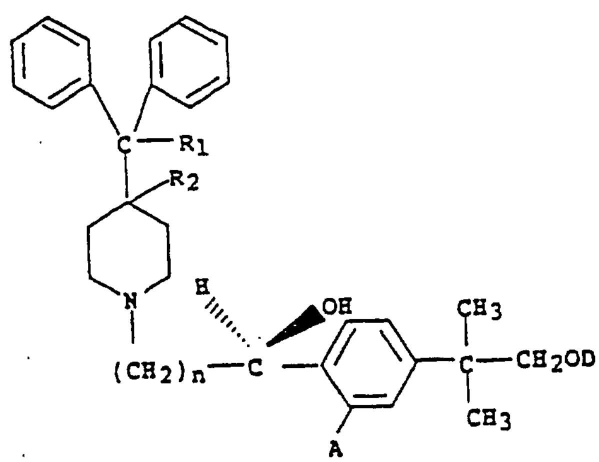

wobei $R_1$, $R_2$, n und D wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

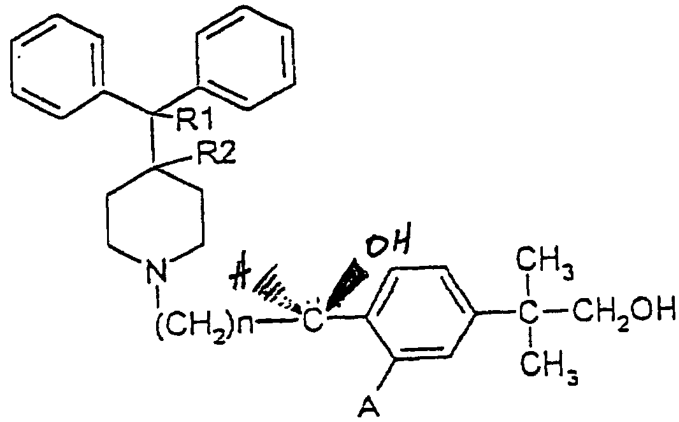

wobei $R_1$, $R_2$ und n wie vorstehend definiert sind und A ein Wasserstoffatom ist, herzustellen,

(g) Umsetzen des Piperidinhydroxyethylphenylalkohols mit Chrom(IV)oxid, Kaliumpermanganat, Salpetersäure, Stickstoffdioxid, Ruthenium(VIII)oxid, Nickelperoxid, Silberoxid, t-Butylchromat oder Xenonsäure, um einen Piperidincarboxyphenylalkohol der Formel

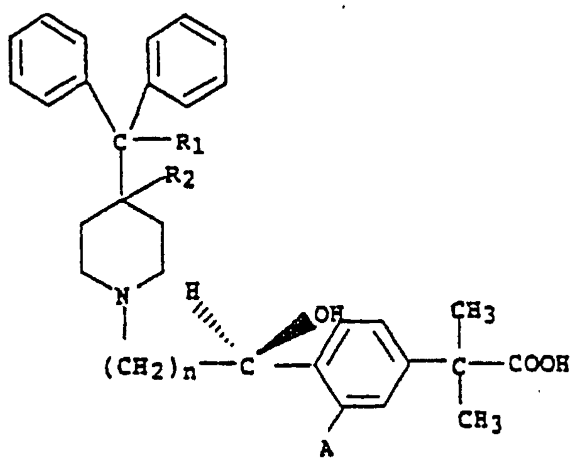

wobei $R_1$, $R_2$ und n wie vorstehend definiert sind und A ein Wasserstoffatom ist, herzustellen,

(h) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

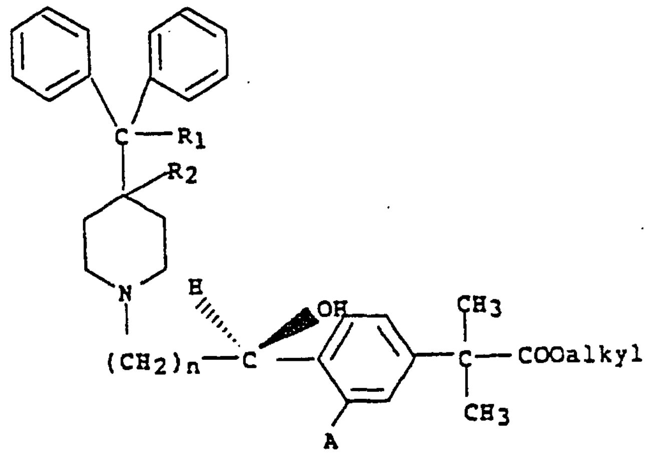

wobei $R_1$, $R_2$, n und Alkyl wie vorstehend definiert sind und A ein Wasserstoffatom ist, zu bilden.

**26.** Verfahren zur Herstellung einer Verbindung der Formel

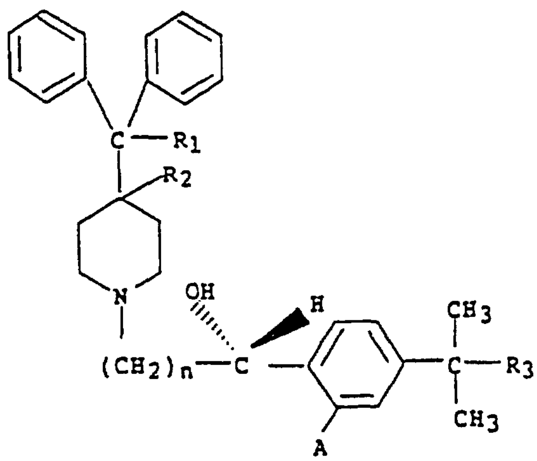

wobei A
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

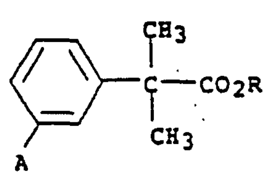

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

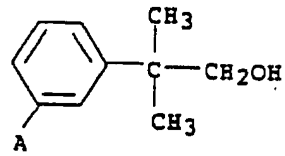

wobei A ein Wasserstoffatom ist, zu erhalten,

(b) Schützen der Hydroxyethylfunktionalität mit einer geeigneten Schutzgruppe, um einen geschützten Phenethylalkohol der Formel

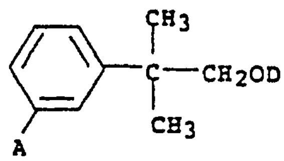

wobei A ein Wasserstoffatom ist und D eine geeignete Schutzgruppe ist, zu erhalten,

(c) Umsetzen des geschützten Phenethylalkohols mit einer ω-Halogenverbindung der Formel

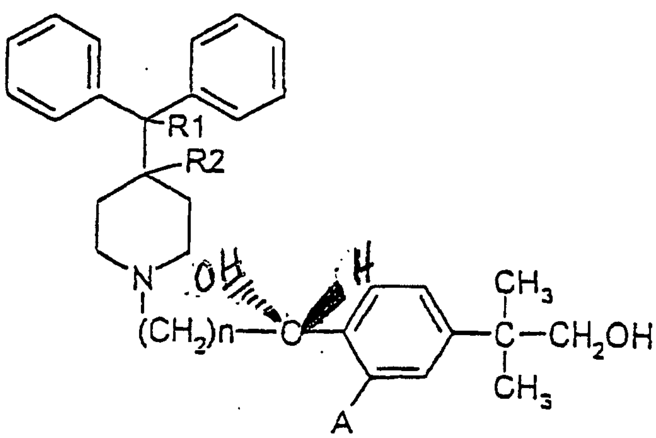

wobei $R_1$, $R_2$ und n wie vorstehend definiert sind und A ein Wasserstoffatom ist, herzustellen,

(g) Umsetzen des Piperidinhydroxyethylphenylalkohols mit Dimethylsulfoxid, Oxyalylchlorid und Triethylamin oder mit Dess-Martin-Reagenz, Chrom(IV)oxid, Nickelperoxid, Natriumdichromat, Kaliumdichromat, t-Butylchromat, Silberoxid, Silberpicolinatmangandioxidbleitetraacetat, Dicyclohexylcarbodiimid, 2,3-Dichlor-5,6-dicyanochinon, Tetrachlor-1,2-benzochinon, 2,2,6,6-Tetramethylpiperidinyl-1-oxy (TEMPO) oder Chinoliniumchlorchromat, um einen Piperidinformylphenylalkohol der Formel

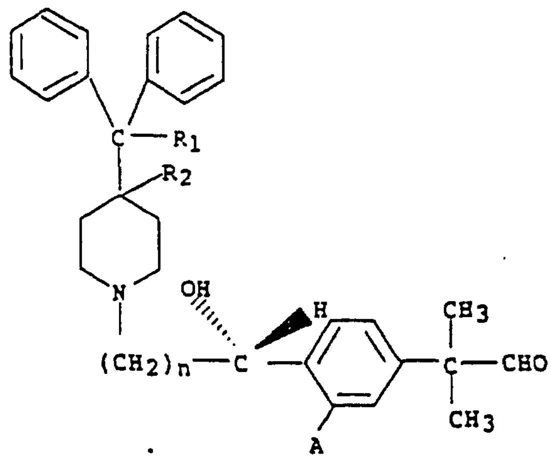

wobei $R_1$, $R_2$ und n wie vorstehend definiert sind und A ein Wasserstoffatom ist, herzustellen,

(h) Umsetzen des Piperidinformylphenylalkohols mit Kaliumpermanganat, Chrom(IV)oxid, Silber(I)oxid, Silberoxid, Silberpicolinat, Peroxid, Salpetersäure, m-Chlorperbenzoesäure und Peressigsäure, um einen Piperidincarboxyphenylalkohol der Formel

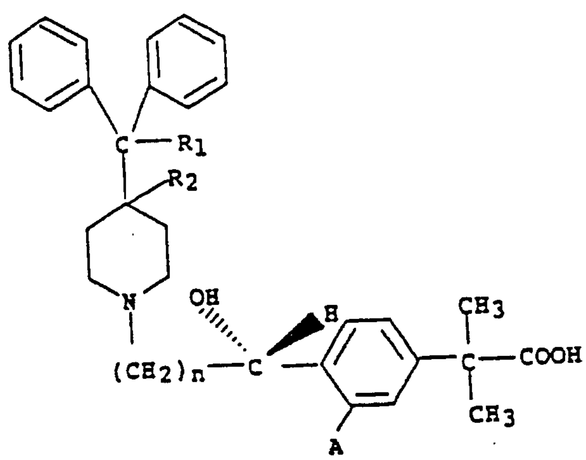

wobei $R_1$, $R_2$ und n wie vorstehend definiert sind und A ein Wasserstoffatom ist, herzustellen,

(i) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

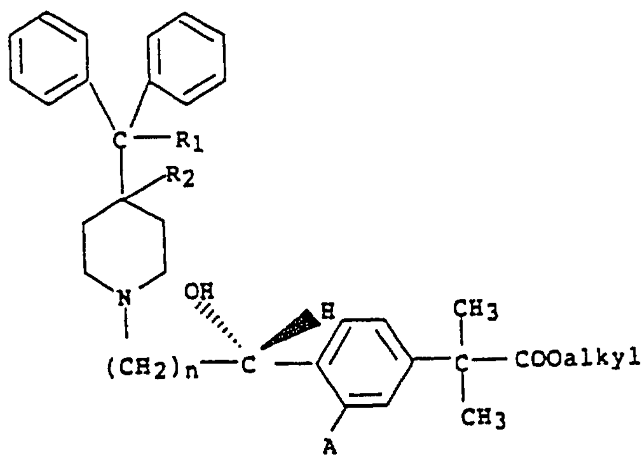

wobei $R_1$, $R_2$, n und Alkyl wie vorstehend definiert sind und A ein Wasserstoffatom ist, zu bilden.

**27.** Verfahren zur Herstellung einer Verbindung der Formel

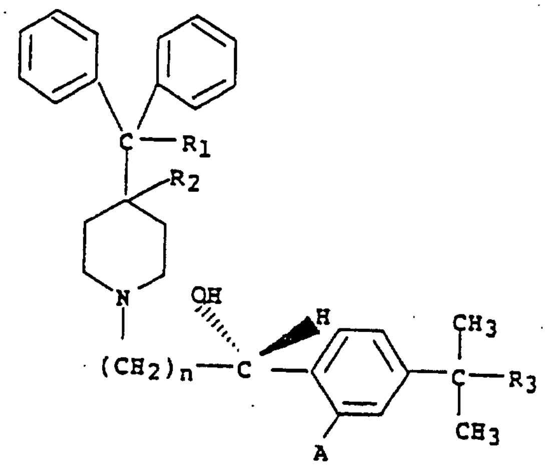

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

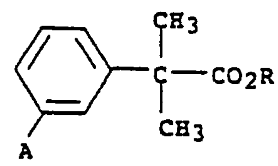

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

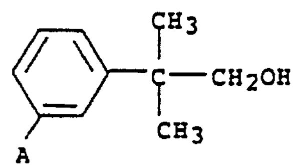

wobei A ein Wasserstoffatom ist, zu erhalten,

(b) Schützen der Hydroxyethylfunktionalität mit einer geeigneten Schutzgruppe, um einer geschützten Phenethylalkohol der Formel

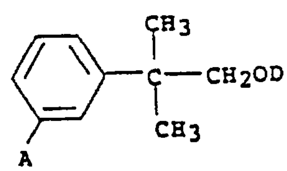

wobei A ein Wasserstoffatom ist und D eine geeignete Schutzgruppe ist, zu erhalten,

(c) Umsetzen des geschützten Phenethylalkohols mit einer ω-Halogenverbindung der Formel

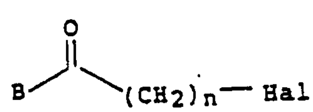

wobei B ein Halogenatom oder eine Hydroxygruppe ist, Hal Cl, Br oder I darstellt und n wie vorstehend definiert ist, in Anwesenheit von Trichlorid, Aluminiumchlorid, Titantetrachlorid, Bortrifluorid, Zinntetrachlorid und Zinkchlorid, um ein geschütztes ω-Halogenhydroxyethylphenylketon der Formel

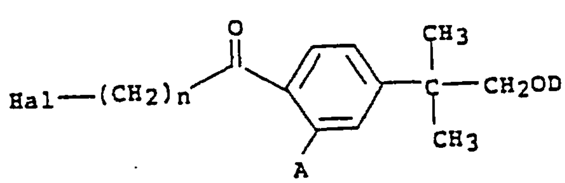

wobei Hal, n und D wie vorstehend definiert sind und A ein Wasserstoffatom ist, herzustellen,

(d) Umsetzen des geschützten ω-Halogenhydroxyethylphenylketons mit (-)-B-Chlordiisopinocamphenylboran, um einen geschützten ω-Halogenhydroxyethylphenylalkohol der Formel

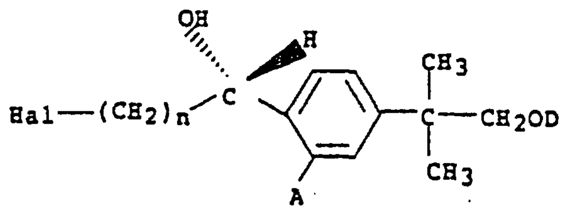

wobei Hal, n und D wie vorstehend definiert sind und A ein Wasserstoffatom ist, herzustellen,

(e) Umsetzen des geschützten ω-Halogenhydroxyethylphenylalkohols mit einer Piperidinverbindung der Formel

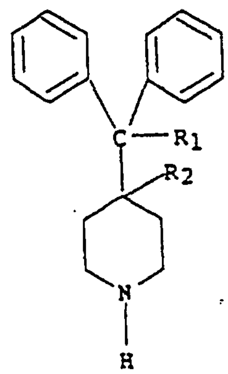

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um einen geschützten Piperidinhydroxyethylphenylalkohol der Formel

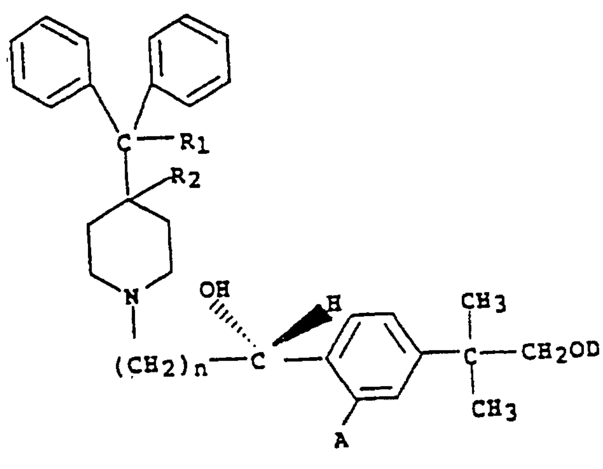

wobei $R_1$, $R_2$, n und D wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

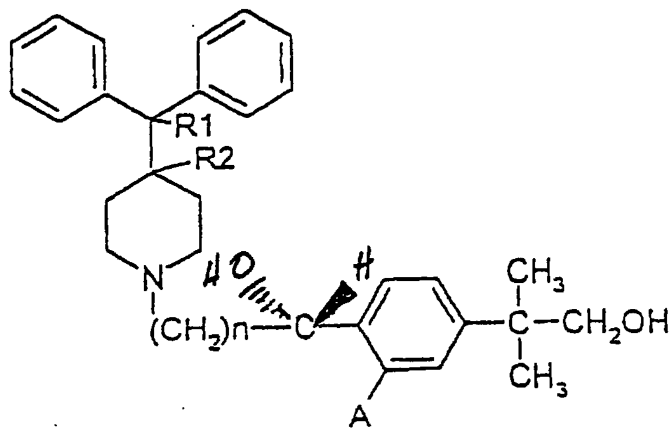

wobei $R_1$, $R_2$ und n wie vorstehend definiert sind und A ein Wasserstoffatom ist, herzustellen,

(g) Umsetzen des Piperidinhydroxyethylphenylalkohols mit Chrom(IV)oxid, Kaliumpermanganat, Salpetersäure, Stickstoffdioxid, Ruthenium(VIII)oxid, Nickelperoxid, Silberoxid, t-Butylchromat oder Xenonsäure, um einen Piperidincarboxyphenylalkohol der Formel

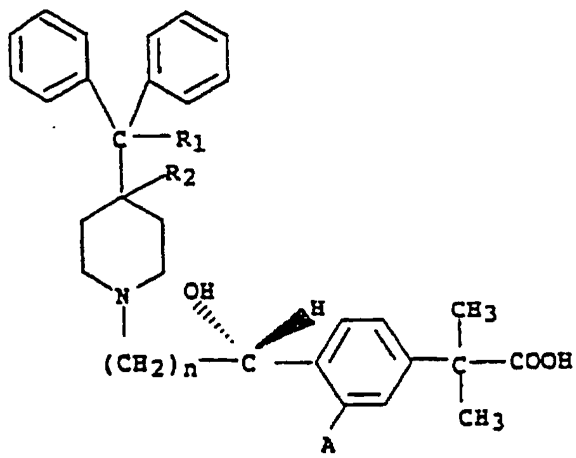

wobei $R_1$, $R_2$ und n wie vorstehend definiert sind und A ein Wasserstoffatom ist, herzustellen,

(h) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

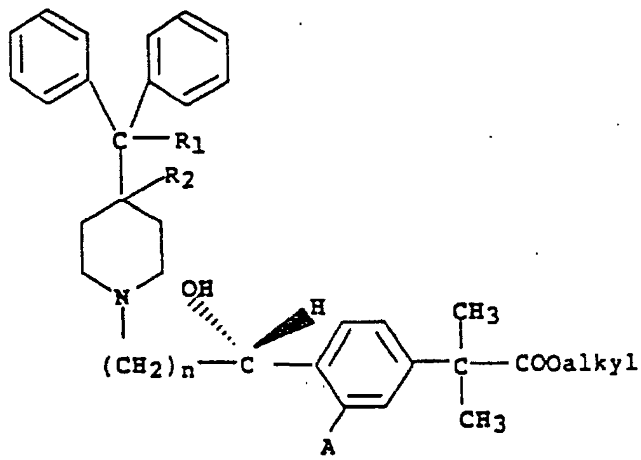

wobei $R_1$, $R_2$, n und Alkyl wie vorstehend definiert sind und A ein Wasserstoffatom ist, zu bilden.

**28.** Verfahren zur Herstellung einer Verbindung der Formel

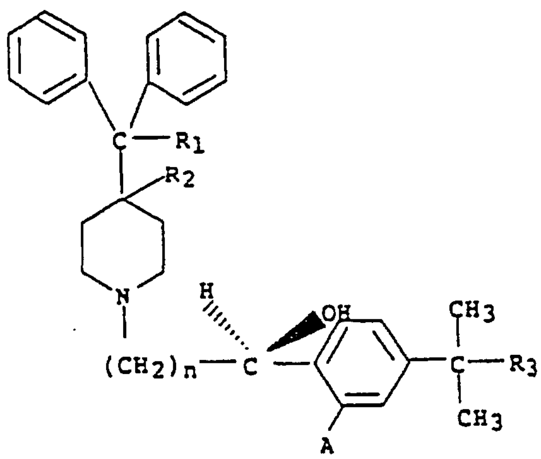

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe $-CH_2OH$ ist,
A ein Wasserstoffatom ist und
pharmazeutisch verträgliche Salze davon,
umfassend die Schritte:

(a) Umsetzen einer Benzolessigsäureverbindung der Formel

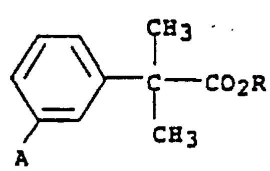

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

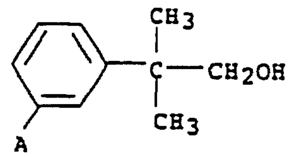

wobei A ein Wasserstoffatom ist, zu erhalten,

(b) Schützen der Hydroxyethylfunktionalität mit einer geeigneten Schutzgruppe, um einen geschützten Phenethylalkohol der Formel

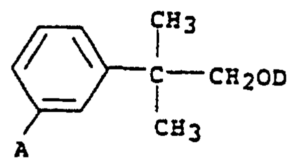

wobei A ein Wasserstoffatom ist und D eine geeignete Schutzgruppe ist, zu erhalten,

(c) Umsetzen des geschützten Phenethylalkohols mit einer ω-Halogenverbindung der Formel

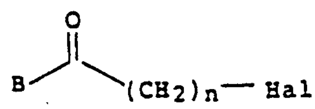

wobei B ein Halogenatom oder eine Hydroxygruppe ist, Hal Cl, Br oder I darstellt und n wie vorstehend definiert ist, in Anwesenheit von Trichlorid, Aluminiumchlorid, Titantetrachlorid, Bortrifluorid, Zinntetrachlorid und Zinkchlorid, um ein geschütztes ω-Halogenhydroxyethylphenylketon der Formel

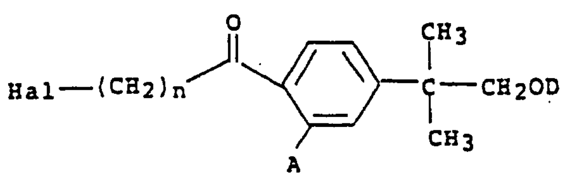

wobei Hal, n und D wie vorstehend definiert sind und A ein Wasserstoffatom ist, herzustellen,

(d) Umsetzen des geschützten ω-Halogenhydroxyethylphenylketons mit (+)-B-Chlordiisopinocamphenylboran, um einen geschützten ω-Halogenhydroxyethylphenylalkohol der Formel

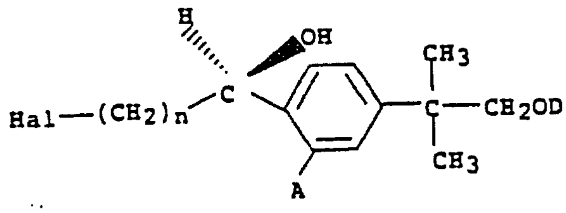

wobei Hal, n und D wie vorstehend definiert sind und A ein Wasserstoffatom ist, herzustellen,

(e) Umsetzen des geschützten ω-Halogenhydroxyethylphenylalkohols mit einer Piperidinverbindung der Formel

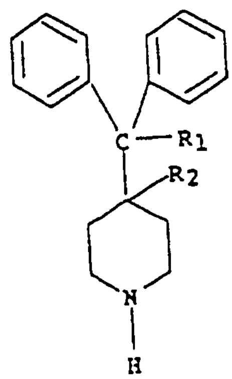

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um einen geschützten Piperidinhydroxyethylphenylalkohol der Formel

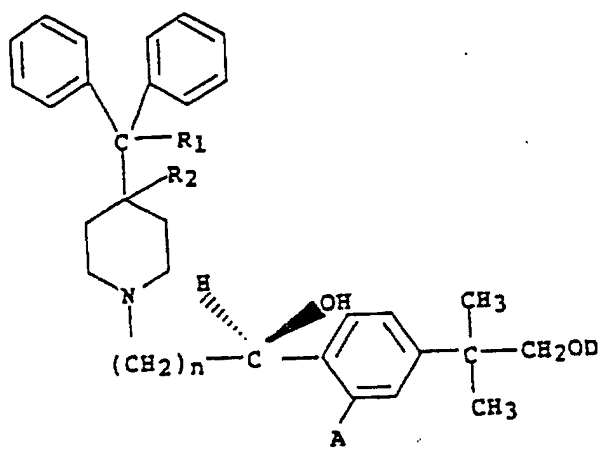

wobei $R_1$, $R_2$, n und D wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

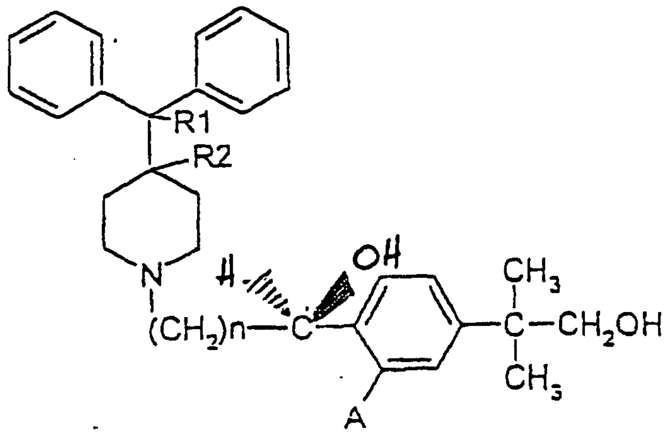

wobei $R_1$, $R_2$ und n wie vorstehend definiert sind und A ein Wasserstoffatom ist, herzustellen.

**29.** Verfahren zur Herstellung einer Verbindung der Formel

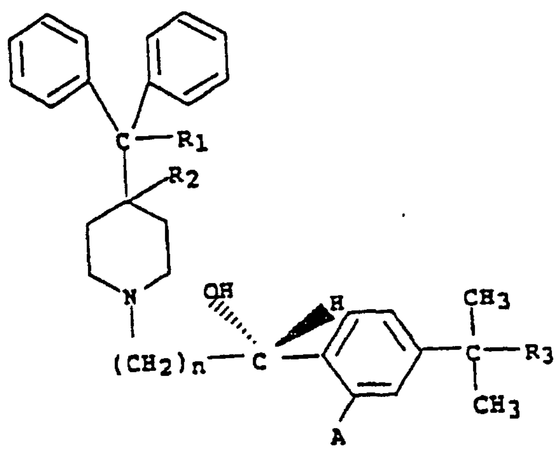

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe $-CH_2OH$ ist,
A ein Wasserstoffatom ist und
pharmazeutisch verträgliche Salze davon,
umfassend die Schritte:

(a) Umsetzen einer Benzolessigsäureverbindung der Formel

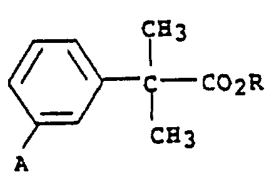

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

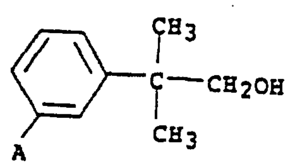

wobei A ein Wasserstoffatom ist, zu erhalten,

(b) Schützen der Hydroxyethylfunktionalität mit einer geeigneten Schutzgruppe, um einen geschützten Phenethylalkohol der Formel

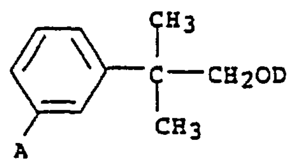

wobei A ein Wasserstoffatom ist und D eine geeignete Schutzgruppe ist, zu erhalten,

(c) Umsetzen des geschützten Phenethylalkohols mit einer ω-Halogenverbindung der Formel

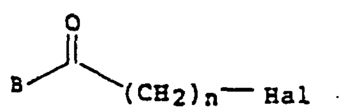

wobei B ein Halogenatom oder eine Hydroxygruppe ist, Hal Cl, Br oder I darstellt und n wie vorstehend definiert ist, in Anwesenheit von Trichlorid, Aluminiumchlorid, Titantetrachlorid, Bortrifluorid, Zinntetrachlorid und Zinkchlorid, um ein geschütztes ω-Halogenhydroxyethylphenylketon der Formel

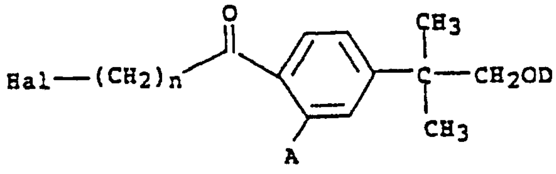

wobei Hal, n und D wie vorstehend definiert sind und A ein Wasserstoffatom ist, herzustellen,

(d) Umsetzen des geschützten ω-Halogenhydroxyethylphenylketons mit (-)-B-Chlordiisopinocamphenylboran, um einen geschützten ω-Halogenhydroxyethylphenylalkohol der Formel

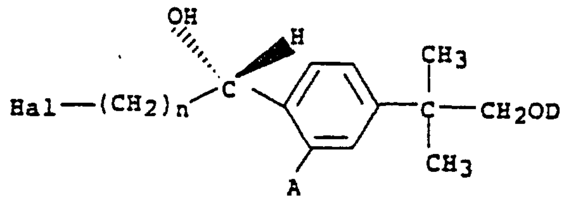

wobei Hal, n und D wie vorstehend definiert sind und A ein Wasserstoffatom ist, herzustellen,

(e) Umsetzen des geschützten ω-Halogenhydroxyethylphenylalkohols mit einer Piperidinverbindung der Formel

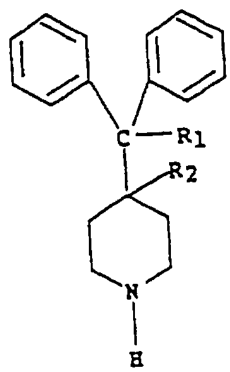

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um einen geschützten Piperidinhydroxyethylphenylalkohol der Formel

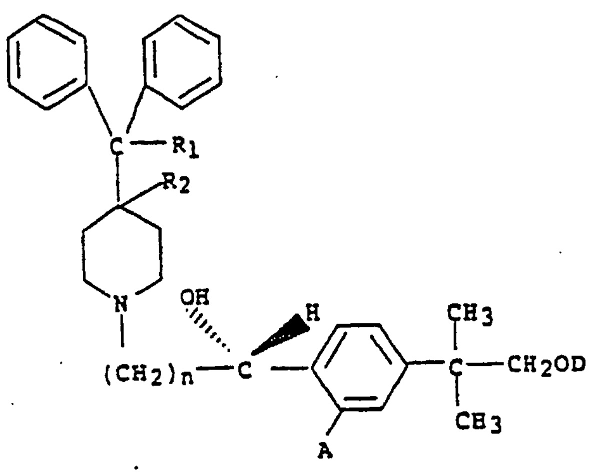

wobei $R_1$, $R_2$, n und D wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

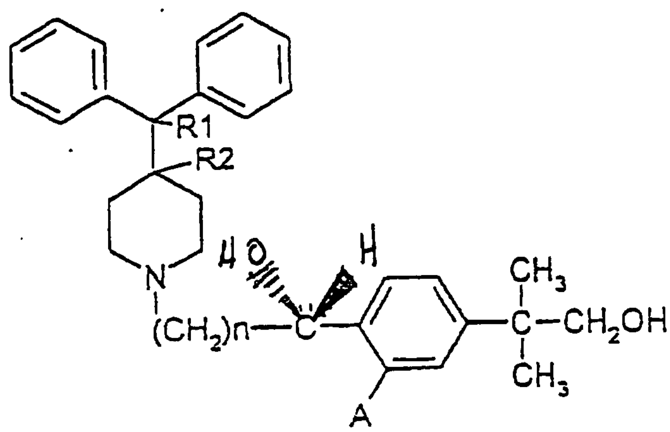

wobei $R_1$, $R_2$ und n wie vorstehend definiert sind und A ein Wasserstoffatom ist, herzustellen.

**30.** Verfahren zur Herstellung einer Verbindung der Formel

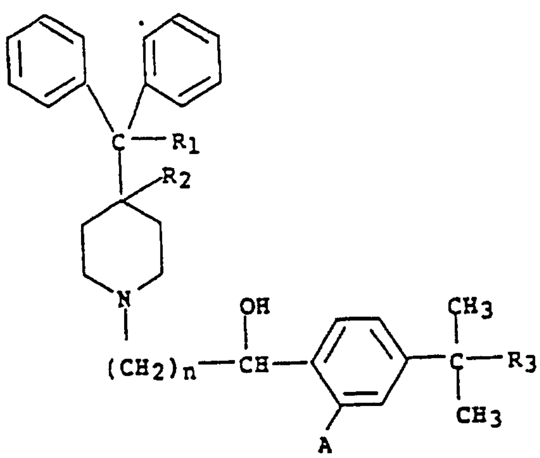

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

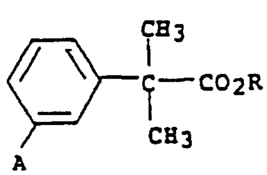

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

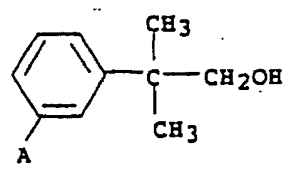

wobei A ein Wasserstoffatom ist, zu erhalten,

(b) Schützen der Hydroxyethylfunktionalität mit einer geeigneten Schutzgruppe, um einen geschützten Phenethylalkohol der Formel

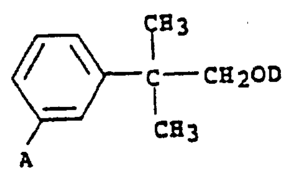

wobei A ein Wasserstoffatom ist und D eine geeignete Schutzgruppe ist, zu erhalten,

(c) Umsetzen des geschützten Phenethylalkohols mit einer ω-Halogenverbindung der Formel

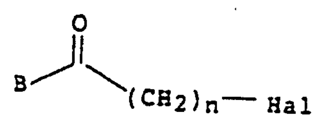

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

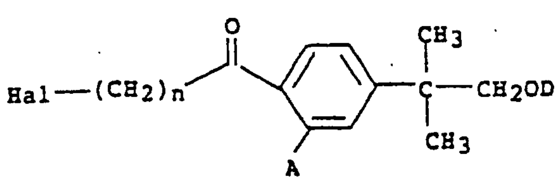

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

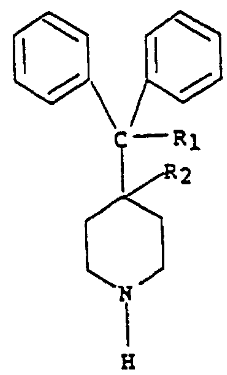

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

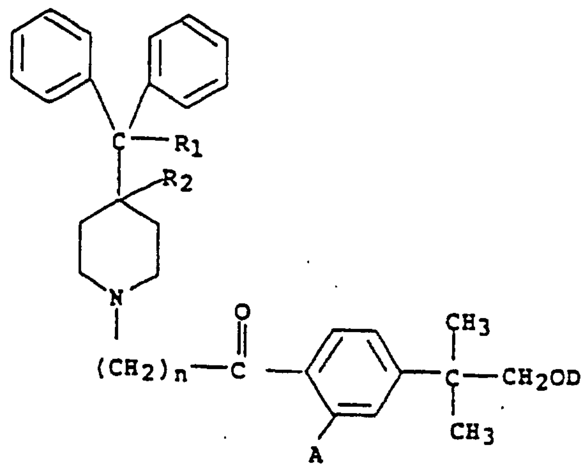

wobei $R_1$, $R_2$, n und D wie vorstehend definiert sind und A ein Wasserstoffatom ist, herzustellen,

(e) Umsetzen des geschützten Piperidinhydroxyethylphenylketons mit Natriumborhydrid, Kaliumborhydrid, Natriumcyanoborhydrid, Tetramethylammoniumborhydrid, Lithiumtri-tert-butylaluminohydrid, Diisobutylaluminiumhydrid oder katalytische Hydrierung, um einen geschützten Piperidinhydroxyethylphenylalkohol der Formel

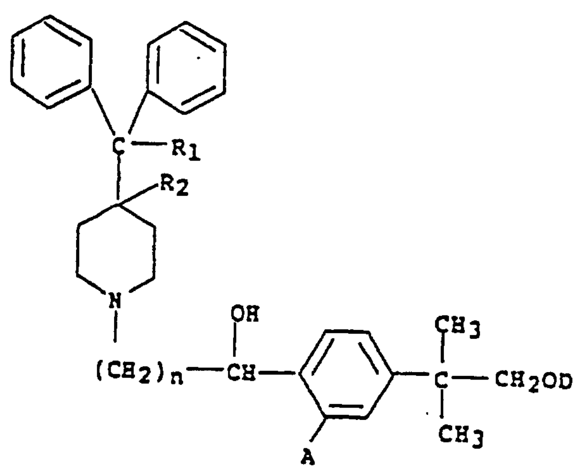

wobei $R_1$, $R_2$, D und n wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

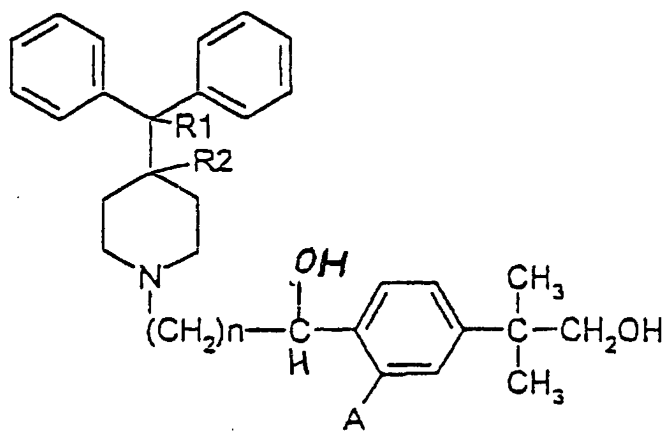

wobei $R_1$, $R_2$ und n wie vorstehend definiert sind und A ein Wasserstoffatom ist, herzustellen,

(g) Umsetzen des Piperidinhydroxyethylphenylalkohols mit Dimethylsulfoxid, Oxyalylchlorid und Triethylamin oder mit Dess-Martin-Reagenz, Chrom(IV)oxid, Nickelperoxid, Natriumdichromat, Kaliumdichromat, t-Butylchromat, Silberoxid, Silberpicolinatmangandioxidbleitetraacetat, Dicyclohexylcarbodiimid, 2,3-Dichlor-5,6-dicyanochinon, Tetrachlor-1,2-benzochinon, 2,2,6,6-Tetramethylpiperidinyl-1-oxy (TEMPO) oder Chinoliniumchlorchromat, um einen Piperidinformylphenylalkohol der Formel

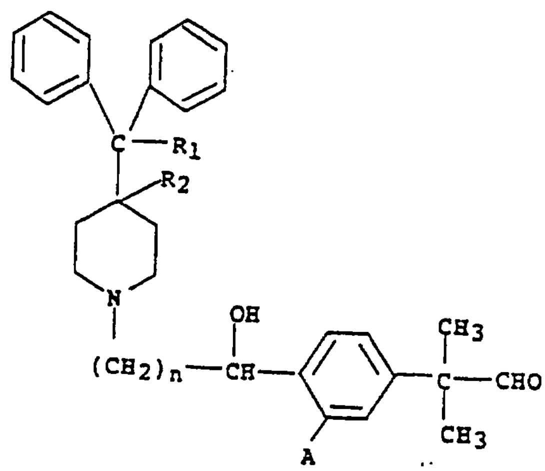

wobei $R_1$, $R_2$ und n wie vorstehend definiert sind und A ein Wasserstoffatom ist, herzustellen,

(h) Umsetzen des Piperidinformylphenylalkohols mit Kaliumpermanganat, Chrom(IV)oxid, Silber(I)oxid, Silberoxid, Silberpicolinat, Peroxid, Salpetersäure, m-Chlorperbenzoesäure und Peressigsäure, um einen Piperidincarboxyphenylalkohol der Formel

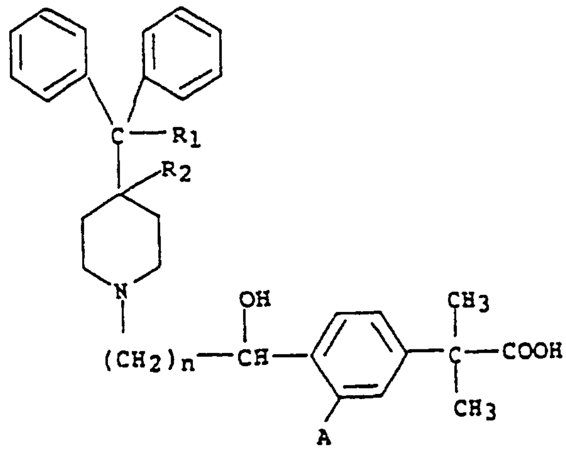

wobei $R_1$, $R_2$ und n wie vorstehend definiert sind und A ein Wasserstoffatom ist, herzustellen,

(i) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

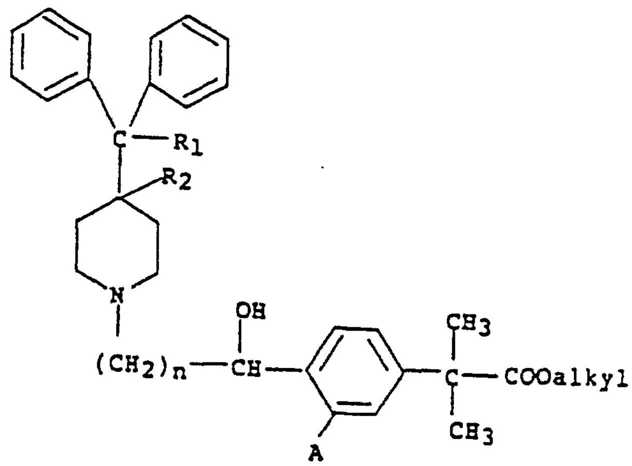

wobei $R_1$, $R_2$, n und Alkyl wie vorstehend definiert sind und A ein Wasserstoffatom ist, zu bilden.

**31.** Verfahren zur Herstellung einer Verbindung der Formel

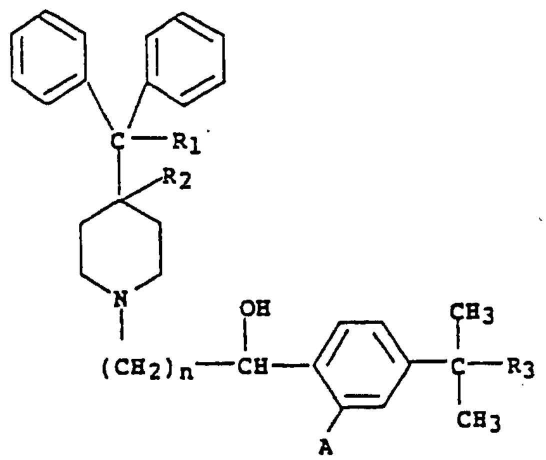

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

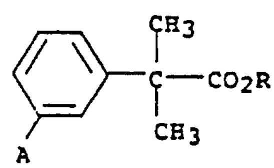

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

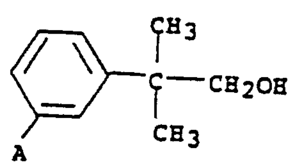

wobei A ein Wasserstoffatom ist, zu erhalten,

(b) Schützen der Hydroxyethylfunktionalität mit einer geeigneten Schutzgruppe, um einen geschützten Phenethylalkohol der Formel

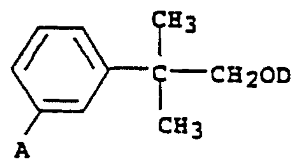

wobei A ein Wasserstoffatom ist und D eine geeignete Schutzgruppe ist, zu erhalten,

(c) Umsetzen des geschützten Phenethylalkohols mit einer ω-Halogenverbindung der Formel

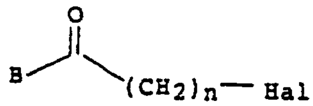

wobei B ein Halogenatom oder eine Hydroxygruppe ist, Hal Cl, Br oder I darstellt und n wie vorstehend definiert ist, in Anwesenheit von Trichlorid, Aluminiumchlorid, Titantetrachlorid, Bortrifluorid, Zinntetrachlorid und Zinkchlorid, um ein geschütztes ω-Halogenhydroxyethylphenylketon der Formel

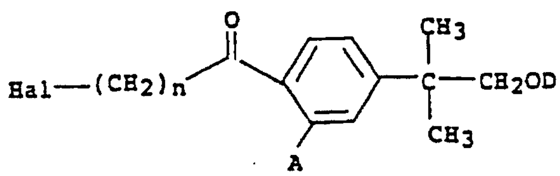

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

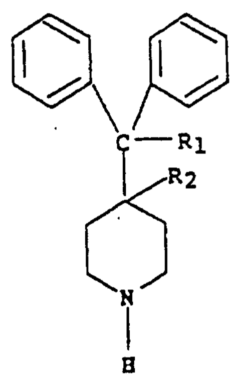

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

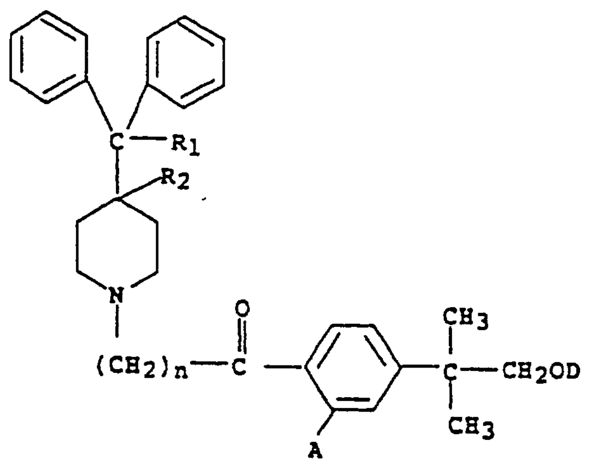

wobei $R_1$, $R_2$, n und D wie vorstehend definiert sind und A ein Wasserstoffatom ist, herzustellen,

(e) Umsetzen des geschützten Piperidinhydroxyethylphenylketons mit Natriumborhydrid, Kaliumborhydrid, Natriumcyanoborhydrid, Tetramethylammoniumborhydrid, Lithiumtri-tert-butylaluminohydrid, Diisobutylaluminiumhydrid oder katalytische Hydrierung, um einen geschützten Piperidinhydroxyethylphenylalkohol der Formel

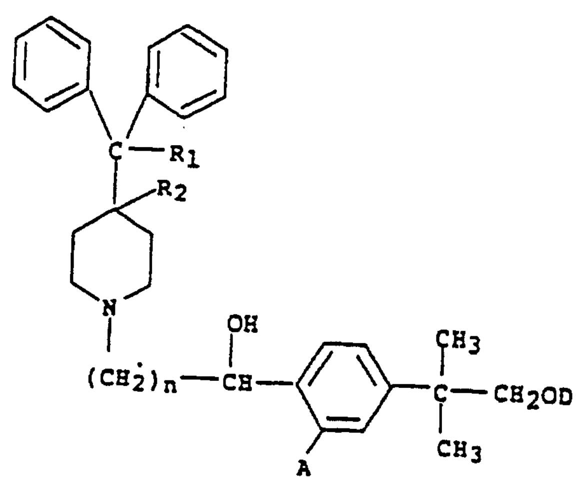

wobei $R_1$, $R_2$, D und n wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

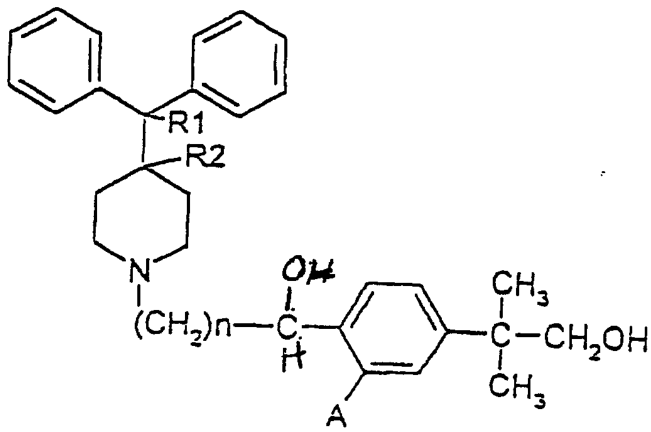

wobei $R_1$, $R_2$ und n wie vorstehend definiert sind und A ein Wasserstoffatom ist, herzustellen,

(g) Umsetzen des Piperidinhydroxyethylphenylalkohols mit Chrom(IV)oxid, Kaliumpermanganat, Salpetersäure, Stickstoffdioxid, Ruthenium(VIII)oxid, Nickelperoxid, Silberoxid, t-Butylchromat oder Xenonsäure, um einen Piperidincarboxyphenylalkohol der Formel

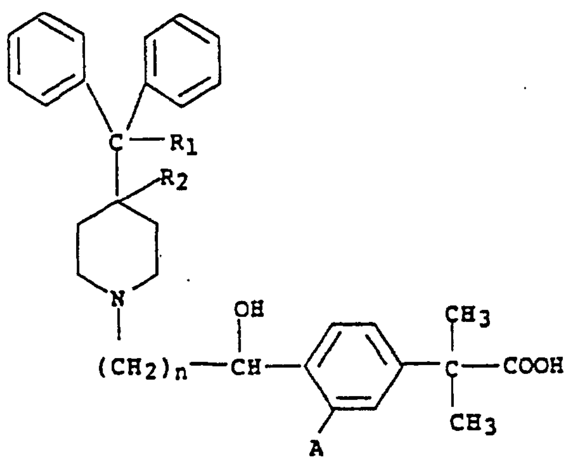

wobei $R_1$, $R_2$ und n wie vorstehend definiert sind und A ein Wasserstoffatom ist, herzustellen,

(h) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

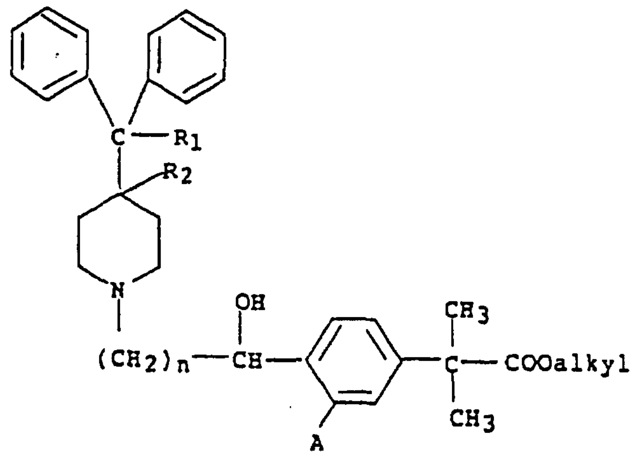

wobei $R_1$, $R_2$, n und Alkyl wie vorstehend definiert sind und A ein Wasserstoffatom ist; zu bilden.

**32.** Verfahren gemäß Anspruch 31, wobei der in Schritt f hergestellte Piperidinhydroxyethylphenylalkohol mit Ruthenium(VIII)oxid umgesetzt wird, um das Piperidincarboxyphenylketon von Schritt g zu erhalten.

**33.** Verfahren zur Herstellung einer Verbindung der Formel

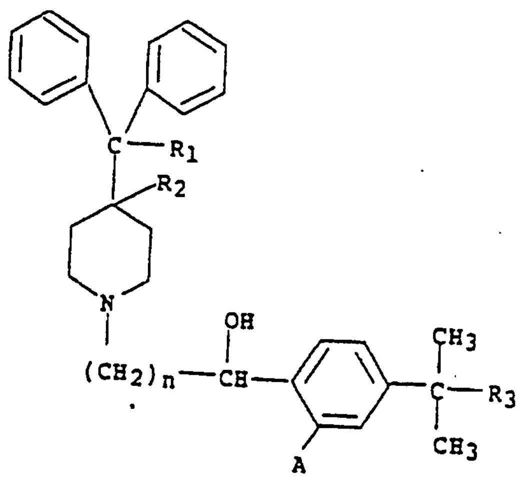

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe $-CH_2OH$ ist,
A ein Wasserstoffatom ist und
pharmazeutisch verträgliche Salze davon,
umfassend die Schritte:

(a) Umsetzen einer Benzolessigsäureverbindung der Formel

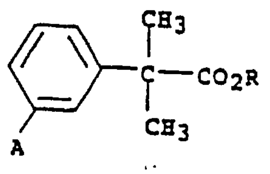

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

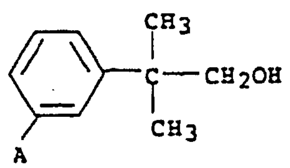

wobei A ein Wasserstoffatom ist, zu erhalten,

(b) Schützen der Hydroxyethylfunktionalität mit einer geeigneten Schutzgruppe, um einen geschützten Phenethylalkohol der Formel

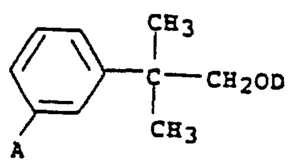

wobei A ein Wasserstoffatom ist und D eine geeignete Schutzgruppe ist, zu erhalten,

(c) Umsetzen des geschützten Phenethylalkohols mit einer ω-Halogenverbindung der Formel

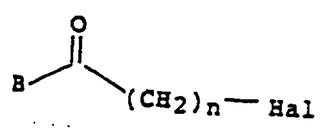

wobei B ein Halogenatom oder eine Hydroxygruppe ist, Hal Cl, Br oder I darstellt und n wie vorstehend definiert ist, in Anwesenheit von Trichlorid, Aluminiumchlorid, Titantetrachlorid, Bortrifluorid, Zinntetrachlorid und Zinkchlorid, um ein geschütztes ω-Halogenhydroxyethylphenylketon der Formel

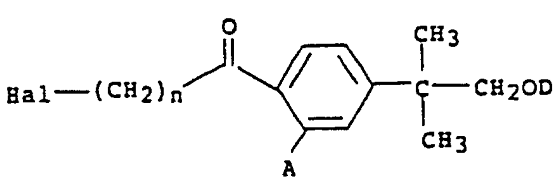

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

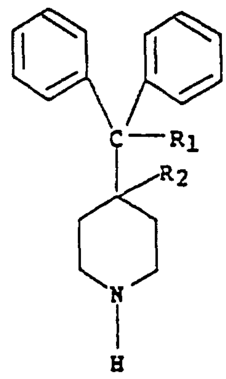

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

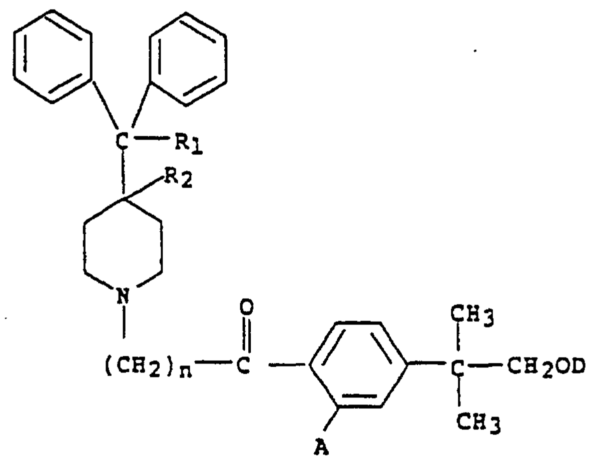

wobei $R_1$, $R_2$, n und D wie vorstehend definiert sind und A ein Wasserstoffatom ist, herzustellen,

(e) Umsetzen des geschützten Piperidinhydroxyethylphenylketons mit Natriumborhydrid, Kaliumborhydrid, Natriumcyanoborhydrid, Tetramethylammoniumborhydrid, Lithiumtri-tert-butylaluminohydrid, Diisobutylaluminiumhydrid oder katalytische Hydrierung, um einen geschützten Piperidinhydroxyethylphenylalkohol der Formel

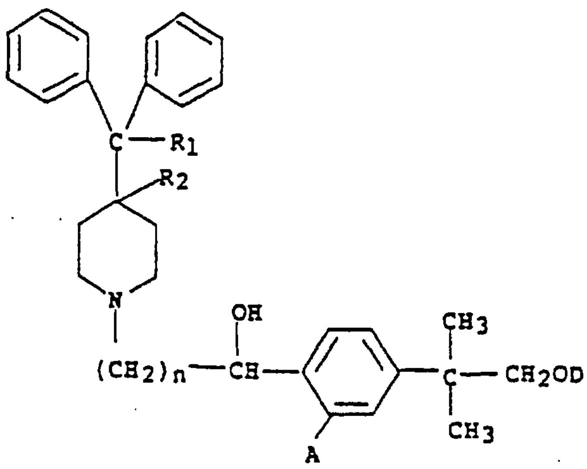

wobei $R_1$, $R_2$, n und D wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

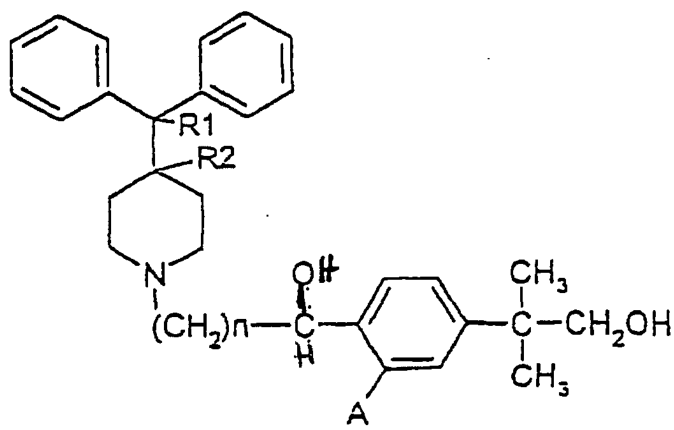

wobei $R_1$, $R_2$ und n wie vorstehend definiert sind und A ein Wasserstoffatom ist, herzustellen.

**34.** Verfahren zur Herstellung einer Verbindung der Formel

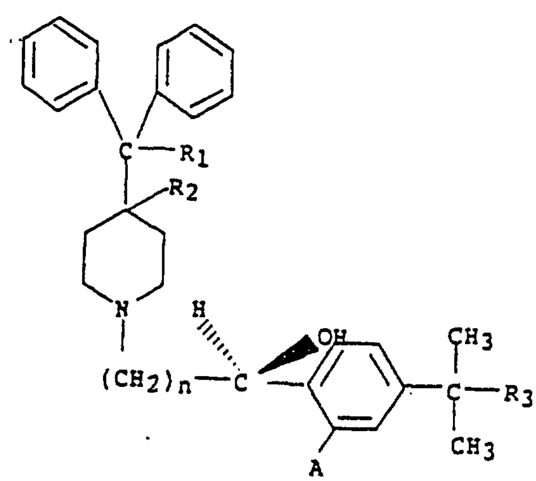

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

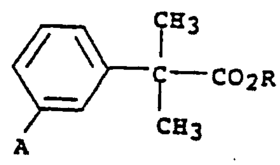

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

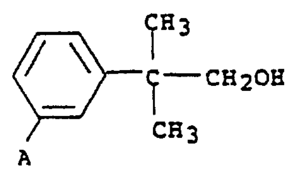

wobei A ein Wasserstoffatom ist, zu erhalten,

(b) Schützen der Hydroxyethylfunktionalität mit einer geeigneten Schutzgruppe, um einen geschützten Phenethylalkohol der Formel

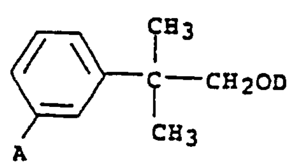

wobei A ein Wasserstoffatom ist und D eine geeignete Schutzgruppe ist, zu erhalten,

(c) Umsetzen des geschützten Phenethylalkohols mit einer ω-Halogenverbindung der Formel

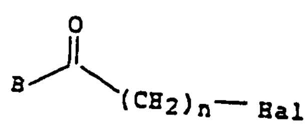

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

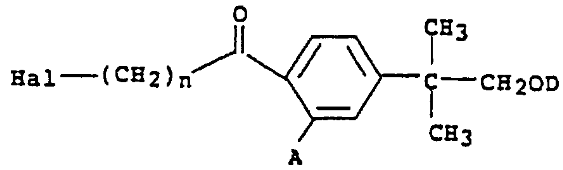

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

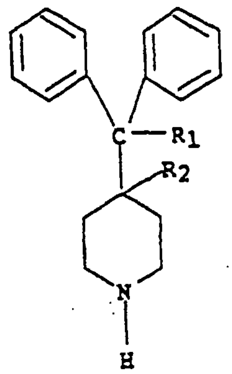

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

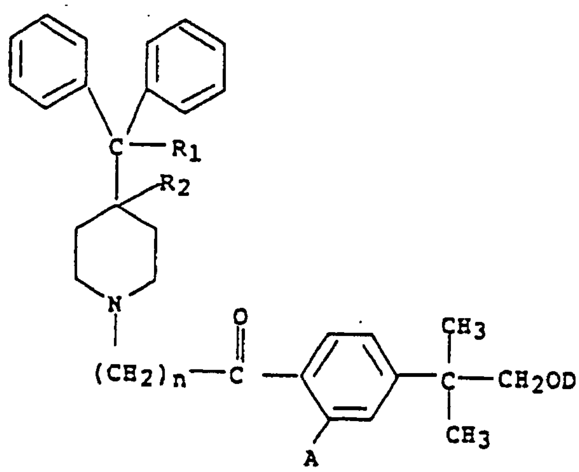

wobei $R_1$, $R_2$, n und D wie vorstehend definiert sind und A ein Wasserstoffatom ist, herzustellen,

(e) Umsetzen des geschützten Piperidinhydroxyethylphenylketons mit (+)-B-Chlordiisopinocamphenylboran, um einen geschützten Piperidinhydroxyethylphenylalkohol der Formel.

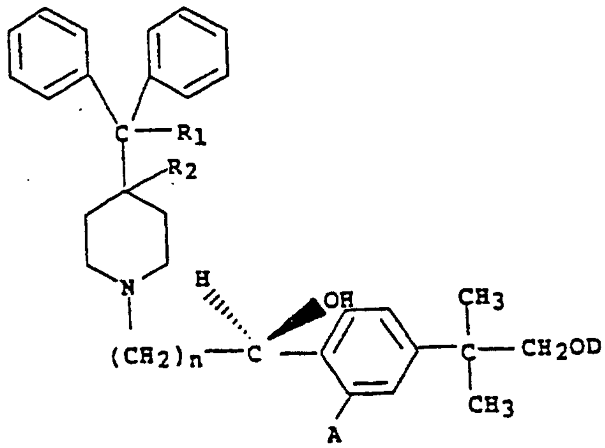

wobei $R_1$, $R_2$, D und n wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

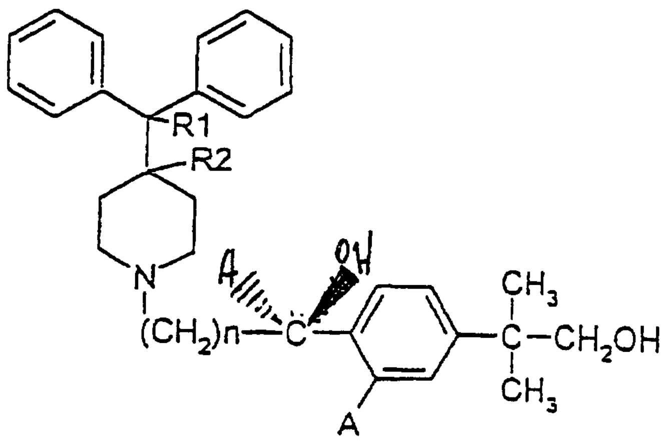

wobei $R_1$, $R_2$ und n wie vorstehend definiert sind und A ein Wasserstoffatom ist, herzustellen,

(g) Umsetzen des Piperidinhydroxyethylphenylalkohols mit Dimethylsulfoxid, Oxyalylchlorid und Triethylamin oder mit Dess-Martin-Reagenz, Chrom(IV)oxid, Nickelperoxid, Natriumdichromat, Kaliumdichromat, t-Butylchromat, Silberoxid, Silberpicolinatmangandioxidbleitetraacetat, Dicyclohexylcarbodiimid, 2,3-Dichlor-5,6-dicyanochinon, Tetrachlor-1,2-benzochinon, 2,2,6,6-Tetramethylpiperidinyl-1-oxy (TEMPO) oder Chinoliniumchlorchromat, um einen Piperidinformylphenylalkohol der Formel

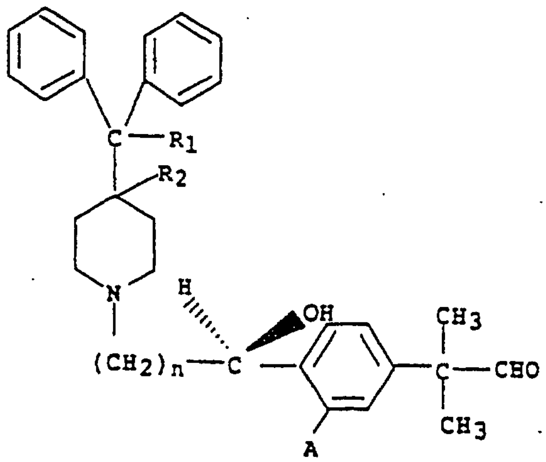

wobei $R_1$, $R_2$ und n wie vorstehend definiert sind und A ein Wasserstoffatom ist, herzustellen,

(h) Umsetzen des Piperidinformylphenylalkohols mit Kaliumpermanganat, Chrom(IV)oxid, Silber(I)oxid, Silberoxid, Silberpicolinat, Peroxid, Salpetersäure, m-Chlorperbenzoesäure und Peressigsäure, um einen Piperidincarboxyphenylalkohol der Formel

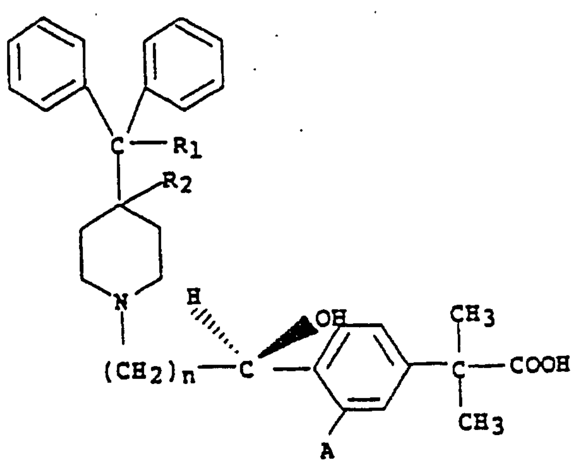

wobei $R_1$, $R_2$ und n wie vorstehend definiert sind und A ein Wasserstoffatom ist, herzustellen,

(i) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

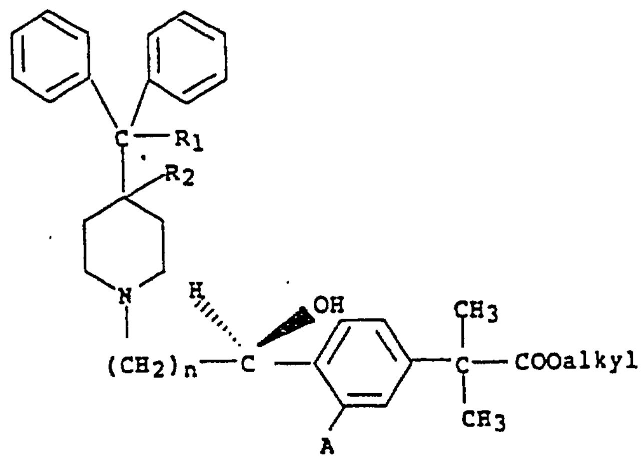

wobei $R_1$, $R_2$, n und Alkyl wie vorstehend definiert sind und A ein Wasserstoffatom ist, zu bilden.

**35.** Verfahren zur Herstellung einer Verbindung der Formel

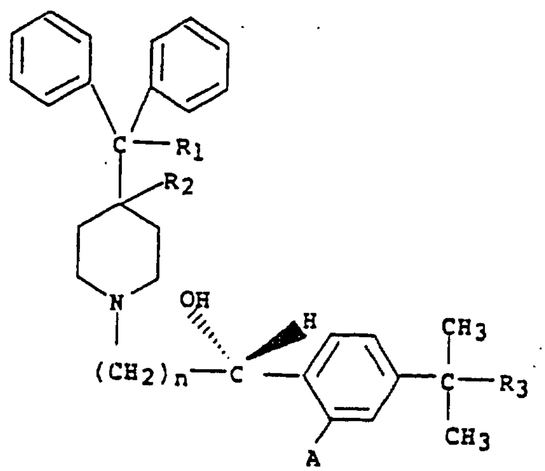

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe -COOH oder -COOalkyl ist, wobei die Alkyleinheit 1 bis 6 Kohlenstoffatome aufweist und geradkettig oder verzweigt ist,
A ein Wasserstoffatom ist und
pharmazeutisch verträgliche Salze davon,
umfassend die Schritte:

(a) Umsetzen einer Benzolessigsäureverbindung der Formel

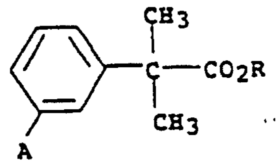

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

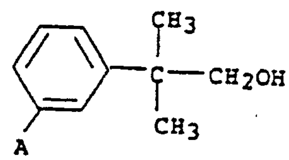

wobei A ein Wasserstoffatom ist, zu erhalten,

(b) Schützen der Hydroxyethylfunktionalität mit einer geeigneten Schutzgruppe, um einen geschützten Phenethylalkohol der Formel

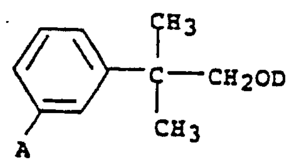

wobei A ein Wasserstoffatom ist und D eine geeignete Schutzgruppe ist, zu erhalten,

(c) Umsetzen des geschützten Phenethylalkohols mit einer ω-Halogenverbindung der Formel

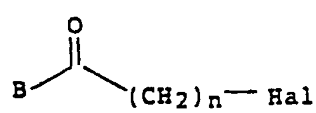

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

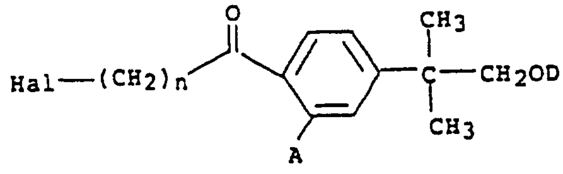

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

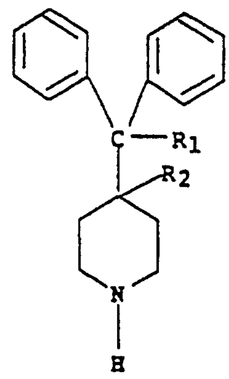

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

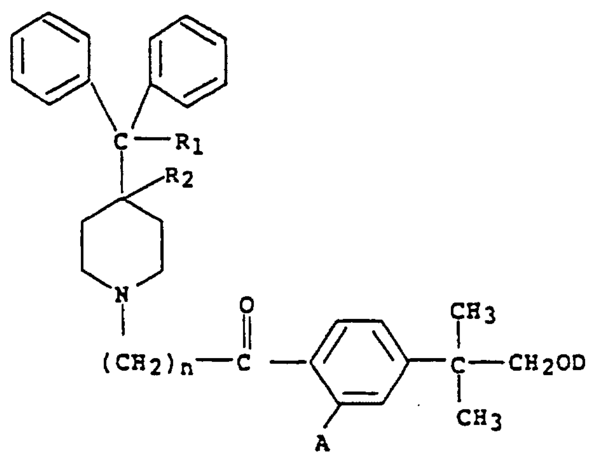

wobei $R_1$, $R_2$, n und D wie vorstehend definiert sind und A ein Wasserstoffatom ist, herzustellen,

(e) Umsetzen des geschützten Piperidinhydroxyethylphenylketons mit (-)-B-Chlordiisopinocamphenylboran, um einen geschützten Piperidinhydroxyethylphenylalkohol der Formel

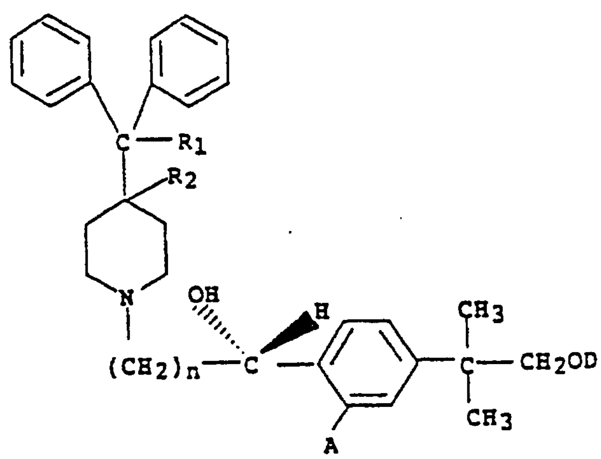

wobei $R_1$, $R_2$, D und n wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

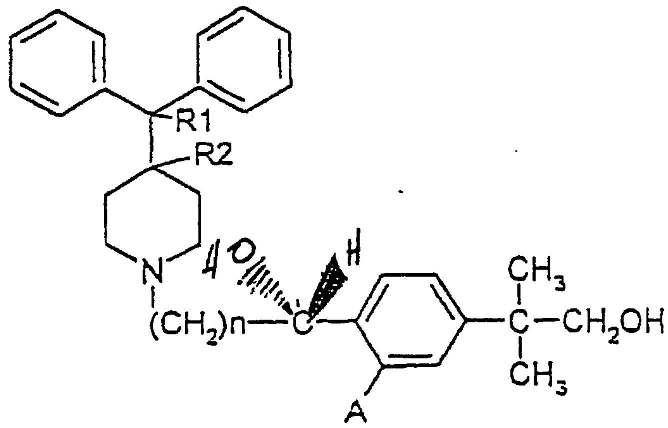

wobei $R_1$, $R_2$ und n wie vorstehend definiert sind und A ein Wasserstoffatom ist, herzustellen,

(g) Umsetzen des Piperidinhydroxyethylphenylalkohols mit Dimethylsulfoxid, Oxyalylchlorid und Triethylamin oder mit Dess-Martin-Reagenz, Chrom(IV)oxid, Nickelperoxid, Natriumdichromat, Kaliumdichromat, t-Butylchromat, Silberoxid, Silberpicolinatmangandioxidbleitetraacetat, Dicyclohexylcarbodiimid, 2,3-Dichlor-5,6-dicyanochinon, Tetrachlor-1,2-benzochinon, 2,2,6,6-Tetramethylpiperidinyl-1-oxy (TEMPO) oder Chinoliniumchlorchromat, um einen Piperidinformylphenylalkohol der Formel

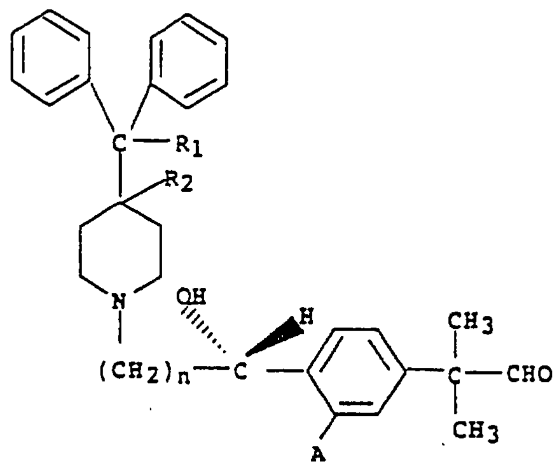

wobei $R_1$, $R_2$ und n wie vorstehend definiert sind und A ein Wasserstoffatom ist, herzustellen,

(h) Umsetzen des Piperidinformylphenylalkohols mit Kaliumpermanganat, Chrom(IV)oxid, Silber(I)oxid, Silberoxid, Silberpicolinat, Peroxid, Salpetersäure, m-Chlorperbenzoesäure und Peressigsäure, um einen Piperidincarboxyphenylalkohol der Formel

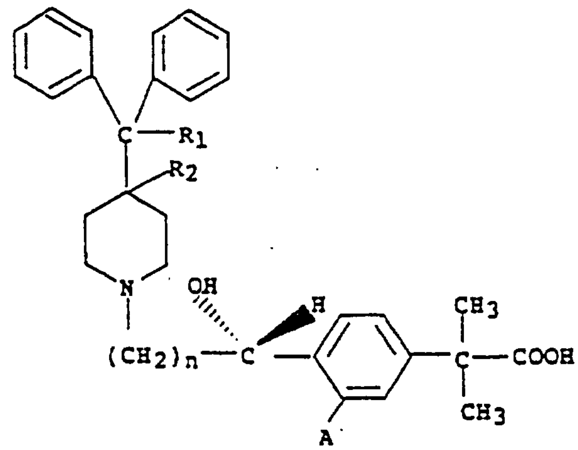

wobei $R_1$, $R_2$ und n wie vorstehend definiert sind und A ein Wasserstoffatom ist, herzustellen,

(i) gegebenenfalls Umsetzen des Piperidincarboxyphenylalkohols mit einem Überschuss an Diazomethan oder einem Überschuss an 2,2-Dimethoxypropan oder mit Thionylchlorid, gefolgt von einem Alkohol der Formel HOalkyl, wobei die Alkyleinheit 1 bis 6 Kohlenstoffatome aufweist und geradkettig oder verzweigt ist, um einen Piperidincarboxyphenylalkoholester der Formel

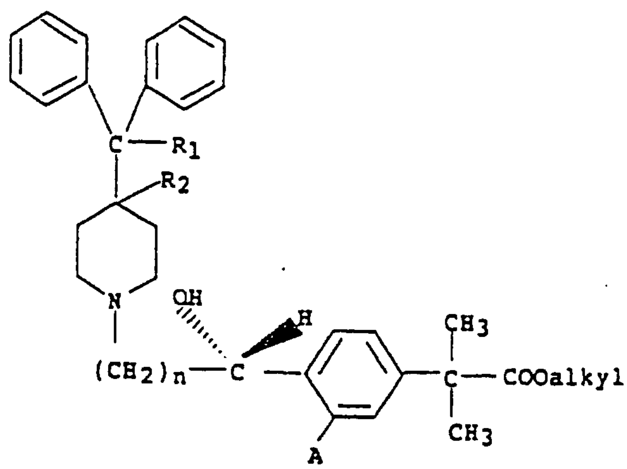

wobei $R_1$, $R_2$, n und Alkyl wie vorstehend definiert sind und A ein Wasserstoffatom ist, zu bilden.

**36.** Verfahren zur Herstellung einer Verbindung der Formel

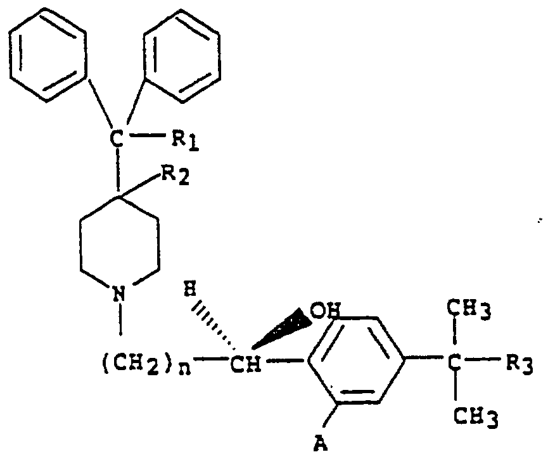

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe $-CH_2OH$ ist,
A ein Wasserstoffatom ist und
pharmazeutisch verträgliche Salze davon,
umfassend die Schritte:

(a) Umsetzen einer Benzolessigsäureverbindung der Formel

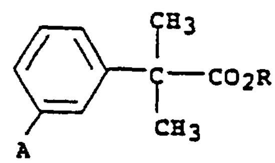

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

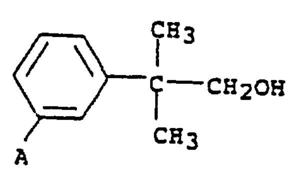

wobei A ein Wasserstoffatom ist, zu erhalten,

(b) Schützen der Hydroxyethylfunktionalität mit einer geeigneten Schutzgruppe, um einen geschützten Phenethylalkohol der Formel

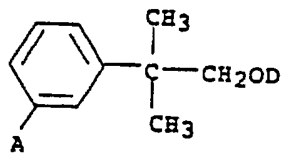

wobei A ein Wasserstoffatom ist und D eine geeignete Schutzgruppe ist, zu erhalten,

(c) Umsetzen des geschützten Phenethylalkohols mit einer ω-Halogenverbindung der Formel

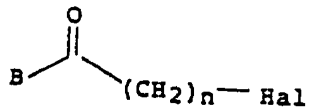

wobei B ein Halogenatom oder eine Hydroxygruppe ist, Hal Cl, Br oder I darstellt und n wie vorstehend definiert ist, in Anwesenheit von Trichlorid, Aluminiumchlorid, Titantetrachlorid, Bortrifluorid, Zinntetrachlorid und Zinkchlorid, um ein geschütztes ω-Halogenhydroxyethylphenylketon der Formel

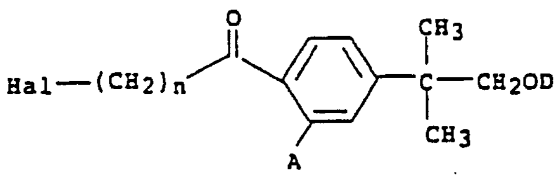

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

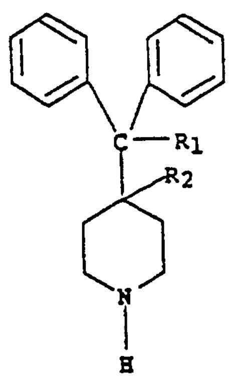

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

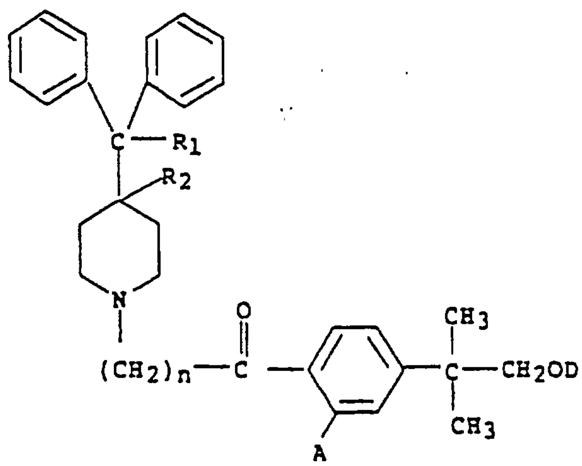

wobei $R_1$, $R_2$, n und D wie vorstehend definiert sind und A ein Wasserstoffatom ist, herzustellen,

(e) Umsetzen des geschützten Piperidinhydroxyethylphenylketons mit (+)-B-Chlordiisopinocamphenylboran, um einen geschützten Piperidinhydroxyethylphenylalkohol der Formel

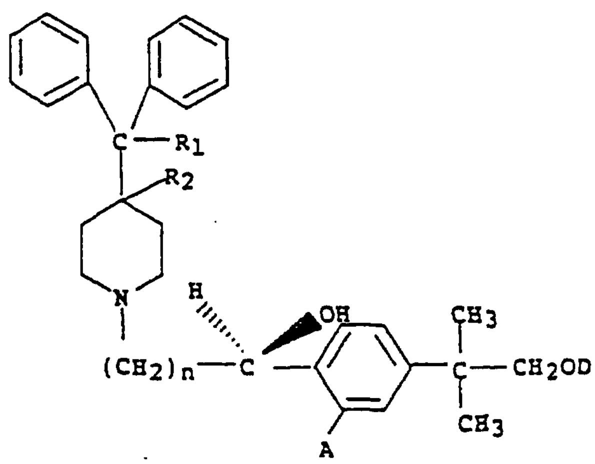

wobei $R_1$, $R_2$, n und D wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

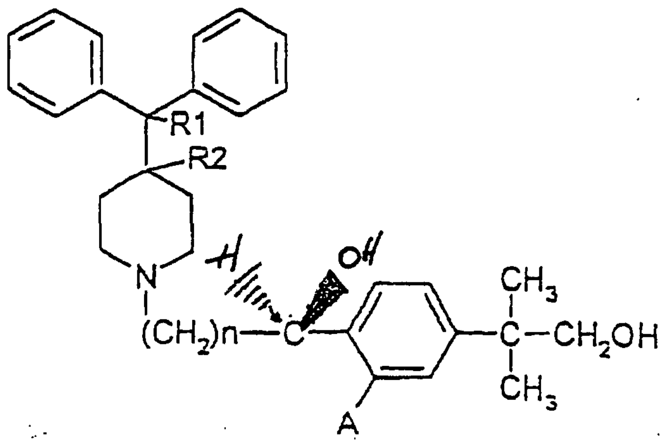

wobei $R_1$, $R_2$ und n wie vorstehend definiert sind und A ein Wasserstoffatom ist, herzustellen.

**37.** Verfahren zur Herstellung einer Verbindung der Formel

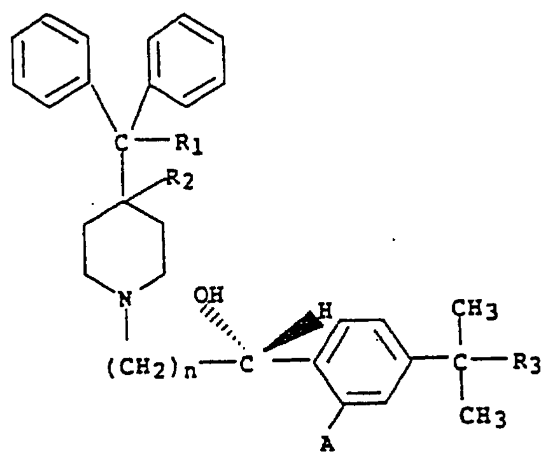

wobei
$R_1$ ein Wasserstoffatom oder eine Hydroxygruppe darstellt,
$R_2$ ein Wasserstoffatom darstellt oder
$R_1$ und $R_2$ zusammen genommen eine zweite Bindung zwischen den $R_1$ und $R_2$ tragenden Kohlenstoffatomen bilden,
n eine ganze Zahl von 1 bis 5 ist,
$R_3$ die Gruppe $-CH_2OH$ ist,
A ein Wasserstoffatom ist und
pharmazeutisch verträgliche Salze davon,
umfassend die Schritte:

(a) Umsetzen einer Benzolessigsäureverbindung der Formel

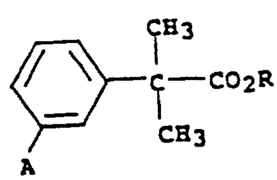

wobei A ein Wasserstoffatom ist und R ein Wasserstoffatom oder ein $C_1$-$C_6$-Alkylrest ist, mit Natriumbis(2-methoxyethoxy)aluminiumhydrid, Lithiumaluminiumhydrid, Diboran oder Aluminiumhydrid, wenn R ein Wasserstoffatom ist, und mit Natriumbis(2-methoxyethoxy)aluminiumhydrid, Lithiumaluminiumhydrid, Lithiumborhydrid, Lithiumtriethylborhydrid, Lithiumtri-sec-butylborhydrid oder Aluminiumhydrid, wenn R ein $C_1$-$C_6$-Alkylrest ist, um einen Phenethylalkohol der Formel

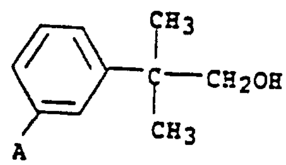

wobei A ein Wasserstoffatom ist, zu erhalten,

(b) Schützen der Hydroxyethylfunktionalität mit einer geeigneten Schutzgruppe, um einen geschützten Phenethylalkohol der Formel

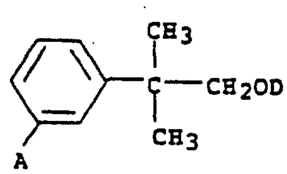

wobei A ein Wasserstoffatom ist und D eine geeignete Schutzgruppe ist, zu erhalten,

(c) Umsetzen des geschützten Phenethylalkohols mit einer ω-Halogenverbindung der Formel

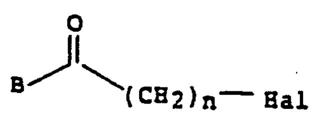

wobei B ein Halogenatom oder eine Hydroxygruppe ist, Hal Cl, Br oder I darstellt und n wie vorstehend definiert ist, in Anwesenheit von Bortrichlorid, Aluminiumchlorid, Titantetrachlorid, Bortrifluorid, Zinntetrachlorid und Zinkchlorid, um ein geschütztes ω-Halogenhydroxyethylphenylketon der Formel

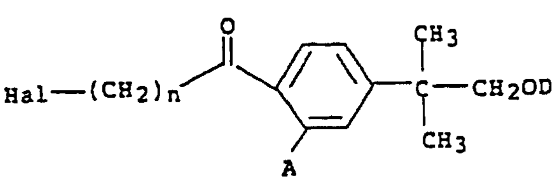

wobei Hal, n und D wie vorstehend definiert sind und A ein Wasserstoffatom ist, herzustellen,

(d) Umsetzen des geschützten ω-Halogenhydroxyethylphenylketons mit einer Piperidinverbindung der Formel

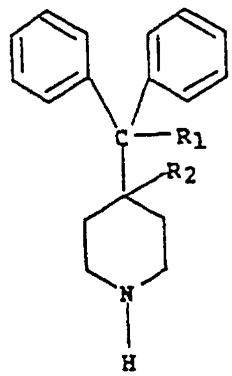

wobei $R_1$ und $R_2$ wie vorstehend definiert sind, in Anwesenheit eines Natriumbicarbonats, Kaliumcarbonats, Kaliumbicarbonats, Triethylamins, Pyridins, oder ein Überschuss an der Piperidinverbindung kann verwendet werden, um ein geschütztes Piperidinhydroxyethylphenylketon der Formel

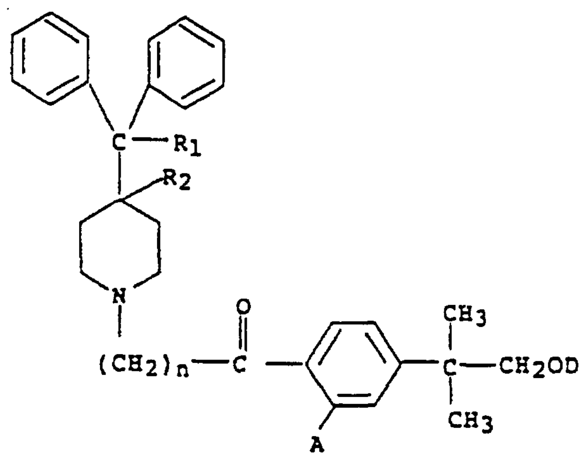

wobei $R_1$, $R_2$, n und D wie vorstehend definiert sind und A ein Wasserstoffatom ist, herzustellen,

(e) Umsetzen des geschützten Piperidinhydroxyethylphenylketons mit (-)-B-Chlordiisopinocamphenylboran, um einen geschützten Piperidinhydroxyethylphenylalkohol der Formel

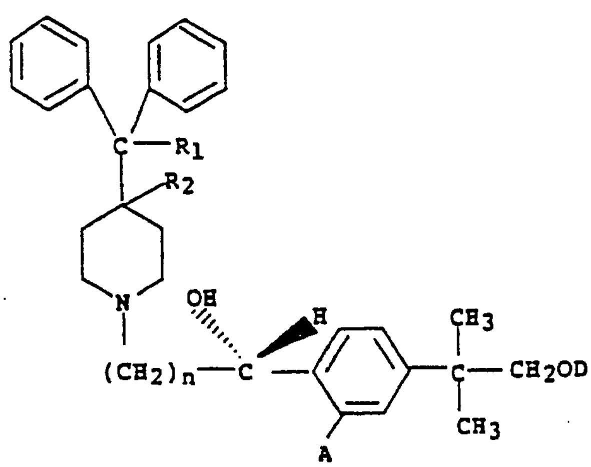

wobei $R_1$, $R_2$, n und D wie vorstehend definiert sind und A ein Wasserstoffatom ist, herzustellen,

(f) Schutzgruppenabspaltung der Hydroxyethylfunktionalität des geschützten Piperidinhydroxyethylphenylalkohols mit einem geeigneten Reagenz zur Schutzgruppenabspaltung, um einen Piperidinhydroxyethylphenylalkohol der Formel

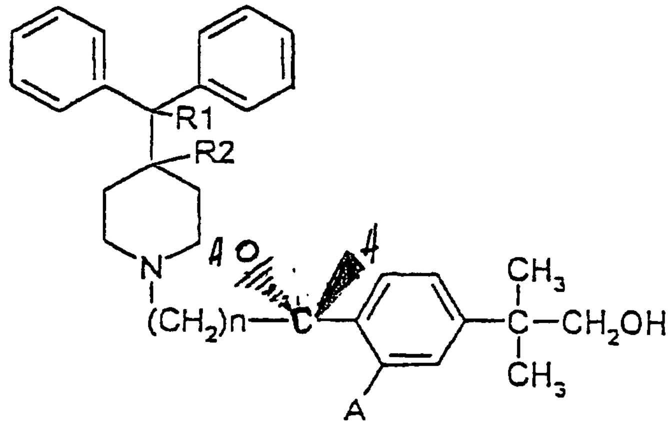

wobei $R_1$, $R_2$ und n wie vorstehend definiert sind und A ein Wasserstoffatom ist, herzustellen.

## Revendications

**1.** Composé de formylphénylalcool pipéridine de formule :

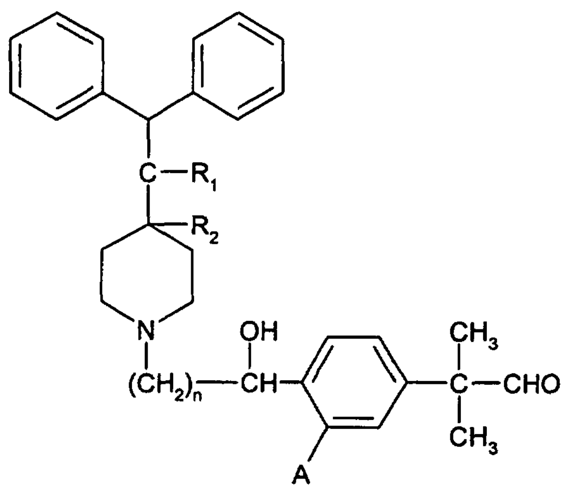

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ; et
A est l'hydrogène.

**2.** Composé de formylphénylcétone pipéridine de formule

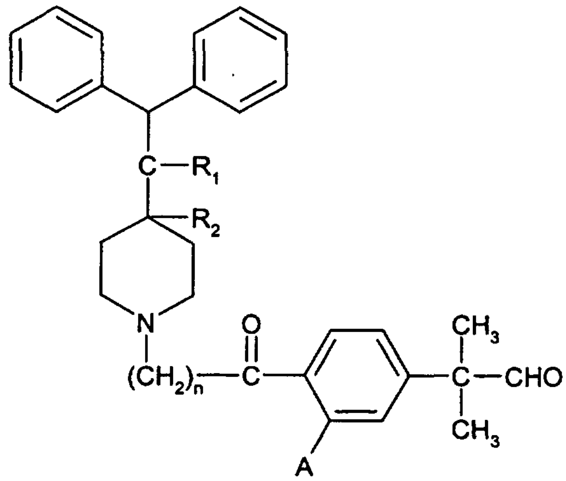

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier.de 1 à 5 ; et
A est l'hydrogène.

**3.** Composé de hydroxyéthylphénylalcool pipéridine protégé de formule :

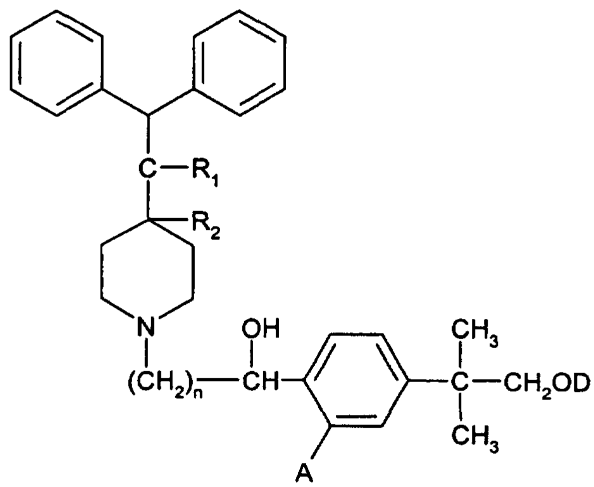

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
A est l'hydrogène ; et
D est $C(=O)CH_3$ ou $C(=O)C_6H_5$.

**4.** Composé de hydroxyéthylphénylcétone pipéridine protégé de formule :

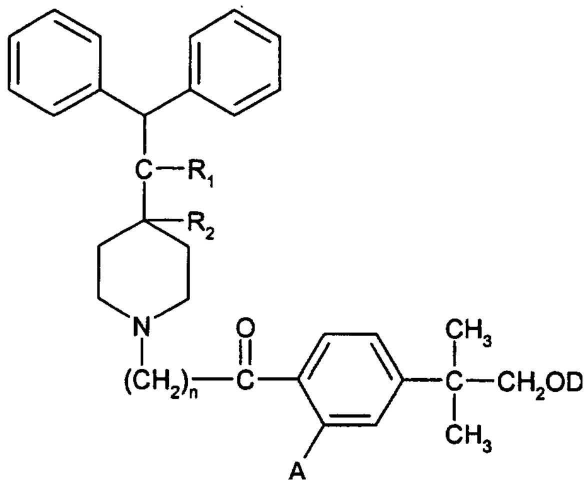

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
A est l'hydrogène ; et
D est $C(=O)CH_3$ ou $C(=O)C_6H_5$.

**5.** Procédé pour préparer un composé de formylphénylalcool pipéridine de formule :

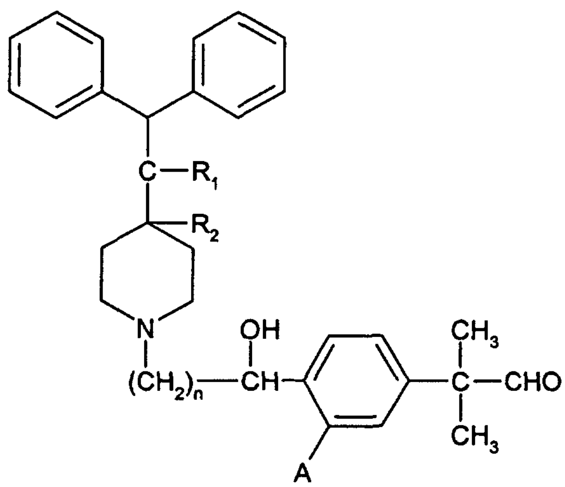

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ; et
A est l'hydrogène,
comprenant les étapes consistant à :

(a) faire réagir un composé d'acide benzène-acétique de formule :

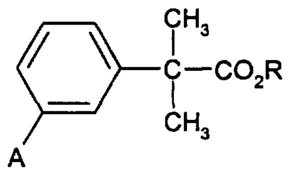

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

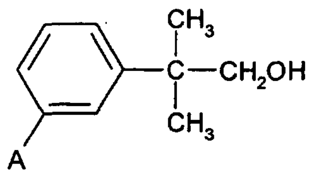

dans laquelle A est l'hydrogène ;

(b) protéger la fonctionnalité hydroxyéthyle avec un groupe protecteur convenable pour donner un alcool phénéthylique protégé de formule :

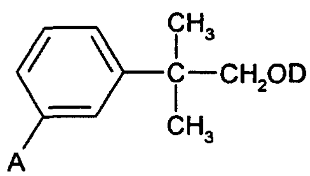

dans laquelle A est l'hydrogène et D est un groupe protecteur convenable ;

(c) faire réagir l'alcool phénéthylique protégé avec un composé ω-halogéno de formule :

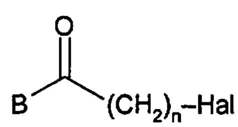

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

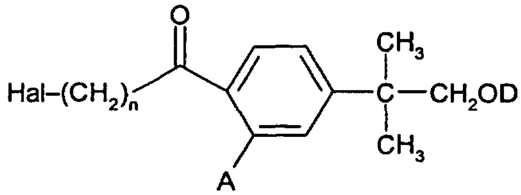

dans laquelle Hal, n et D sont définis ci-dessus et A est l'hydrogène ;

(d) faire réagir l'hydroxyéthylphénylcétone ω-halogéno protégée avec un composé de pipéridine de formule :

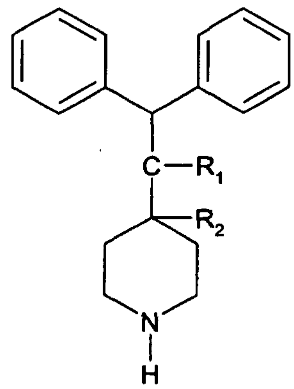

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

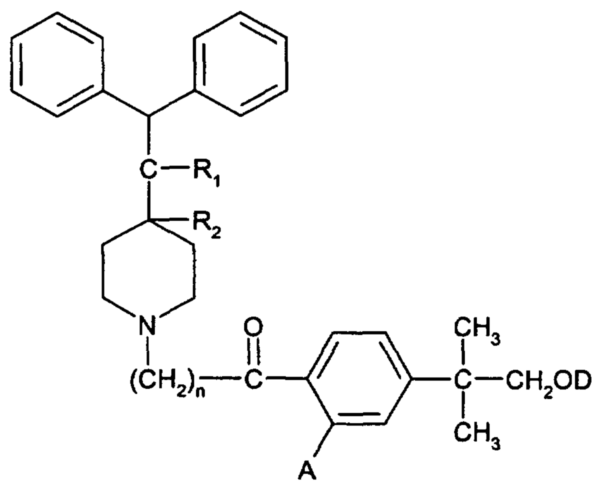

dans laquelle $R_1$, $R_2$, n et D sont définis ci-dessus et A est l'hydrogène ;

(e) faire réagir l'hydroxyéthylphénylcétone pipéridine protégée avec du borohydrure de sodium, du borohydrure de potassium, du cyanoborohydrure de sodium, du borohydrure de tétraméthylammonium, de l'hydrure de lithium et de tri-tert-butyl-aluminium, de l'hydrure de diisobutyl-aluminium ou catalyser une hydrogénation pour produire un hydroxyéthylphénylalcool pipéridine protégé de formule :

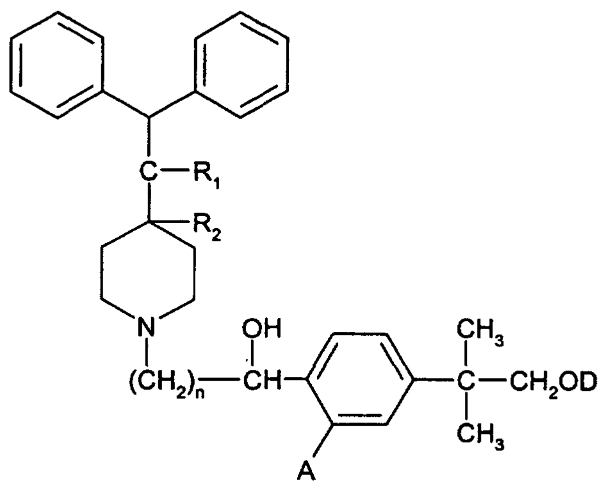

dans laquelle $R_1$, $R_2$, D et n sont définis ci-dessus et A est l'hydrogène ;
(f) déprotéger la fonctionnalité hydroxyéthyle de l'hydroxyéthylphénylalcool pipéridine protégé avec un réactif de déprotection approprié pour produire un hydroxyéthylphénylalcool pipéridine de formule :

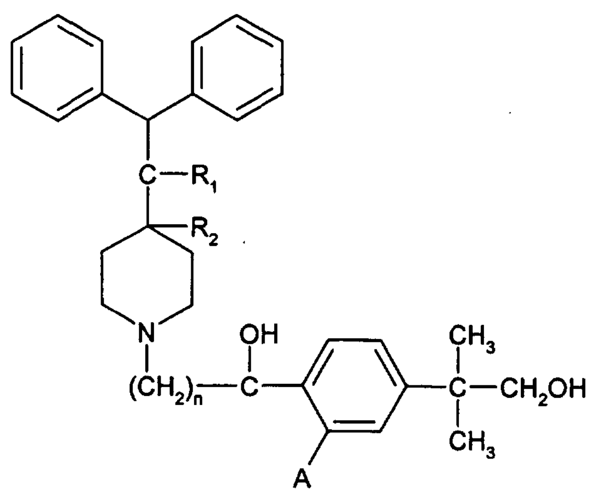

dans laquelle $R_1$, $R_2$ et n sont définis ci-dessus et A est l'hydrogène ;
(g) faire réagir l'hydroxyéthylphénylalcool pipéridine avec du diméthylsulfoxyde, du chlorure d'oxalyle et de la triéthylamine ou avec un réactif de Dess-Martin, de l'oxyde de chrome(IV), du peroxyde de nickel, du dichromate de sodium, du dichromate de potassium, du chromate de t-butyle, de l'oxyde d'argent, du picolinate argentique du dioxyde de manganèse du tétraacétate de plomb, du dicyclohexylcarbodiimide, de la 2,3-dichloro-5,6-dicyanoquinone, de la tétrachloro-1,2-benzoquinone, du 2,2,6,6-tétraméthylpipéridinyl-1-oxy (TEMPO) ou du chlorochromate de quinolinium pour produire un formylphénylalcool pipéridine de formule :

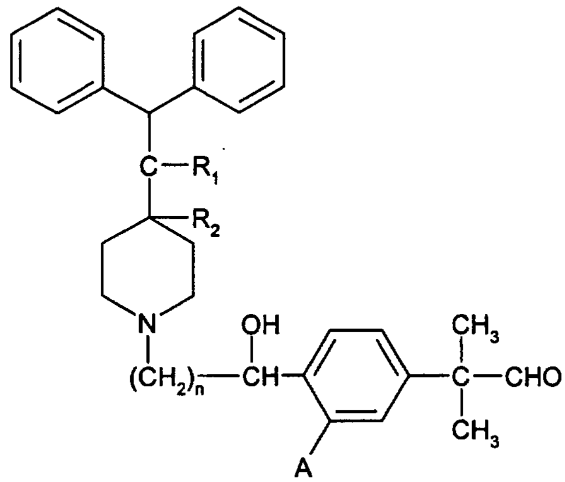

dans laquelle $R_1$, $R_2$ et n sont définis ci-dessus et A est l'hydrogène.

**6.** Procédé pour préparer un composé de formylphénylcétone pipéridine de formule :

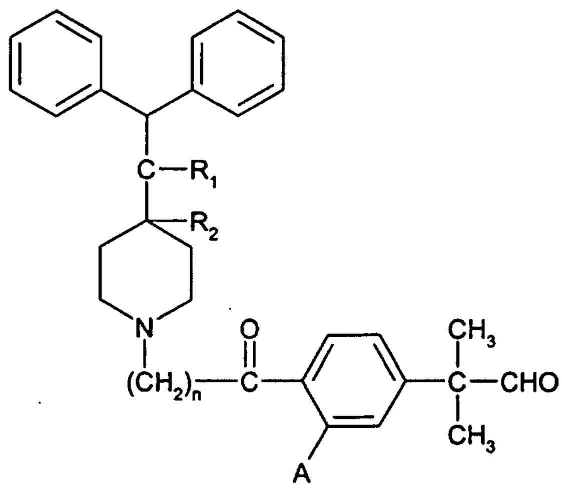

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ; et
A est l'hydrogène,
comprenant les étapes consistant à :

(a) faire réagir un composé d'acide benzène-acétique de formule :

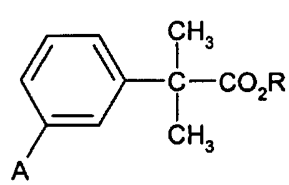

dans laquelle A est tel que défini ci-dessus et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy) aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

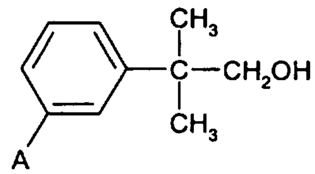

dans laquelle A est défini ci-dessus ;

(b) protéger la fonctionnalité hydroxyéthyle avec un groupe protecteur convenable pour donner un alcool phénéthylique protégé de formule :

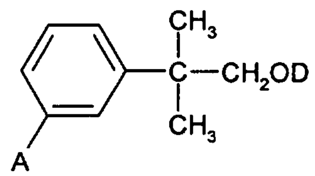

dans laquelle A est l'hydrogène et D est un groupe protecteur convenable ;

(c) faire réagir l'alcool phénéthylique protégé avec un composé ω-halogéno de formule :

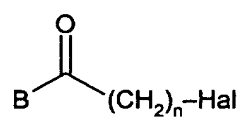

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

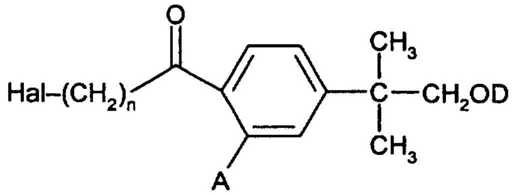

dans laquelle Hal, n, A et D sont définis ci-dessus ;

(d) faire réagir l'hydroxyéthylphénylcétone ω-halogéno protégée avec un composé de pipéridine de formule :

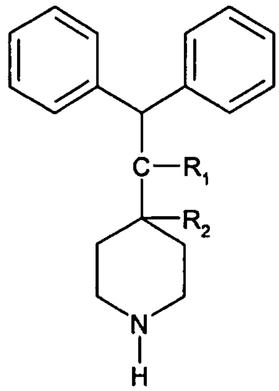

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire le composé hydroxyéthylphénylcétone pipéridine protégée de formule :

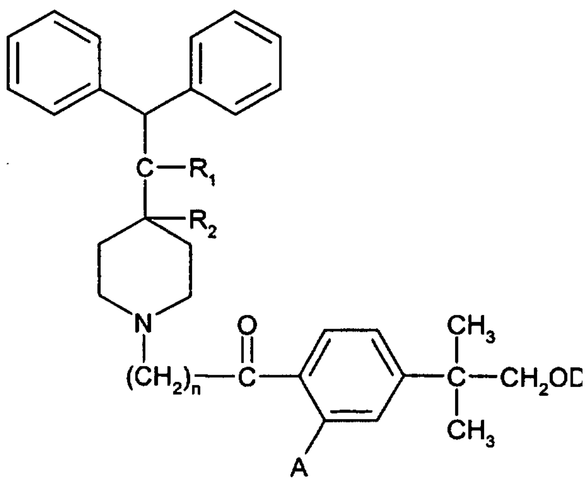

dans laquelle $R_1$, $R_2$, n et A sont définis ci-dessus et D est $-C(=O)CH_3$ ou $-C(=O)C_6H_5$ ;

(e) déprotéger la fonctionnalité hydroxyéthyle de l'hydroxyéthylphénylcétone pipéridine protégée avec un réactif de déprotection approprié pour produire une hyxroxyéthylphénylcétone pipéridine de formule :

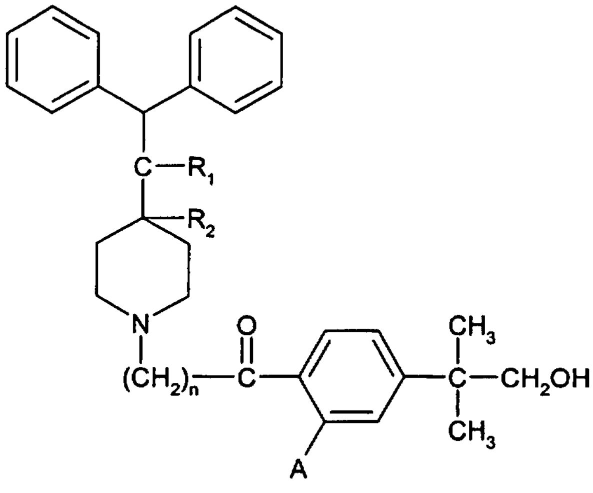

dans laquelle $R_1$, $R_2$, n et A sont définis ci-dessus ; et
(g) faire réagir le composé hydroxyéthylphénylcétone pipéridine avec du diméthylsulfoxyde, du chlorure d'oxalyle et de la triéthylamine ou avec un réactif de Dess-Martin, de l'oxyde de chrome(IV), du peroxyde de nickel, du dichromate de sodium, du dichromate de potassium, du chromate de t-butyle, de l'oxyde d'argent, du picolinate argentique du dioxyde de manganèse du tétraacétate de plomb, du dicyclohexylcarbodiimide, de la 2,3-dichloro-5,6-dicyanoquinone, de la tétrachloro-1,2-benzoquinone, du 2,2,6,6-tétraméthylpipéridinyl-1-oxy (TEMPO) ou du chlorochromate de quinolinium pour produire la formylphénylcétone pipéridine.

**7.** Procédé pour préparer un composé d'hydroxyphénylalcool pipéridine protégé de formule :

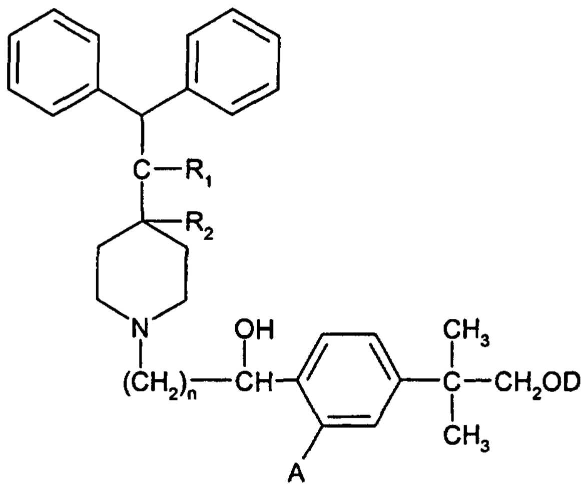

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
A est l'hydrogène ; et
D est $-C(=O)CH_3$ ou $-C(=O)C_6H_5$,
comprenant les étapes consistant à :

(a) faire réagir un composé d'acide benzène-acétique de formule :

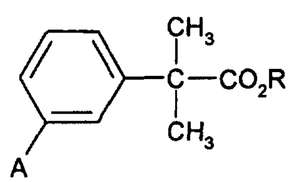

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

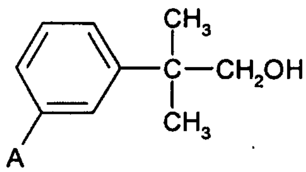

dans laquelle A est l'hydrogène ;

(b) protéger la fonctionnalité hydroxyéthyle avec un groupe protecteur convenable pour donner un alcool phénéthylique protégé de formule :

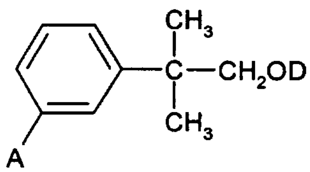

dans laquelle A est l'hydrogène et D est un groupe protecteur convenable ;

(c) faire réagir l'alcool phénéthylique protégé avec un composé ω-halogéno de formule :

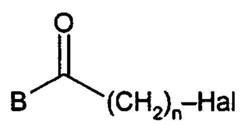

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

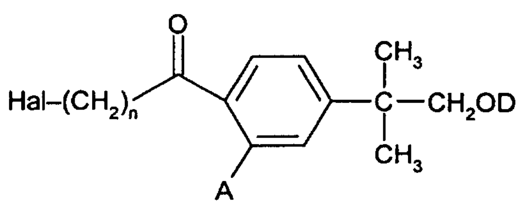

dans laquelle Hal, n et D sont définis ci-dessus et A est l'hydrogène ;

(d) faire réagir l'hydroxyéthylphénylcétone ω-halogéno protégée avec un composé de pipéridine de formule :

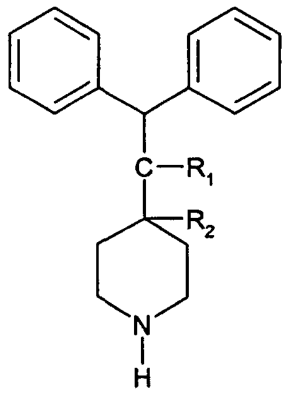

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

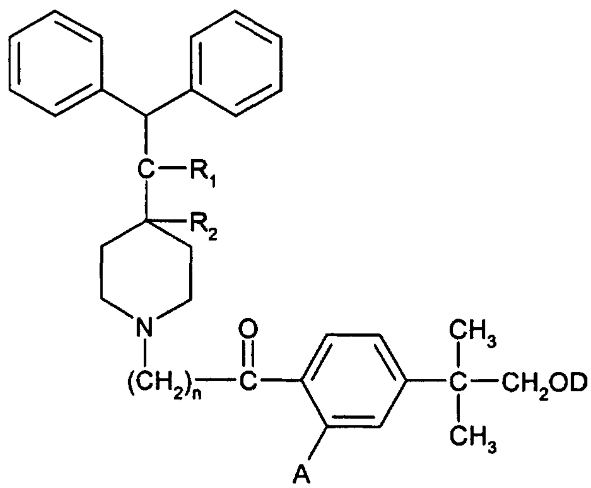

dans laquelle $R_1$, $R_2$, n et D sont définis ci-dessus et A est l'hydrogène ;

(e) faire réagir l'hydroxyéthylphénylcétone pipéridine protégée avec du borohydrure de sodium, du borohydrure de potassium, du cyanoborohydrure de sodium, du borohydrure de tétraméthylammonium, de l'hydrure de lithium et de tri-tert-butyl-aluminium, de l'hydrure de diisobutyl-aluminium ou catalyser une hydrogénation pour produire un hydroxyéthylphénylalcool pipéridine protégé de formule :

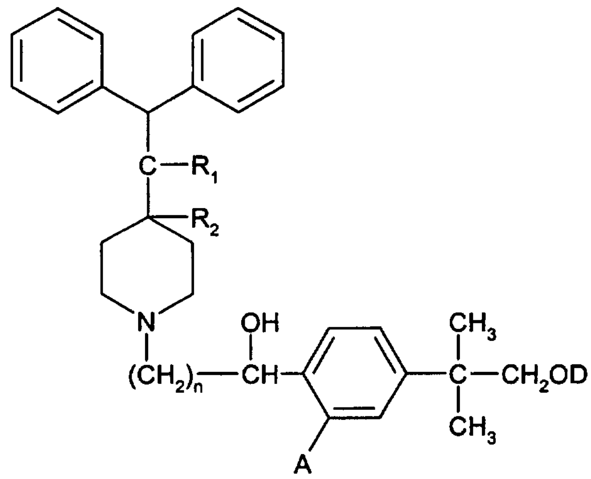

dans laquelle $R_1$, $R_2$, D et n sont définis ci-dessus et A est l'hydrogène.

**8.** Procédé pour préparer un composé hydroxyéthylphénylcétone pipéridine protégé de formule :

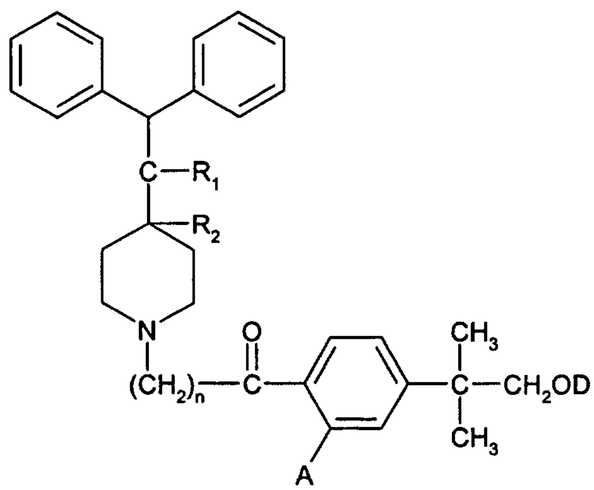

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
A est l'hydrogène ; et
D est $-C(=O)CH_3$ ou $-C(=O)C_6H_5$,
comprenant les étapes consistant à :

(a) faire réagir un composé d'acide benzène-acétique de formule :

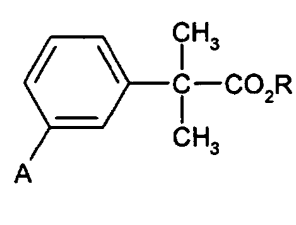

dans laquelle A est défini ci-dessus et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

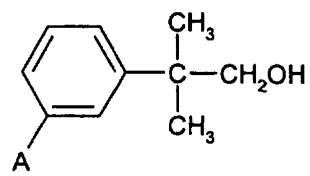

dans laquelle A est défini ci-dessus ;

(b) protéger la fonctionnalité hydroxyéthyle avec un groupe protecteur convenable pour donner un alcool phénéthylique protégé de formule :

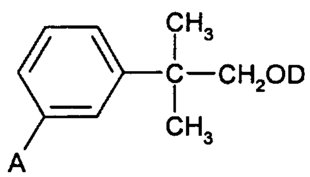

dans laquelle A est l'hydrogène et D est un groupe protecteur convenable ;

(c) faire réagir l'alcool phénéthylique protégé avec un composé ω-halogéno de formule :

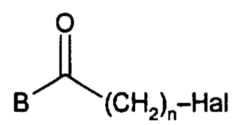

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

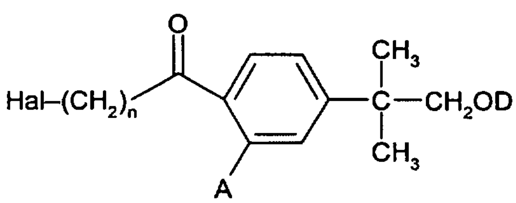

dans laquelle Hal, n, A et D sont définis ci-dessus ;

(d) faire réagir l'hydroxyéthylphénylcétone ω-halogéno protégée avec un composé de pipéridine de formule :

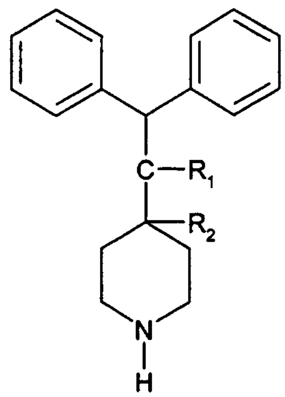

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire l'hydroxyéthylphénylcétone pipéridine protégée.

**9.** Procédé pour préparer un composé de formule :

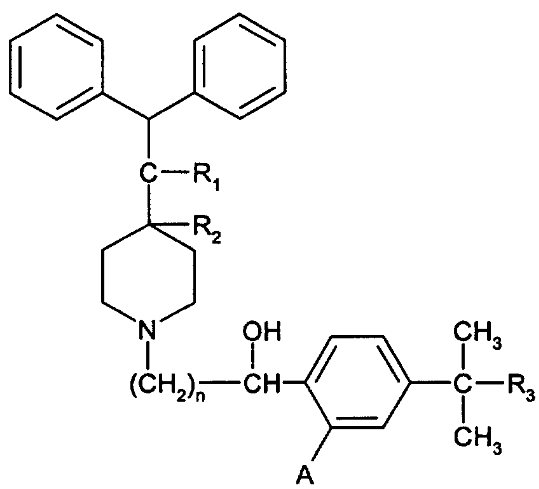

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

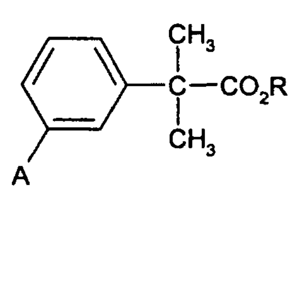

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

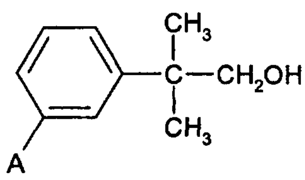

dans laquelle A est l'hydrogène ;
(b) protéger la fonctionnalité hydroxyéthyle avec un groupe protecteur convenable pour donner un alcool phénéthylique protégé de formule :

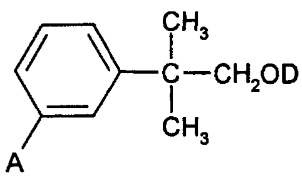

dans laquelle A est l'hydrogène et D est un groupe protecteur convenable ;
(c) faire réagir l'alcool phénéthylique protégé avec un composé ω-halogéno de formule :

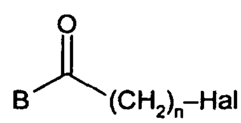

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

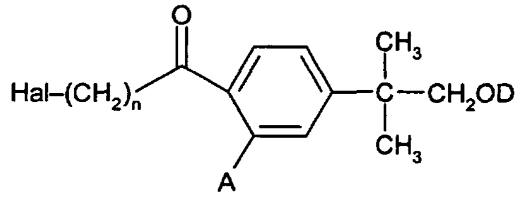

dans laquelle Hal, n et D sont définis ci-dessus et A est l'hydrogène ;
(d) faire réagir l'hydroxyéthylphénylcétone ω-halogéno protégée avec un composé de pipéridine de formule :

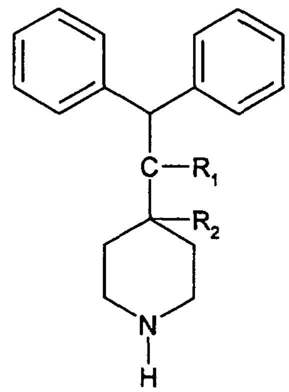

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

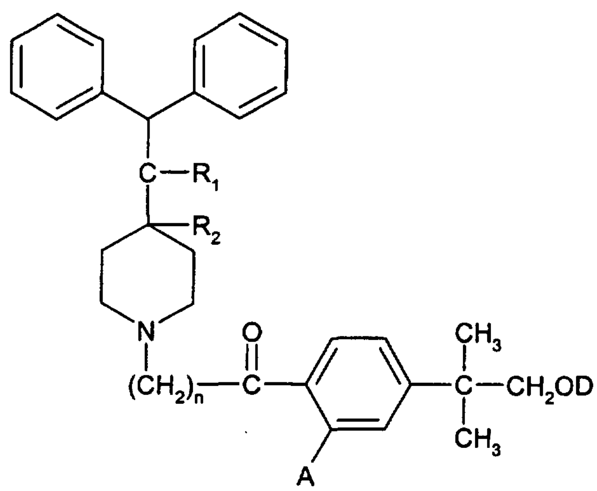

dans laquelle $R_1$, $R_2$, n et D sont définis ci-dessus et A est l'hydrogène ;

(e) déprotéger la fonctionnalité hydroxyéthyle de l'hydroxyéthylphénylcétone pipéridine protégée avec un réactif de déprotection approprié pour produire une hyxroxyéthylphénylcétone pipéridine de formule :

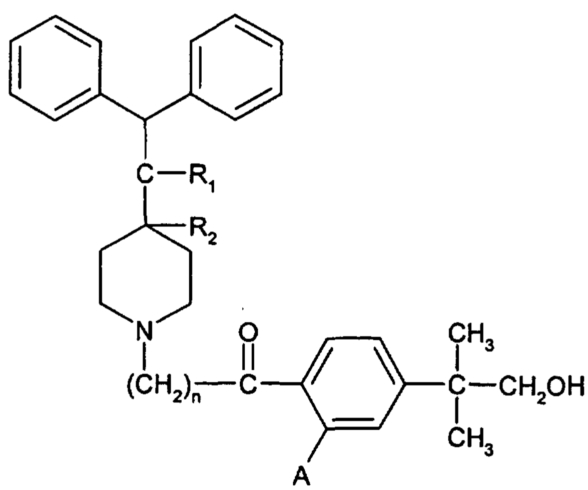

dans laquelle $R_1$, $R_2$, n et A sont définis ci-dessus ; et

(f) faire réagir le composé hydroxyéthylphénylcétone pipéridine avec du diméthylsulfoxyde, du chlorure d'oxalyle et de la triéthylamine ou avec un réactif de Dess-Martin, de l'oxyde de chrome(IV), du peroxyde de nickel, du dichromate de sodium, du dichromate de potassium, du chromate de t-butyle, de l'oxyde d'argent, du picolinate argentique du dioxyde de manganèse du tétraacétate de plomb, du dicyclohexylcarbodiimide, de la 2,3-dichloro-5,6-dicyanoquinone, de la tétrachloro-1,2-benzoquinone, du 2,2,6,6-tétraméthylpipéridinyl-1-oxy (TEMPO) ou du chlorochromate de quinolinium pour produire une formylphénylcétone pipéridine de formule :

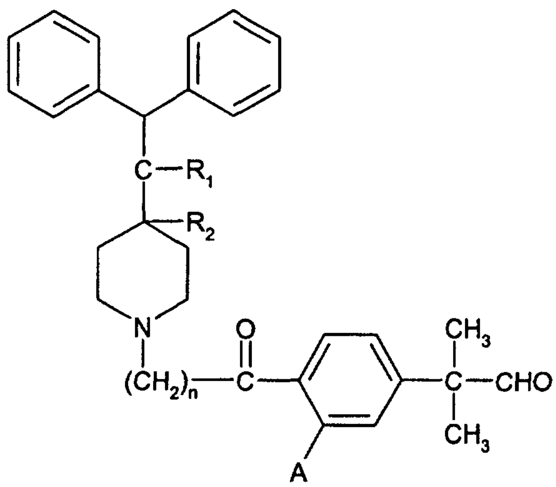

dans laquelle $R_1$, $R_2$ et n sont définis ci-dessus et A est l'hydrogène ;

(g) faire réagir la formylphénylcétone pipéridine avec du permanganate de potassium, de l'oxyde de chrome (IV), de l'oxyde d'argent(I), de l'oxyde d'argent, du picolinate argentique, un peroxyde, de l'acide nitrique, de l'acide m-chloroperbenzoïque et de l'acide peracétique pour produire une carboxyphénylcétone pipéridine de formule :

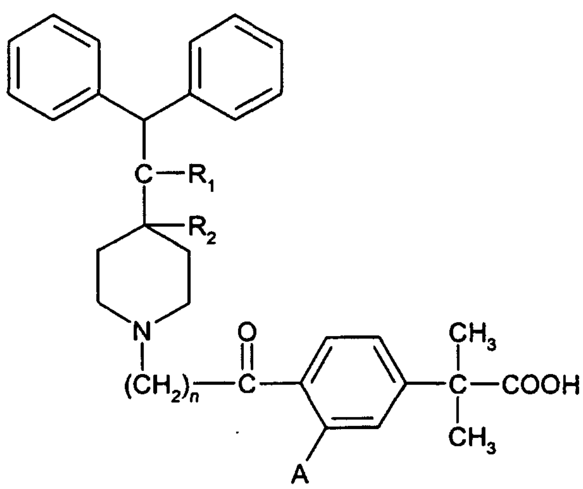

dans laquelle $R_1$, $R_2$ et n sont définis ci-dessus et A est l'hydrogène ;

(h) faire réagir la carboxyphénylcétone pipéridine avec du borohydrure de sodium, du borohydrure de potassium, du cyanoborohydrure de sodium, du borohydrure de tétraméthylammonium, de l'hydrure de lithium et de tri-tert-butyl-aluminium, de l'hydrure de diisobutyl-aluminium ou catalyser une hydrogénation pour produire une hydroxyphénylalcool pipéridine de formule :

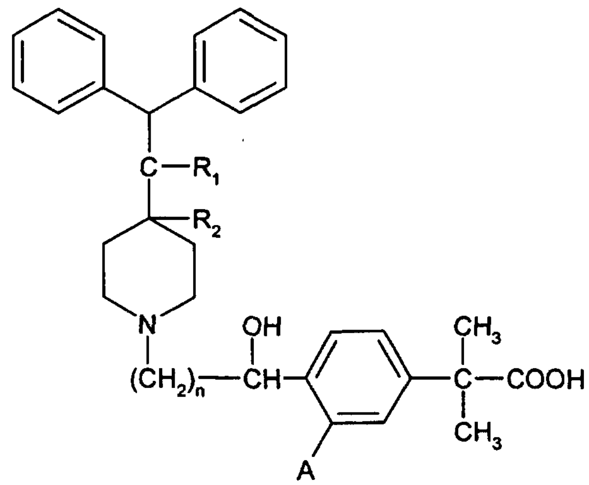

dans laquelle $R_1$, $R_2$ et n sont définis ci-dessus et A est l'hydrogène ;

(i) éventuellement faire réagir la carboxyphénylalcool pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylalcool pipéridine de formule :

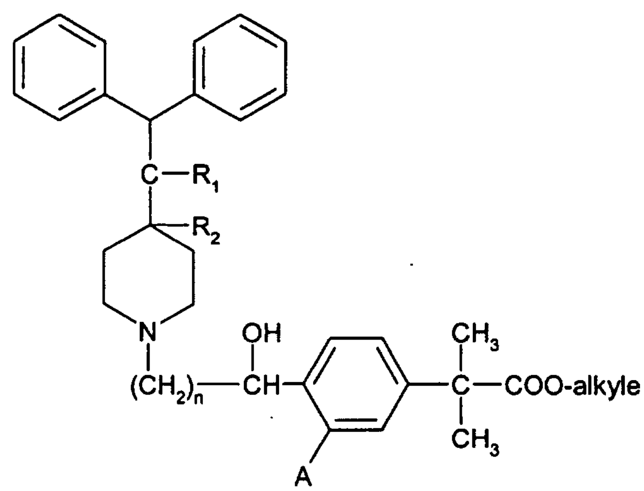

dans laquelle $R_1$, $R_2$, n et alkyle sont définis ci-dessus et A est l'hydrogène.

**10.** Procédé pour préparer un composé de formule :

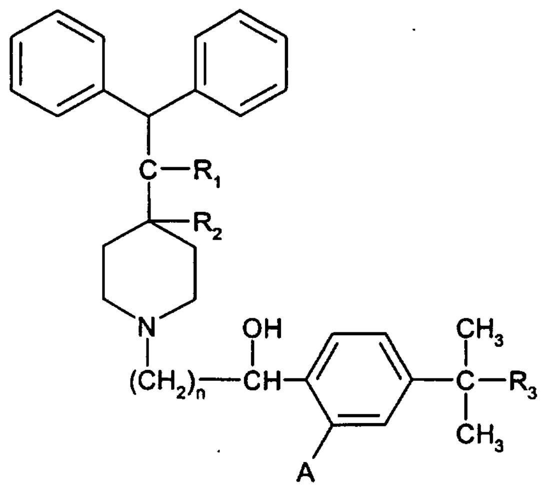

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

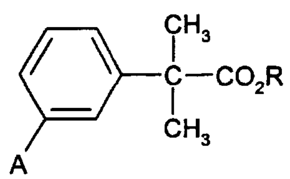

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

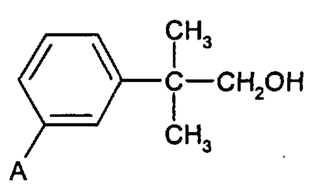

dans laquelle A est l'hydrogène ;
(b) protéger la fonctionnalité hydroxyéthyle avec un groupe protecteur convenable pour donner un alcool phénéthylique protégé de formule :

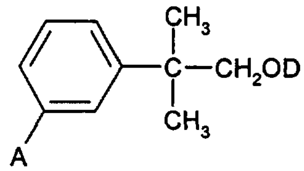

dans laquelle A est l'hydrogène et D est un groupe protecteur convenable ;
(c) faire réagir l'alcool phénéthylique protégé avec un composé ω-halogéno de formule :

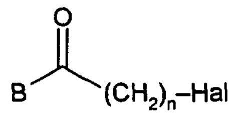

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

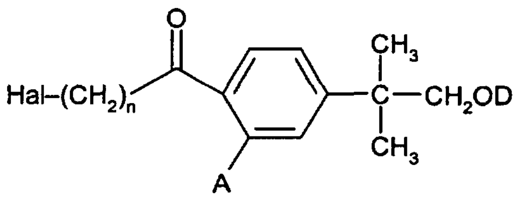

dans laquelle Hal, n et D sont définis ci-dessus et A est l'hydrogène ;
(d) faire réagir l'hydroxyéthylphénylcétone ω-halogéno protégée avec un composé de pipéridine de formule :

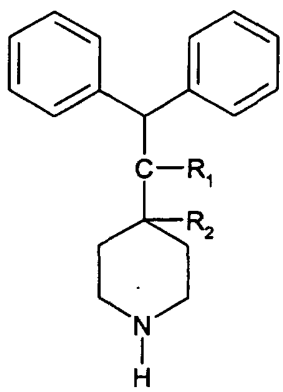

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

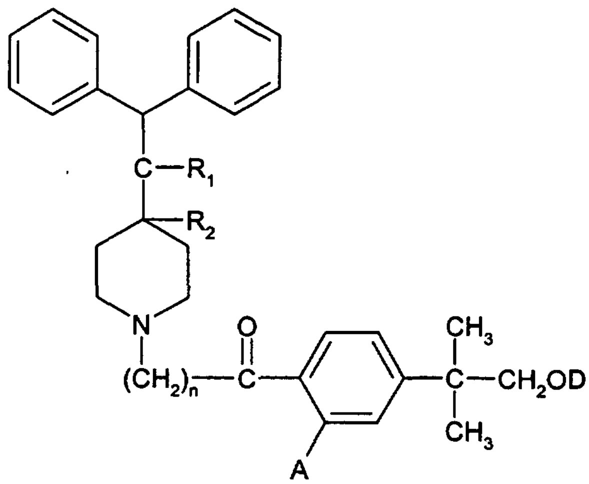

dans laquelle $R_1$, $R_2$, n et D sont définis ci-dessus et A est l'hydrogène ;
(e) déprotéger la fonctionnalité hydroxyéthyle de l'hydroxyéthylphénylcétone pipéridine protégée avec un réactif de déprotection approprié pour produire une hydroxyéthylphénylcétone pipéridine de formule :

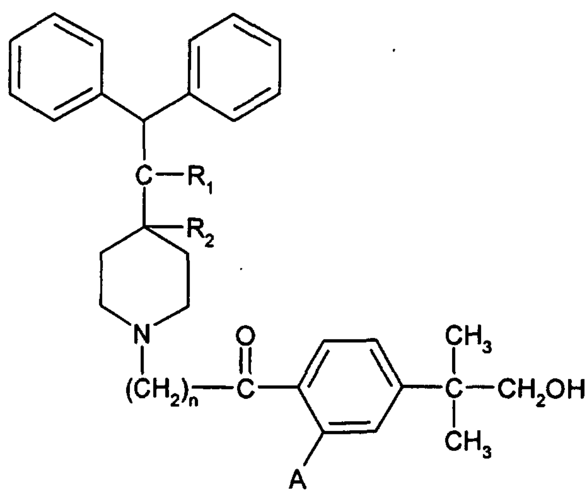

dans laquelle $R_1$, $R_2$, n et A sont définis ci-dessus ;
(f) faire réagir le composé hydroxyéthylphénylcétone pipéridine avec de l'oxyde de chrome(IV), du permanganate de potassium, de l'acide nitrique, du dioxyde d'azote, de l'oxyde de ruthénium(VIII), du peroxyde de nickel, de l'oxyde d'argent, du chromate de t-butyle ou de l'acide xénique, pour produire une carboxyphénylcétone pipéridine de formule :

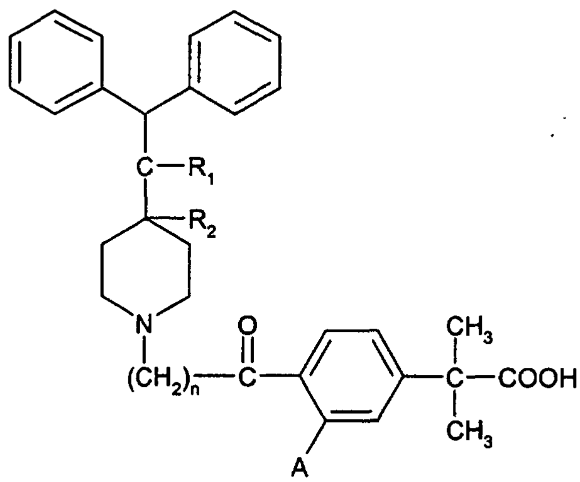

dans laquelle $R_1$, $R_2$ et n sont définis ci-dessus et A est l'hydrogène ; et
(g) faire réagir la carboxyphénylcétone pipéridine avec du borohydrure de sodium, du borohydrure de potassium, du cyanoborohydrure de sodium, du borohydrure de tétraméthylammonium, de l'hydrure de lithium et de tri-tert-butyl-aluminium, de l'hydrure de diisobutyl-aluminium ou catalyser une hydrogénation pour produire une carboxyphénylalcool pipéridine de formule :

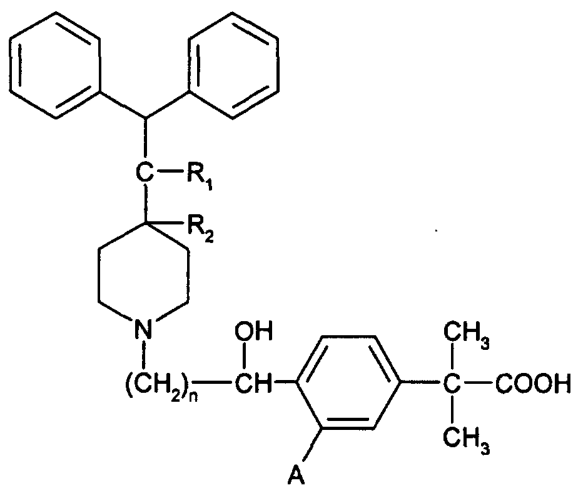

dans laquelle $R_1$, $R_2$ et n sont définis ci-dessus et A est l'hydrogène ;
(h) éventuellement faire réagir la carboxyphénylalcool pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylalcool pipéridine de formule :

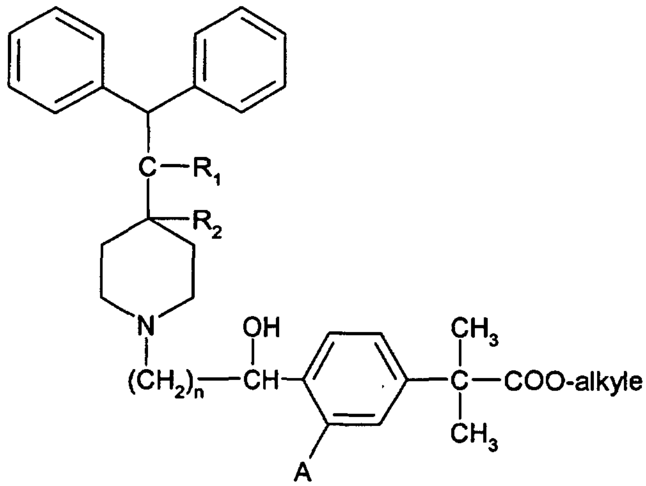

dans laquelle $R_1$, $R_2$, n et alkyle sont définis ci-dessus et A est l'hydrogène.

**11.** Procédé selon la revendication 10, dans lequel l'hydroxyéthylphénylcétone pipéridine produite dans l'étape e est mise à réagir avec de l'oxyde de ruthénium(VIII) pour produire la carboxyphénylcétone pipéridine de l'étape f.

**12.** Procédé pour préparer un composé de formule :

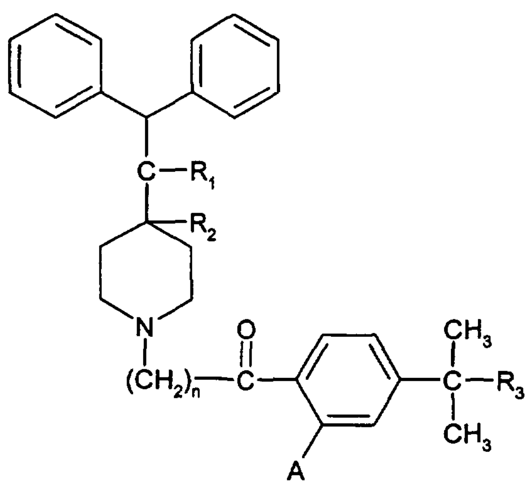

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

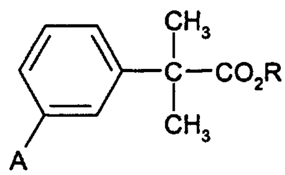

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

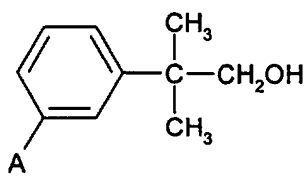

dans laquelle A est l'hydrogène ;

(b) protéger la fonctionnalité hydroxyéthyle avec un groupe protecteur convenable pour donner un alcool phénéthylique protégé de formule :

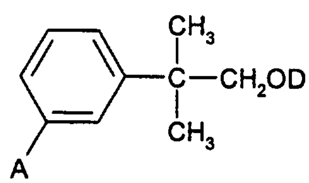

dans laquelle A est l'hydrogène et D est un groupe protecteur convenable ;

(c) faire réagir l'alcool phénéthylique protégé avec un composé ω-halogéno de formule :

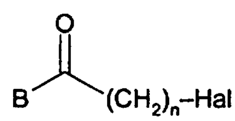

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

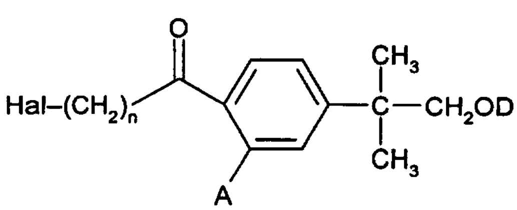

dans laquelle Hal, n et D sont définis ci-dessus et A est l'hydrogène ;

(d) faire réagir l'hydroxyéthylphénylcétone ω-halogéno protégée avec un composé de pipéridine de formule :

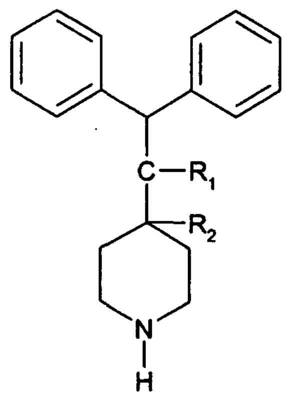

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

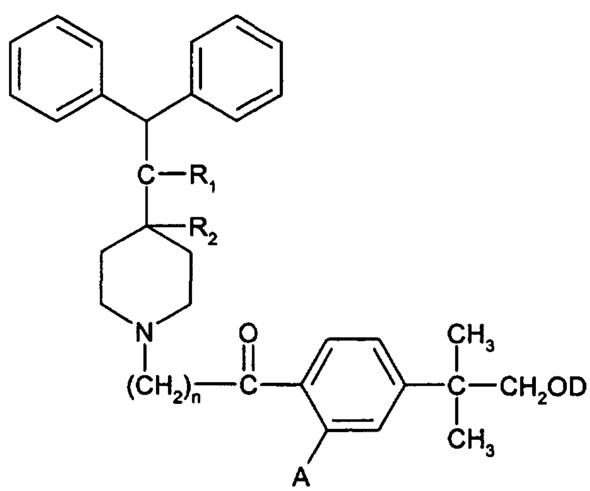

dans laquelle $R_1$, $R_2$, n et D sont définis ci-dessus et A est l'hydrogène ;
(e) déprotéger la fonctionnalité hydroxyéthyle de l'hydroxyéthylphénylcétone pipéridine protégée avec un réactif de déprotection approprié pour produire une hyxroxyéthylphénylcétone pipéridine de formule :

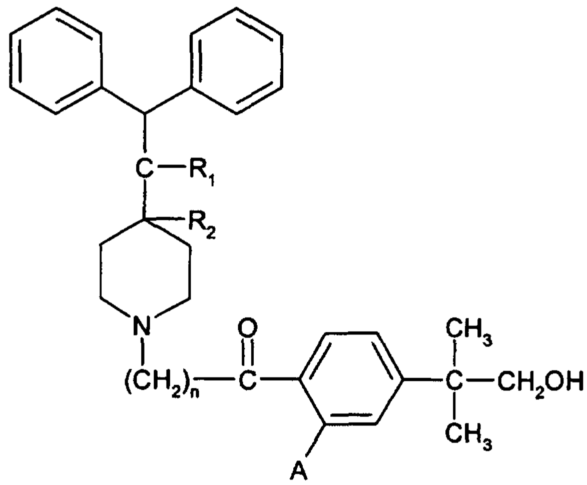

dans laquelle $R_1$, $R_2$, n sont définis ci-dessus et A est l'hydrogène ;
(f) faire réagir le composé hydroxyéthylphénylcétone pipéridine avec du diméthylsulfoxyde, du chlorure d'oxalyle et de la triéthylamine ou avec un réactif de Dess-Martin, de l'oxyde de chrome(IV), du peroxyde de nickel, du dichromate de sodium, du dichromate de potassium, du chromate de t-butyle, de l'oxyde d'argent, du picolinate argentique du dioxyde de manganèse du tétraacétate de plomb, du dicyclohexylcarbodiimide, de la 2,3-dichloro-5,6-dicyanoquinone, de la tétrachloro-1,2-benzoquinone, du 2,2,6,6-tétraméthylpipéridinyl-1-oxy (TEMPO) ou du chlorochromate de quinolinium pour produire une formylphénylcétone pipéridine de formule :

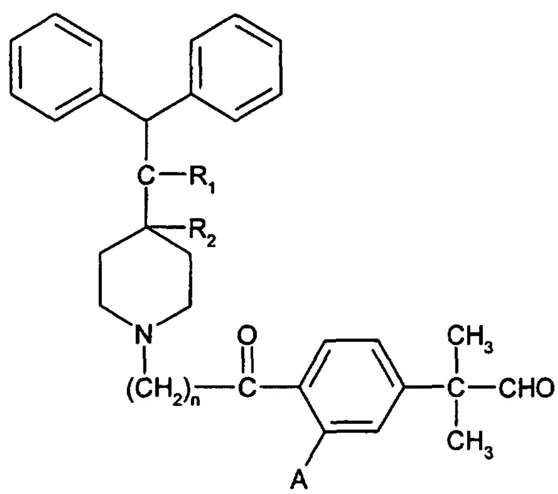

dans laquelle $R_1$, $R_2$ et n sont définis ci-dessus et A est l'hydrogène ;
(g) faire réagir la formylphénylcétone pipéridine avec du permanganate de potassium, de l'oxyde de chrome (IV), de l'oxyde d'argent(I), de l'oxyde d'argent, du picolinate argentique, un peroxyde, de l'acide nitrique, de l'acide m-chloroperbenzoïque et de l'acide peracétique pour produire une carboxyphénylcétone pipéridine de formule :

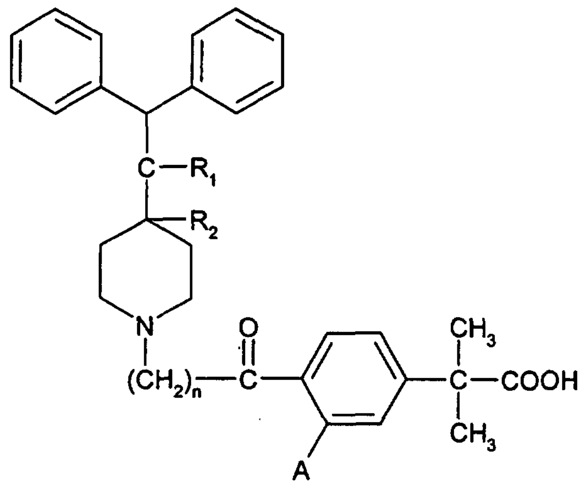

dans laquelle $R_1$, $R_2$ et n sont définis ci-dessus et A est l'hydrogène ;

(h) éventuellement faire réagir la carboxyphénylcétone pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylcétone pipéridine de formule :

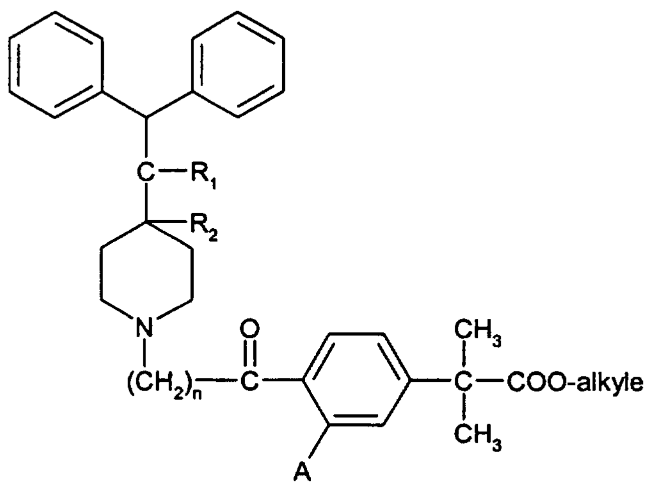

dans laquelle $R_1$, $R_2$, n et alkyle sont définis ci-dessus et A est l'hydrogène.

**13.** Procédé pour préparer un composé de formule :

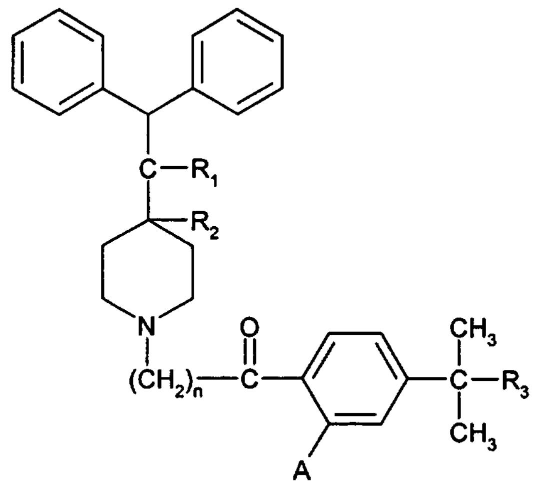

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

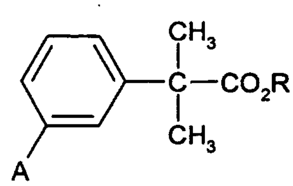

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

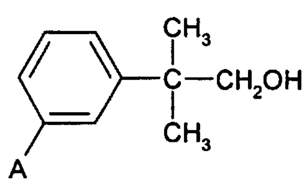

dans laquelle A est l'hydrogène ;
(b) protéger la fonctionnalité hydroxyéthyle avec un groupe protecteur convenable pour donner un alcool phénéthylique protégé de formule :

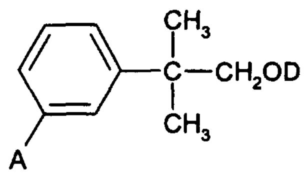

dans laquelle A est l'hydrogène et D est un groupe protecteur convenable ;

(c) faire réagir l'alcool phénéthylique protégé avec un composé ω-halogéno de formule :

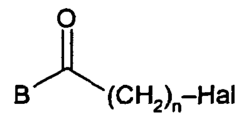

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

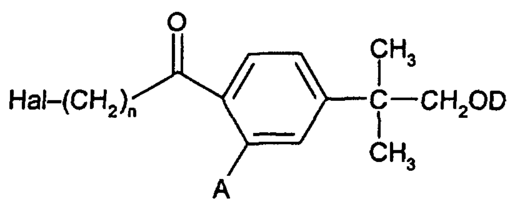

dans laquelle Hal, n et D sont définis ci-dessus et A est l'hydrogène ;

(d) faire réagir l'hydroxyéthylphénylcétone ω-halogéno protégée avec un composé de pipéridine de formule :

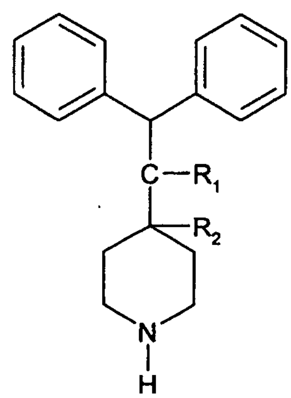

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

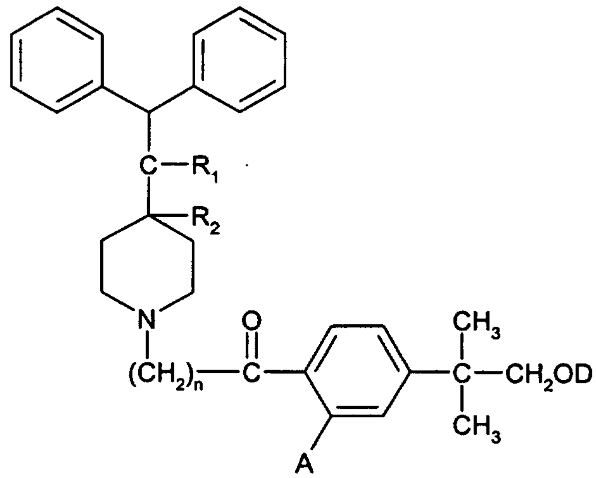

dans laquelle $R_1$, $R_2$, n et D sont définis ci-dessus et A est l'hydrogène ;

(e) déprotéger la fonctionnalité hydroxyéthyle de l'hydroxyéthylphénylcétone pipéridine protégée avec un réactif de déprotection approprié pour produire une hydroxyéthylphénylcétone pipéridine de formule :

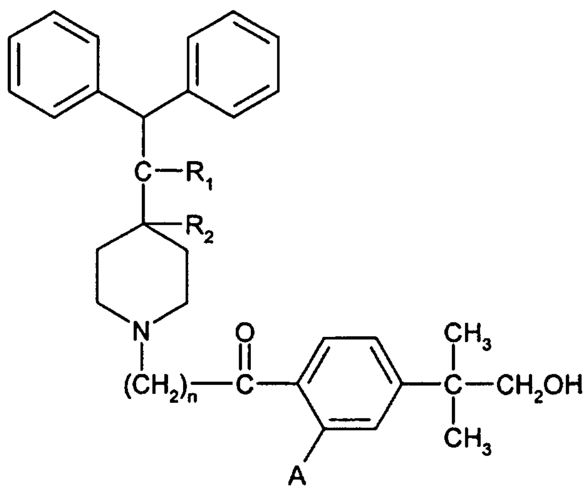

dans laquelle $R_1$, $R_2$, n et A sont définis ci-dessus ; et

(f) faire réagir le composé hydroxyéthylphénylcétone pipéridine avec de l'oxyde de chrome(IV), du permanganate de potassium, de l'acide nitrique, du dioxyde d'azote, de l'oxyde de ruthénium(VIII), du peroxyde de nickel, de l'oxyde d'argent, du chromate de t-butyle ou de l'acide xénique pour produire la carboxyphénylcétone pipéridine de formule :

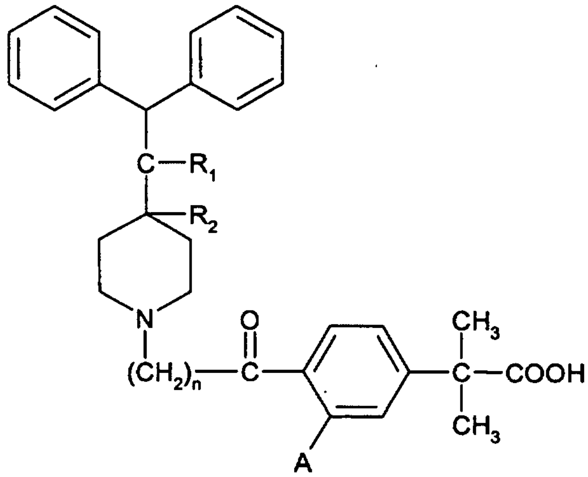

dans laquelle $R_1$, $R_2$ et n sont définis ci-dessus et A est l'hydrogène ;

(g) éventuellement faire réagir la carboxyphénylcétone pipéridine avec du diazométhane ou du 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylcétone pipéridine de formule :

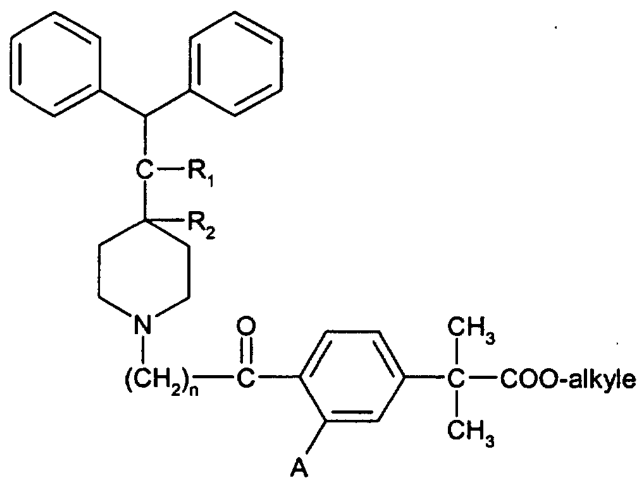

dans laquelle $R_1$, $R_2$, n et alkyle sont définis ci-dessus et A est l'hydrogène.

**14.** Procédé selon la revendication 13, dans lequel l'hydroxyéthylphénylcétone pipéridine produite dans l'étape e est mise à réagir avec de l'oxyde de ruthénium(VIII) pour produire la carboxyphénylcétone pipéridine de l'étape f.

**15.** Procédé pour préparer un composé hydroxyéthylphénylcétone pipéridine de formule :

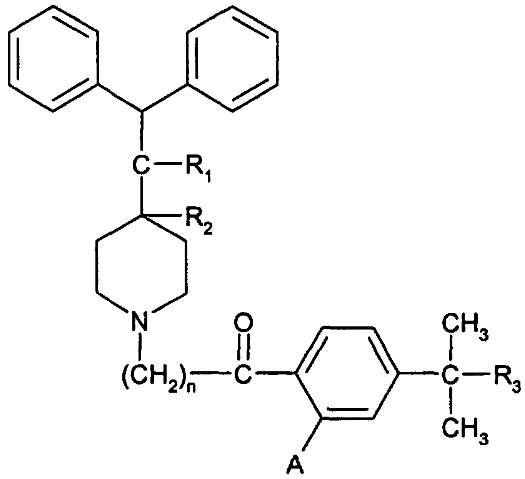

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est $-CH_2OH$ ;
A est l'hydrogène ; et
ses sels acceptables en pharmacie, comprenant les étapes consistant à :

(a) faire réagir un composé d'acide benzène-acétique de formule :

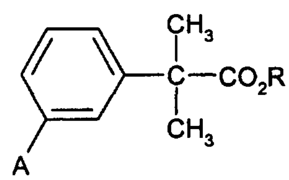

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

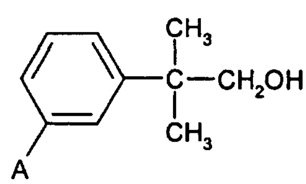

dans laquelle A est l'hydrogène ;
(b) protéger la fonctionnalité hydroxyéthyle avec un groupe protecteur convenable pour donner un alcool phénéthylique protégé de formule :

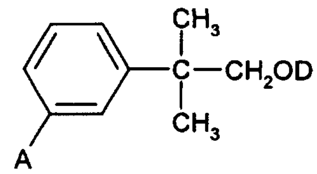

dans laquelle A est l'hydrogène et D est un groupe protecteur convenable ;

(c) faire réagir l'alcool phénéthylique protégé avec un composé ω-halogéno de formule :

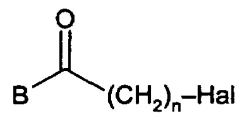

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

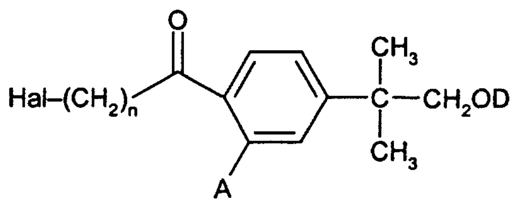

dans laquelle Hal, n et D sont définis ci-dessus et A est l'hydrogène ;

(d) faire réagir l'hydroxyéthylphénylcétone ω-halogéno protégée avec un composé de pipéridine de formule :

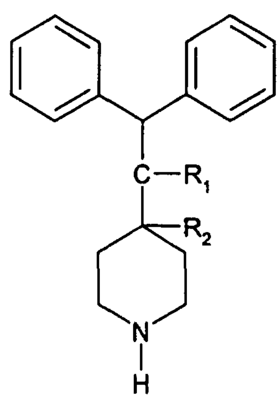

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

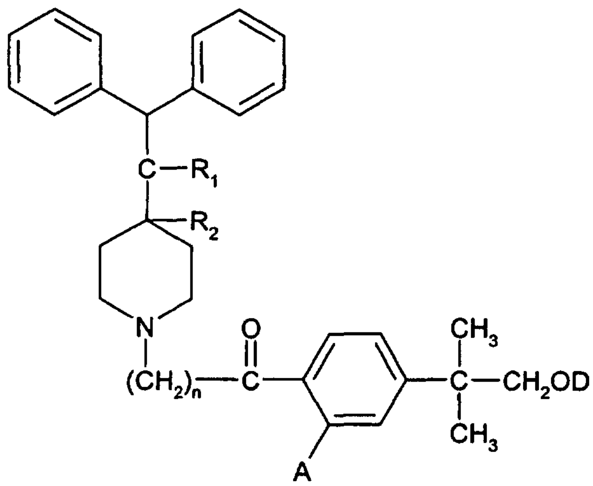

dans laquelle $R_1$, $R_2$, n et D sont définis ci-dessus et A est l'hydrogène ;
(e) déprotéger la fonctionnalité hydroxyéthyle de l'hydroxyéthylphénylcétone pipéridine protégée avec un réactif de déprotection approprié pour produire une hydroxyéthylphénylcétone pipéridine de formule :

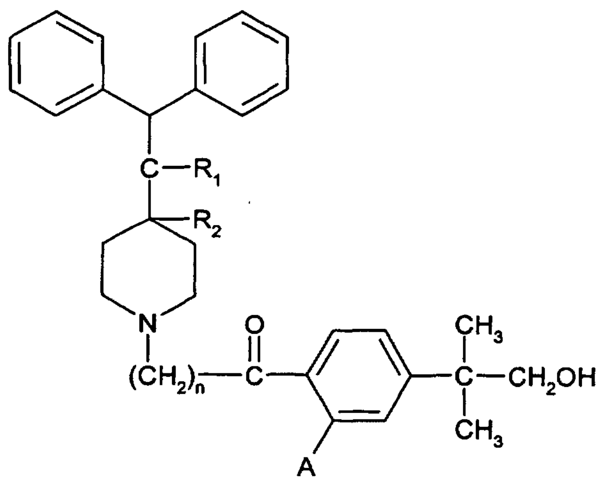

dans laquelle $R_1$, $R_2$, n et A sont définis ci-dessus.

**16.** Procédé pour préparer un composé de formule :

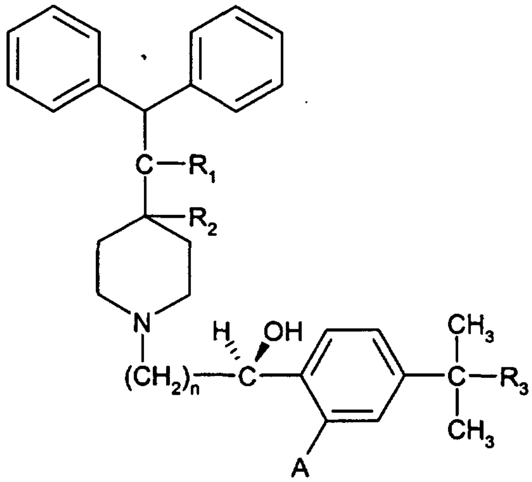

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

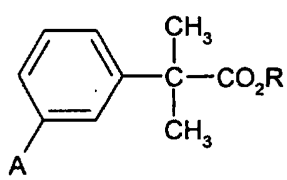

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

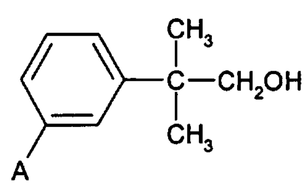

dans laquelle A est l'hydrogène ;

(b) protéger la fonctionnalité hydroxyéthyle avec un groupe protecteur convenable pour donner un alcool phénéthylique protégé de formule :

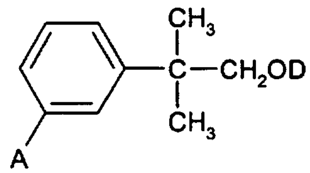

dans laquelle A est l'hydrogène et D est un groupe protecteur convenable ;

(c) faire réagir l'alcool phénéthylique protégé avec un composé ω-halogéno de formule :

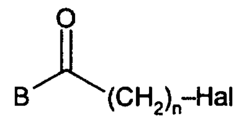

dans laquelle B est un radical halogéno ou hydroxy, Hal représente Cl, Br ou I et n est tel que défini ci-dessus, en présence de trichlorure, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

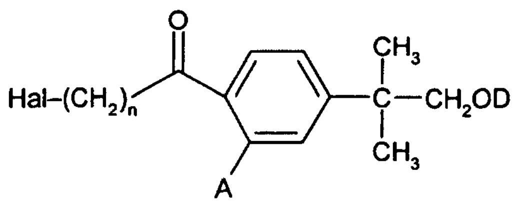

dans laquelle Hal, n et D sont définis ci-dessus et A est l'hydrogène ;

(d) faire réagir l'hydroxyéthylphénylcétone ω-halogéno protégée avec un composé de pipéridine de formule :

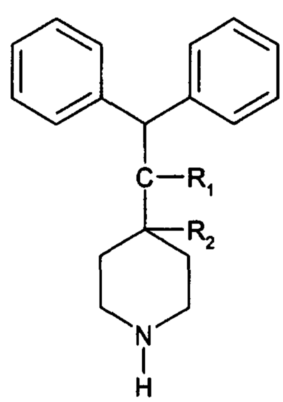

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

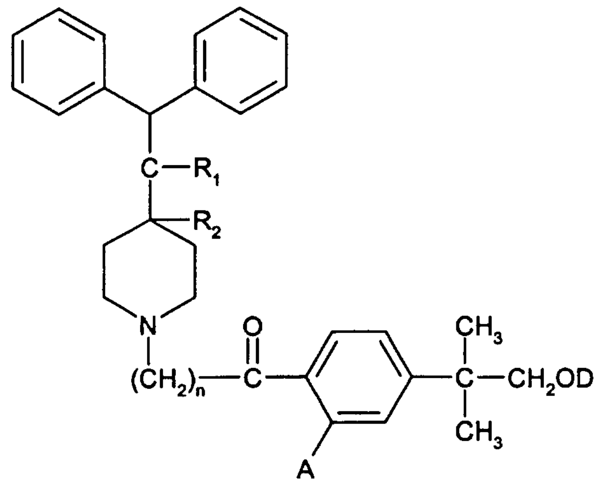

dans laquelle $R_1$, $R_2$, n et D sont définis ci-dessus et A est l'hydrogène ;

(e) déprotéger la fonctionnalité hydroxyéthyle de l'hydroxyéthylphénylcétone pipéridine protégée avec un réactif de déprotection approprié pour produire une hydroxyéthylphénylcétone pipéridine de formule :

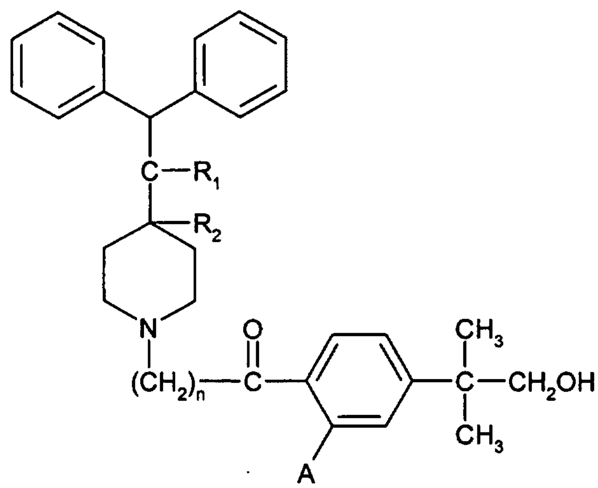

dans laquelle $R_1$, $R_2$, n et A sont définis ci-dessus ; et

(f) faire réagir le composé hydroxyéthylphénylcétone pipéridine avec du diméthylsulfoxyde, du chlorure d'oxalyle et de la triéthylamine ou avec un réactif de Dess-Martin, de l'oxyde de chrome(IV), du peroxyde de nickel, du dichromate de sodium, du dichromate de potassium, du chromate de t-butyle, de l'oxyde d'argent, du picolinate argentique du dioxyde de manganèse du tétraacétate de plomb, du dicyclohexylcarbodiimide, de la 2,3-dichloro-5,6-dicyanoquinone, de la tétrachloro-1,2-benzoquinone, du 2,2,6,6-tétraméthylpipéridinyl-1-oxy (TEMPO) ou du chlorochromate de quinolinium pour produire une formylphénylcétone pipéridine de formule :

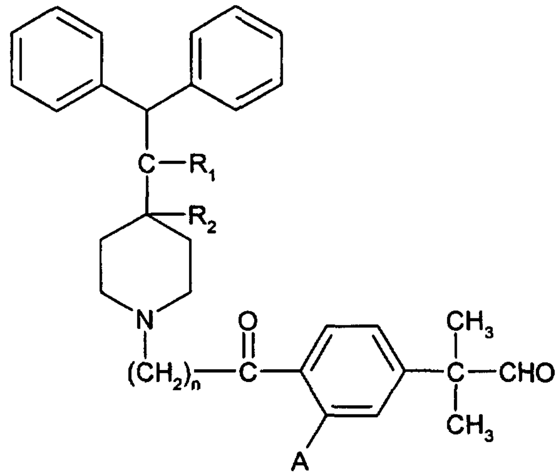

dans laquelle $R_1$, $R_2$ et n sont définis ci-dessus et A est l'hydrogène ;

(g) faire réagir la formylphénylcétone pipéridine avec du permanganate de potassium, de l'oxyde de chrome (IV), de l'oxyde d'argent(I), de l'oxyde d'argent, du picolinate argentique, un peroxyde, de l'acide nitrique, de l'acide m-chloroperbenzoïque et de l'acide peracétique pour produire une carboxyphénylcétone pipéridine de formule :

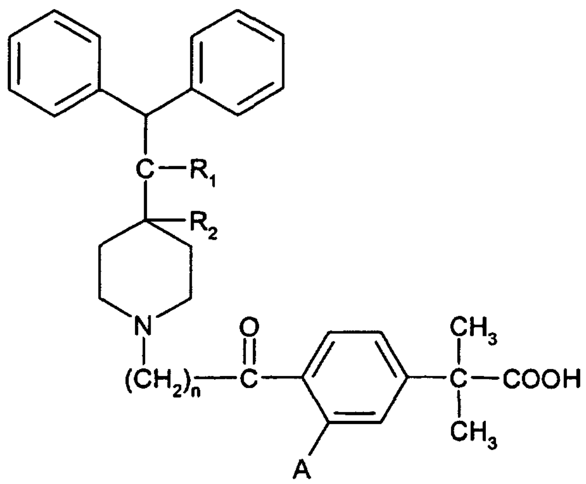

dans laquelle $R_1$, $R_2$ et n sont définis ci-dessus et A est l'hydrogène ;

(h) faire réagir la carboxyphénylcétone pipéridine avec du (+)-B-chlorodiisopinocamphénylborane pour produire un carboxyphénylalcool pipéridine de formule :

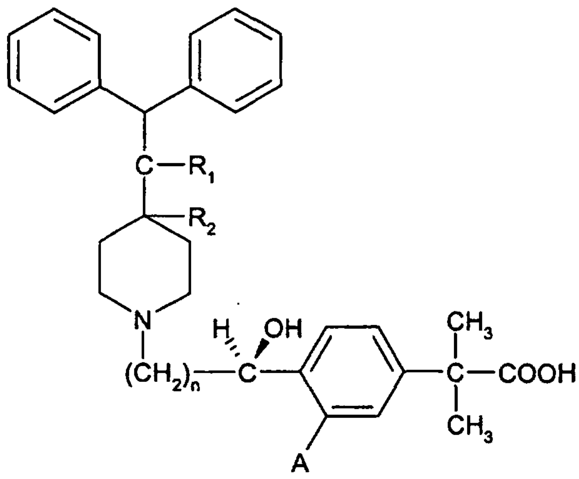

dans laquelle $R_1$, $R_2$ et n sont définis ci-dessus et A est l'hydrogène ;

(i) éventuellement faire réagir la carboxyphénylalcool pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylalcool pipéridine de formule :

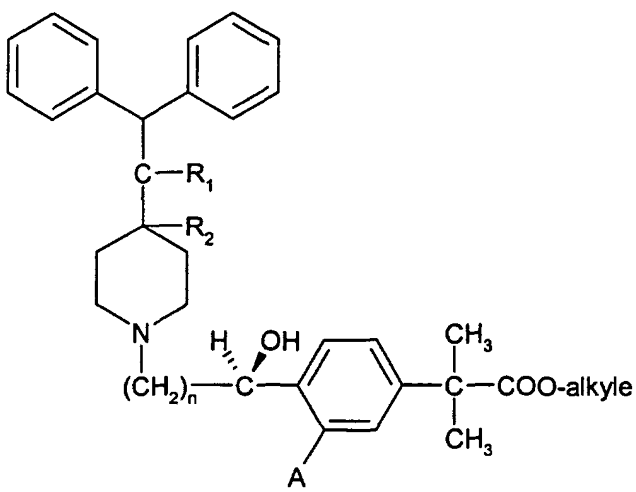

dans laquelle $R_1$, $R_2$, n et alkyle sont définis ci-dessus et A est l'hydrogène.

**17.** Procédé pour préparer un composé de formule :

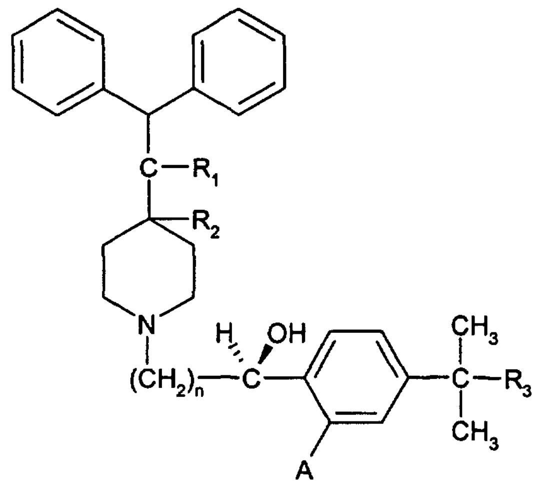

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

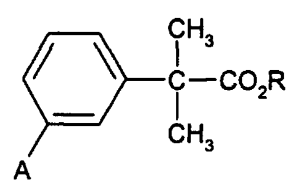

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

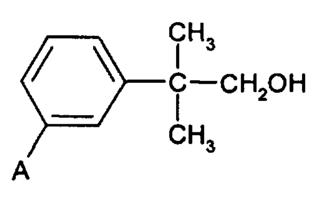

dans laquelle A est l'hydrogène ;
(b) protéger la fonctionnalité hydroxyéthyle avec un groupe protecteur convenable pour donner un alcool phénéthylique protégé de formule :

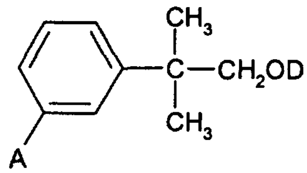

dans laquelle A est l'hydrogène et D est un groupe protecteur convenable ;
(c) faire réagir l'alcool phénéthylique protégé avec un composé ω-halogéno de formule :

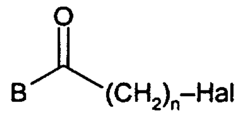

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

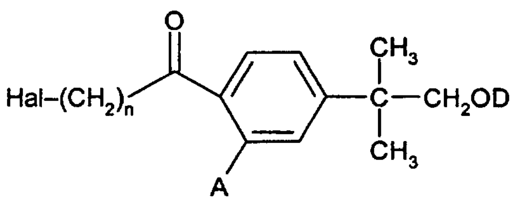

dans laquelle Hal, n et D sont définis ci-dessus et A est l'hydrogène ;
(d) faire réagir l'hydroxyéthylphénylcétone ω-halogéno protégée avec un composé de pipéridine de formule :

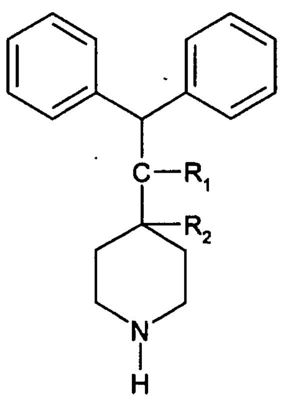

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

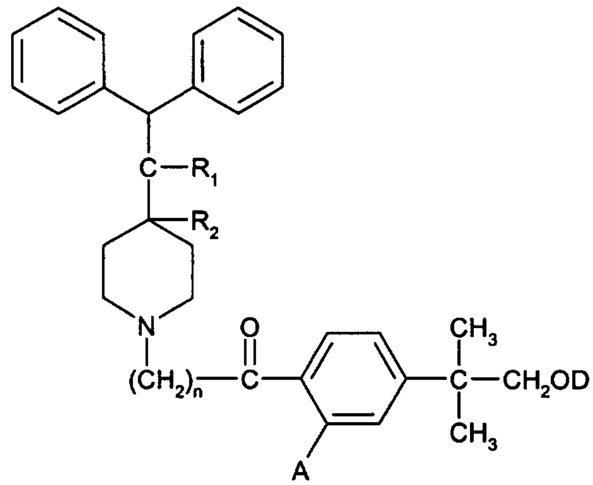

dans laquelle $R_1$, $R_2$, n et D sont définis ci-dessus et A est l'hydrogène ;

(e) déprotéger la fonctionnalité hydroxyéthyle de l'hydroxyéthylphénylcétone pipéridine protégée avec un réactif de déprotection approprié pour produire une hydroxyéthylphénylcétone pipéridine de formule :

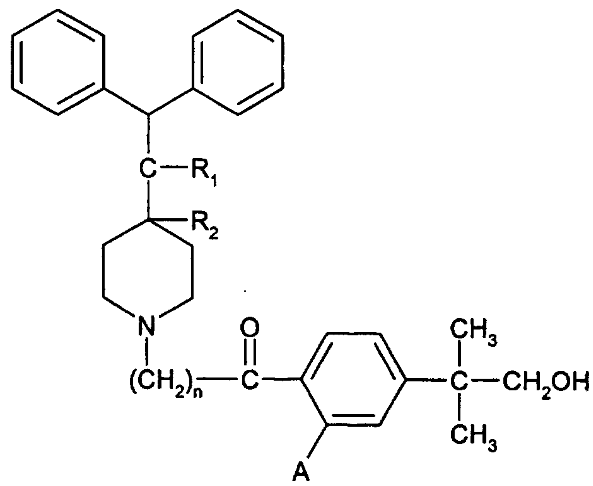

dans laquelle $R_1$, $R_2$, n et A sont définis ci-dessus ; et

(f) faire réagir le composé hydroxyéthylphénylcétone pipéridine avec de l'oxyde de chrome(IV), du permanganate de potassium, de l'acide nitrique, du dioxyde d'azote, de l'oxyde de ruthénium(VIII), du peroxyde de nickel, de l'oxyde d'argent, du chromate de t-butyle ou de l'acide xénique pour produire une carboxyphénylcétone pipéridine de formule :

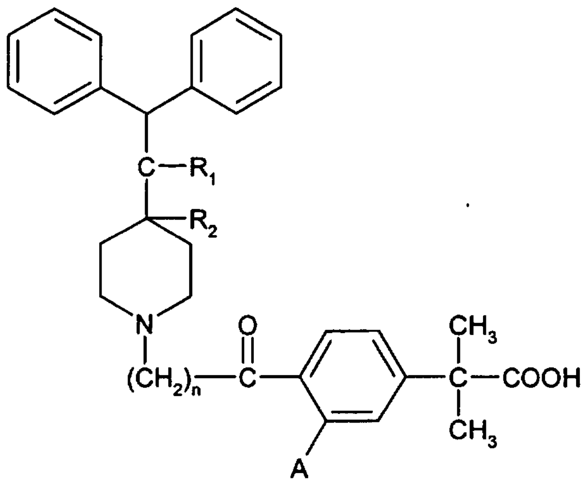

dans laquelle $R_1$, $R_2$ et n sont définis ci-dessus et A est l'hydrogène ;

(g) faire réagir la carboxyphénylcétone pipéridine avec du (+)-B-chlorodiisopinocamphénylborane pour produire un carboxyphénylalcool pipéridine de formule :

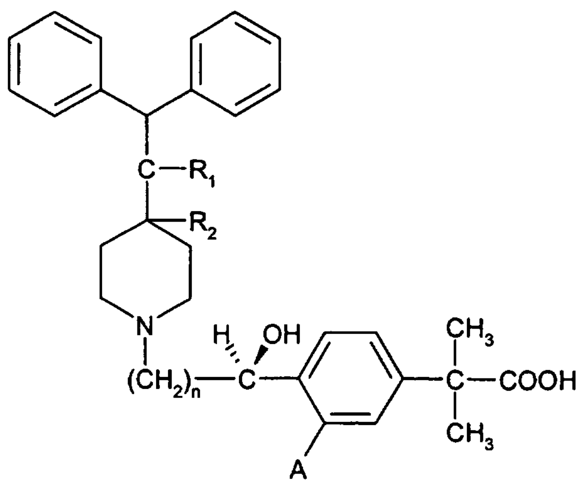

dans laquelle $R_1$, $R_2$ et n sont définis ci-dessus et A est l'hydrogène ;

(h) éventuellement faire réagir la carboxyphénylalcool pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylalcool pipéridine de formule :

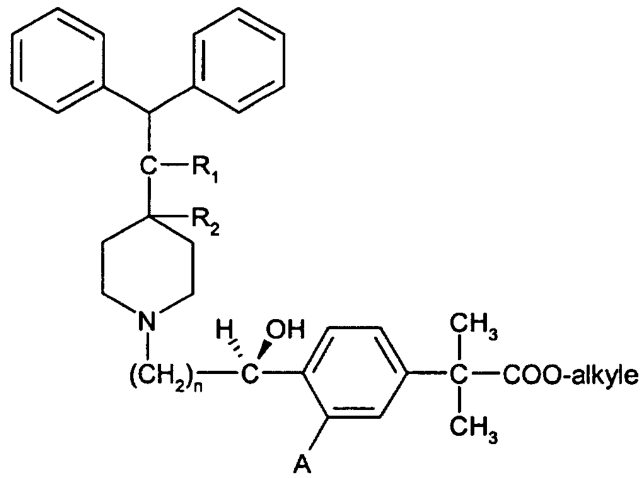

dans laquelle $R_1$, $R_2$, n et alkyle sont définis ci-dessus et A est l'hydrogène.

**18.** Procédé pour préparer un composé de formule :

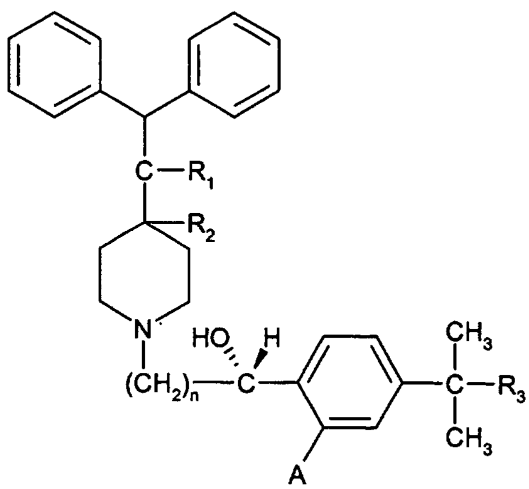

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

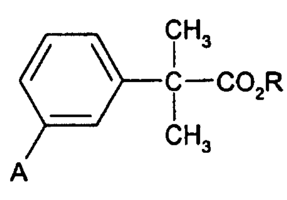

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

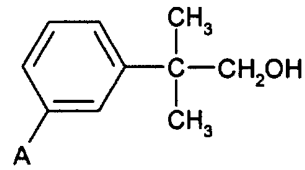

dans laquelle A est l'hydrogène ;

(b) protéger la fonctionnalité hydroxyéthyle avec un groupe protecteur convenable pour donner un alcool phénéthylique protégé de formule :

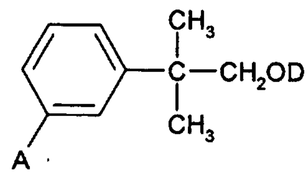

dans laquelle A est l'hydrogène et D est un groupe protecteur convenable ;

(c) faire réagir l'alcool phénéthylique protégé avec un composé ω-halogéno de formule :

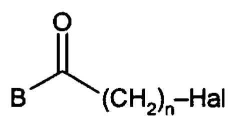

dans laquelle B est un radical halogéno ou hydroxy, Hal représente Cl, Br ou I et n est tel que défini ci-dessus, en présence de trichlorure, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

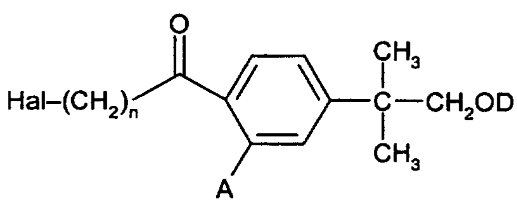

dans laquelle Hal, n et D sont définis ci-dessus et A est l'hydrogène ;

(d) faire réagir l'hydroxyéthylphénylcétone ω-halogéno protégée avec un composé de pipéridine de formule :

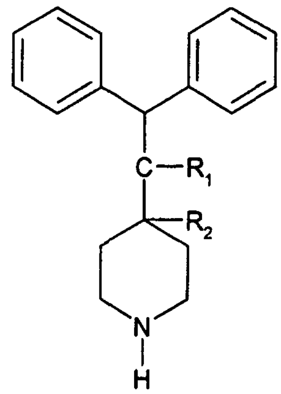

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

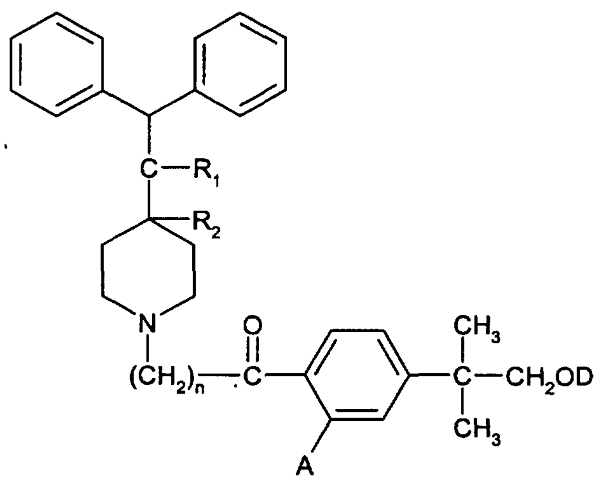

dans laquelle $R_1$, $R_2$, n et D sont définis ci-dessus et A est l'hydrogène ;
(e) déprotéger la fonctionnalité hydroxyéthyle de l'hydroxyéthylphénylcétone pipéridine protégée avec un réactif de déprotection approprié pour produire une hydroxyéthylphénylcétone pipéridine de formule :

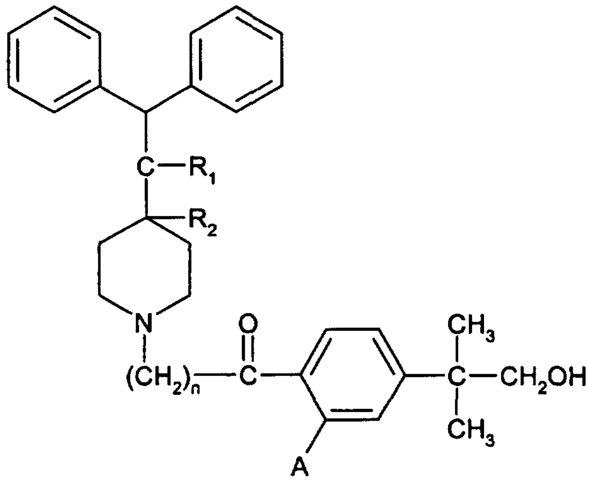

dans laquelle $R_1$, $R_2$, n et A sont définis ci-dessus ; et

(f) faire réagir le composé hydroxyéthylphénylcétone pipéridine avec du diméthylsulfoxyde, du chlorure d'oxalyle et de la triéthylamine ou avec un réactif de Dess-Martin, de l'oxyde de chrome(IV), du peroxyde de nickel, du dichromate de sodium, du dichromate de potassium, du chromate de t-butyle, de l'oxyde d'argent, du picolinate argentique du dioxyde de manganèse du tétraacétate de plomb, du dicyclohexylcarbodiimide, de la 2,3-dichloro-5,6-dicyanoquinone, de la tétrachloro-1,2-benzoquinone, du 2,2,6,6-tétraméthylpipéridinyl-1-oxy (TEMPO) ou du chlorochromate de quinolinium pour produire une formylphénylcétone pipéridine de formule :

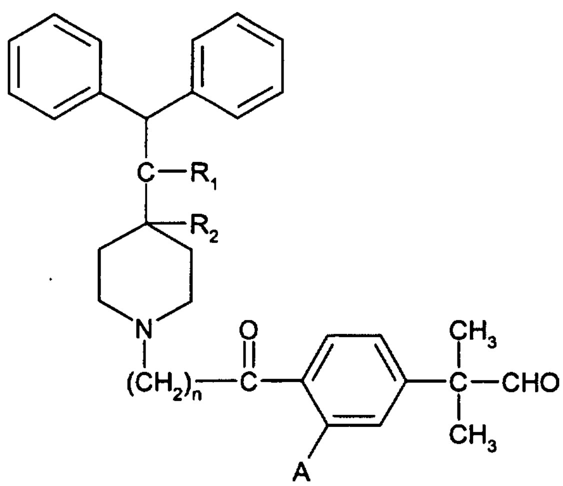

dans laquelle $R_1$, $R_2$ et n sont définis ci-dessus et A est l'hydrogène ;

(g) faire réagir la formylphénylcétone pipéridine avec du permanganate de potassium, de l'oxyde de chrome (IV), de l'oxyde d'argent(I), de l'oxyde d'argent, du picolinate argentique, un peroxyde, de l'acide nitrique, de l'acide m-chloroperbenzoïque et de l'acide peracétique pour produire une carboxyphénylcétone pipéridine de formule :

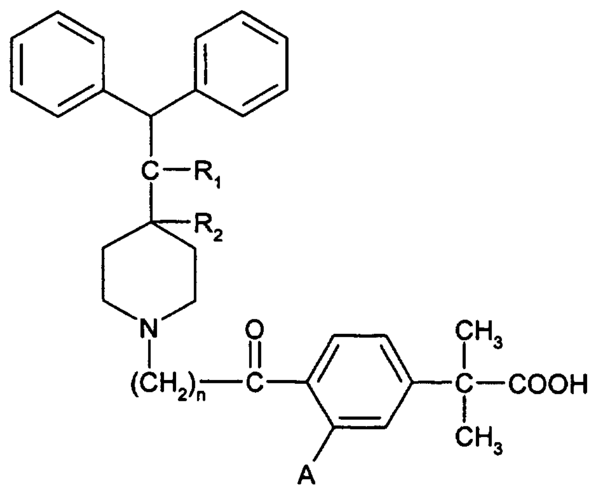

dans laquelle $R_1$, $R_2$ et n sont définis ci-dessus et A est l'hydrogène ;
(h) faire réagir la carboxyphénylcétone pipéridine avec du (-)-B-chlorodiisopinocamphénylborane pour produire un carboxyphénylalcool pipéridine de formule :

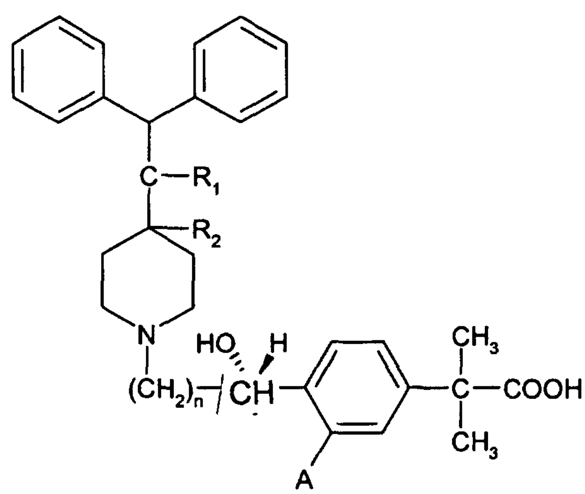

dans laquelle $R_1$, $R_2$ et n sont définis ci-dessus et A est l'hydrogène ;
(i) éventuellement faire réagir la carboxyphénylalcool pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylalcool pipéridine de formule :

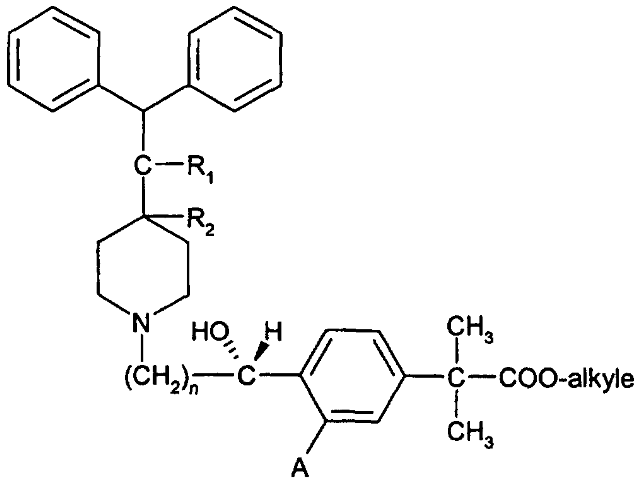

dans laquelle $R_1$, $R_2$, n et alkyle sont définis ci-dessus et A est l'hydrogène.

**19.** Procédé pour préparer un composé de formule :

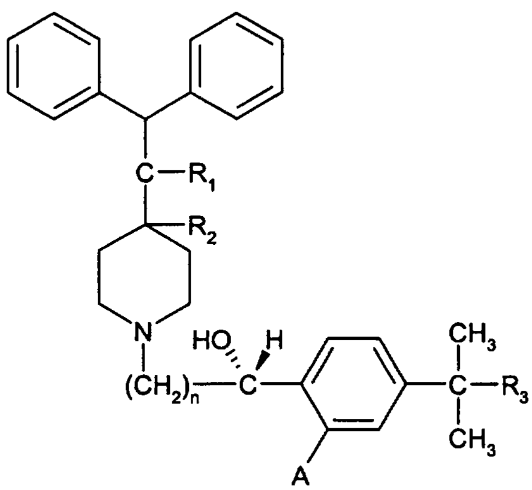

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

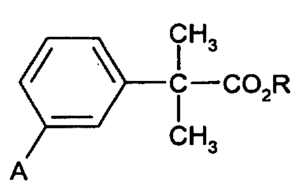

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

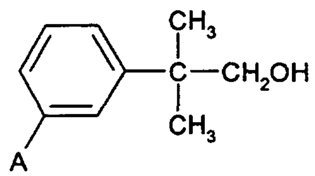

dans laquelle A est l'hydrogène ;
(b) protéger la fonctionnalité hydroxyéthyle avec un groupe protecteur convenable pour donner un alcool phénéthylique protégé de formule :

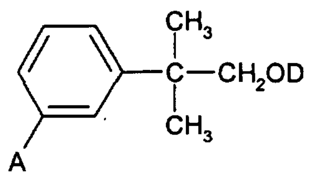

dans laquelle A est l'hydrogène et D est un groupe protecteur convenable ;
(c) faire réagir l'alcool phénéthylique protégé avec un composé ω-halogéno de formule :

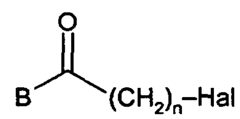

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

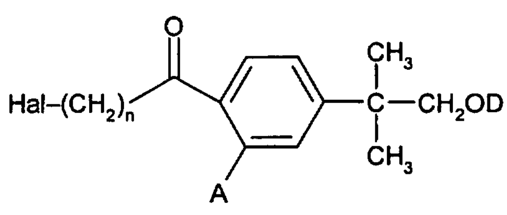

dans laquelle Hal, n et D sont définis ci-dessus et A est l'hydrogène ;
(d) faire réagir l'hydroxyéthylphénylcétone ω-halogéno protégée avec un composé de pipéridine de formule :

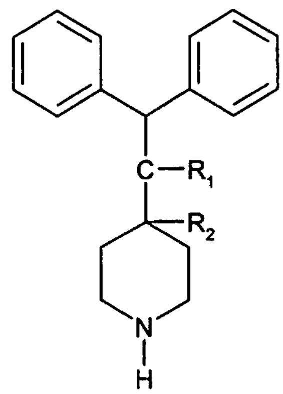

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

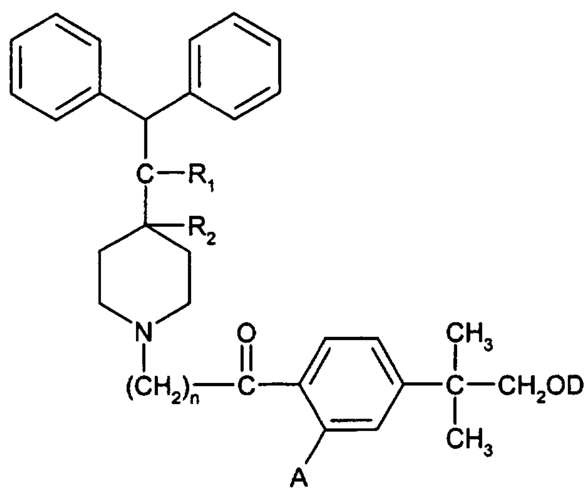

dans laquelle $R_1$, $R_2$, n et D sont définis ci-dessus et A est l'hydrogène ;
(e) déprotéger la fonctionnalité hydroxyéthyle de l'hydroxyéthylphénylcétone pipéridine protégée avec un réactif de déprotection approprié pour produire une hydroxyéthylphénylcétone pipéridine de formule :

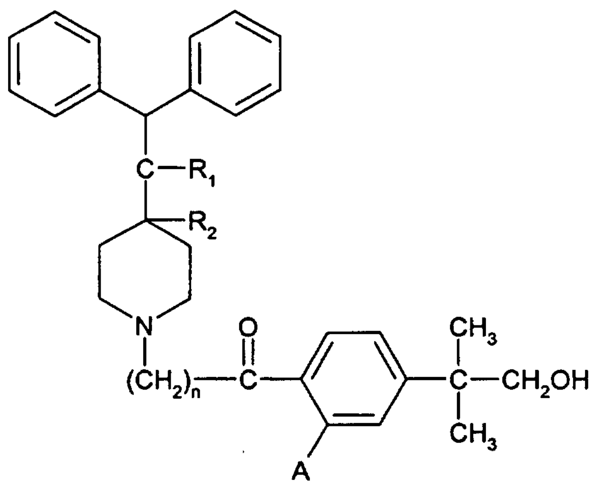

dans laquelle $R_1$, $R_2$, n et A sont définis ci-dessus ; et

(f) faire réagir le composé hydroxyéthylphénylcétone pipéridine avec de l'oxyde de chrome(IV), du permanganate de potassium, de l'acide nitrique, du dioxyde d'azote, de l'oxyde de ruthénium(VIII), du peroxyde de nickel, de l'oxyde d'argent, du chromate de t-butyle ou de l'acide xénique pour produire une carboxyphénylcétone pipéridine de formule :

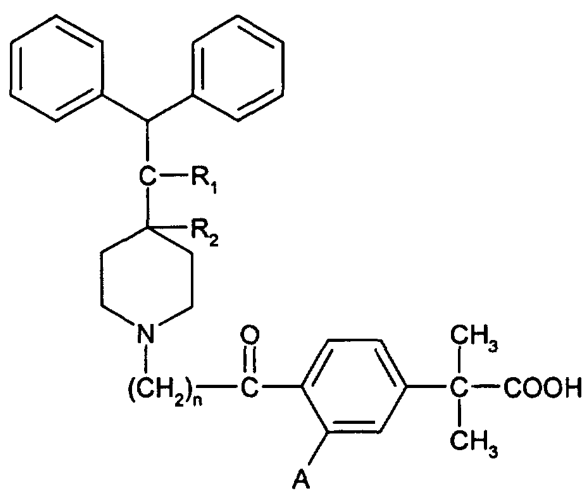

dans laquelle $R_1$, $R_2$ et n sont définis ci-dessus et A est l'hydrogène ;

(g) faire réagir la carboxyphénylcétone pipéridine avec du (-)-B-chlorodiisopinocamphénylborane pour produire un carboxyphénylalcool pipéridine de formule :

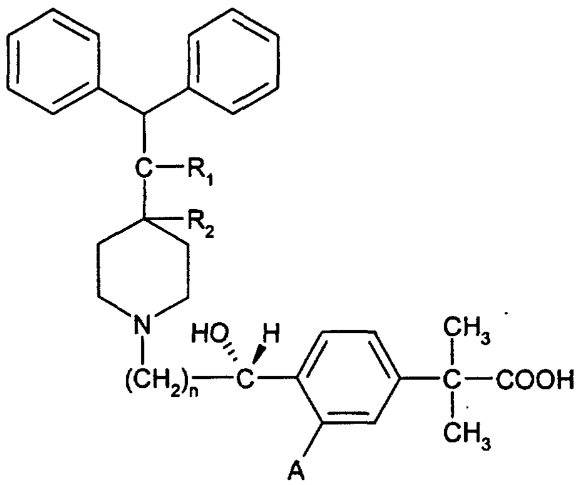

dans laquelle $R_1$, $R_2$ et n sont définis ci-dessus et A est l'hydrogène ;
(h) éventuellement faire réagir la carboxyphénylalcool pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylalcool pipéridine de formule :

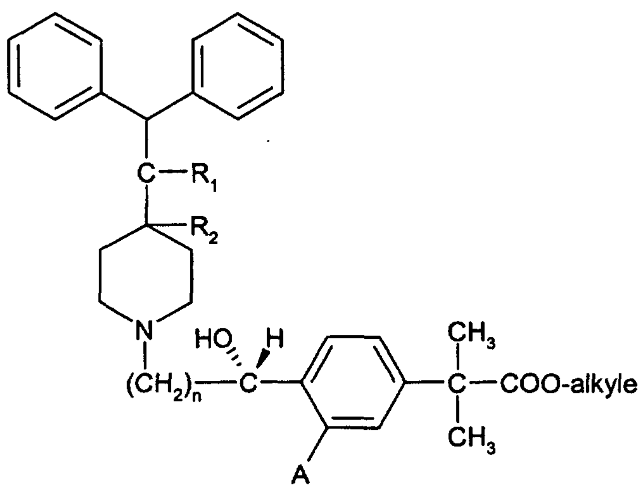

dans laquelle $R_1$, $R_2$, n et alkyle sont définis ci-dessus et A est l'hydrogène.

**20.** Procédé pour préparer un composé de formule :

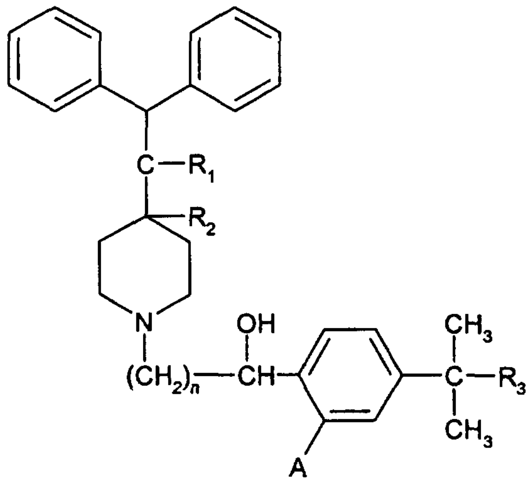

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

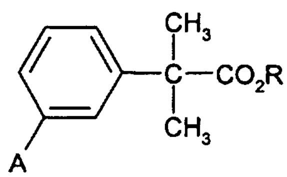

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

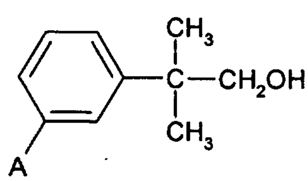

dans laquelle A est l'hydrogène ;
(b) protéger la fonctionnalité hydroxyéthyle avec un groupe protecteur convenable pour donner un alcool phénéthylique protégé de formule :

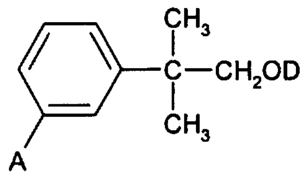

dans laquelle A est l'hydrogène et D est un groupe protecteur convenable ;

(c) faire réagir l'alcool phénéthylique protégé avec un composé ω-halogéno de formule :

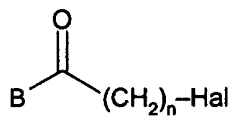

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

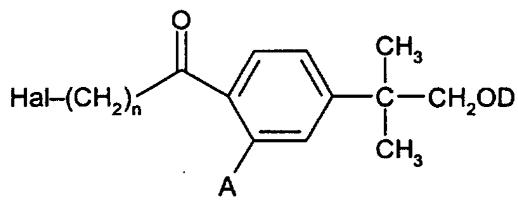

dans laquelle Hal, n et D sont définis ci-dessus et A est l'hydrogène ;

(d) faire réagir l'hydroxyéthylphénylcétone ω-halogéno protégée avec borohydrure de sodium, du borohydrure de potassium, du cyanoborohydrure de sodium, du borohydrure de tétraméthylammonium, de l'hydrure de lithium et de tri-tert-butyl-aluminium, de l'hydrure de diisobutyl-aluminium ou catalyser une hydrogénation pour produire un hydroxyéthylphénylalcool ω-halogéno protégé de formule :

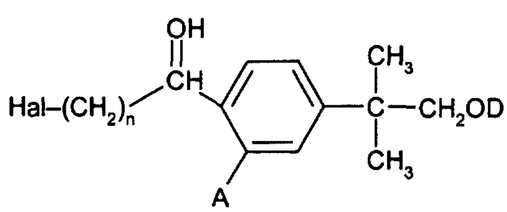

dans laquelle Hal, n et D sont définis ci-dessus et A est l'hydrogène ;

(e) faire réagir l'hydroxyéthylphénylalcool ω-halogéno protégé avec un composé de pipéridine de formule :

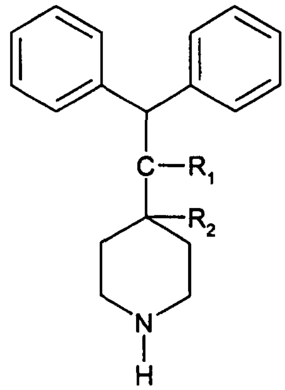

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylalcool pipéridine protégée de formule :

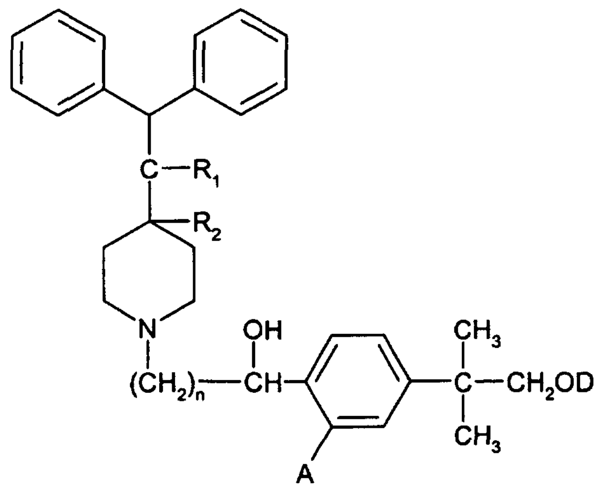

dans laquelle $R_1$, $R_2$, n et D sont définis ci-dessus et A est l'hydrogène ;
(f) déprotéger la fonctionnalité hydroxyéthyle de l'hydroxyéthylphénylalcool pipéridine protégée avec un réactif de déprotection approprié pour produire une hydroxyéthylphénylalcool pipéridine de formule :

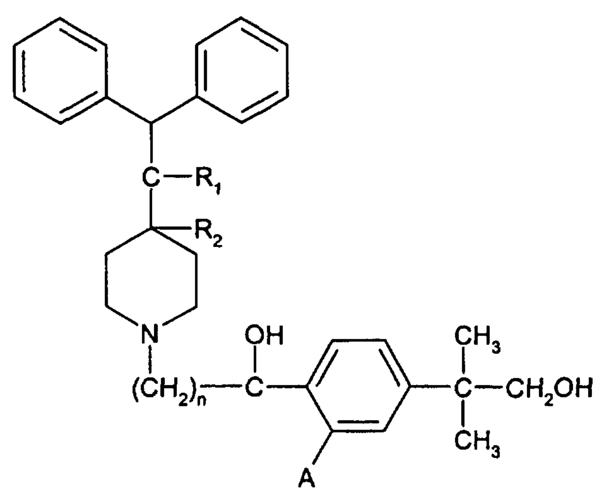

dans laquelle $R_1$, $R_2$ et n sont définis ci-dessus et A est l'hydrogène ;

(g) faire réagir le hydroxyéthylphénylalcool pipéridine avec du diméthylsulfoxyde, du chlorure d'oxalyle et de la triéthylamine ou avec un réactif de Dess-Martin, de l'oxyde de chrome(IV), du peroxyde de nickel, du dichromate de sodium, du dichromate de potassium, du chromate de t-butyle, de l'oxyde d'argent, du picolinate argentique du dioxyde de manganèse du tétraacétate de plomb, du dicyclohexylcarbodiimide, de la 2,3-dichloro-5,6-dicyanoquinone, de la tétrachloro-1,2-benzoquinone, du 2,2,6,6-tétraméthylpipéridinyl-1-oxy (TEMPO) ou du chlorochromate de quinolinium pour produire une formylphénylalcool pipéridine de formule :

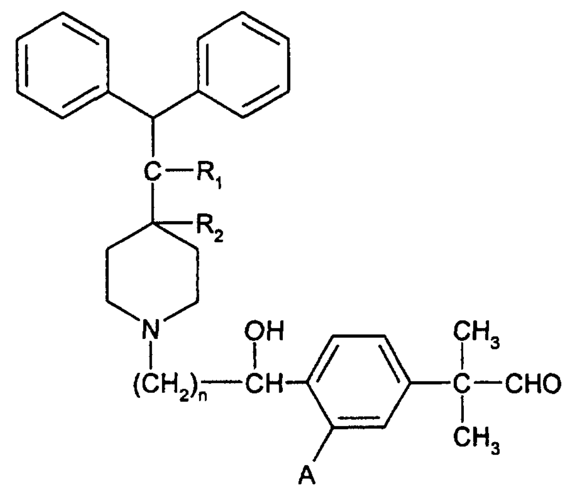

dans laquelle $R_1$, $R_2$ et n sont définis ci-dessus et A est l'hydrogène ;

(h) faire réagir la formylphénylalcool pipéridine avec du permanganate de potassium, de l'oxyde de chrome (IV), de l'oxyde d'argent(I), de l'oxyde d'argent, du picolinate argentique, un peroxyde, de l'acide nitrique, de l'acide m-chloroperbenzoïque et de l'acide peracétique pour produire une carboxyphénylalcool pipéridine de formule :

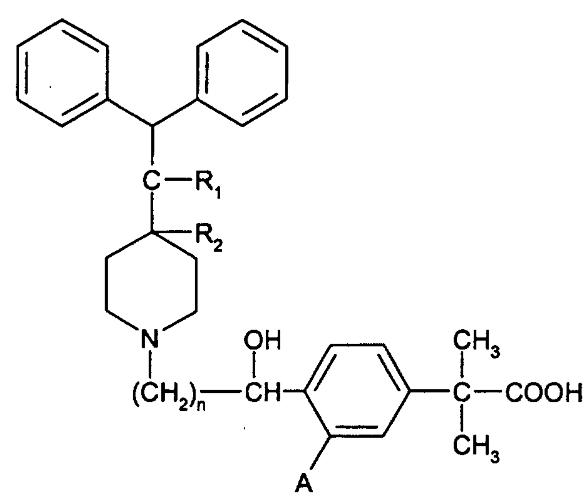

dans laquelle $R_1$, $R_2$ et n sont définis ci-dessus et A est l'hydrogène ;

(i) éventuellement faire réagir la carboxyphénylalcool pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylalcool pipéridine de formule :

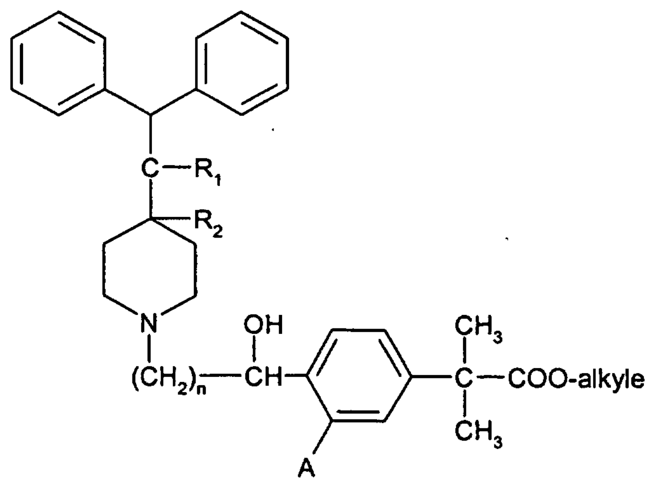

dans laquelle $R_1$, $R_2$, n et alkyle sont définis ci-dessus et A est l'hydrogène.

**21.** Procédé pour préparer un composé de formule :

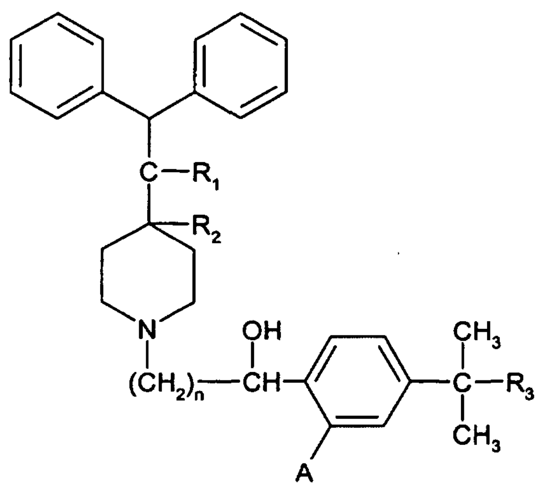

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

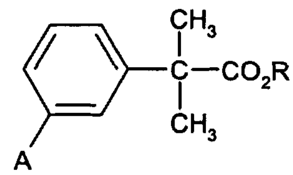

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

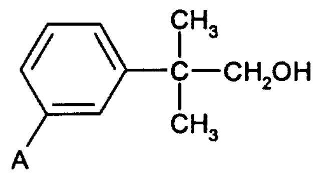

dans laquelle A est l'hydrogène ;
(b) protéger la fonctionnalité hydroxyéthyle avec un groupe protecteur convenable pour donner un alcool phénéthylique protégé de formule :

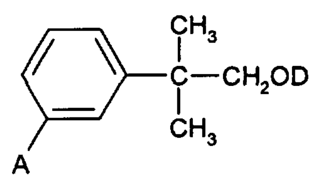

dans laquelle A est l'hydrogène et D est un groupe protecteur convenable ;
(c) faire réagir l'alcool phénéthylique protégé avec un composé ω-halogéno de formule :

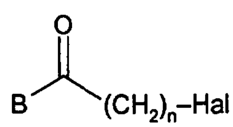

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

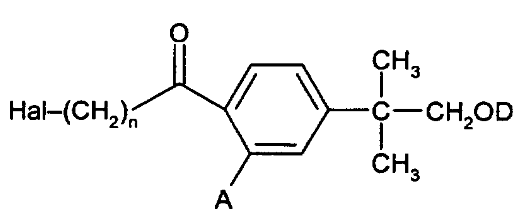

dans laquelle Hal, n et D sont définis ci-dessus et A est l'hydrogène ;

(d) faire réagir l'hydroxyéthylphénylcétone ω-halogéno protégée avec borohydrure de sodium, du borohydrure de potassium, du cyanoborohydrure de sodium, du borohydrure de tétraméthylammonium, de l'hydrure de lithium et de tri-tert-butyl-aluminium, de l'hydrure de diisobutyl-aluminium ou catalyser une hydrogénation pour produire un hydroxyéthylphénylalcool ω-halogéno protégé de formule :

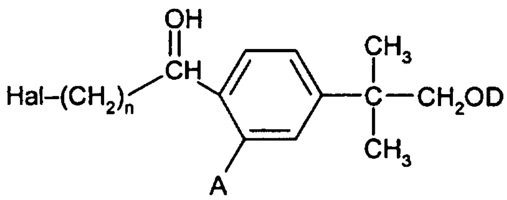

dans laquelle Hal, n et D sont définis ci-dessus et A est l'hydrogène ;

(e) faire réagir l'hydroxyéthylphénylalcool ω-halogéno protégé avec un composé de pipéridine de formule :

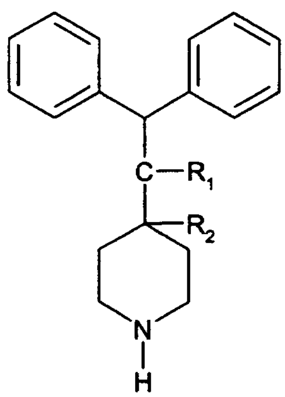

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylalcool pipéridine protégée de formule :

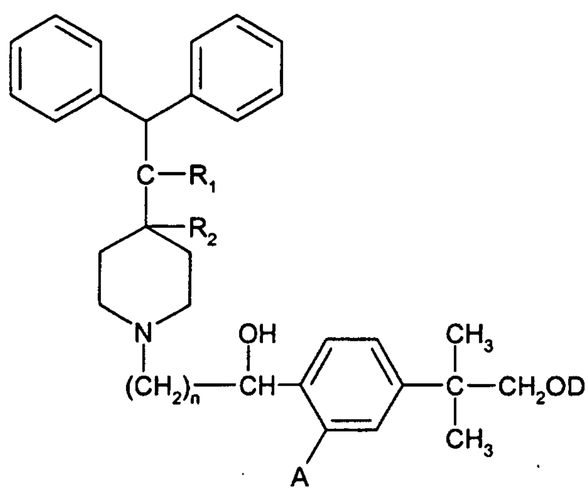

dans laquelle $R_1$, $R_2$, n et D sont définis ci-dessus et A est l'hydrogène ;

(f) déprotéger la fonctionnalité hydroxyéthyle de l'hydroxyéthylphénylalcool pipéridine protégée avec un réactif de déprotection approprié pour produire une hydroxyéthylphénylalcool pipéridine de formule :

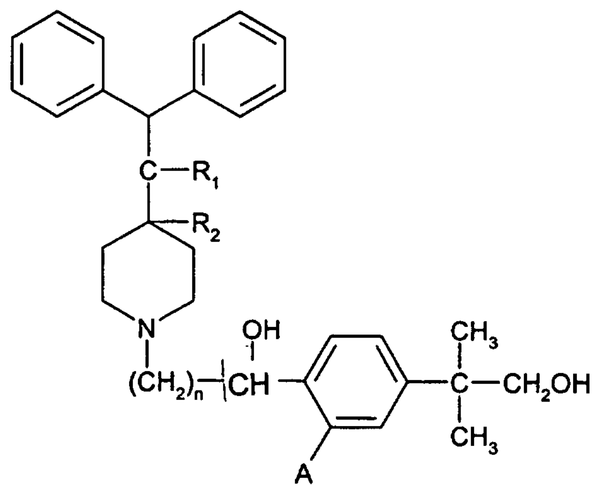

dans laquelle $R_1$, $R_2$ et n sont définis ci-dessus et A est l'hydrogène ;
(g) faire réagir le composé hydroxyéthylphénylalcool pipéridine avec de l'oxyde de chrome(IV), du permanganate de potassium, de l'acide nitrique, du dioxyde d'azote, de l'oxyde de ruthénium(VIII), du peroxyde de nickel, de l'oxyde d'argent, du chromate de t-butyle ou de l'acide xénique pour produire une carboxyphénylalcool pipéridine de formule :

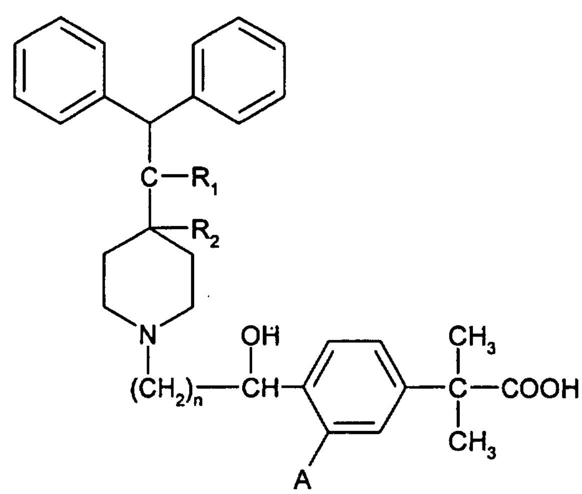

dans laquelle $R_1$, $R_2$ et n sont définis ci-dessus et A est l'hydrogène ;
(h) éventuellement faire réagir la carboxyphénylalcool pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylalcool pipéridine de formule :

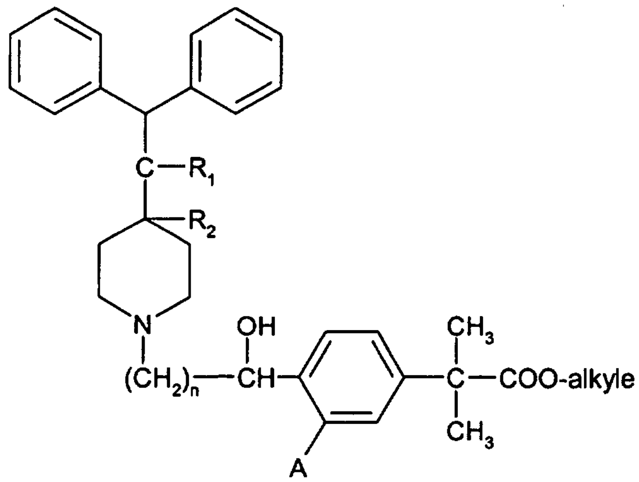

dans laquelle $R_1$, $R_2$, n et alkyle sont définis ci-dessus et A est l'hydrogène.

**22.** Procédé selon la revendication 21, dans lequel l'hydroxyphénylalcool pipéridine produite dans l'étape f est mise à réagir avec un oxyde de ruthénium(VIII) pour produire une carboxyphénylalcool pipéridine de l'étape g.

**23.** Procédé pour préparer un composé de formule :

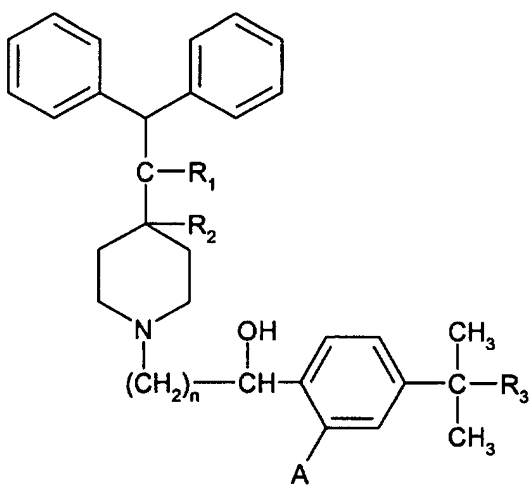

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est --$CH_2OH$ ;
A est l'hydrogène ; et
ses sels acceptables en pharmacie, comprenant les étapes consistant à :

(a) faire réagir un composé d'acide benzène-acétique de formule :

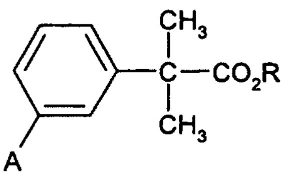

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

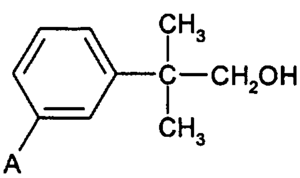

dans laquelle A est l'hydrogène ;

(b) protéger la fonctionnalité hydroxyéthyle avec un groupe protecteur convenable pour donner un alcool phénéthylique protégé de formule :

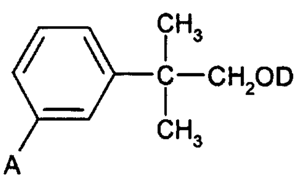

dans laquelle A est l'hydrogène et D est un groupe protecteur convenable ;

(c) faire réagir l'alcool phénéthylique protégé avec un composé ω-halogéno de formule :

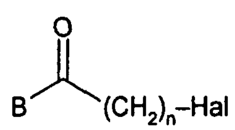

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

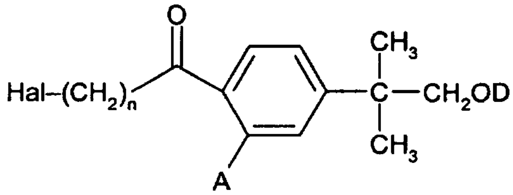

dans laquelle Hal, n et D sont définis ci-dessus et A est l'hydrogène ;
(d) faire réagir l'hydroxyéthylphénylcétone ω-halogéno protégée avec du borohydrure de sodium, du borohydrure de potassium, du cyanoborohydrure de sodium, du borohydrure de tétraméthylammonium, de l'hydrure de lithium et de tri-tert-butyl-aluminium, de l'hydrure de diisobutyl-aluminium ou catalyser une hydrogénation pour produire un hydroxyéthylphénylalcool ω-halogéno protégé de formule :

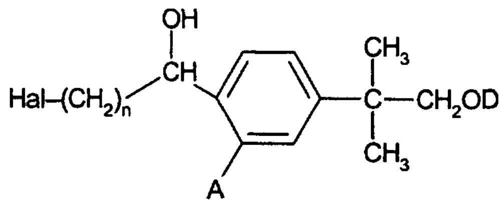

dans laquelle Hal, n et D sont définis ci-dessus et A est l'hydrogène ;
(e) faire réagir l'hydroxyéthylphénylalcool ω-halogéno protégé avec un composé de pipéridine de formule :

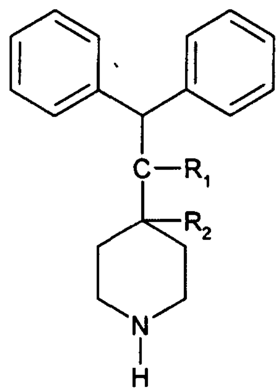

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylalcool pipéridine protégée de formule :

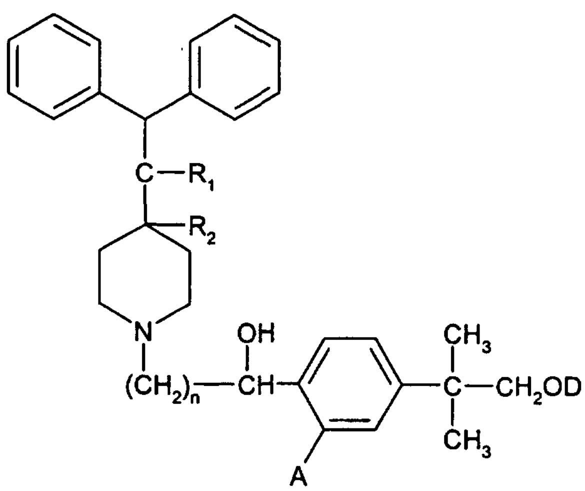

dans laquelle $R_1$, $R_2$, n et D sont définis ci-dessus et A est l'hydrogène ;
(f) déprotéger la fonctionnalité hydroxyéthyle de l'hydroxyéthylphénylalcool pipéridine protégée avec un réactif de déprotection approprié pour produire une hydroxyéthylphénylalcool pipéridine de formule :

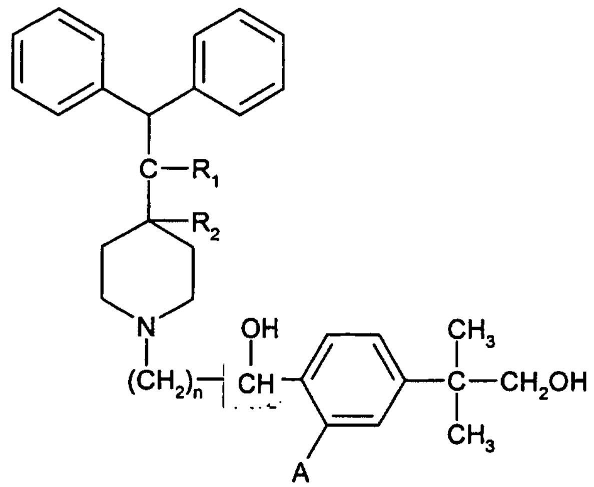

dans laquelle $R_1$, $R_2$ et n sont définis ci-dessus et A est l'hydrogène.

**24.** Procédé pour préparer un composé de formule :

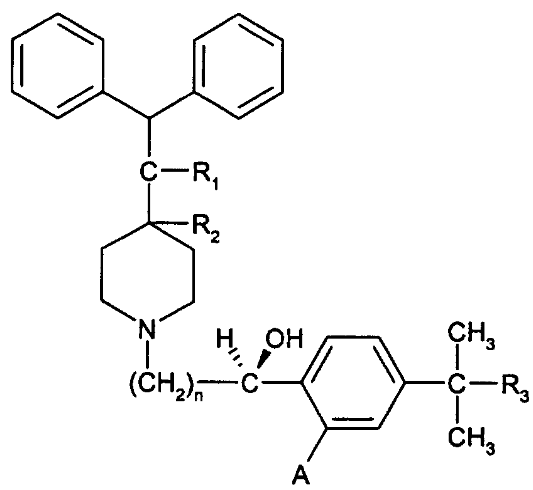

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

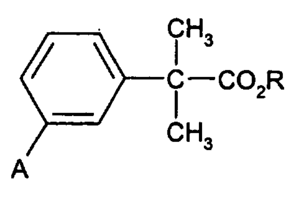

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

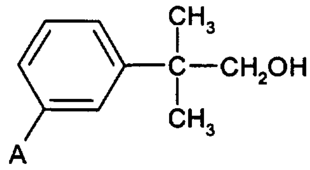

dans laquelle A est l'hydrogène ;

(b) protéger la fonctionnalité hydroxyéthyle avec un groupe protecteur convenable pour donner un alcool phénéthylique protégé de formule :

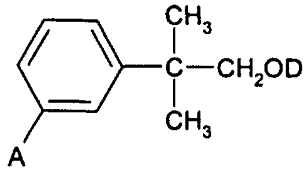

dans laquelle A est l'hydrogène et D est un groupe protecteur convenable ;

(c) faire réagir l'alcool phénéthylique protégé avec un composé ω-halogéno de formule :

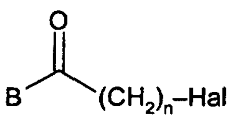

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

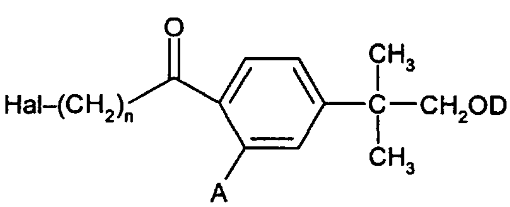

dans laquelle Hal, n et D sont définis ci-dessus et A est l'hydrogène ;

(d) faire réagir l'hydroxyéthylphénylcétone ω-halogéno protégée avec du (+)-B-chlorodiisopinocamphénylborane pour produire un hydroxyéthylphénylalcool ω-halogéno protégé de formule :

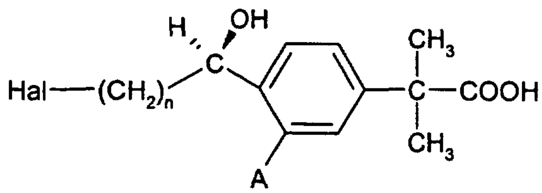

dans laquelle Hal, n et D sont définis ci-dessus et A est l'hydrogène ;
(e) faire réagir l'hydroxyéthylphénylalcool ω-halogéno protégé avec un composé de pipéridine de formule :

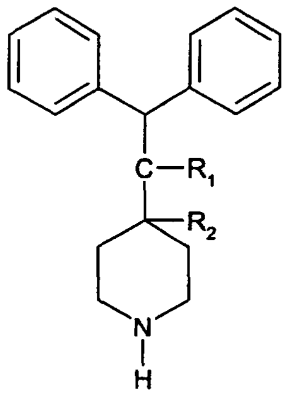

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylalcool pipéridine protégée de formule :

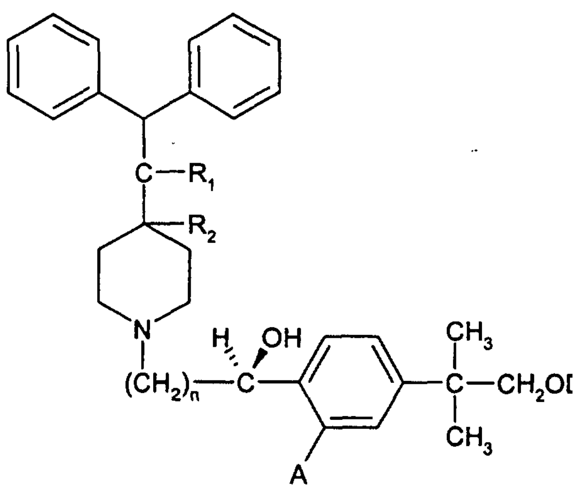

dans laquelle $R_1$, $R_2$, n et D sont définis ci-dessus et A est l'hydrogène ;
(f) déprotéger la fonctionnalité hydroxyéthyle de l'hydroxyéthylphénylalcool pipéridine protégée avec un réactif de déprotection approprié pour produire une hydroxyéthylphénylalcool pipéridine de formule :

einf

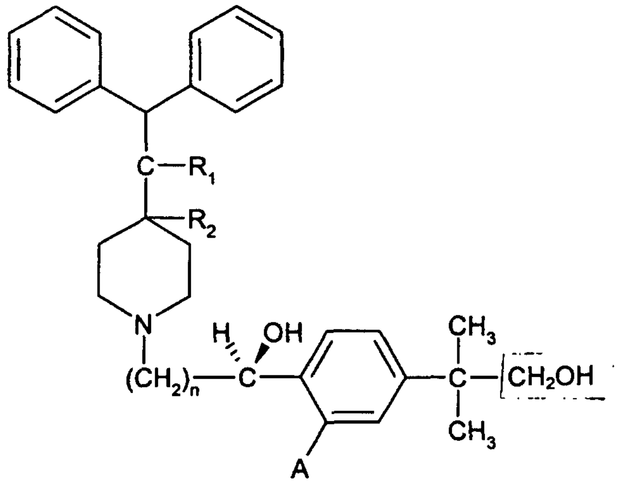

dans laquelle $R_1$, $R_2$ et n sont définis ci-dessus et A est l'hydrogène ;

(g) faire réagir le hydroxyéthylphénylalcool pipéridine avec du diméthylsulfoxyde, du chlorure d'oxalyl et de la triéthylamine ou avec un réactif de Dess-Martin, de l'oxyde de chrome(IV), du peroxyde de nickel, du dichromate de sodium, du dichromate de potassium, du chromate de t-butyle, de l'oxyde d'argent, du picolinate argentique du dioxyde de manganèse du tétraacétate de plomb, du dicyclohexylcarbodiimide, de la 2,3-dichloro-5,6-dicyanoquinone, de la tétrachloro-1,2-benzoquinone, du 2,2,6,6-tétraméthylpipéridinyl-1-oxy (TEMPO) ou du chlorochromate de quinolinium pour produire une formylphénylalcool pipéridine de formule :

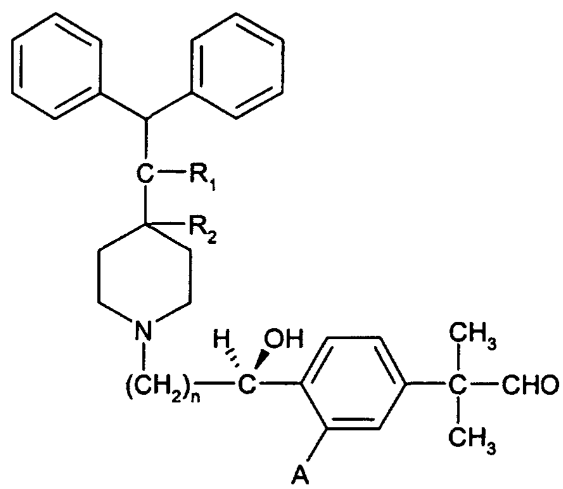

dans laquelle $R_1$, $R_2$ et n sont définis ci-dessus et A est l'hydrogène ;

(h) faire réagir la formylphénylalcool pipéridine avec du permanganate de potassium, de l'oxyde de chrome (IV), de l'oxyde d'argent(I), de l'oxyde d'argent, du picolinate argentique, un peroxyde, de l'acide nitrique, de l'acide m-chloroperbenzoïque et de l'acide peracétique pour produire une carboxyphénylalcool pipéridine de formule :

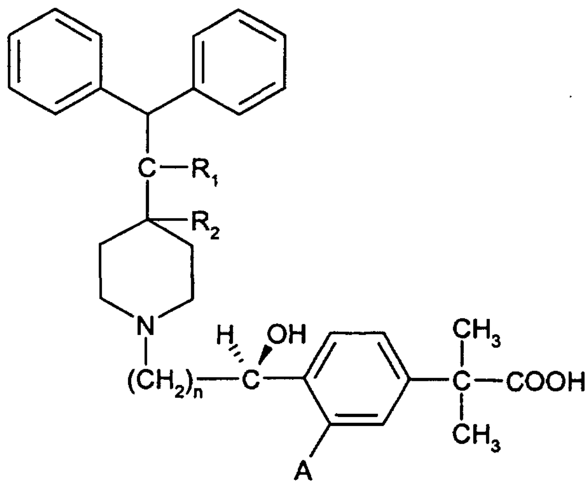

dans laquelle $R_1$, $R_2$ et n sont définis ci-dessus et A est l'hydrogène ;

(i) éventuellement faire réagir la carboxyphénylalcool pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylalcool pipéridine de formule :

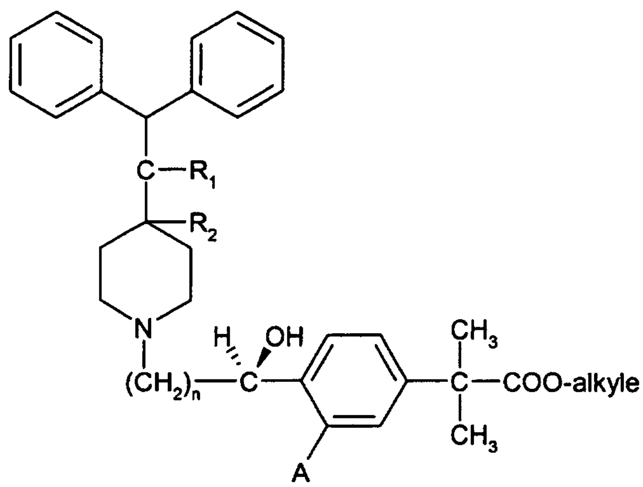

dans laquelle $R_1$, $R_2$, n et alkyle sont définis ci-dessus et A est l'hydrogène.

**25.** Procédé pour préparer un composé de formule :

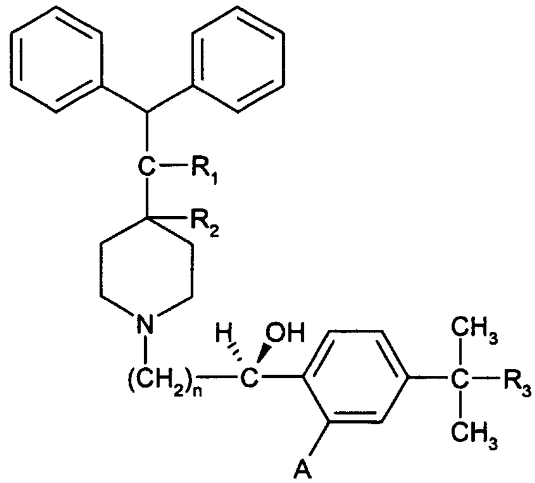

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

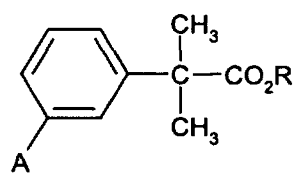

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

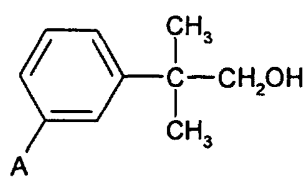

dans laquelle A est l'hydrogène ;
(b) protéger la fonctionnalité hydroxyéthyle avec un groupe protecteur convenable pour donner un alcool phénéthylique protégé de formule :

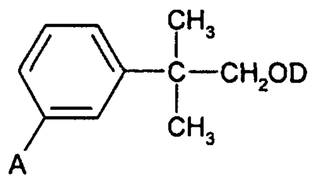

dans laquelle A est l'hydrogène et D est un groupe protecteur convenable ;

(c) faire réagir l'alcool phénéthylique protégé avec un composé ω-halogéno de formule :

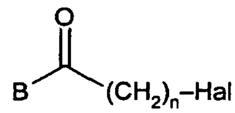

dans laquelle B est un radical halogéno ou hydroxy, Hal représente Cl, Br ou I et n est tel que défini ci-dessus, en présence de trichlorure, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

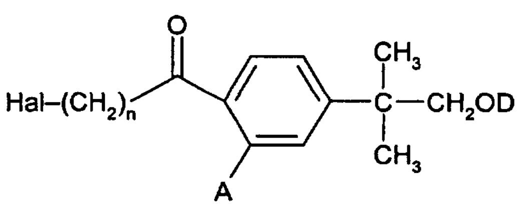

dans laquelle Hal, n et D sont définis ci-dessus et A est l'hydrogène ;

(d) faire réagir l'hydroxyéthylphénylcétone ω-halogéno protégée avec du (+)-B-chlorodiisopinocamphénylborane pour produire un hydroxyéthylphénylalcool ω-halogéno protégé de formule :

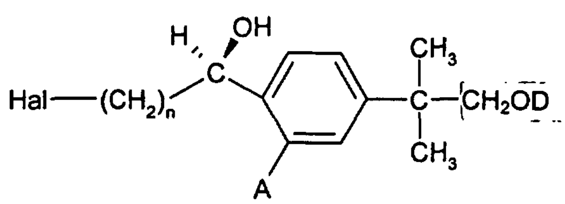

dans laquelle Hal, n et D sont définis ci-dessus et A est l'hydrogène ;

(e) faire réagir l'hydroxyéthylphénylalcool ω-halogéno protégé avec un composé de pipéridine de formule :

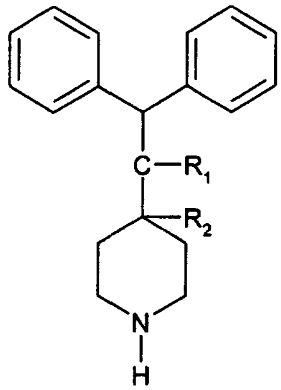

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylalcool pipéridine protégée de formule :

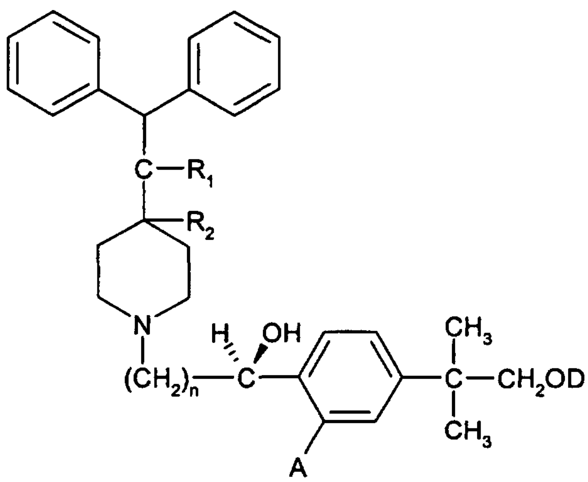

dans laquelle $R_1$, $R_2$, n et D sont définis ci-dessus et A est l'hydrogène ;
(f) déprotéger la fonctionnalité hydroxyéthyle de l'hydroxyéthylphénylalcool pipéridine protégée avec un réactif de déprotection approprié pour produire une hydroxyéthylphénylalcool pipéridine de formule :

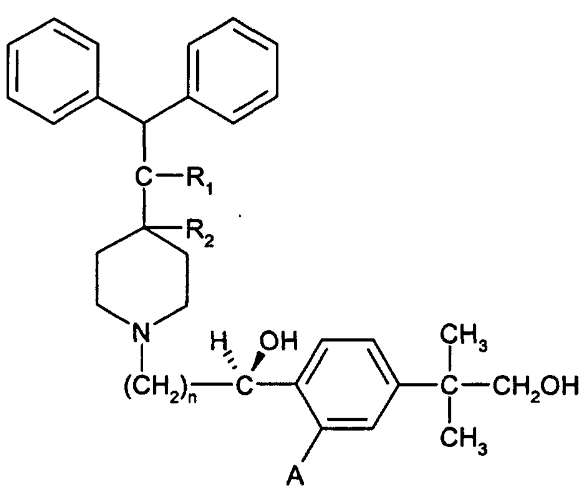

dans laquelle $R_1$, $R_2$ et n sont définis ci-dessus et A est l'hydrogène ;

(g) faire réagir l'hydroxyéthylphénylalcool pipéridine avec de l'oxyde de chrome(IV), du permanganate de potassium, de l'acide nitrique, du dioxyde d'azote, de l'oxyde de ruthénium(VIII), du peroxyde de nickel, de l'oxyde d'argent, du chromate de t-butyle ou de l'acide xénique, pour produire une carboxyphénylalcool pipéridine de formule :

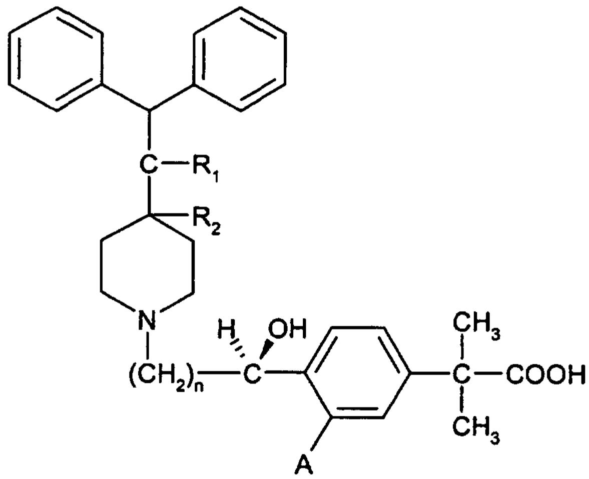

dans laquelle $R_1$, $R_2$ et n sont définis ci-dessus et A est l'hydrogène ;

(h) éventuellement faire réagir la carboxyphénylalcool pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylalcool pipéridine de formule :

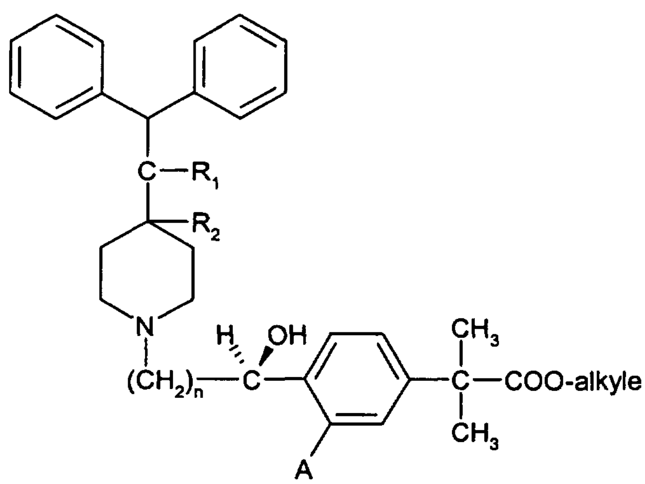

dans laquelle $R_1$, $R_2$, n et alkyle sont définis ci-dessus et A est l'hydrogène.

**26.** Procédé pour préparer un composé de formule :

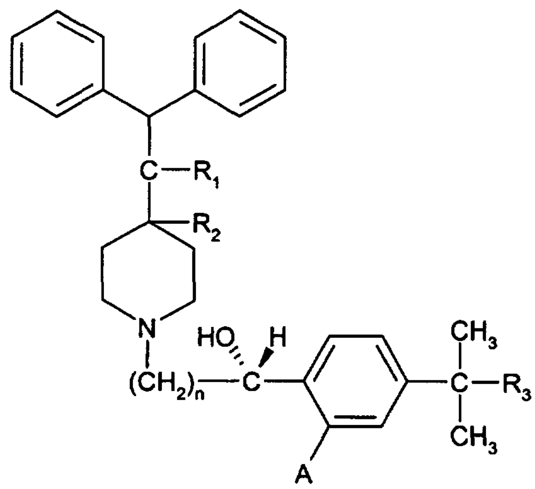

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

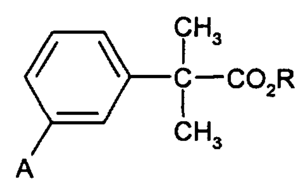

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

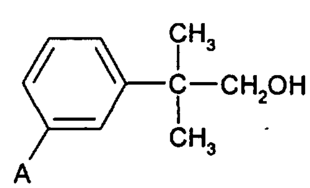

dans laquelle A est l'hydrogène ;
(b) protéger la fonctionnalité hydroxyéthyle avec un groupe protecteur convenable pour donner un alcool phénéthylique protégé de formule :

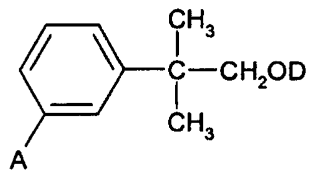

dans laquelle A est l'hydrogène et D est un groupe protecteur convenable ;
(c) faire réagir l'alcool phénéthylique protégé avec un composé ω-halogéno de formule :

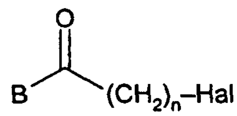

dans laquelle B est un radical halogéno ou hydroxy, Hal représente Cl, Br ou I et n est tel que défini ci-dessus, en présence de trichlorure, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

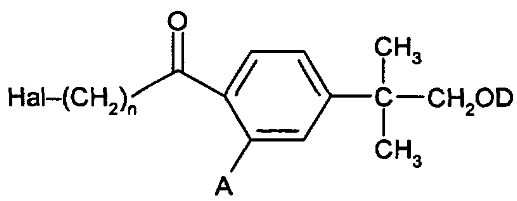

dans laquelle Hal, n et D sont définis ci-dessus et A est l'hydrogène ;
(d) faire réagir l'hydroxyéthylphénylcétone ω-halogéno protégée avec du (-)-B-chlorodiisopinocamphénylborane pour produire un hydroxyéthylphénylalcool ω-halogéno protégé de formule :

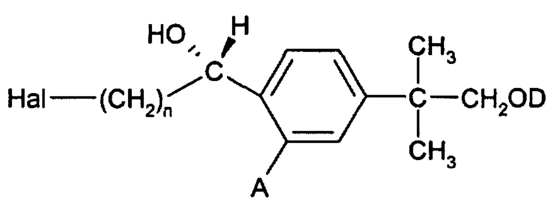

dans laquelle Hal, n et D sont définis ci-dessus et A est l'hydrogène ;
(e) faire réagir l'hydroxyéthylphénylalcool ω-halogéno protégé avec un composé de pipéridine de formule :

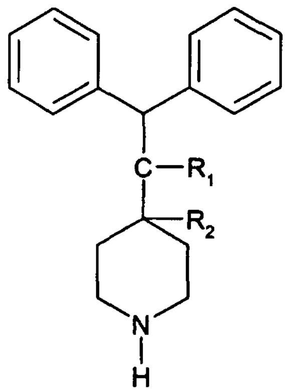

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylalcool pipéridine protégée de formule :

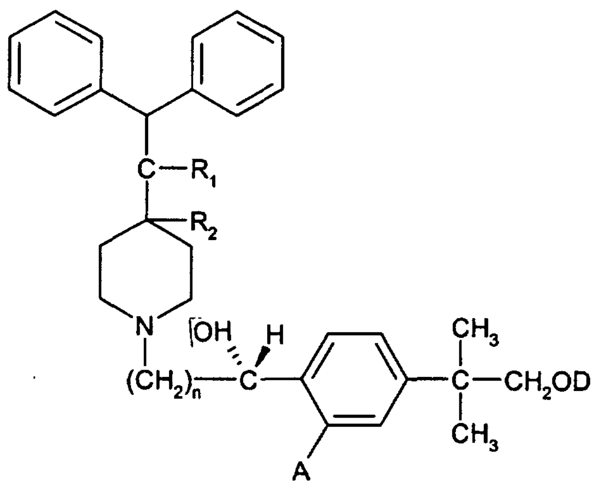

dans laquelle $R_1$, $R_2$, n et D sont définis ci-dessus et A est l'hydrogène ;

(f) déprotéger la fonctionnalité hydroxyéthyle de l'hydroxyéthylphénylalcool pipéridine protégée avec un réactif de déprotection approprié pour produire une hydroxyéthylphénylalcool pipéridine de formule :

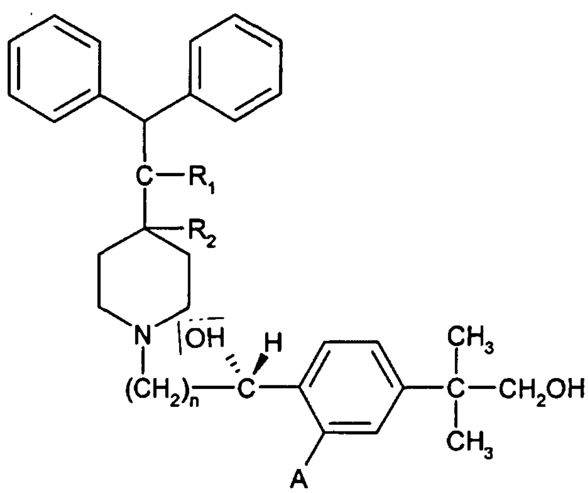

dans laquelle $R_1$, $R_2$ et n sont définis ci-dessus et A est l'hydrogène ;
(g) faire réagir l'hydroxyéthylphénylalcool pipéridine avec du diméthylsulfoxyde, du chlorure d'oxalyle et de la triéthylamine ou avec un réactif de Dess-Martin, de l'oxyde de chrome(IV), du peroxyde de nickel, du dichromate de sodium, du dichromate de potassium, du chromate de t-butyle, de l'oxyde d'argent, du picolinate argentique du dioxyde de manganèse du tétraacétate de plomb, du dicyclohexylcarbodiimide, de la 2,3-dichloro-5,6-dicyanoquinone, de la tétrachloro-1,2-benzoquinone, du 2,2,6,6-tétraméthylpipéridinyl-1-oxy (TEMPO) ou du chlorochromate de quinolinium pour produire une formylphénylalcool pipéridine de formule :

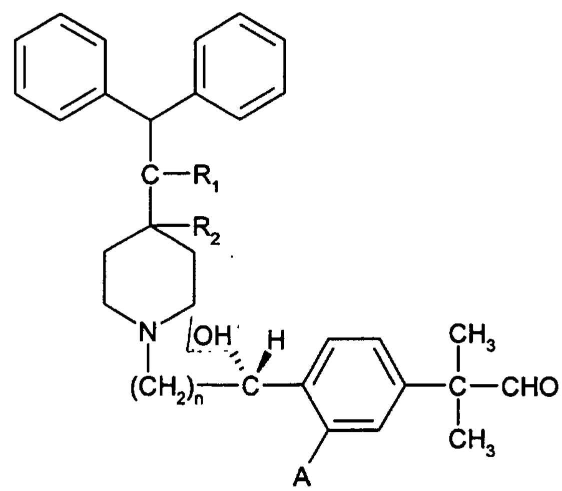

dans laquelle $R_1$, $R_2$ et n sont définis ci-dessus et A est l'hydrogène ;
(h) faire réagir la formylphénylalcool pipéridine avec du permanganate de potassium, de l'oxyde de chrome (IV), de l'oxyde d'argent(I), de l'oxyde d'argent, du picolinate argentique, un peroxyde, de l'acide nitrique, de l'acide m-chloroperbenzoïque et de l'acide peracétique pour produire une carboxyphénylalcool pipéridine de formule :

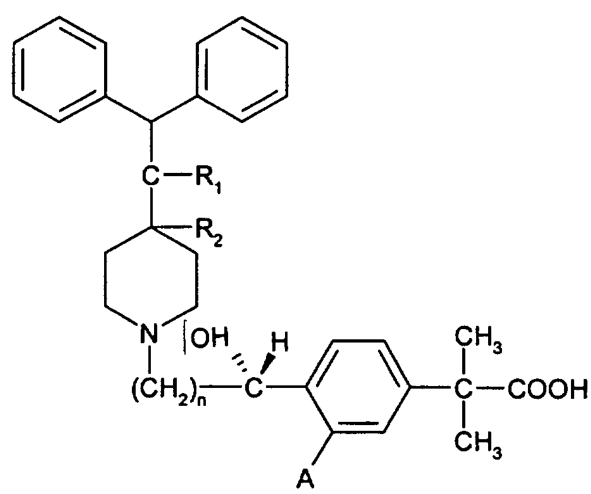

dans laquelle $R_1$, $R_2$ et n sont définis ci-dessus et A est l'hydrogène ;
(i) éventuellement faire réagir la carboxyphénylalcool pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylalcool pipéridine de formule :

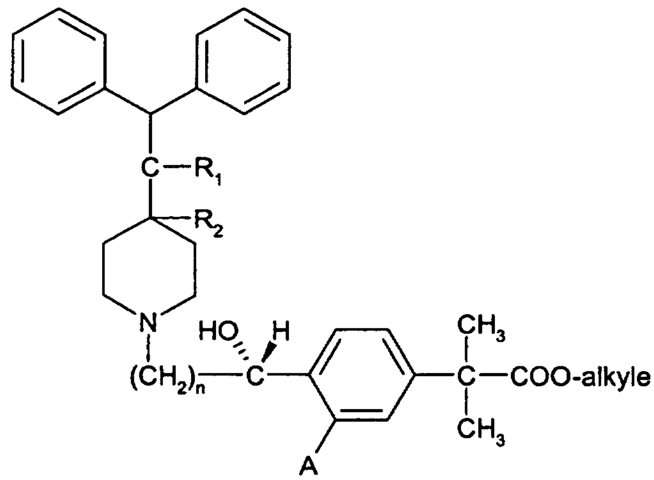

dans laquelle $R_1$, $R_2$, n et alkyle sont définis ci-dessus et A est l'hydrogène.

**27.** Procédé pour préparer un composé de formule :

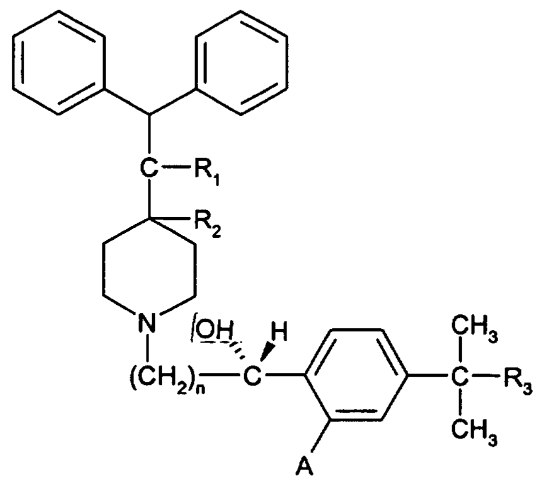

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

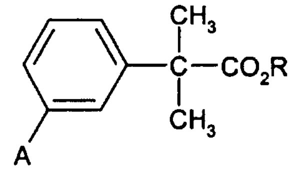

dans laquelle A est l'hydrogène et R est l'hydrogène ou un radical alkyle en $C_1$ à $C_6$, avec de l'hydrure

de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

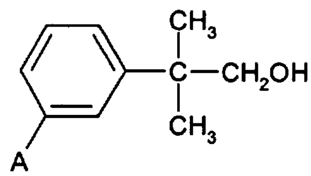

dans laquelle A est l'hydrogène ;

(b) protéger la fonctionnalité hydroxyéthyle avec un groupe protecteur convenable pour donner un alcool phénéthylique protégé de formule :

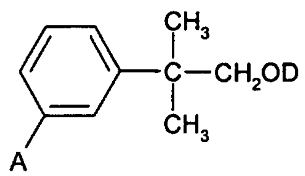

dans laquelle A est l'hydrogène et D est un groupe protecteur convenable ;

(c) faire réagir l'alcool phénéthylique protégé avec un composé ω-halogéno de formule :

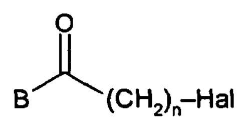

dans laquelle B est un radical halogéno ou hydroxy, Hal représente Cl, Br ou I et n est tel que défini ci-dessus, en présence de trichlorure, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

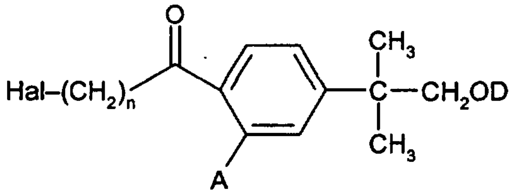

dans laquelle Hal, n et D sont définis ci-dessus et A est l'hydrogène ;

(d) faire réagir l'hydroxyéthylphénylcétone ω-halogéno protégée avec du (-)-B-chlorodiisopinocamphénylborane pour produire un hydroxyéthylphénylalcool ω-halogéno protégé de formule :

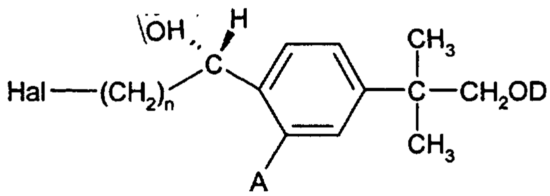

dans laquelle Hal, n et D sont définis ci-dessus et A est l'hydrogène ;

(e) faire réagir l'hydroxyéthylphénylalcool ω-halogéno protégé avec un composé de pipéridine de formule :

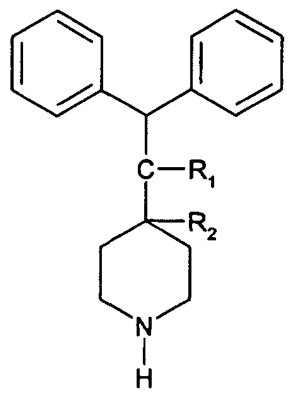

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylalcool pipéridine protégée de formule :

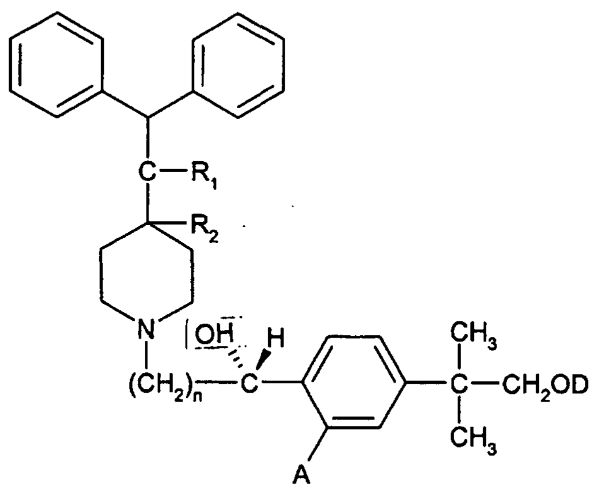

dans laquelle $R_1$, $R_2$, n et D sont définis ci-dessus et A est l'hydrogène ;

(f) déprotéger la fonctionnalité hydroxyéthyle de l'hydroxyéthylphénylalcool pipéridine protégée avec un réactif de déprotection approprié pour produire une hydroxyéthylphénylalcool pipéridine de formule :

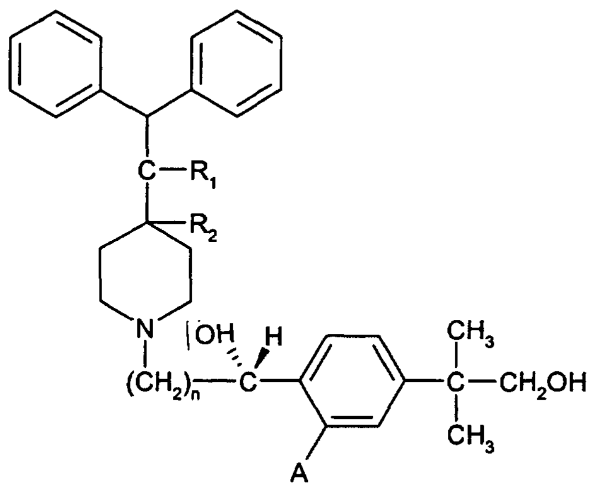

dans laquelle $R_1$, $R_2$ et n sont définis ci-dessus et A est l'hydrogène ;

(g) faire réagir l'hydroxyéthylphénylalcool pipéridine avec de l'oxyde de chrome(IV), du permanganate de potassium, de l'acide nitrique, du dioxyde d'azote, de l'oxyde de ruthénium(VIII), du peroxyde de nickel, de l'oxyde d'argent, du chromate de t-butyle ou de l'acide xénique pour produire une carboxyphénylalcool pipéridine de formule :

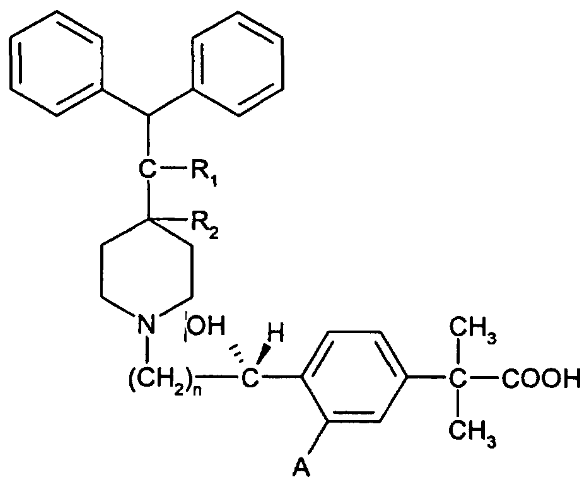

dans laquelle $R_1$, $R_2$ et n sont définis ci-dessus et A est l'hydrogène ;

(h) éventuellement faire réagir la carboxyphénylalcool pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylalcool pipéridine de formule :

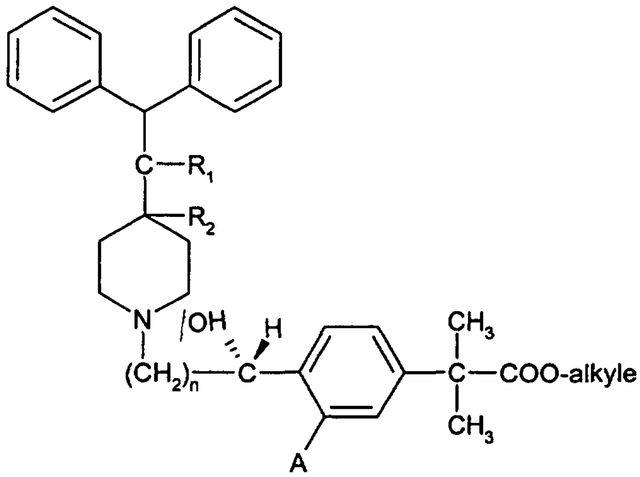

dans laquelle $R_1$, $R_2$, n et alkyle sont définis ci-dessus et A est l'hydrogène.

**28.** Procédé pour préparer un composé de formule :

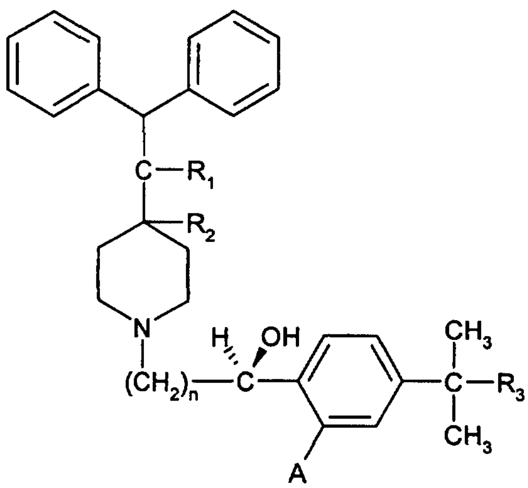

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est $-CH_2OH$ ;
A est l'hydrogène ; et
ses sels acceptables en pharmacie, comprenant les étapes consistant à :

(a) faire réagir un composé d'acide benzène-acétique de formule :

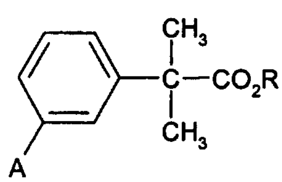

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

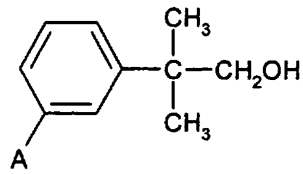

dans laquelle A est l'hydrogène ;

(b) protéger la fonctionnalité hydroxyéthyle avec un groupe protecteur convenable pour donner un alcool phénéthylique protégé de formule :

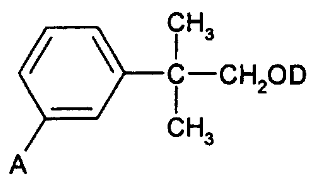

dans laquelle A est l'hydrogène et D est un groupe protecteur convenable ;

(c) faire réagir l'alcool phénéthylique protégé avec un composé ω-halogéno de formule :

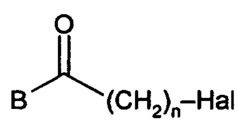

dans laquelle B est un radical halogéno ou hydroxy, Hal représente Cl, Br ou I et n est tel que défini ci-dessus, en présence de trichlorure, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

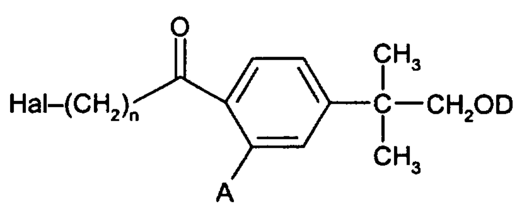

dans laquelle Hal, n et D sont définis ci-dessus et A est l'hydrogène ;

(d) faire réagir l'hydroxyéthylphénylcétone ω-halogéno protégée avec du (+)-B-chlorodiisopinocamphénylborane pour produire un hydroxyéthylphénylalcool ω-halogéno protégé de formule :

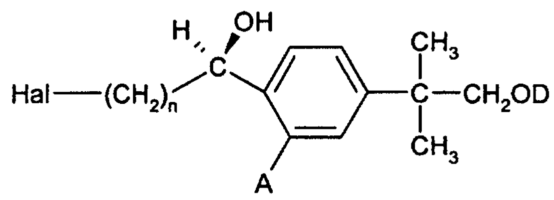

dans laquelle Hal, n et D sont définis ci-dessus et A est l'hydrogène ;
(e) faire réagir l'hydroxyéthylphénylalcool ω-halogéno protégé avec un composé de pipéridine de formule :

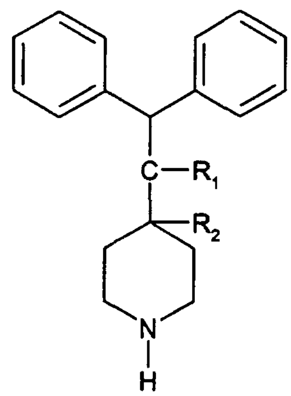

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylalcool pipéridine protégée de formule :

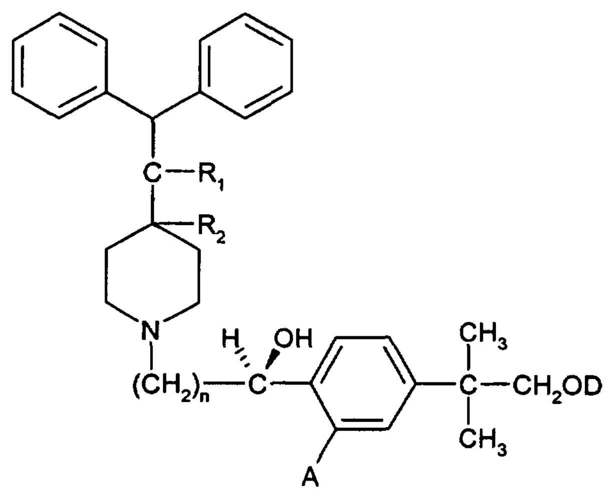

dans laquelle $R_1$, $R_2$, n et D sont définis ci-dessus et A est l'hydrogène ;
(f) déprotéger la fonctionnalité hydroxyéthyle de l'hydroxyéthylphénylalcool pipéridine protégée avec un réactif de déprotection approprié pour produire une hydroxyéthylphénylalcool pipéridine de formule :

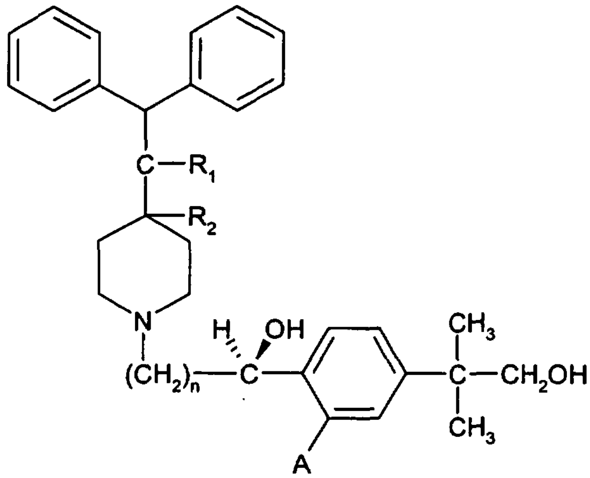

dans laquelle $R_1$, $R_2$ et n sont définis ci-dessus et A est l'hydrogène.

**29.** Procédé pour préparer un composé de formule :

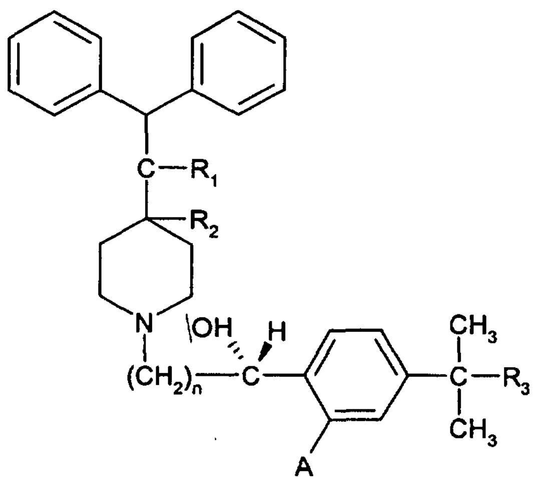

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est $-CH_2OH$ ;
A est l'hydrogène ; et
ses sels acceptables en pharmacie, comprenant les étapes consistant à :

(a) faire réagir un composé d'acide benzène-acétique de formule :

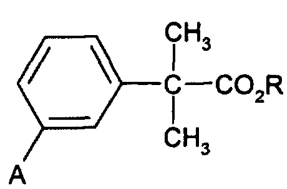

dans laquelle A est l'hydrogène et R est l'hydrogène ou un radical alkyle en $C_1$ à $C_6$, avec de l'hydrure

de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

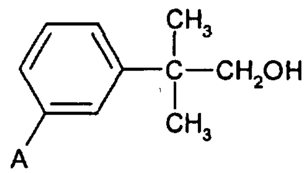

dans laquelle A est l'hydrogène ;

(b) protéger la fonctionnalité hydroxyéthyle avec un groupe protecteur convenable pour donner un alcool phénéthylique protégé de formule :

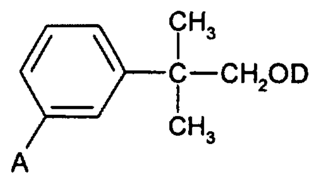

dans laquelle A est l'hydrogène et D est un groupe protecteur convenable ;

(c) faire réagir l'alcool phénéthylique protégé avec un composé ω-halogéno de formule :

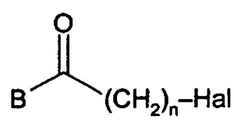

dans laquelle B est un radical halogéno ou hydroxy, Hal représente Cl, Br ou I et n est tel que défini ci-dessus, en présence de trichlorure, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

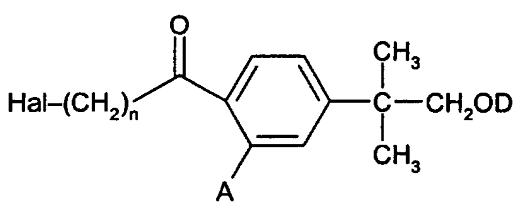

dans laquelle Hal, n et D sont définis ci-dessus et A est l'hydrogène ;

(d) faire réagir l'hydroxyéthylphénylcétone ω-halogéno protégée avec du (-)-B-chlorodiisopinocamphénylborane pour produire un hydroxyéthylphénylalcool ω-halogéno protégé de formule :

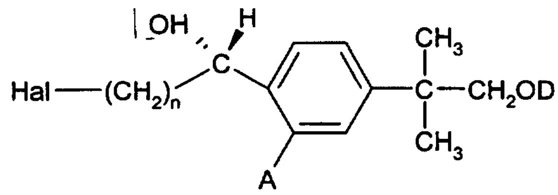

dans laquelle Hal, n et D sont définis ci-dessus et A est l'hydrogène ;

(e) faire réagir l'hydroxyéthylphénylalcool ω-halogéno protégé avec un composé de pipéridine de formule :

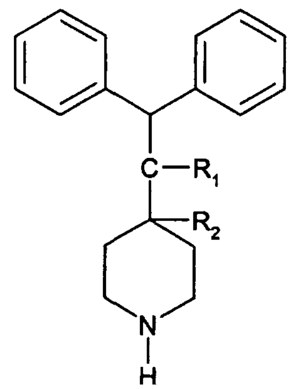

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylalcool pipéridine protégée de formule :

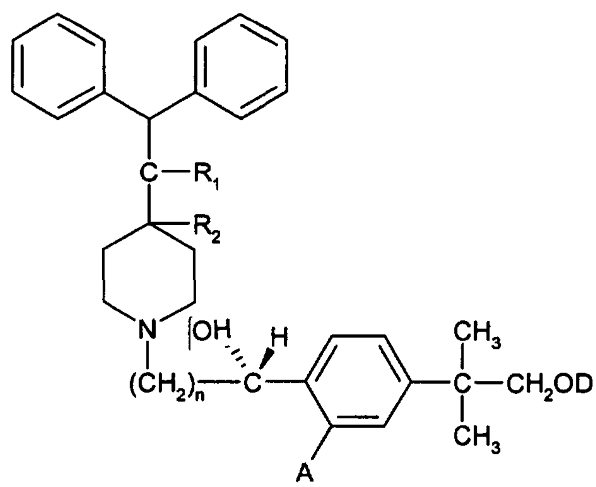

dans laquelle $R_1$, $R_2$, n et D sont définis ci-dessus et A est l'hydrogène ;

(f) déprotéger la fonctionnalité hydroxyéthyle de l'hydroxyéthylphénylalcool pipéridine protégée avec un réactif de déprotection approprié pour produire une hydroxyéthylphénylalcool pipéridine de formule :

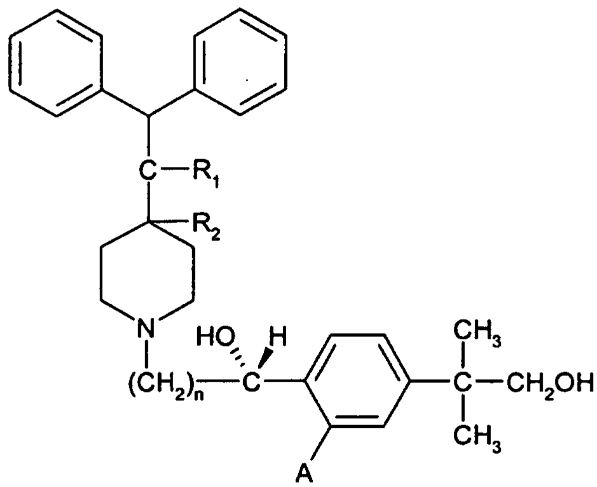

dans laquelle $R_1$, $R_2$ et n sont définis ci-dessus et A est l'hydrogène.

**30.** Procédé pour préparer un composé de formule :

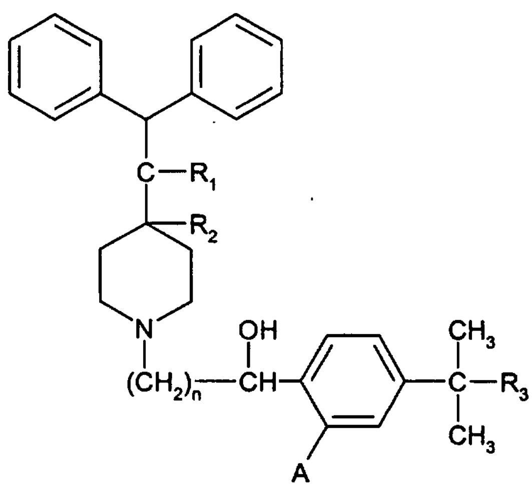

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

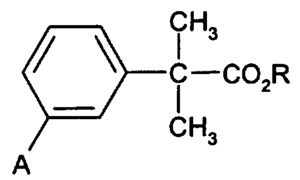

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

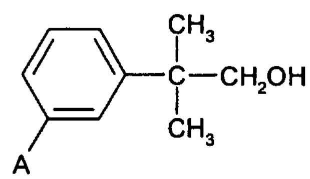

dans laquelle A est l'hydrogène ;

(b) protéger la fonctionnalité hydroxyéthyle avec un groupe protecteur convenable pour donner un alcool phénéthylique protégé de formule :

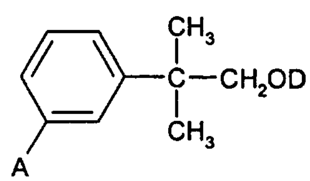

dans laquelle A est l'hydrogène et D est un groupe protecteur convenable ;

(c) faire réagir l'alcool phénéthylique protégé avec un composé ω-halogéno de formule :

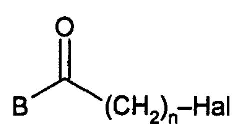

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

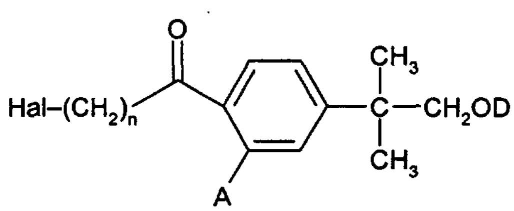

dans laquelle Hal, n et D sont définis ci-dessus et A est l'hydrogène ;

(d) faire réagir l'hydroxyéthylphénylcétone ω-halogéno protégée avec un composé de pipéridine de formule :

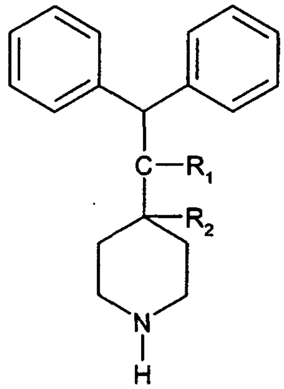

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

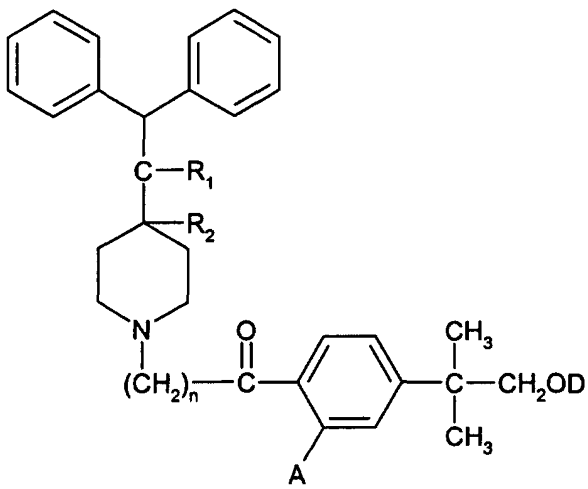

dans laquelle $R_1$, $R_2$, n et D sont définis ci-dessus et A est l'hydrogène ;

(e) faire réagir l'hydroxyéthylphénylcétone pipéridine protégée avec du borohydrure de sodium, du borohydrure de potassium, du cyanoborohydrure de sodium, du borohydrure de tétraméthylammonium, de l'hydrure de lithium et de tri-tert-butyl-aluminium, de l'hydrure de diisobutyl-aluminium ou catalyser une hydrogénation pour produire une hydroxyéthylphénylalcool pipéridine protégée de formule :

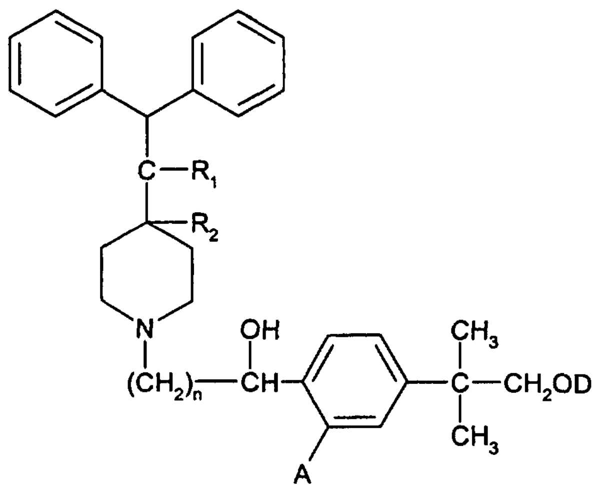

dans laquelle $R_1$, $R_2$, D et n sont définis ci-dessus et A est l'hydrogène ;

(f) déprotéger la fonctionnalité hydroxyéthyle de l'hydroxyéthylphénylalcool pipéridine protégée avec un réactif de déprotection approprié pour produire une hydroxyéthylphénylalcool pipéridine de formule :

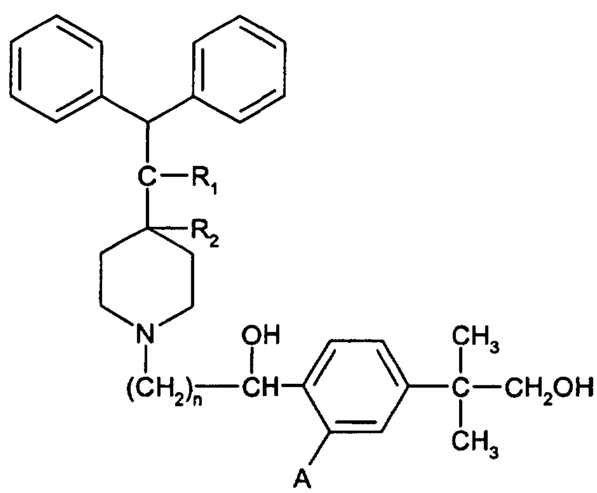

dans laquelle $R_1$, $R_2$ et n sont définis ci-dessus et A est l'hydrogène ;

(g) faire réagir l'hydroxyéthylphénylalcool pipéridine avec du diméthylsulfoxyde, du chlorure d'oxalyle et de la triéthylamine ou avec un réactif de Dess-Martin, de l'oxyde de chrome(IV), du peroxyde de nickel, du dichromate de sodium, du dichromate de potassium, du chromate de t-butyle, de l'oxyde d'argent, du picolinate argentique du dioxyde de manganèse du tétraacétate de plomb, du dicyclohexylcarbodiimide, de la 2,3-dichloro-5,6-dicyanoquinone, de la tétrachloro-1,2-benzoquinone, du 2,2,6,6-tétraméthylpipéridinyl-1-oxy (TEMPO) ou du chlorochromate de quinolinium pour produire un formylphénylalcool pipéridine de formule :

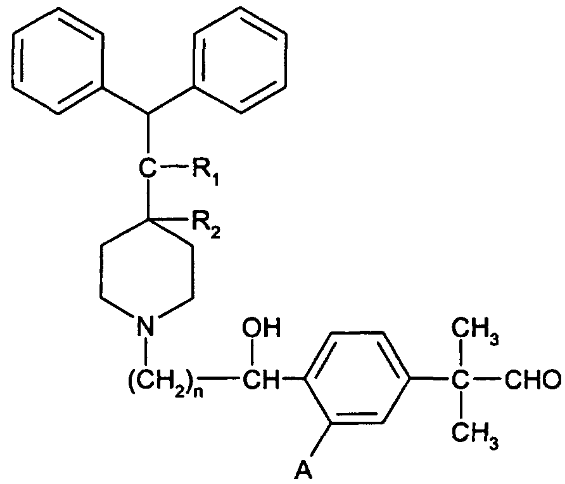

dans laquelle $R_1$, $R_2$ et n sont définis ci-dessus et A est l'hydrogène ;

(h) faire réagir la formylphénylalcool pipéridine avec du permanganate de potassium, de l'oxyde de chrome (IV), de l'oxyde d'argent(I), de l'oxyde d'argent, du picolinate argentique, un peroxyde, de l'acide nitrique, de l'acide m-chloroperbenzoïque et de l'acide peracétique pour produire une carboxyphénylalcool pipéridine de formule :

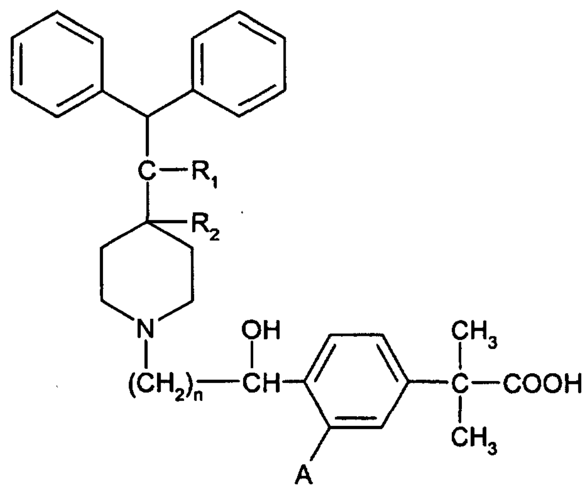

dans laquelle $R_1$, $R_2$ et n sont définis ci-dessus et A est l'hydrogène ;

(i) éventuellement faire réagir la carboxyphénylalcool pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylalcool pipéridine de formule :

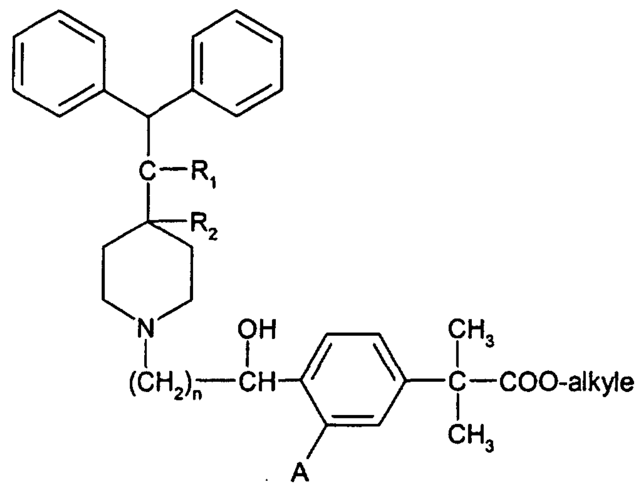

dans laquelle $R_1$, $R_2$, n et alkyle sont définis ci-dessus et A est l'hydrogène.

**31.** Procédé pour préparer un composé de formule :

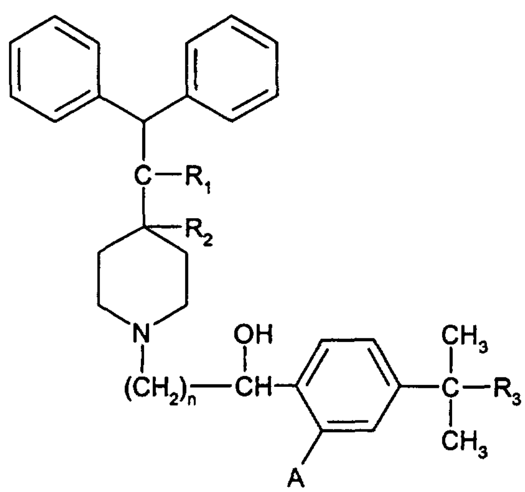

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

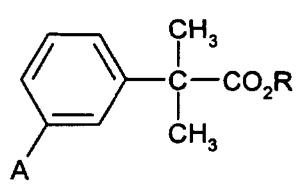

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

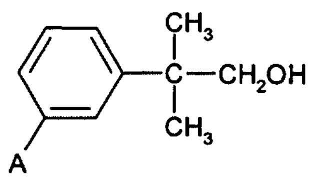

dans laquelle A est l'hydrogène ;
(b) protéger la fonctionnalité hydroxyéthyle avec un groupe protecteur convenable pour donner un alcool phénéthylique protégé de formule :

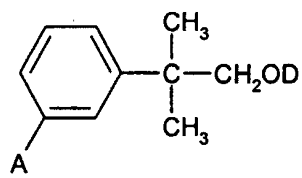

dans laquelle A est l'hydrogène et D est un groupe protecteur convenable ;
(c) faire réagir l'alcool phénéthylique protégé avec un composé ω-halogéno de formule :

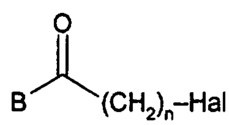

dans laquelle B est un radical halogéno ou hydroxy, Hal représente Cl, Br ou I et n est tel que défini ci-dessus, en présence de trichlorure, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

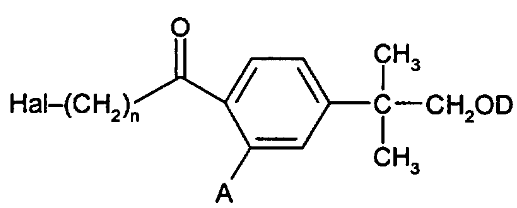

dans laquelle Hal, n et D sont définis ci-dessus et A est l'hydrogène ;
(d) faire réagir l'hydroxyéthylphénylcétone ω-halogéno protégée avec un composé de pipéridine de formule :

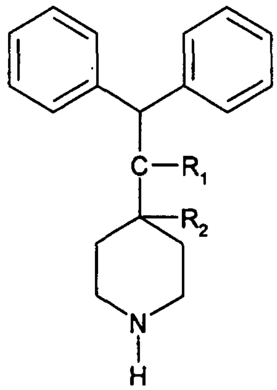

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

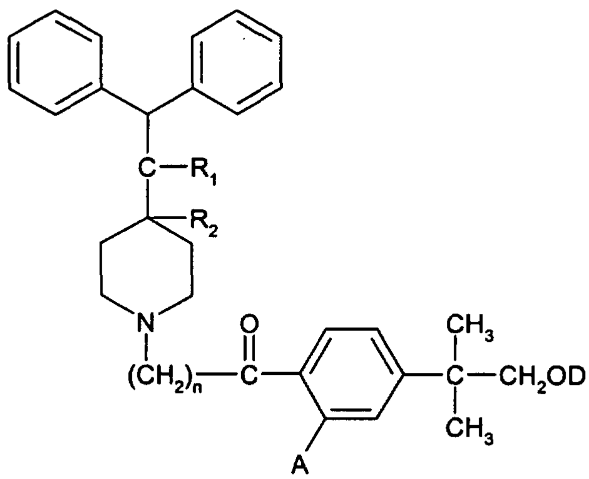

dans laquelle $R_1$, $R_2$, n et D sont définis ci-dessus et A est l'hydrogène ;

(e) faire réagir l'hydroxyéthylphénylcétone pipéridine protégée avec du borohydrure de sodium, du borohydrure de potassium, du cyanoborohydrure de sodium, du borohydrure de tétraméthylammonium, de l'hydrure de lithium et de tri-tert-butyl-aluminium, de l'hydrure de diisobutyl-aluminium ou catalyser une hydrogénation pour produire une hydroxyéthylphénylalcool pipéridine protégée de formule :

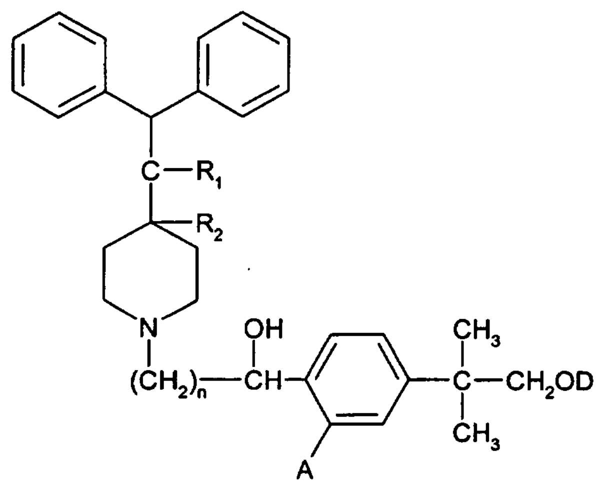

dans laquelle $R_1$, $R_2$, D et n sont définis ci-dessus et A est l'hydrogène ;

(f) déprotéger la fonctionnalité hydroxyéthyle de l'hydroxyéthylphénylalcool pipéridine protégée avec un réactif de déprotection approprié pour produire une hydroxyéthylphénylalcool pipéridine de formule :

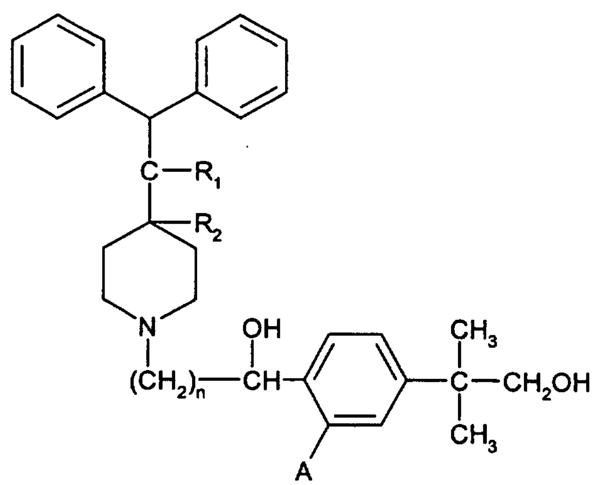

dans laquelle $R_1$, $R_2$ et n sont définis ci-dessus et A est l'hydrogène ;

(g) faire réagir l'hydroxyéthylphénylalcool pipéridine avec de l'oxyde de chrome(IV), du permanganate de potassium, de l'acide nitrique, du dioxyde d'azote, de l'oxyde de ruthénium(VIII), du peroxyde de nickel, de l'oxyde d'argent, du chromate de t-butyle ou de l'acide xénique, pour produire une carboxyphénylalcool pipéridine de formule :

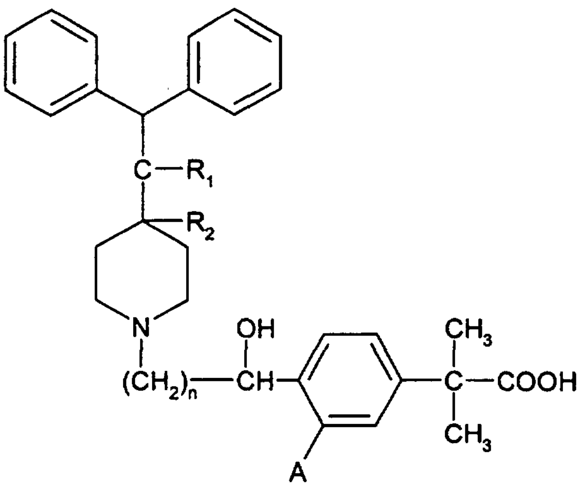

dans laquelle $R_1$, $R_2$ et n sont définis ci-dessus et A est l'hydrogène ;

(h) éventuellement faire réagir la carboxyphénylalcool pipéridine avec un excès de diazométhane ou un excès de 2,2-diméthoxypropane ou avec du chlorure de thionyle puis un alcool, de formule HO-alkyle où le fragment alkyle a de 1 à 6 atomes de carbone et est linéaire ou ramifié, pour former un ester de carboxyphénylalcool pipéridine de formule :

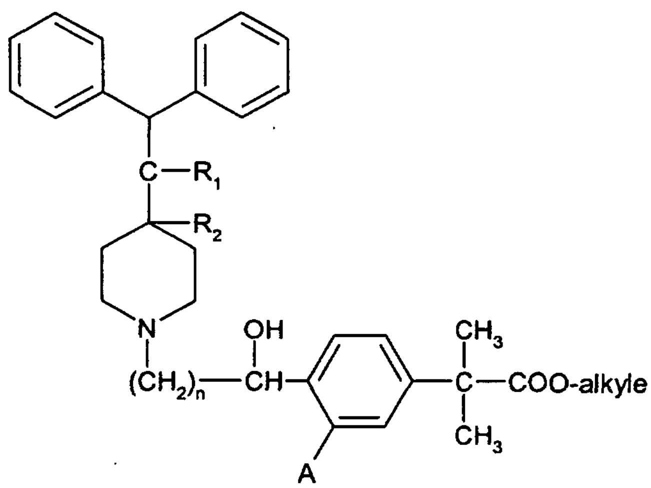

dans laquelle $R_1$, $R_2$, n et alkyle sont définis ci-dessus et A est l'hydrogène.

**32.** Procédé selon la revendication 31, dans lequel l'hydroxyéthylphénylalcool pipéridine produite dans l'étape f est mise à réagir avec de l'oxyde de ruthénium(VIII) pour produire la carboxyphénylcétone pipéridine de l'étape g.

**33.** Procédé pour préparer un composé de formule :

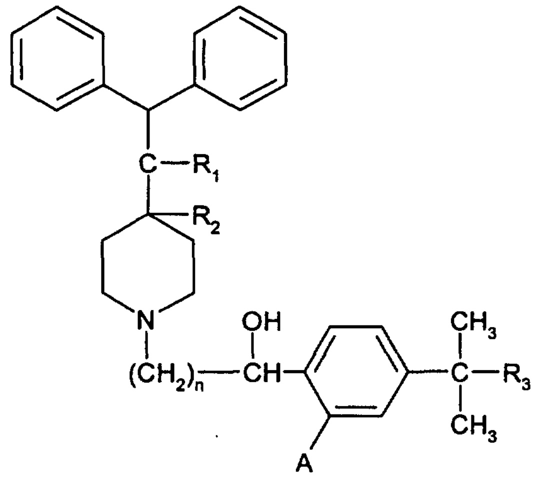

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
A est l'hydrogène ;
$R_3$ est $-CH_2OH$ ; et
ses sels acceptables en pharmacie, comprenant les étapes consistant à :

(a) faire réagir un composé d'acide benzène-acétique de formule :

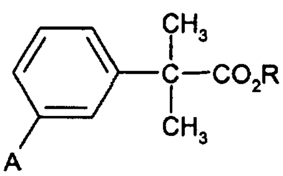

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

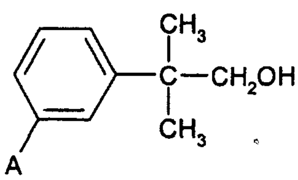

dans laquelle A est l'hydrogène ;
(b) protéger la fonctionnalité hydroxyéthyle avec un groupe protecteur convenable pour donner un alcool phénéthylique protégé de formule :

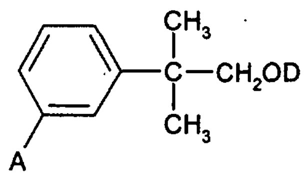

dans laquelle A est l'hydrogène et D est un groupe protecteur convenable ;
(c) faire réagir l'alcool phénéthylique protégé avec un composé ω-halogéno de formule :

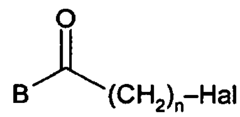

dans laquelle B est un radical halogéno ou hydroxy, Hal représente Cl, Br ou I et n est tel que défini ci-dessus, en présence de trichlorure, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

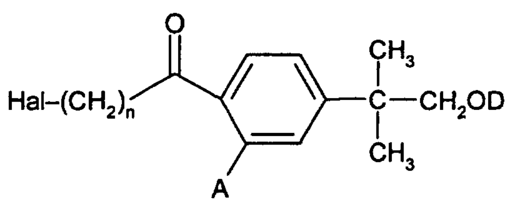

dans laquelle Hal, n et D sont définis ci-dessus et A est l'hydrogène ;
(d) faire réagir l'hydroxyéthylphénylcétone ω-halogéno protégée avec un composé de pipéridine de formule :

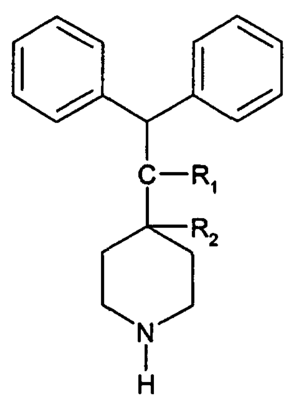

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

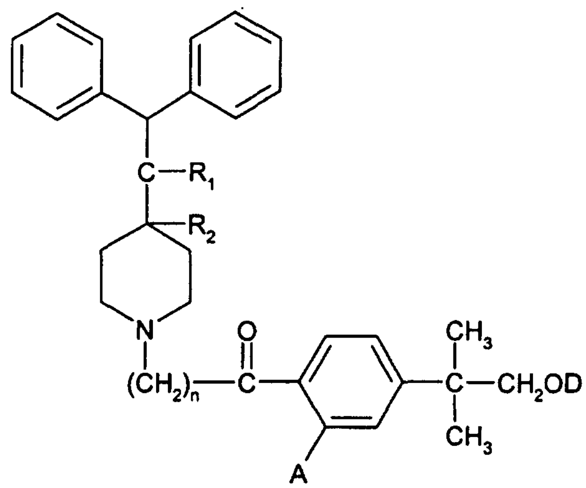

dans laquelle $R_1$, $R_2$, n et D sont définis ci-dessus et A est l'hydrogène ;

(e) faire réagir l'hydroxyéthylphénylcétone pipéridine protégée avec du borohydrure de sodium, du borohydrure de potassium, du cyanoborohydrure de sodium, du borohydrure de tétraméthylammonium, de l'hydrure de lithium et de tri-tert-butyl-aluminium, de l'hydrure de diisobutyl-aluminium ou catalyser une hydrogénation pour produire une hydroxyéthylphénylalcool pipéridine protégée de formule :

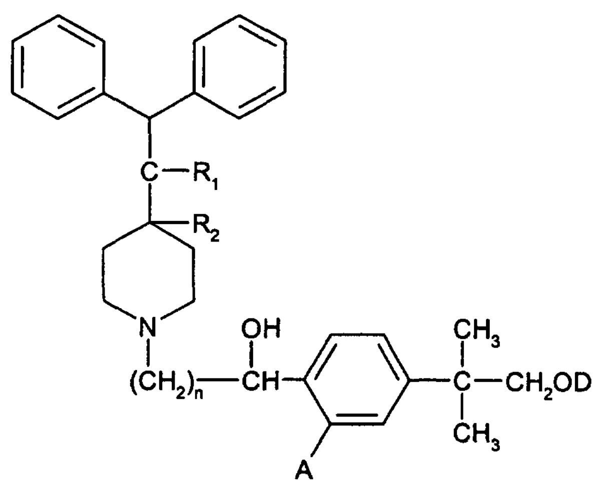

dans laquelle $R_1$, $R_2$, D et n sont définis ci-dessus et A est l'hydrogène ;

(f) déprotéger la fonctionnalité hydroxyéthyle de l'hydroxyéthylphénylalcool pipéridine protégée avec un réactif de déprotection approprié pour produire une hydroxyéthylphénylalcool pipéridine de formule :

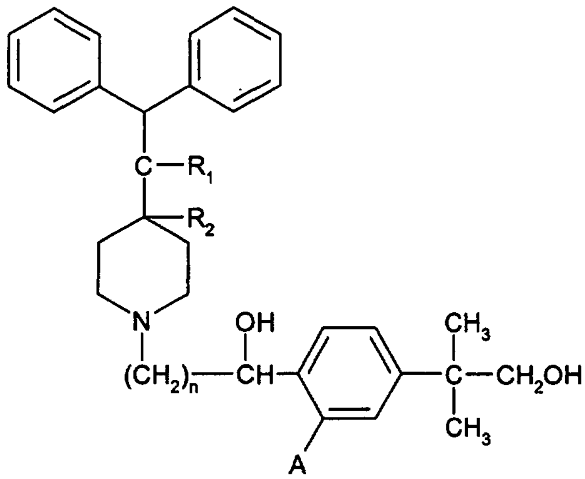

dans laquelle $R_1$, $R_2$ et n sont définis ci-dessus et A est l'hydrogène.

**34.** Procédé pour préparer un composé de formule :

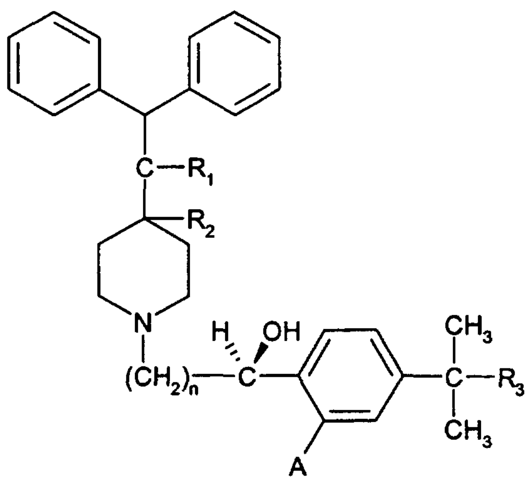

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

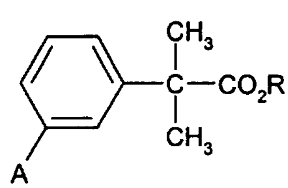

dans laquelle A est l'hydrogène et R est l'hydrogène ou un radical alkyle en $C_1$ à $C_6$, avec de l'hydrure

de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

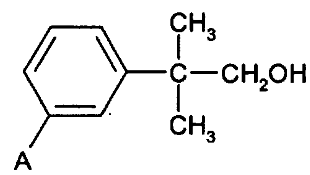

dans laquelle A est l'hydrogène ;

(b) protéger la fonctionnalité hydroxyéthyle avec un groupe protecteur convenable pour donner un alcool phénéthylique protégé de formule :

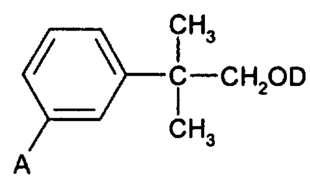

dans laquelle A est l'hydrogène et D est un groupe protecteur convenable ;

(c) faire réagir l'alcool phénéthylique protégé avec un composé ω-halogéno de formule :

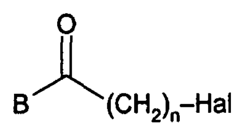

dans laquelle B est un radical halogéno ou hydroxy, Hal représente Cl, Br ou I et n est tel que défini ci-dessus, en présence de trichlorure, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

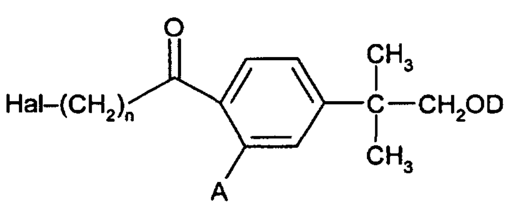

dans laquelle Hal, n et D sont définis ci-dessus et A est l'hydrogène ;

(d) faire réagir l'hydroxyéthylphénylcétone ω-halogéno protégée avec un composé de pipéridine de formule :

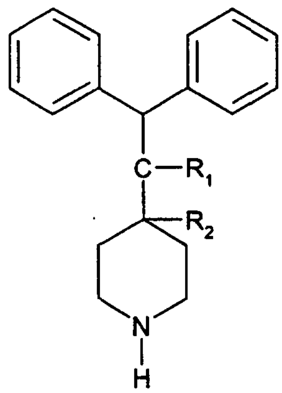

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

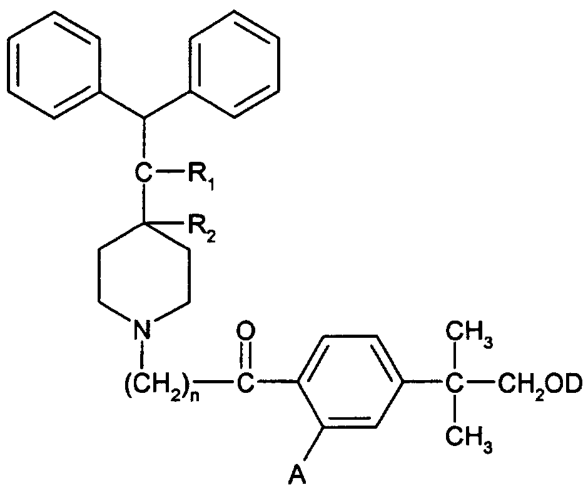

dans laquelle $R_1$, $R_2$, n et D sont définis ci-dessus et A est l'hydrogène ;

(e) faire réagir l'hydroxyéthylphénylcétone pipéridine protégée avec du (+)-B-chlorodiisopinocamphénylborane pour produire une hydroxyéthylphénylalcool pipéridine protégée de formule :

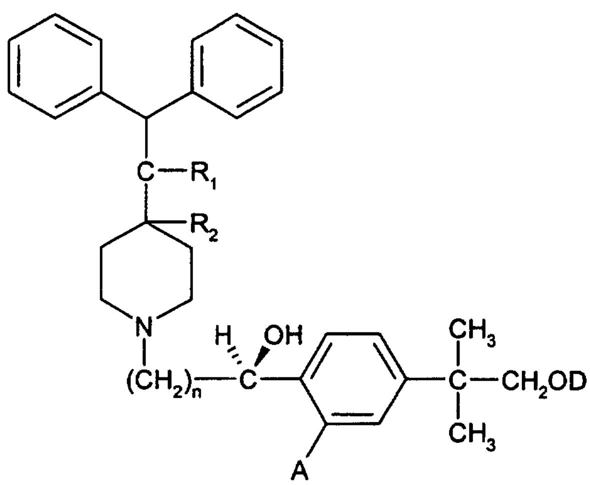

dans laquelle $R_1$, $R_2$, D et n sont définis ci-dessus et A est l'hydrogène ;

(f) déprotéger la fonctionnalité hydroxyéthyle de l'hydroxyéthylphénylalcool pipéridine protégée avec un réactif de déprotection approprié pour produire une hydroxythylphénylalcool pipéridine de formule :

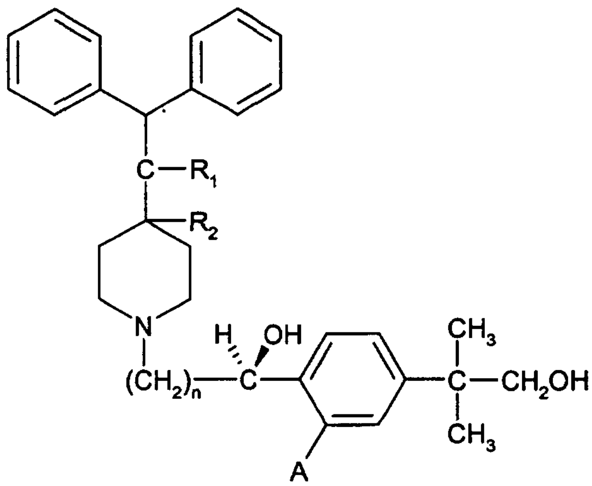

dans laquelle $R_1$, $R_2$ et n sont définis ci-dessus et A est l'hydrogène ;

(g) faire réagir l'hydroxyéthylphénylalcool pipéridine avec du diméthylsulfoxyde, du chlorure d'oxalyle et de la triéthylamine ou avec un réactif de Dess-Martin, de l'oxyde de chrome(IV), du peroxyde de nickel, du dichromate de sodium, du dichromate de potassium, du chromate de t-butyle, de l'oxyde d'argent, du picolinate argentique du dioxyde de manganèse du tétraacétate de plomb, du dicyclohexylcarbodiimide, de la 2,3-dichloro-5,6-dicyanoquinone, de la tétrachloro-1,2-benzoquinone, du 2,2,6,6-tétraméthylpipéridinyl-1-oxy (TEMPO) ou du chlorochromate de quinolinium pour produire une formylphénylalcool pipéridine de formule :

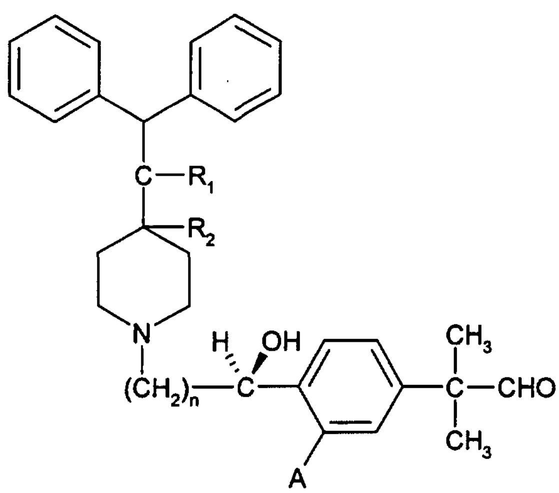

dans laquelle $R_1$, $R_2$ et n sont définis ci-dessus et A est l'hydrogène ;

(h) faire réagir la formylphénylalcool pipéridine avec du permanganate de potassium, de l'oxyde de chrome (IV), de l'oxyde d'argent(I), de l'oxyde d'argent, du picolinate argentique, un peroxyde, de l'acide nitrique, de l'acide m-chloroperbenzoïque et de l'acide peracétique pour produire une carboxyphénylalcool pipéridine de formule :

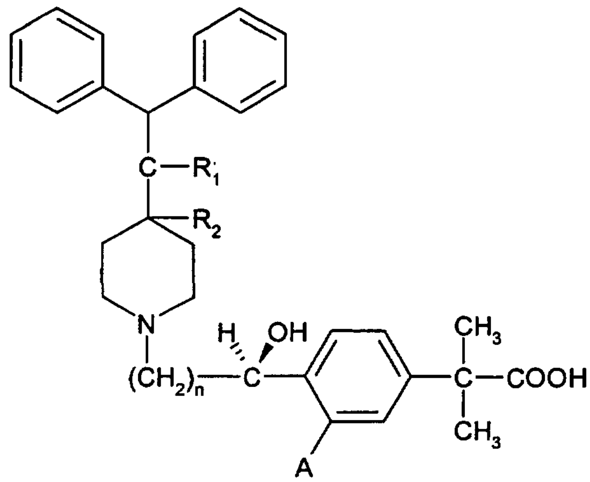

dans laquelle $R_1$, $R_2$ et n sont définis ci-dessus et A est l'hydrogène ;

(i) éventuellement faire réagir la carboxyphénylalcool pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylalcool pipéridine de formule :

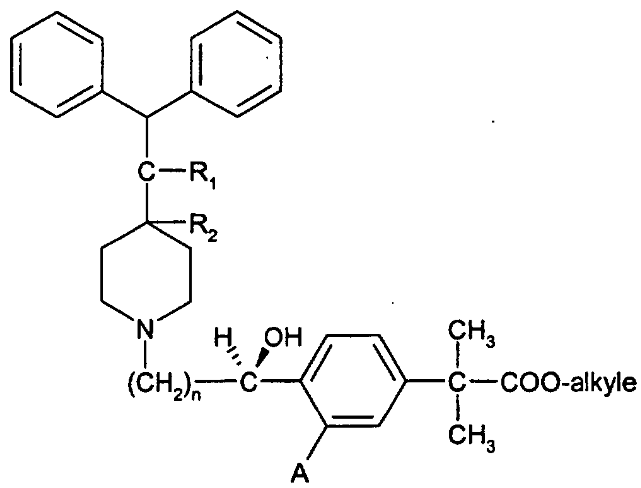

dans laquelle $R_1$, $R_2$, n et alkyle sont définis ci-dessus et A est l'hydrogène.

**35.** Procédé pour préparer un composé de formule :

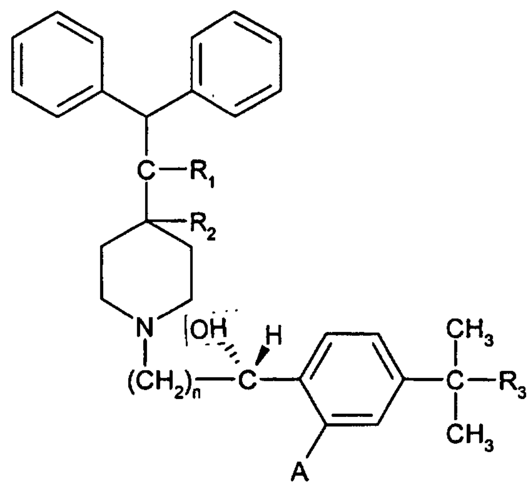

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
$R_3$ est -COOH ou -COO-alkyle où le fragment alkyle a de 1 à 6 atomes de carbone et est linéaire ou ramifié ;
A est l'hydrogène ; et
ses sels acceptables en pharmacie, comprenant les étapes consistant à :

(a) faire réagir un composé d'acide benzène-acétique de formule :

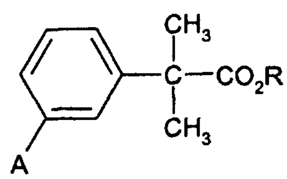

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

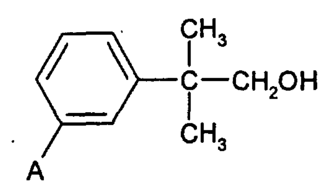

dans laquelle A est l'hydrogène ;
(b) protéger la fonctionnalité hydroxyéthyle avec un groupe protecteur convenable pour donner un alcool phénéthylique protégé de formule :

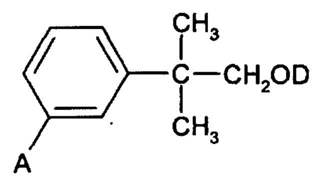

dans laquelle A est l'hydrogène et D est un groupe protecteur convenable ;
(c) faire réagir l'alcool phénéthylique protégé avec un composé ω-halogéno de formule :

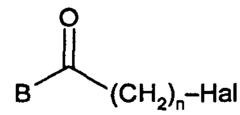

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

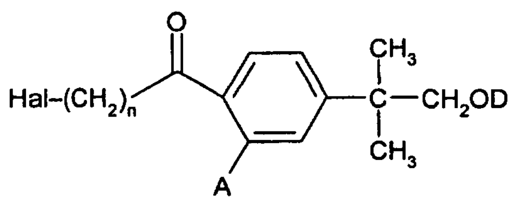

dans laquelle Hal, n et D sont définis ci-dessus et A est l'hydrogène ;
(d) faire réagir l'hydroxyéthylphénylcétone ω-halogéno protégée avec un composé de pipéridine de formule :

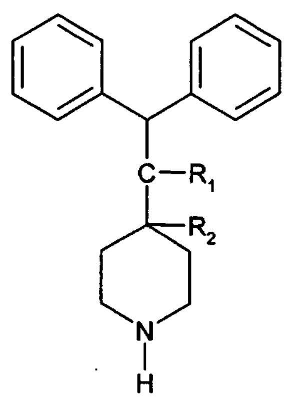

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

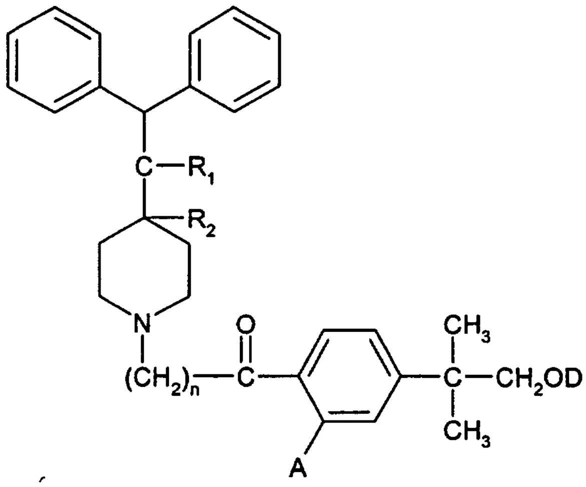

dans laquelle $R_1$, $R_2$, n et D sont définis ci-dessus et A est l'hydrogène ;

(e) faire réagir, l'hydroxyéthylphénylcétone pipéridine protégée avec du (-)-B-chlorodiisopinocamphénylborane pour produire une hydroxyéthylphénylalcool pipéridine protégée de formule :

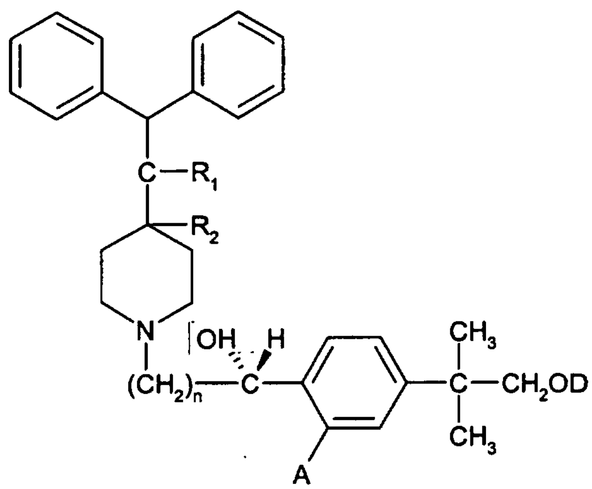

dans laquelle $R_1$, $R_2$, D et n sont définis ci-dessus et A est l'hydrogène ;

(f) déprotéger la fonctionnalité hydroxyéthyle de l'hydroxyéthylphénylalcool pipéridine protégée avec un réactif de déprotection approprié pour produire une hydroxyéthylphénylalcool pipéridine de formule :

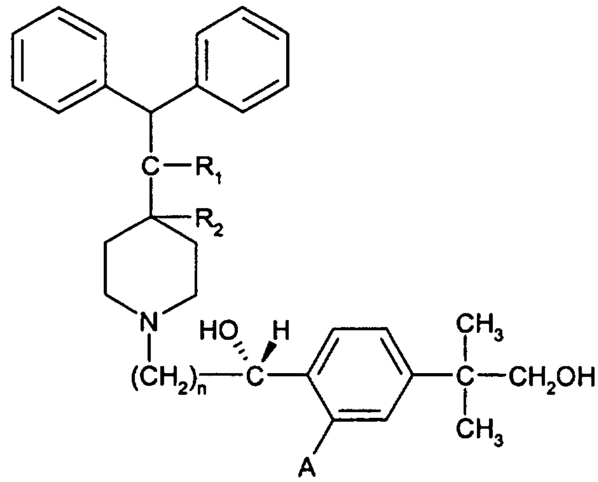

dans laquelle $R_1$, $R_2$ et n sont définis ci-dessus et A est l'hydrogène ;

(g) faire réagir l'hydroxyéthylphénylalcool pipéridine avec du diméthylsulfoxyde, du chlorure d'oxalyle et de la triéthylamine ou avec un réactif de Dess-Martin, de l'oxyde de chrome(IV), du peroxyde de nickel, du dichromate de sodium, du dichromate de potassium, du chromate de t-butyle, de l'oxyde d'argent, du picolinate argentique du dioxyde de manganèse du tétraacétate de plomb, du dicyclohexylcarbodiimide, de la 2,3-dichloro-5,6-dicyanoquinone, de la tétrachloro-1,2-benzoquinone, du 2,2,6,6-tétraméthylpipéridinyl-1-oxy (TEMPO) ou du chlorochromate de quinolinium pour produire une formylphénylalcool pipéridine de formule :

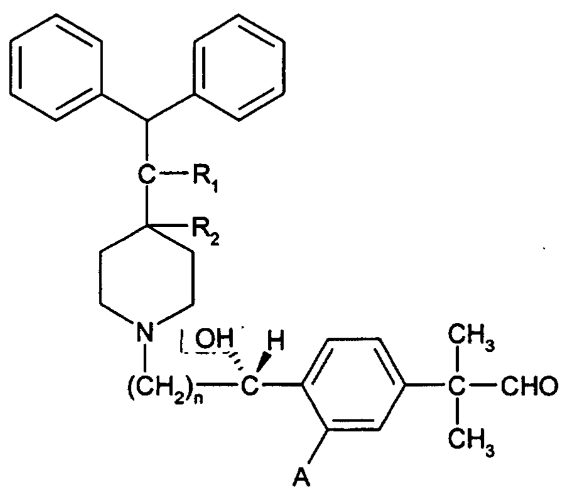

dans laquelle $R_1$, $R_2$ et n sont définis ci-dessus et A est l'hydrogène ;

(h) faire réagir la formylphénylalcool pipéridine avec du permanganate de potassium, de l'oxyde de chrome (IV), de l'oxyde d'argent(I), de l'oxyde d'argent, du picolinate argentique, un peroxyde, de l'acide nitrique, de l'acide m-chloroperbenzoïque et de l'acide peracétique pour produire une carboxyphénylalcool pipéridine de formule :

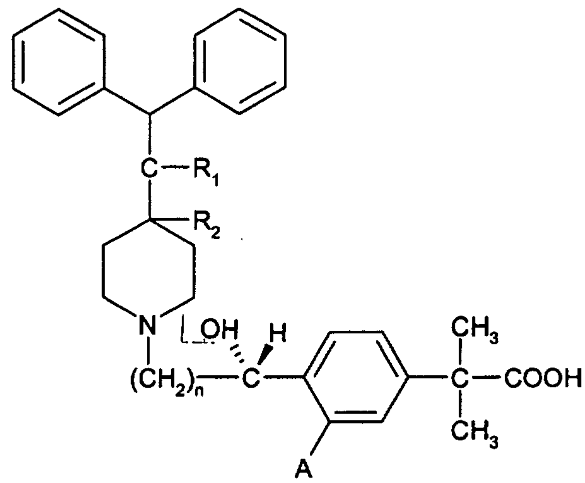

dans laquelle $R_1$, $R_2$ et n sont définis ci-dessus et A est l'hydrogène ;

(i) éventuellement faire réagir la carboxyphénylalcool pipéridine avec un excès de diazométhane ou un excès de 2,2-diméthoxypropane ou avec du chlorure de thionyle puis un alcool de formule HO-alkyle où le fragment alkyle a de 1 à 6 atomes de carbone et est linéaire ou ramifié, pour former un ester de carboxyphénylalcool pipéridine de formule :

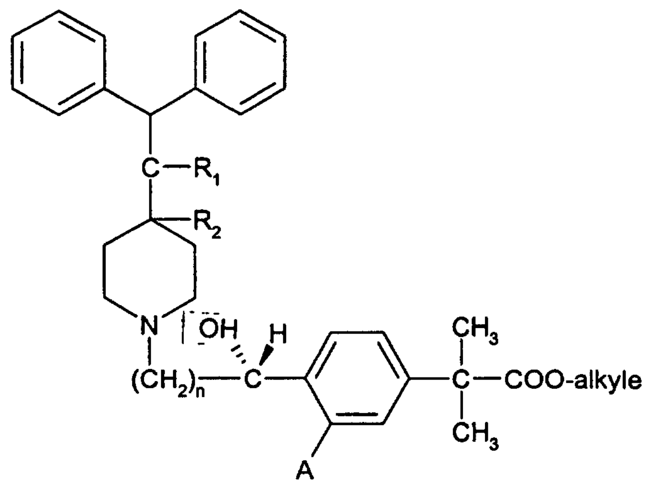

dans laquelle $R_1$, $R_2$, n et alkyle sont définis ci-dessus et A est l'hydrogène.

**36.** Procédé pour préparer un composé de formule :

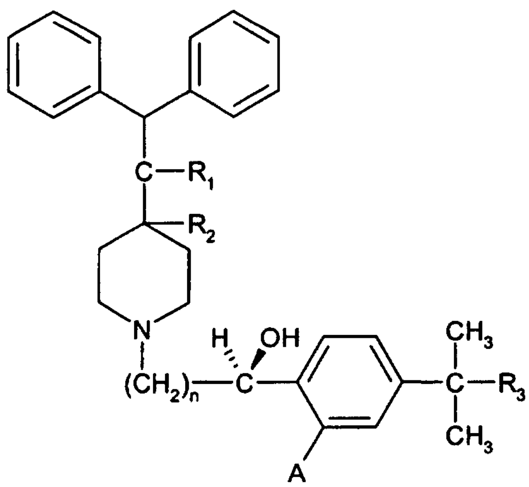

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
A est l'hydrogène ;
$R_3$ est $-CH_2OH$ ; et
ses sels acceptables en pharmacie, comprenant les étapes consistant à :

(a) faire réagir un composé d'acide benzène-acétique de formule :

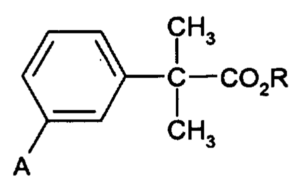

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

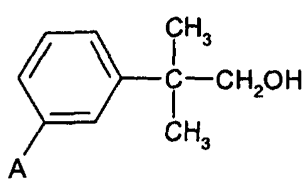

dans laquelle A est l'hydrogène ;
(b) protéger la fonctionnalité hydroxyéthyle avec un groupe protecteur convenable pour donner un alcool phénéthylique protégé de formule :

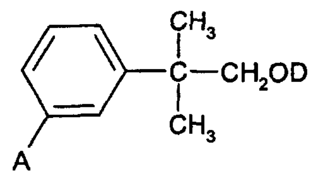

dans laquelle A est l'hydrogène et D est un groupe protecteur convenable ;
(c) faire réagir l'alcool phénéthylique protégé avec un composé ω-halogéno de formule :

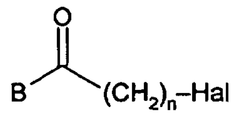

dans laquelle B est un radical halogéno ou hydroxy, Hal représente Cl, Br ou I et n est tel que défini ci-dessus, en présence de trichlorure, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

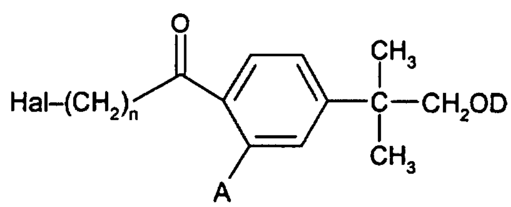

dans laquelle Hal, n et D sont définis ci-dessus et A est l'hydrogène ;
(d) faire réagir l'hydroxyéthylphénylcétone ω-halogéno protégée avec un composé de pipéridine de formule :

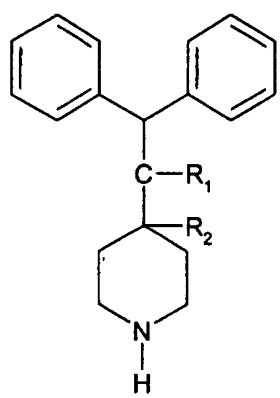

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

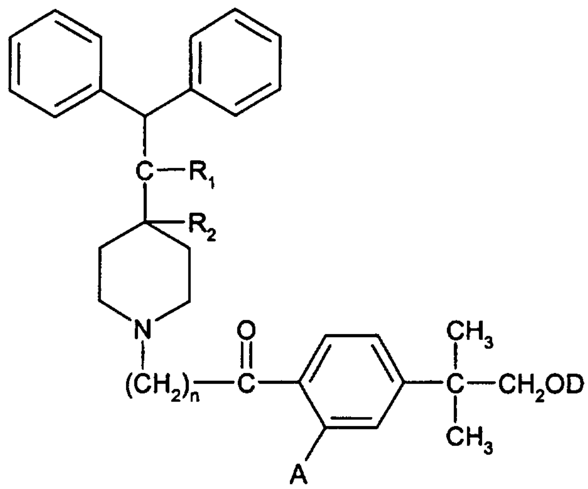

dans laquelle $R_1$, $R_2$, n et D sont définis ci-dessus et A est l'hydrogène ;

(e) faire réagir l'hydroxyéthylphénylcétone pipéridine protégée avec du (+)-B-chlorodiisopinocamphénylborane pour produire une hydroxyéthylphénylalcool pipéridine protégée de formule :

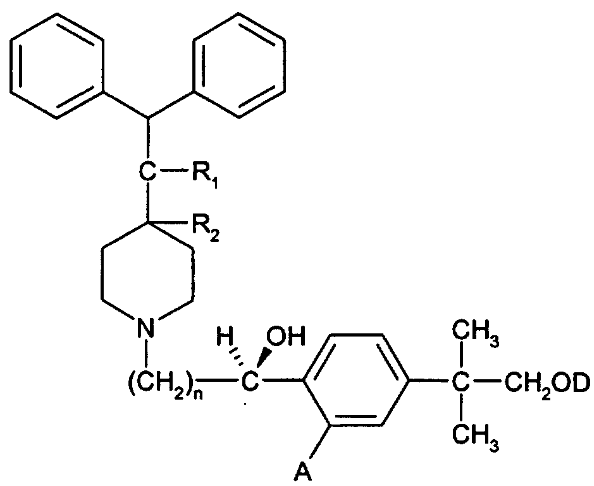

dans laquelle $R_1$, $R_2$, D et n sont définis ci-dessus et A est l'hydrogène ;

(f) déprotéger la fonctionnalité hydroxyéthyle de l'hydroxyéthylphénylalcool pipéridine protégée avec un réactif de déprotection approprié pour produire une hydroxyéthylphénylalcool pipéridine de formule :

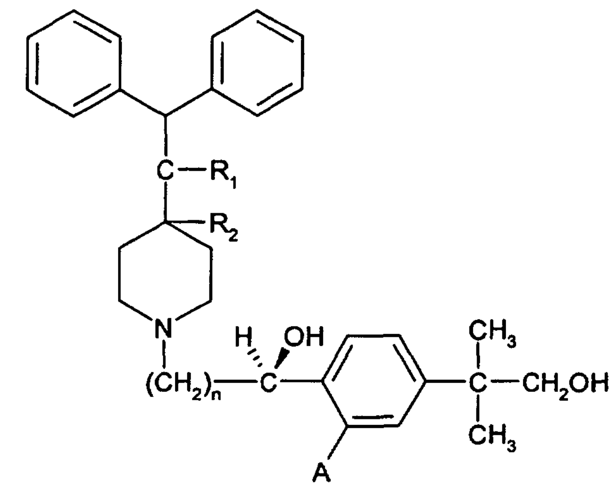

dans laquelle $R_1$, $R_2$ et n sont définis ci-dessus et A est l'hydrogène.

**37.** Procédé pour préparer un composé de formule :

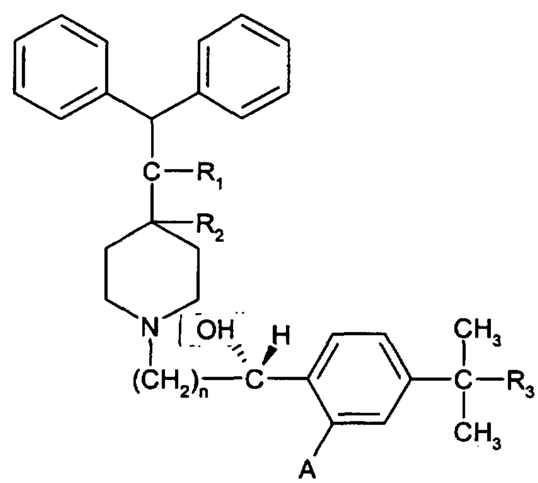

dans laquelle
$R_1$ représente l'hydrogène ou un radical hydroxy ;
$R_2$ représente l'hydrogène ; ou
$R_1$ et $R_2$, pris ensemble, forment une deuxième liaison entre les atomes de carbone portant $R_1$ et $R_2$ ;
n est un entier de 1 à 5 ;
A est l'hydrogène ;
$R_3$ est $-CH_2OH$ ; et
ses sels acceptables en pharmacie, comprenant les étapes consistant à :

(a) faire réagir un composé d'acide benzène-acétique de formule :

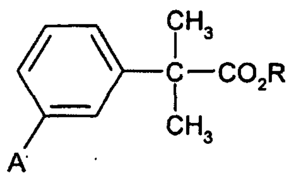

dans laquelle A est l'hydrogène et R est l'hydrogène ou un radical alkyle en $C_1$ à $C_6$, avec de l'hydrure de sodium-bis(2-méthoxyéthoxy)aluminium, de l'hydrure de lithium et d'aluminium, du diborane, ou de l'hydrure d'aluminium quand R est l'hydrogène, et avec de l'hydrure de sodium-bis(2-méthoxyéthoxy)aluminium, de l'hydrure de lithium et d'aluminium, du borohydrure de lithium, du triéthylborohydrure de lithium, du tri-sec-butylborohydrure de lithium ou de l'hydrure d'aluminium quand R est un radical alkyle en $C_1$ à $C_6$, pour donner un alcool phénéthylique de formule :

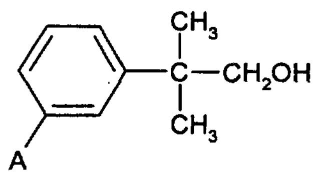

dans laquelle A est l'hydrogène ;

(b) protéger la fonctionnalité hydroxyéthyle avec un groupe protecteur convenable pour donner un alcool phénéthylique protégé de formule :

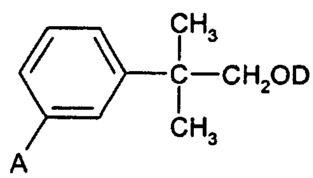

dans laquelle A est l'hydrogène et D est un groupe protecteur convenable ;

(c) faire réagir l'alcool phénéthylique protégé avec un composé ω-halogéno de formule :

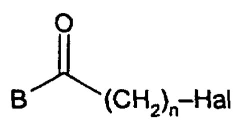

dans laquelle B est un radical halogéno ou hydroxy, Hal représente Cl, Br ou I et n est tel que défini ci-dessus, en présence de trichlorure de bore, de chlorure d'aluminium, de tétrachlorure de titane, de trifluorure de bore, de tétrachlorure d'étain et de chlorure de zinc, pour produire une hydroxyéthylphénylcétone ω-halogéno protégée de formule :

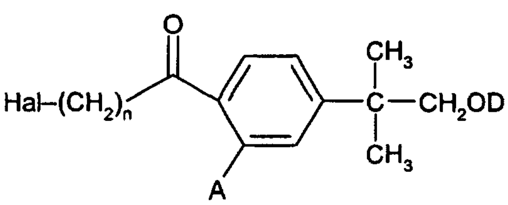

dans laquelle Hal, n et D sont définis ci-dessus et A est l'hydrogène ;

(d) faire réagir l'hydroxyéthylphénylcétone ω-halogéno protégée avec un composé de pipéridine de formule :

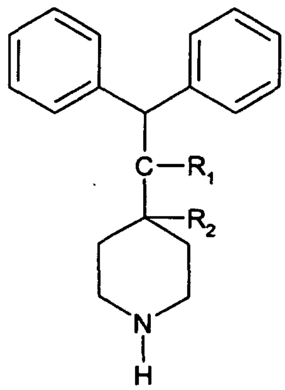

dans laquelle $R_1$ et $R_2$ sont tels que définis ci-dessus, en présence de bicarbonate de sodium, de carbonate de potassium, de bicarbonate de potassium, de triéthylamine, de pyridine, ou bien on peut utiliser un excès du composé pipéridine pour produire une hydroxyéthylphénylcétone pipéridine protégée de formule :

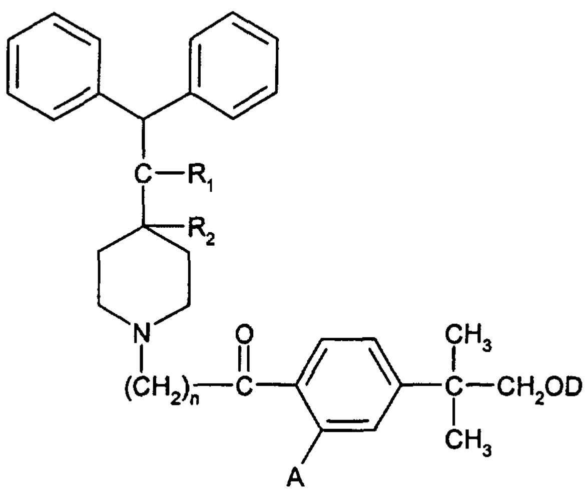

dans laquelle $R_1$, $R_2$, n et D sont définis ci-dessus et A est l'hydrogène ;
(e) faire réagir l'hydroxyéthylphénylcétone pipéridine protégée avec du (-)-B-chlorodiisopinocamphénylborane pour produire une hydroxyéthylphénylalcool pipéridine protégée de formule :

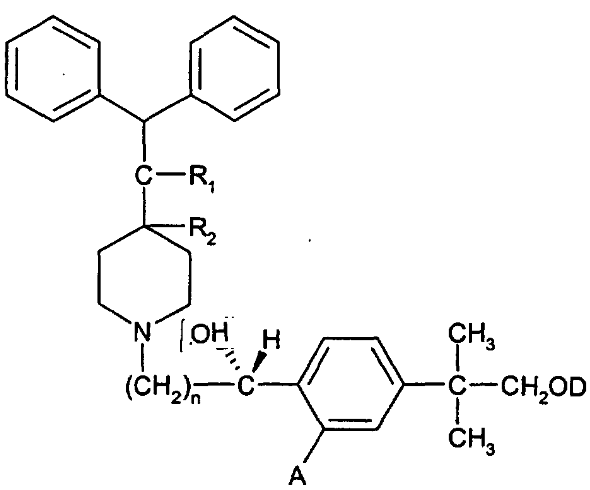

dans laquelle $R_1$, $R_2$, D et n sont définis ci-dessus et A est l'hydrogène ;

(f) déprotéger la fonctionnalité hydroxyéthyle de l'hydroxyéthylphénylalcool pipéridine protégée avec un réactif de déprotection approprié pour produire une hydroxyéthylphénylalcool pipéridine de formule :

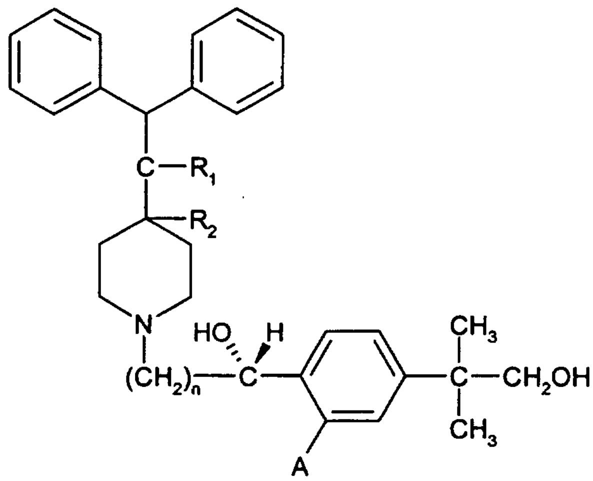

dans laquelle $R_1$, $R_2$ et n sont définis ci-dessus et A est l'hydrogène.